# EUROPEAN PATENT SPECIFICATION

(11) **EP 0 030 138 B1**
(45) Date of publication and mention of the grant of the patent: **13.06.1984**
(21) Application number: 80304283.7
(22) Date of filing: 28.11.1980
(51) Int. Cl.: A01N 47/36, C07D 239/42, C07D 239/46, C07D 239/52, C07D 251/16, C07D 251/46

(54) **Herbicidal sulfonamides, and compositions, preparation and use thereof**
Herbizide Sulfonamide und deren Zusammensetzungen, Herstellung und Verwendung
Sulfonamides à activité herbicide, compositions, préparation et leur utilisation

(30) Priority: 30.11.1979 US 98781
(43) Date of publication of application: 10.06.1981
(73) Proprietor: E.I. DU PONT DE NEMOURS AND COMPANY, Wilmington Delaware 19898 (US)
(72) Inventor: Levitt, George, Wilmington Delaware 19810 (US)
(74) Representative: Hildyard, Edward Martin

## Description

### Background of the Invention

This invention relates to novel N-(heterocyclicaminocarbonyl)arylsulfonamides in which the aryl radical is substituted by a carboxyl radical, ester, thioester or amide thereof. The compounds of this invention and their agriculturally suitable salts, are useful as agricultural chemicals, e.g. plant growth regulants and herbicides.

Netherlands Patent 121,788, published September 15, 1966, discloses the preparation of compounds of the following Formula and their use as general or selective herbicides: 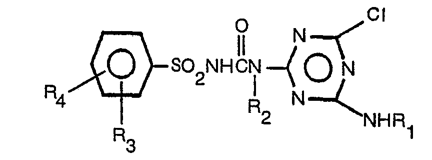 wherein
R₁ and R₂ may independently be alkyl of 1―4. carbon atoms; and
R₃ and R₄ may independently be hydrogen, chlorine or alkyl of 1-4 carbon atoms.

U.S. Patent 3,637,366 discloses compounds having the formula: 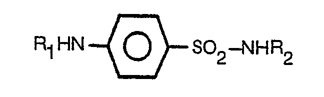 wherein
R₁ is hydrogen or lower saturated aliphatic acyl and
R₂ is hydrogen, 2-pyrimidinyl, pyridyl, amidino, acetyl or carbamoyl.

The disclosed compounds are said to provide control of crabgrass, cress, endhive, clover and Poa annua.

French Patent No. 1,468,747 discloses the following para-substituted phenylsulfonamides as being useful as antidiabetic agents: 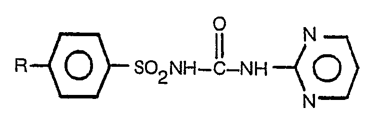 wherein
R = H, halogen, CF₃ or alkyl.

Logemann et al. Chem Ab., 53, 18052 g (1959), disclose a number of sulfonamides, including uracil derivatives and those having the formula: 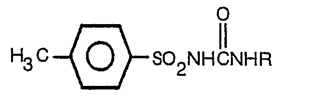 wherein
R is butyl, phenyl, or 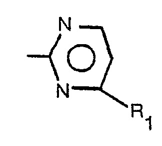 and
Rᵢ is hydrogen or methyl.

When tested for hypoglycemic effect in rats (oral doses of 25 mg/100 g), the compounds in which R is butyl and phenyl were most potent. The others were of low potency or inactive.

Wojciechowski, J. Acta. Polon. Pharm 19, p. 121-5 (1962) [Chem. Ab., 59 1633 e] describes ⁱ the synthesis of N-[(2,6-dimethoxypyrimidin-4-yl)aminocarbonyl]-4-methylbenzenesulfonamide:

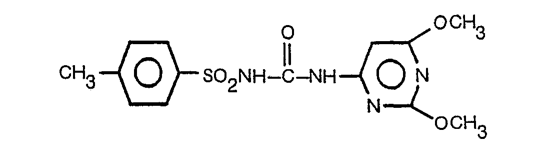

Based upon similarity to a known compound, the author speculated that the foregoing compound might have a hypoglycemic activity.

Substituted-pyrimidinyl sulfonylureas of the following formula, which are also para-substituted on the phenyl ring, are disclosed in Farmco Ed. Sci., 12, 586 (1957) [Chem. Ab., 53, 18052 g (1959]: 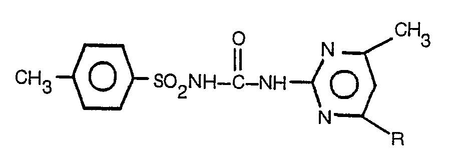 wherein
R = H or CH₃.

Herbicidal sulfonamides which are structurally distinct from, but related to, those of the present invention are also disclosed in our US-A-4,127,405; US-A-4,169,719; US-A-4,120,691; EP-A-0,001,485; EP-A-0,001,514; and EP-A-0,001,515.

The presence of undesired vegetation causes substantial damage to useful crops, especially agricultural products that satisfy man's basic food and fiber needs, such as cotton, rice, corn, wheat, and the like. The current population explosion and concomitant world food and fiber shortage demand- improvements in the efficiency of producing these crops. Preventing or minimizing loss of a portion of such valuable crops by killing, or inhibiting the growth of undesired vegetation is one way of improving this efficiency. A wide variety of materials useful for killing or inhibiting (controlling) the growth of undesired vegetation is available; such materials are commonly referred to as herbicides. The need still exists however, for more effective herbicides.

### SUMMARY OF THE INVENTION

According to this invention, there are provided novel compounds of Formula I and their agriculturally suitable salts, e.g. Na, K, alkyl ammonium, trichloroacetic acid, suitable agricultural compositions containing them and methods of using them as general or selective pre-emergence and post-emergence herbicides and as plant growth regulants: 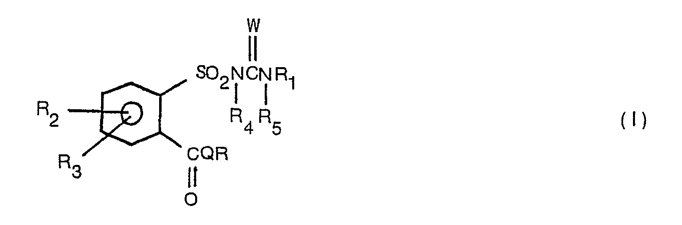 wherein Q is O, S or 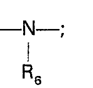 when Q is 0 or S then R is C₁―C₁₂ alkyl; C₃―C₁₀ alkenyl; C₂―C₆ alkyl substituted with one to four substituents selected from 0-3 atoms of F, CI or Br, 0-2 methoxy groups and 0-1 cyano groups; --CH₂CN; ―CH₂CO₂CH₃; ―CH₂CO₂C₂H₅; C₃―C₆ alkenyl substituted with 1-3 atoms of F, Cl, or Br; C₅―C₈ cycloalkyl; C₅―C₈ cycloalkenyl; C₅―C₆ cycloalkyl substituted with any of one to four methyl groups, OCH₃, alkyl of C₂―C₄, F, CI or Br; C₄―C₁₀ cycloalkylalkyl; C₄―C₈ cycloalkylalkyl with 1-2 CH₃; C₇―C₁₀ bicycloalkyl; C₇―C₁₀ bicycloalkenyl; C₁₀ tricycloalkyl, C₁₀ tricycloalkenyl; 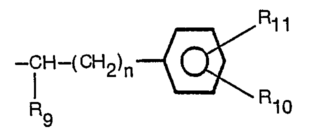 where R₉ is C₁―C₃ alkyl or hydrogen, R₁₀ and R₁₁ are independently hydrogen, C₁―C₃ alkyl, Cl, Br, -OCH₃, or ―OC₂H₅, or R₁₀ and R₁₁ may be taken together to form a 5 or 6 member ring: 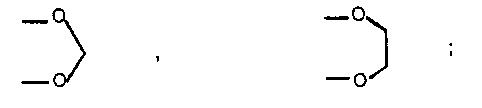 and n is 0, 1, 2 or 3 provided that R has a total number of carbon atoms of less than or equal to 12; 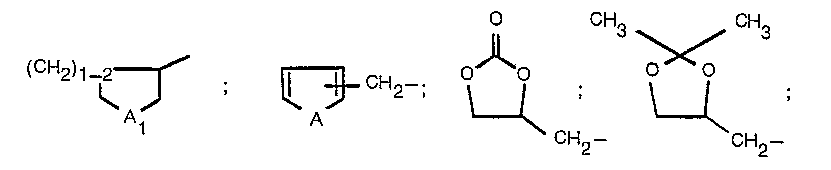 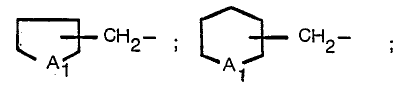 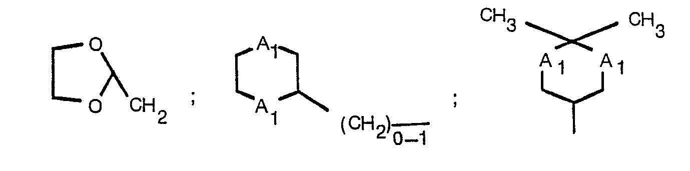
A is 0 or S;
A₁ is 0, S or SO₂; when Q is 0 or S then
   1) R may also be C₃―C₁₀, alkynyl; C₃―C₆ alkynyl substituted with F, CI or Br; 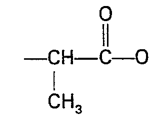 (C,-C₂ alkyl); or 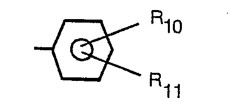 where R₁₀ and R₁₁ are as previously defined; when Q is 0 then R is H, M, ―C₂CH₂OR₇, ―CH₂CH₂CH₂OR₇, 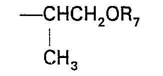 where R₇ is ―CH₂CH₃, ―CH(CH₃)₂, phenyl, ―CH₂CH₂Cl or ―CH₂CCl₃; (̵CH₂CH₂O)̵ₙ, R₈ or 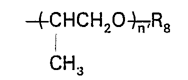 where R₈ is -CH₃, -CH₂CH₃, ―CH(CH₃)₂, phenyl, -CH₂CH₂CI or ―CH₂CCl₃, and n' is 2 or 3; provided that R has a total number of carbon atoms of less than or equal to 13; 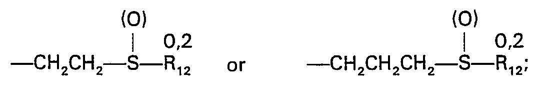 where R₁₂ is -CH₃, ―CH₂CH₃, ―CH(CH₃)₂, or phenyl; and when Q is 0
   2) R may also be CH₂OR'₈ where R'₈ is CH₃, CH₃CH₂, (CH₃)₂CH-, ClCH₂CH₂―, Cl₃CCH₂, 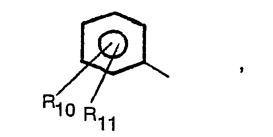 CH₃CH₂OCH₂CH₂ or CH₃OCH₂CH₂; where R₁₀ and R₁₁ are as previously defined; when Q is 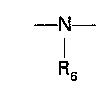 then R is hydrogen; C₁―C₁₂ alkyl; (̵CH₂CH₂O)̵ₙR₁₂, ―CH₂CH₂CH₂OR₁₂ where R₁₂ is as defined above and n"' is 1-3; C₃―C₁₀ alkenyl; C₃―C₈ cycloalkyl; C₅―C₆ cycloalkenyl; C₅―C₈ cycloalkyl substituted with 1 to 3 substituents selected from 0-2 -OCH₃, 0-3 -CH₃ and ―C₂H₅; trifluoromethylcyclohexyl; C₄―C₁₀ cycloalkylalkyl; C₄―C₈ cycloalkylalkyl substituted with 1-2 -CH₃; -CH₂CN; -CH₂CH₂CN; 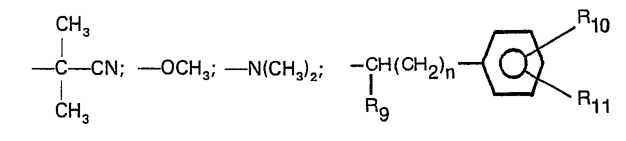 where n, R₉, R₁₀ and R₁₁ are as defined above; 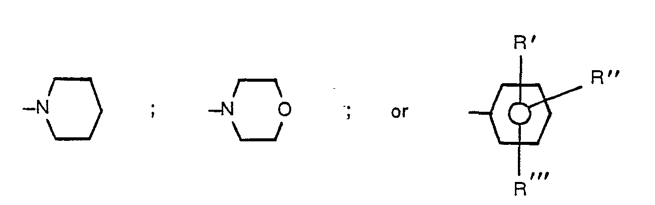 where R' is hydrogen, C₁―C₄ alkyl, -OCH₃, F, Br, Cl, -CF₃, CN, N0₂, ―SO₂CH₃, -SCH₃ or -N(CH₃)₂; R" is hydrogen, C₁―C₄ alkyl, -OCH₃, F, Br or, Cl; R'" is hydrogen, -CH₃, Cl, F or Br; R₆ is hydrogen, C₁―C₆ alkyl, allyl, -CH₂CN; or -CH₂CH₂CN; or R₆ and R can be taken together to form ―(CH₂)₄―, ―(CH₂)₅―, ―(CH₂)₆―, ―CH₂CH₂OCH₂CH₂―; or 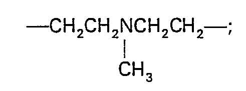 with the proviso that when R is -OCH₃ or OCH₂CH₃ then R₆ is H or -CH₃; when R₆ is -CH₂CH₂CN or -CHN₂CN then R is ―CH₂CH₂CN or CH₂CN; and R and R₆ have a total number of carbon atoms of less than or equal to 13; and when Q is 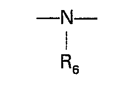
   3) R may also be C₃―C₆ alkynyl; 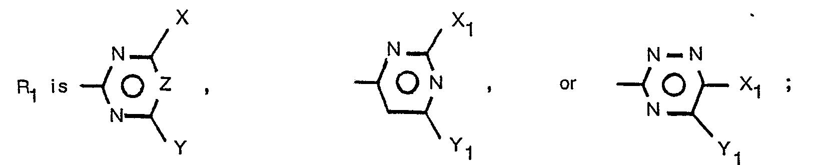
      R₂ is H, Cl, Br, F, C₁―C₃ alkyl, -NO₂, ―SO₂CH₃, -OCH₃, -SCH₃, ―CF₃, ―N(CH₃)₂, -NH₂, or -CN;
      R₃ is H, Cl, Br, F or CH₃;
      R₄ is H, or -CH₃;
      R₅ is H, -CH₃, or -OCH₃;
      M is an alkali metal;
      W is oxygen or sulfur;
      X is H, Cl, -CH₃, -OCH₃, -OCH₂CH₃ or ―OCH₂CH₂OCH₃;
      Y is H; Cl; C₁―C₄ alkyl; C₁―C₄ alkyl substituted with OCH₃, OC₂H₅, CN, CO₂CH₅, CO₂C₂H₅, or 1-3 atoms of F, CI or Br; C₃―C₄ alkenyl; CH₂≡CR₁₃; A (CH₂)ₙ,―A₁ (C₁―C₃ alkyl); 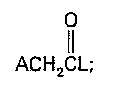 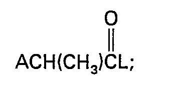 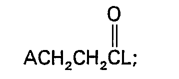 SCN; N₃; MR₁₆R₁₇; OR₁₄ or SR₁₅; wherein
      R₁₃ is H, CH₃ or CH₂Cl;
      n', A and A₁ are as previously defined L is NH₂, N(OCH₃)CH₃, NH(C₁―C₄ alkyl), N(C₁―C₄ alkyl)₂, or C₁―C₆ alkoxy;
      R₁₄ is C₁―C₄ alkyl; C₂―C₄ alkyl substituted with 1-3 atoms of F, CI or Br; C₁―C₄ alkyl substituted with CN; C₃―C₄ alkenyl; CH₂C=CR₁₃ wherein R₁₃ is as defined above; or 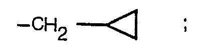
      R₁₅ is C₁―C₄ alkyl; cyanomethyl, cyanoethyl, allyl or propargyl;
      R₁₆ is H or CH₃;
      R₁₇ is H; OCH₃; C₁―C₄ alkyl; C₁―C₄ alkyl substituted with CN, CO₂CH₃ or CO₂C₂H₅; C₃―C₄ alkenyl; or C₂―C₃ alkyl substituted with either OCH or OC₂H₅; or
      R₁₆ and R₁₇ may be taken together to form CH₂CH₂CH₂CH₂ or CH₂CH₂OCH₂CH₂

      with the proviso that when Y contains 4 or more carbon atoms, then R contains not more than 4 carbon atoms; when X is Cl, then Y is Cl; and when X and Y are both H, then R contains not more than 4 carbon atoms,
   4) Y may also be F, Br, 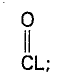 C₁―C₄, alkyl substituted with 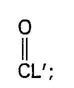 or NR₁₆R₁₇'; wherein
      L is as defined above or OH;
      R₁₆ is as defined above;
      L' is NH₂, OH, N(OCH₃)CH₃, NH(C₁―C₄ alkyl), N(C₁―C₄ alkyl)₂ or C₃―C₆ alkoxy; and
      R₁₇, is C₅―C₆ alkyl or C₁―C₄ alkyl substituted with 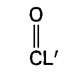 wherein
      L' is as defined above;
      Z is CH or N;
      Y₁ is H, -OCH₃ or -CH₃;
   5) Y₁ may also be OCH₂CH₃; and
      X, is H, Cl, -OCH₃, ―OCH₂CH₃ or -CH₃; providing that X, and Y, are not simultaneously hydrogen and when R₁ is 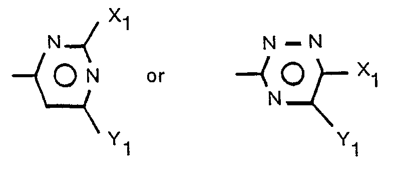 then R₄ and R₅ are both H and R contains 5 or less carbon atoms; provided that
         1) when Y is not as defined in 4), then R must be as defined in 1), 2), or 3;
         2) when Y₁ is not as defined in 5), then R must be as defined in 1), 2) or 3.

More Preferred and in increasing order or preference for reasons of higher biological activity and/or lower cost or both:
1) Compounds of the generic scope in which 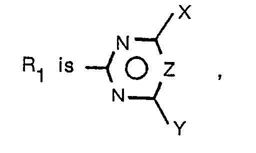 R₄ and R₅ are H, and W is O.
2) Compounds of Preferred 1) in which Q is oxygen.
3) Compounds of Preferred 2) in which R is C₁―C₆ alkyl, C₃―C₄ alkenyl, CH₂CH₂CI, CH₂CH₂OCH₃, or CH₂OR'₈.
4) Compounds of Preferred 3) in which X is CH₃ or OCH₃.
5) Compounds of Preferred 4) in which Y is 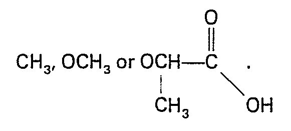
6) Compounds of Preferred 5) in which R₃ is H.
7. Compounds of Preferred 6) in which R₂ is H.
8. Compounds of Preferred 7) in which R'₈ is CH₃ or CH₃CH₂ and R is CH₃, CH₂CH₂0CH₃, or CH₂CH₂OC₂H₅.
   Specifically Preferred for highest activity and/or lowest cost and/or greatest ease of synthesis; [(2-methoxyethoxy)methyl] 2-[[(4,6-dimethoxypyrimidin-2-yl)aminocarbonyl]aminosulfonyl]benzoate. [(2-methoxyethoxy)methyl] 2-[[(4-ethoxy-6-methyl-1,3,5-triazin-2-yl)aminocarbonyl]aminosulfonyl]-benzoate
      [(4-chlorophenoxy)methyl] 2-[[(4,6-dimethylpyrimidin-2-yl)aminocarbonyl]aminosulfonyl]benzoate. methyl 2-[[[4-(1-carboxyethoxy)-6-methyl-1,3,5-triazin-2-yl]aminocarbonyl]aminosulfonyl]benzoate. [(2-methoxyethoxy)methyl 2-[[(4,6-dimethylpyrimidin-2-yl)aminocarbonyl]aminosulfonyl]benzoate. (4-chloro-2-butynyl) 2-[[(4-methoxy-6-methylpyrimidin-2-yl)aminocarbonyl]aminosulfonyl]benzoate.

These compounds may be used in the form of an agriculturally suitable salt, where appropriate.

### Synthesis

Many of the compounds of Formula I are prepared as shown in Equation 3 by the reaction of an appropriately substituted o-carbonylbenzenesulfonyl isocyanate or isothiocyanate with an appropriate aminopyrimidine or aminotriazine. These compounds of Formula I can be converted into other compounds of Formula I as will be shown in subsequent equations. Thus, o-carbonylbenzenesulfonyl isocyanates and sulfonyl isothiocyanates are important intermediates for the preparation of the compounds of this invention. Therefore, the synthesis of these is described in Equations 1 and 2.

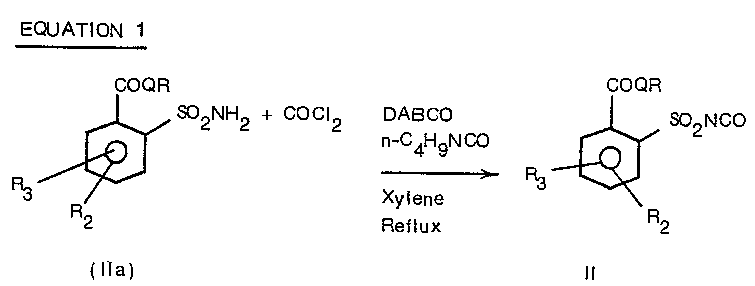

A mixture of the appropriate sulfonamide, e.g. an o-alkoxycarbonyl benzenesulfonamide Ila such as the methyl ester, which is known in the art, an alkyl isocyanate such as butyl isocyanate and a catalytic amount of 1,4-diaza [2.2.2]bicyclooctane (DABCO) in xylene or other inert solvent of sufficiently high boiling point (e.g. >135°) is heated to approximately 135°C. Phosgene is added to the mixture until an excess of phosgene is present as indicated by a drop in the boiling point. (The mixture is heated further to drive off the excess phosgene). After the mixture is cooled and filtered to remove a small amount of insoluble by-products. The solvent and alkyl isocyanate are distilled off in-vacuo leaving a residue which is the crude sulfonyl isocyanate II. In Equation 1
Q is O
R is C₁―C₁₂ alkyl; C₃―C₁₀ alkenyl; C₂―C₆ alkyl substituted with one to four substituents selected from 0-3 atoms of F, Cl, Br, 0-2 methoxy groups; C₃―C₆ alkenyl substituted with 1-3 atoms of F, Cl, Br; C₅―C₈ cycloalkyl; C₅―C₈ cycloalkenyl; C₅―C₆ cycloalkyl substituted with any of one to four methyl groups, methoxy, alkyl substituents of C₂―C₄, F, CI or Br; C₄―C₁₀ cycloalkylalkyl; C₄―C₈ cycloalkylalkyl with 1-2 CH₃; ―CH₂CH₂OR₇; CH₂CH₂CH₂OR₇; 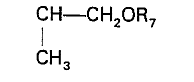 where R₇ is ―CH₂CH₃, CH(CH₃)₂, phenyl -CH₂CH₂Cl, ―CH₂CCl₃; (̵CH₂CH₂O)̵ₙ,―R₈; 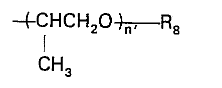 where Rₐ is CH₃, -CH₂CH₃ ―CH(CH₃)₂, phenyl, -CH₂CH₂Cl, ―CH₂CCl₃, and n' is 2 or 3;
R₂ is H, CI, Br, F, C₁―C₃ alkyl, ―NO₂, -OCH₃, -SCH₃, CF₃, SO₂CH₃, N(CH₃)₂, CN;
R₃ is H, Cl, Br or CH₃

Where W = S in Formula I the useful sulfonylisothiocyanate intermediates are prepared according to Equations 2 and 2'.

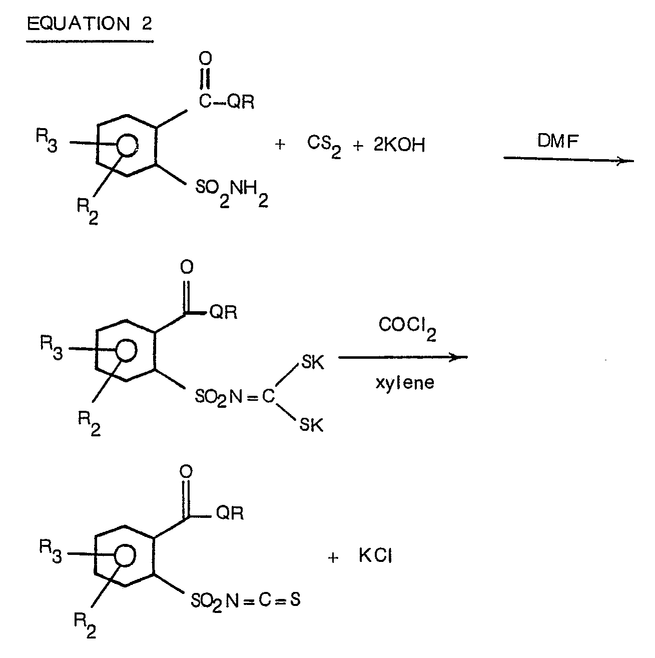

The o-carbonyl substituted sulfonamide is dissolved in dimethylformamide (DMF) with an equivalent amount of carbon disulfide and two equivalents of potassium hydroxide are added portionwise at room temperature. The mixture is stirred for 1-8 hours and diluted with ethyl acetate, ethyl ether or similar aprotic solvent to cause the dipotassium salt of the dithiocarbamic acid to precipitate. The salt is isolated, dried and suspended in an inert solvent such as xylene, benzene, carbon tetrachloride or methylene chloride. Phosgene is added to the stirred suspension at below room temperature and the mixture stirred for 1-3 hours. In place of phosgene, a chloroformic ester (e.g. methyl chloroformate), phosphorus pentachloride, sulfuryl chloride or thionyl chloride can be used.

The sulfonylisothiocyanate which is formed is usually soluble in the solvent and is isolated by filtering off the inorganic potassium chloride and concentrating the filtrate. These isothiocyanates tend to be unstable and dimerize readily, (Equation 2') however, the dimers can be used. 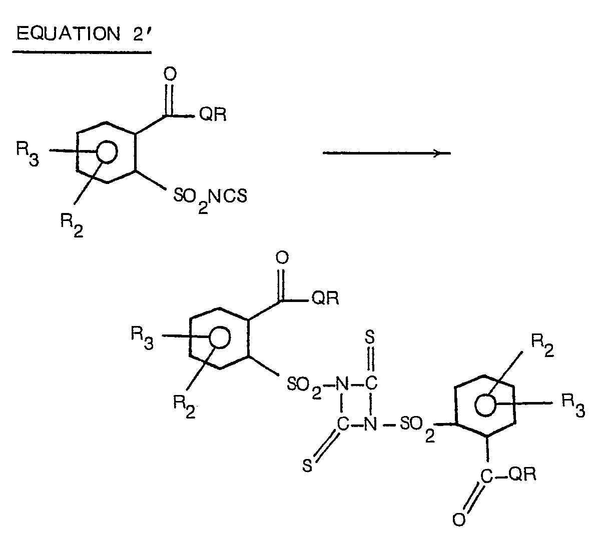 in the same manner as the parent isothiocyanates for the purposes of this invention.

The synthetic method chosen for the preparation of compounds of Formula I depends largely on the substituents R and R₄. As shown in Equation 3, compounds of Formula I, wherein Q, R, R and R₃ are as defined for Equation 1, are conveniently prepared by reacting an appropriately substituted carbonylbenzenesulfonyl isocyanate or isothiocyanate of Formula Ilb with an appropriately substituted aminopyrimidine or aminotriazine of Formula III:

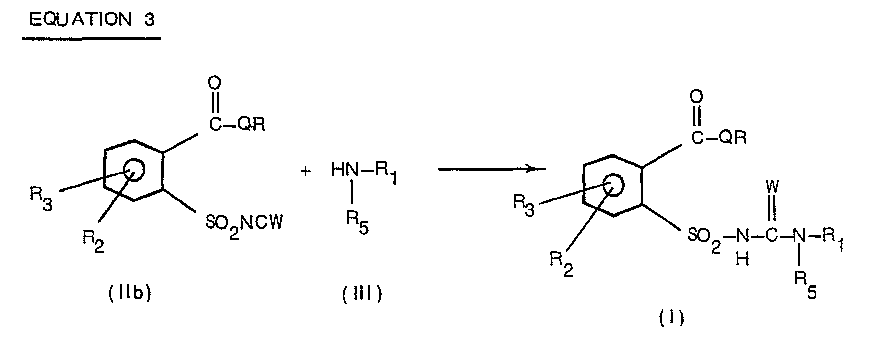

The reaction of Equation 3 is best carried out in inert aprotic organic solvents such as methylene _chloride, tetrahydrofuran or acetonitrile, at ambient pressure and temperature. The mode of addition is not critical; however, it is often convenient to add the sulfonyl isocyanate or isothiocyanate to a stirred suspension of amine III. Since such isocyanates and isothiocyanates are liquids, low melting solids or are readily soluble in solvents such as those listed above, their addition can be easily controlled.

The reaction is generally exothermic. In some cases, the desired product is soluble in the warm reaction medium and on cooling crystallizes in pure form. Other products which are soluble in the reaction medium are isolated by evaporation of the solvent, trituration of the solid residue with solvents such as 1-chlorobutane or ethyl ether, and filtration.

As shown in Equation 3A compounds of Formula la, wherein R is not H or M, W is S and R₅ is H, are alternatively prepared by the reaction of an appropriately substituted o-carbonylbenzene- sulfonamide with the appropriate triazine or pyrimidine isothiocyanate of formula IIIA.

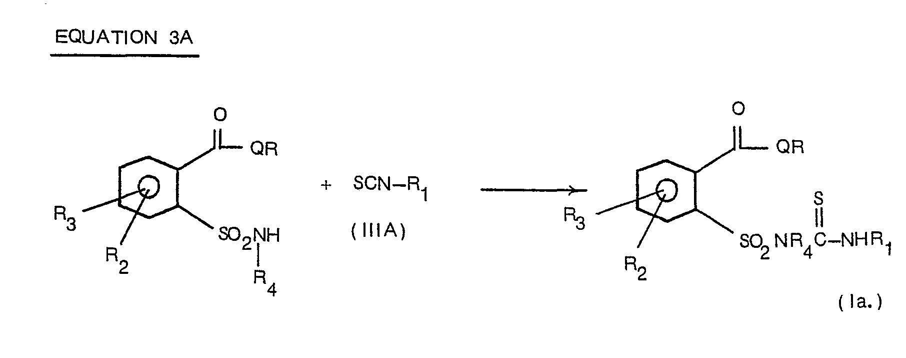

The reaction of Equation 3A is best carried out by dissolving or suspending the sulfonamide and isothiocyanate in a polar solvent such as acetone, acetonitrile, ethyl acetate or methylethyl ketone, adding an equivalent of a base such as potassium carbonate and stirring the mixture at ambient temperature up to the reflux temperature for one to twenty-four hours. In some cases, the product precipitates from the reaction mixture and can be removed by filtration. The product is stirred in dilute mineral acid, filtered and washed with cold water. If the product does not precipitate from the reaction mixture it can be isolated by evaporation of the solvent, trituration of the residue with dilute mineral acid and filtering off the insoluble product.

The heterocyclic isothiocyanates which are used in the procedure of Equation 3A are prepared, for example, according to the method of Japan Patent Application Pub: Kokai 51-143686, June 5, 1976, or that of W. Abraham and G. Barnikow Tetrahedron 29, 691-7 (1973).

As shown in Equation 4, compounds of Formula I, wherein Q is O, S or 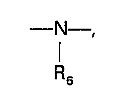 and R is as defined for Equation 1 R₄ is methyl and W is 0, can be prepared by methylation of salts IV wherein M is an alkali metal cation such as sodium (derived from compounds of Formula I wherein R₄ is hydrogen): 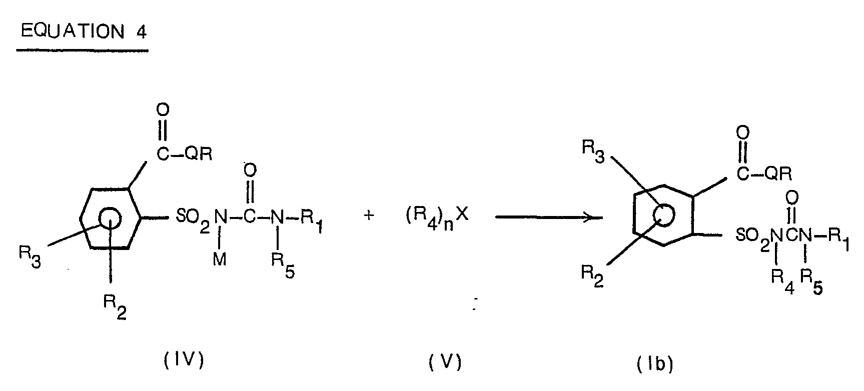
X being an incipient anion and n being an integer corresponding to the valence of X.

The reaction of Equation 4 is best carried out in aprotic organic solvents such as tetrahydrofuran, dimethylformamide, or dimethylacetamide, at ambient pressure and temperature. Methylating agents V, such as dimethyl sulfate or methyl iodide, can be employed. The desired product can be isolated by pouring the reaction mixture into water and filtering off the precipitated solid.

As shown in Equation 5, compounds of Formula lc, wherein Q is O, S or 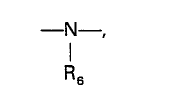 R and R₄ are as defined for Equation 4, can also be prepared by the reaction of an appropriately substituted sulfonyl-N-methylcarbamoyl chloride or sulfonyl-N-methylthiocarbamoyl chloride of Formula VI with an appropriate aminopyrimidine or aminotriazine of Formula III:

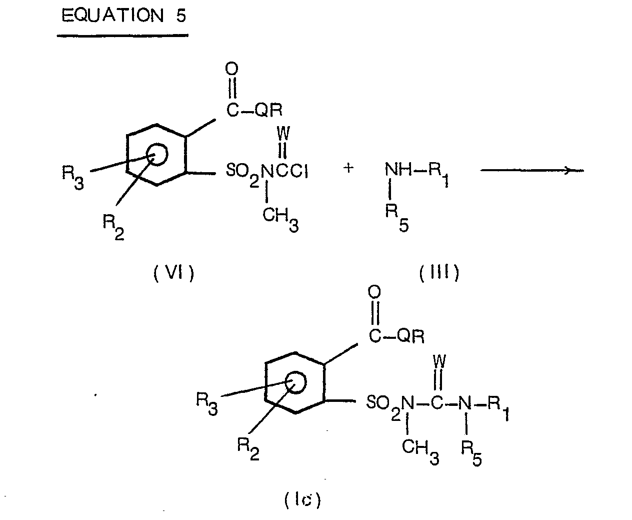

The preparation of ureas and thioureas, like those of Formula Ic, from amines and carbamoyl chlorides and thiocarbamoyl chlorides is well known to the art. The reaction can best be carried out by adding equivalent amounts of the chloride VI and amine III to an inert organic solvent such as tetrahydrofuran, xylene, or methylene chloride, in the presence of an acid acceptor, such as triethylamine, pyridine, or sodium carbonate employing temperatures from 20°-130°C. Soluble products can be isolated by filtering off the precipitated salts and concentration of the filtrate. Insoluble products can be filtered off and washed free of salts with water.

The chlorides of Formula VI can be prepared by phosgenation or thiophosgenation of N-alkylsulfonamide salts. The sulfonamide salt is added to an excess of phosgene or thiophosgene in an inert organic solvent, such as tetrahydrofuran, toluene, or xylene, whereupon, after removal of the excess phosgene, the chloride VI can be isolated or reacted in situ with the amine III.

Compounds of Formula le, wherein R is -H, can be prepared by hydrolysis of esters of Formula Id wherein R is C₁―C₁₂ alkyl. As shown in Equation 6, alkali metal base catalyzed hydrolysis in aqueous methanol produces the alkali metal carboxylate from which the carboxylic acid is obtained by treatment with mineral acids such as HCI:

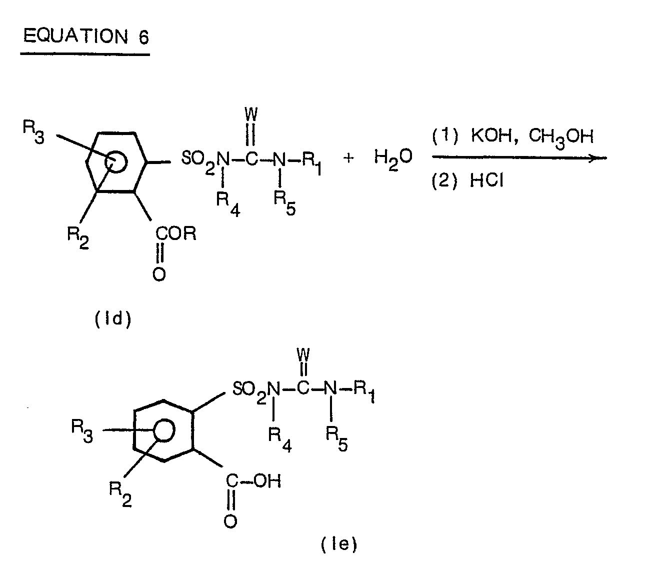

The reaction of Equation 6 is best carried out in a solution containing the compound being hydrolyzed, 2 to 10 parts of methanol, 10-50 parts of water and 2-10 equivalents of a base such as sodium or potassium hydroxide maintaining the temperature at 30-90°C for 3-24 hours. The reaction yields the soluble alkali metal salt of the carboxylic acid, which is suitable for the purposes of this invention. Conversion of these salts to the acid form is easily carried out by addition to the reaction medium of strong mineral acids, such as hydrochloric or sulfuric acid, causing the desired carboxylic acids to precipitate from solution.

Compounds wherein W and Q are 0 and R is H can be converted to compounds of this invention where R is other than hydrogen, as already disclosed herein, by the reaction of salts of the parent acid (R=H) with R-Halogen as shown in Equation 6A

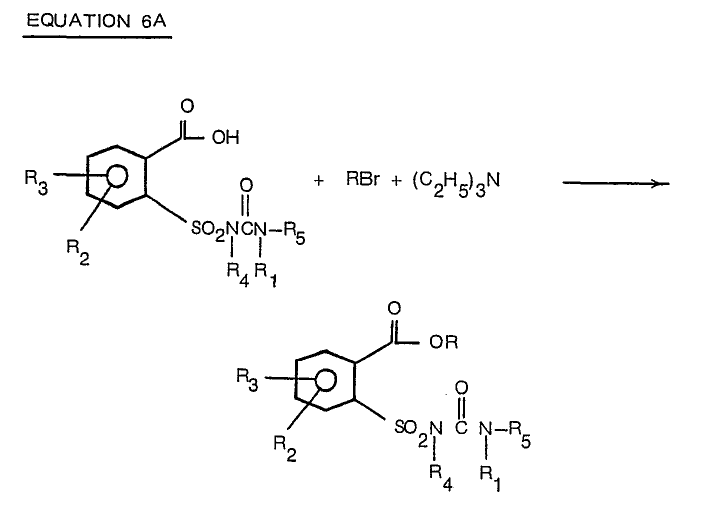

The reaction of Example 6A is of use where the intermediate compound R-Halogen contains a readily replaceable halogen as is the case for substituted or unsubstituted allylic or benzylic halides, a-halonitriles, or a-halocarbonyl compounds.

The procedure of Equation 6A is best carried out in an inert polar solvents such as tetrahydrofuran, acetonitrile or acetone by combining the appropriately substituted carboxylic acid and base such as triethylamine or 1,4-diaza⁅2,2,2⁆bicyciooctane adding the appropriate halide and heating the mixture to reflux with stirring for 1 to 16 hours. The reaction mixture can be evaporated to dryness and the residue triturated with water, filtered and washed with water to separate the desired product from the water soluble salt.

Certain compounds of Formula I, wherein Q is oxygen, are more conveniently prepared by reaction of the silver salt of the carboxylic acid and the appropriate R group containing a suitable leaving group such as iodide. Thus, a substituted iodobenzene is reacted with the silver carboxylate as in Equation 6B. 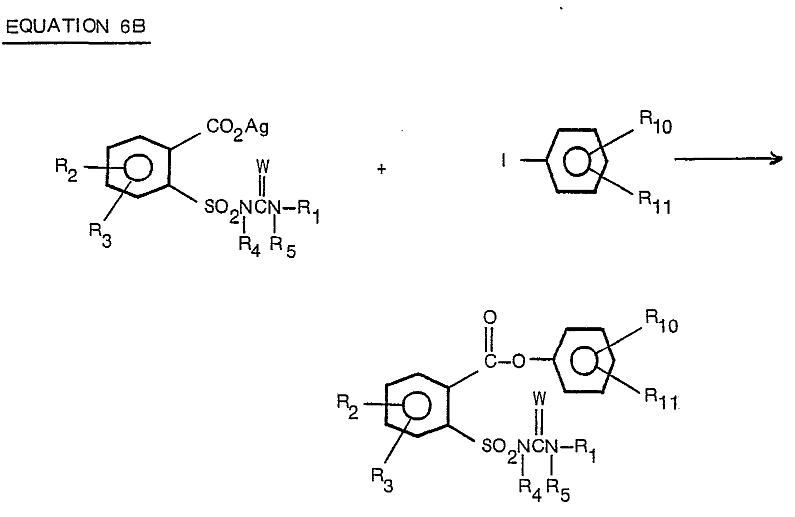 in a suitable solvent such as acetonitrile between 0° and 80°C for 1-6 hours.

Esters where R is C₃-C₉ alkynyl are also conveniently prepared by this method.

The silver salt is prepared by adding an excess of silver nitrate to an aqueous solution of the sodium carboxylate and filtering the precipitate and washing with water.

Compounds of Formula I wherein Y of group R, contains 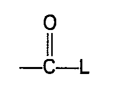 and L is OH can be prepared according to the procedure of Equation 6C wherein R, R₂, R₃, Q, R₄, W, X and R₅ are as previously defined.

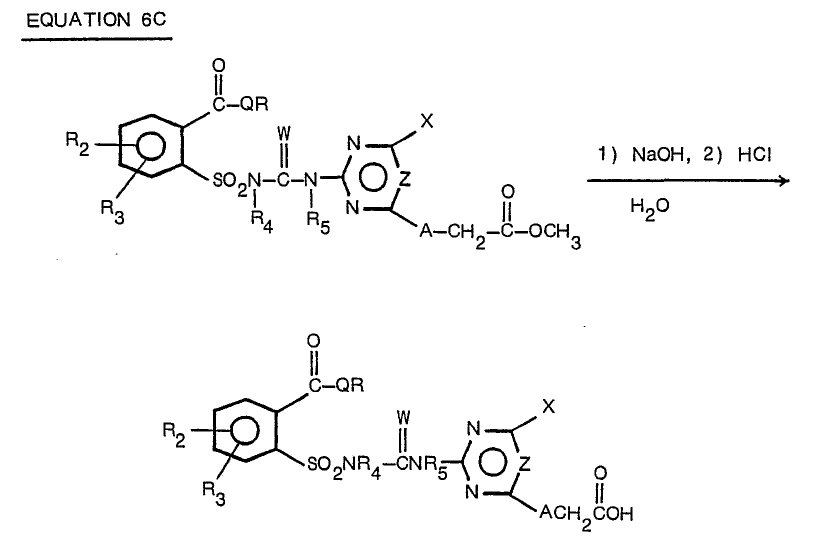

The reaction of Equation 6C is best carried out by suspending the compound being hydrolyzed in 10 to 100 parts of water with enough of a base such as sodium hydroxide or potassium hydroxide to obtain a pH of 10 to 14, ideally, a pH of 12, heating until a clear solution is obtained and then adjusting the pH to 1-3, preferably 3. The product is thus caused to precipitate in some instances and can be removed by filtration or it can be extracted into a polar organic solvent such as methylene chloride and isolated by evaporation of the solvent.

When Q is NR₆, the compounds can be prepared from the esters of this invention where R is C₁―C₄ (preferably C₁) by the reaction of the esters with dialkylaluminium-N-alkylamide derivatives according to Equation 7, R, R₁, R₂, R₃ and R₆ being as previously defined.

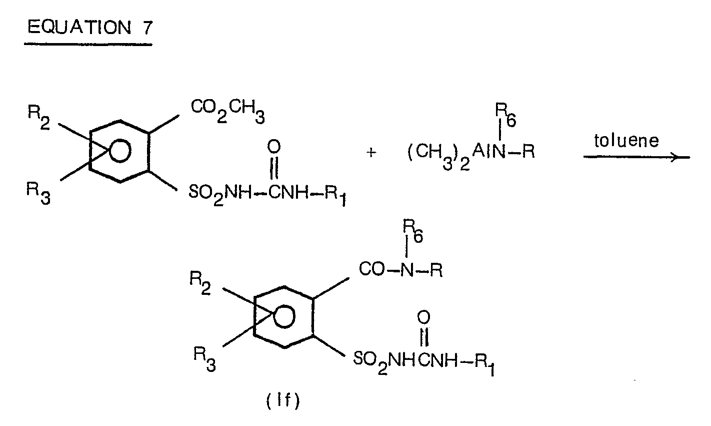

The intermediate alkylaminoaluminium compounds prepared according to A. Basha, M. Lipton and S. W. Weinreb, Tetrahedron Letters 4171 (1977), are co-mingled with a suspension of the esters in toluene or similar inert solvent and the mixture is refluxed for one to six hours. The product can be isolated by evaporation of the solvent toluene, adding methylene chloride and aqueous hydrochloric acid to decompose the residual reaction mass and extracting the desired product into methylene chloride. Evaporation of the methylene chloride yields the desired product in sufficiently pure form for the purpose of this invention.

Compounds of formula lid, wherein Q is NR₆ and R₁, R₂, R₃ and R₄ are as previously defined in the general formula, which are useful as intermediates in Equation 3A, are prepared as shown in Equation 7A.

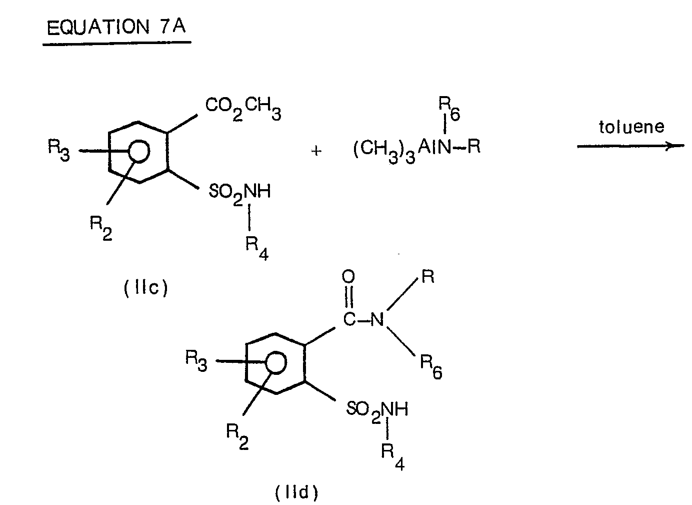

The conditions described for Equation 7 are suitable for the conversion of the esters of formula IIc to the amides lid as shown in Equation 7A.

The products of Equation 7A are especially useful for the preparation of compounds of formula la wherein Y has an ester substituent CO₂(C₁―C₆), by the route described in Equation 3A.

When Q is S, these compounds can be prepared from the esters of this invention wherein QR is C₁―C₄ alkoxy (preferably C₁) by the reaction of the esters with the appropriate dialkylaluminium alkylthiolate according to Equation 8.

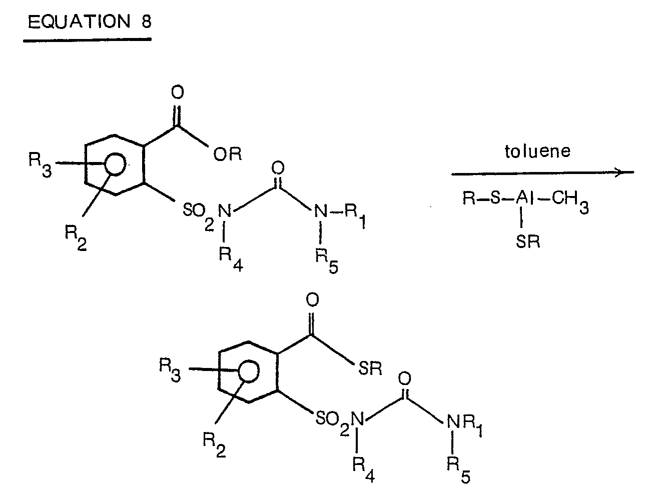

The intermediate aluminium thiolates can be prepared according to R. P. Hatch and S. W. Weinreb Journal of Organic Chemistry, Vol. 42, 3960 (1977). The reaction of the thiolate with the ester of this invention is best carried out in a neutral solvent such as toluene or xylene at reflux for one to three hours. Best results are obtained when the aluminum thiolate compound is present in excess of the stoichiometric amount required.

Sulfonamides of formula Ilb are also converted from carboxylic acid esters to the thiolesters as shown in Equation 8A according to the method of R. P. Hatch and S. W. Weinreb as described for Equation 8 wherein R, R₂, R₃ and R₄ are as previously defined.

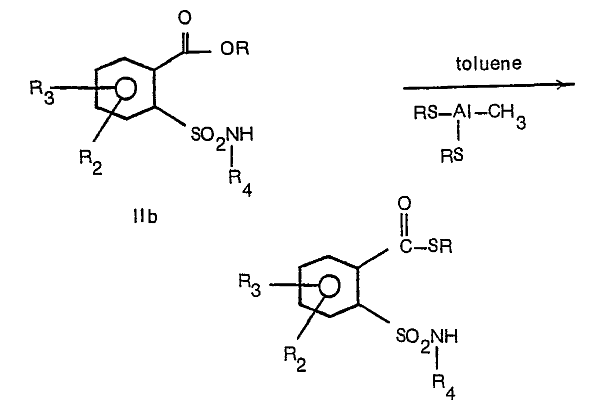

The conditions described for Equation 8 are suitable for the conversion of the sulfonamides of formula Ilb as shown in Equation 8A.

The production of Equation 8A are especially useful for the preparation of compounds of formula la, wherein Y has a substituent (CO₂C₁―C₆) by the route described for Equation 3A.

An alternate route to prepare compounds where R is bonded to Q (Q=O) at a secondary carbon involves the reaction of the appropriate dialkylaluminium alcoholate and an ester of this invention wherein R is a lower primary alkyl group, preferably methyl, according to Equation 9.

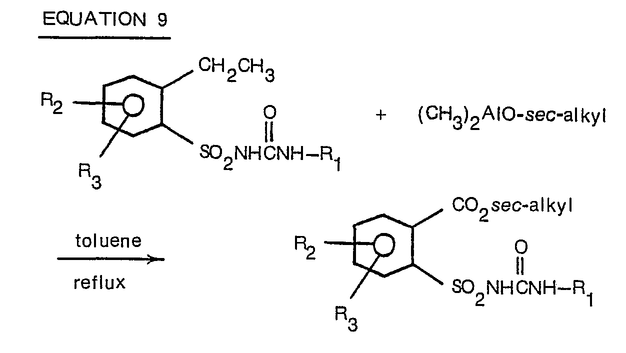

The reaction is carried out in a neutral solvent such as toluene with a boiling point sufficiently high to bring about the desired reaction during reflux. The dialkylaluminium alcoholate being present in greater than an equivalent amount to the ester for best yields. After refluxing for 1-15 hours, the reaction mixture is decomposed with dilute hydrochloric acid and the product extracted into methylene chloride. Evaporation of the methylene chloride yields the desired compound sufficiently pure for the purposes of this invention. The product can be triturated with a solvent, e.g. 1-chlorobutane to remove impurities.

The synthesis of heterocyclic amines has been reviewed in "The Chemistry of Heterocyclic Compounds" a series published by Interscience Publ., New York and London. 2-Aminopyrimidines are described by D. J. Brown in The Pyrimidines, Vol. XVI of this series. The 2-amino-1,3,5-triazines are reviewed by K. R. Huffman and in The Triazines of this same series. The synthesis of triazines are also described by F. C. Schaefer, U.S. Patent No. 3,154,547 and by K. R. Huffman and F. C. Schaeffer, J. Org. Chem. 28, 1816-1821 (1963).

The preparation of agriculturally suitable salts of the compounds of Formula I, as well as starting materials and intermediates for said compounds, not otherwise described herein, is disclosed in our U.S. Patent Specification No. 4,127,405, to which the reader is referred for further information.

The compounds of this invention may in general be made by the methods disclosed in our European Patent Specification No. 0,007,687, or by suitable variants thereof.

The compounds of this invention and their preparation are further illustrated by the following examples wherein temperatures are given in degrees centigrade and parts by weight unless otherwise indicated.

### Example 1

### Methyl 2-(isocyanatosulfonyl)benzoate

A stirred mixture containing 157 g of methyl 2-sulfamoylbenzoate, 73 g of butyl isocyanate 0.3 g of 1,4-diazabicyclo[2.2.2]octane and 1.0 I of xylene was heated to reflux for one half hour. Phosgene gas was then passed into the system under a dry ice reflux condenser allowing the reaction temperature to drop to 120°. This addition was continued until the reflux temperature remained at 120° without further phosgene addition. The temperature of the reaction mixture was then raised to 136° (by removal of the dry ice reflux condenser) after which it was cooled to room temperature and filtered. Evaporation of the filtrate yielded the desired crude sulfonyl isocyanate which could be purified by distillation at 132-138°C under 1.0 to 1.1 mm of mercury pressure. The product is extremely reactive with water so contact with moisture should be scrupulously avoided.

### Example 2

### Isopropyl 2-(isocyanatosuffonyl)benzoate

To 60.7 g (.25 mole) of isopropyl 2-sulfamoylbenzoate in 300 ml dry (molecular sieves) xylenes was added 25.0 g (.25 mole) n-butyl isocyanate and .1 g 1,4-diazabicyclo[2.2.2]octane. The mixture was heated to reflux temperature and phosgene was slowly bubbled through the solution for 2 hours.

An infrared spectrum of the reaction mixture indicated formation of the desired sulfonylisocyanate (2250 cm-'). The resulting cloudy solution was cooled to room temperature and decanted from a small amount of solid impurity. Evaporation of the resulting clear solution yielded the desired crude sulfonyl isocyanate, which was used in subsequent steps without further purification.

### Example 3

### Methyl 2-[[[4-( 1-carboxyethoxy)-6-methyl-1,3,5-triazin-2-yl]aminocarbonyl]aminosulfonyl]benzoate

Methyl 2-(isocyanatosulfonyl)benzoate (10.1 g) and 8.0 g of ethyl 2-[(4-amino-6-methyl-1,3,5-triazin-2-yi)oxy]propanoate were stirred in 80 ml of methylene chloride at ambient temperature for 18 hours. An additional 2 g of the sulfonylisocyanate was added and the reaction mixture was stirred for four more hours and then 2 g more of the sulfonylisocyanate was added. After stirring for four more hours at ambient temperature, 400 ml of water was added and the pH of this mixture was adjusted to pH 8 by the addition of 10% aqueous sodium hydroxide. The aqueous phase was separated and acidified to pH 1 with hydrochloric acid to precipitate a thick white gum. Sufficient 50% aqueous sodium hydroxide was added to achieve pH 12 and the mixture was heated on a steam bath until a clear solution resulted. The solution was again acidified with hydrochloric acid to pH 3 and the resultant cloudy solution was extracted with dichloromethane. The organic phase was dried and the solvent was removed to afford 3.7 g of the title compound as a glass.

By using the procedures of Examples 1 to 3, or the methods described herein, the compounds of Tables I to IX may be prepared.

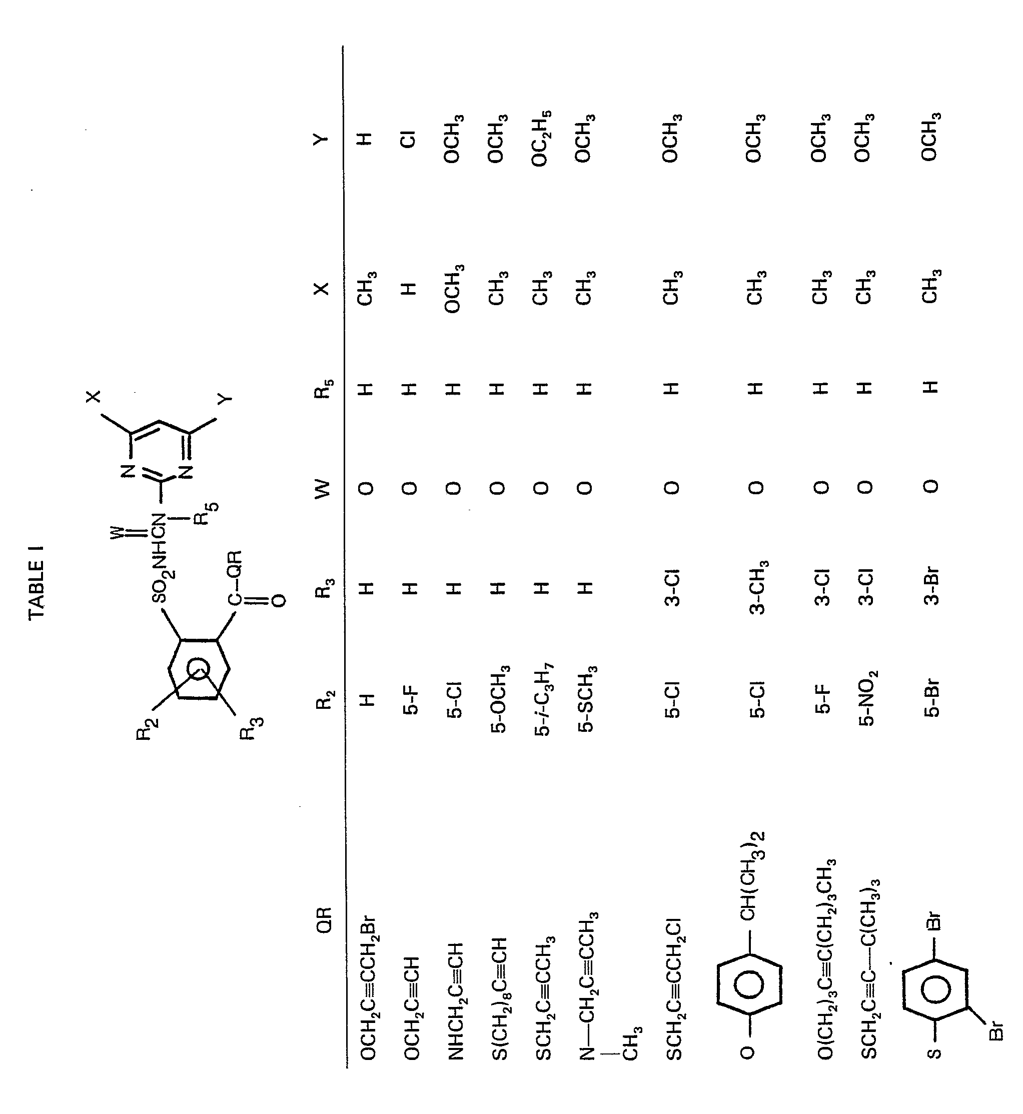

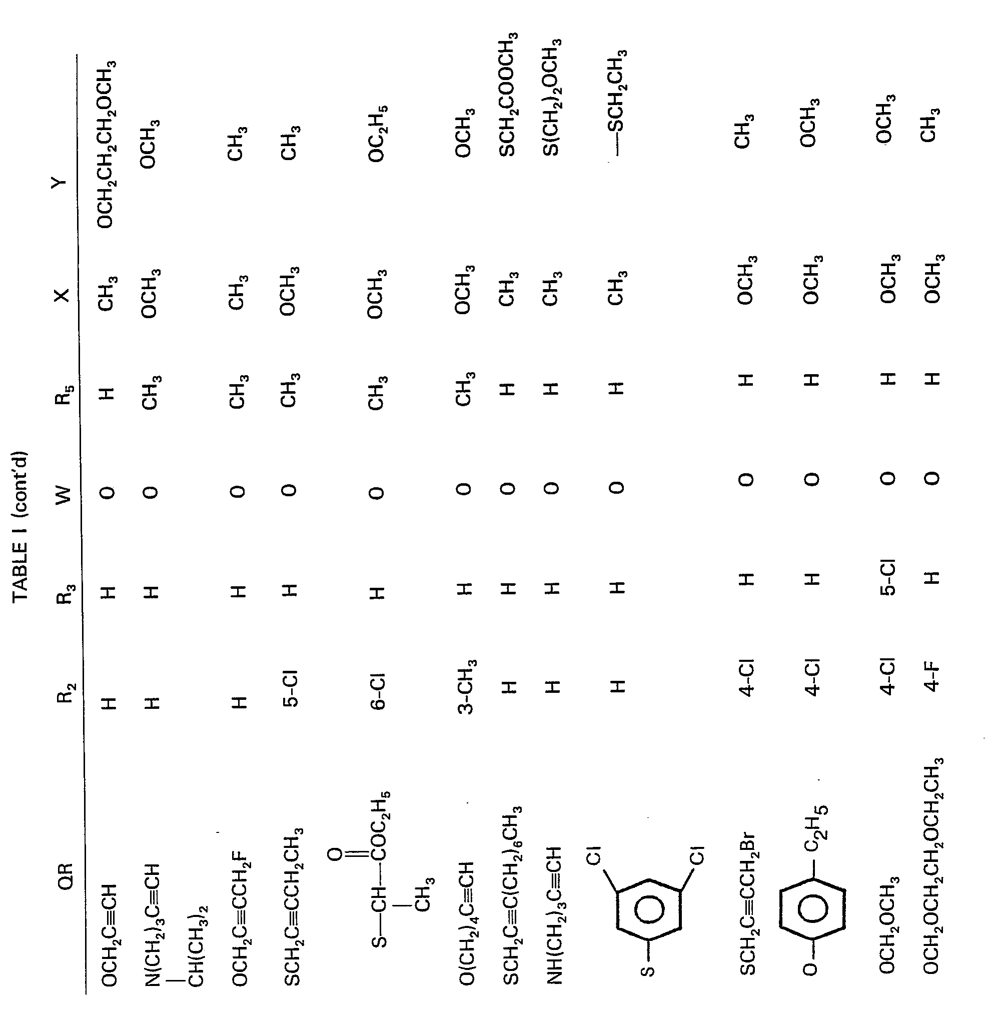

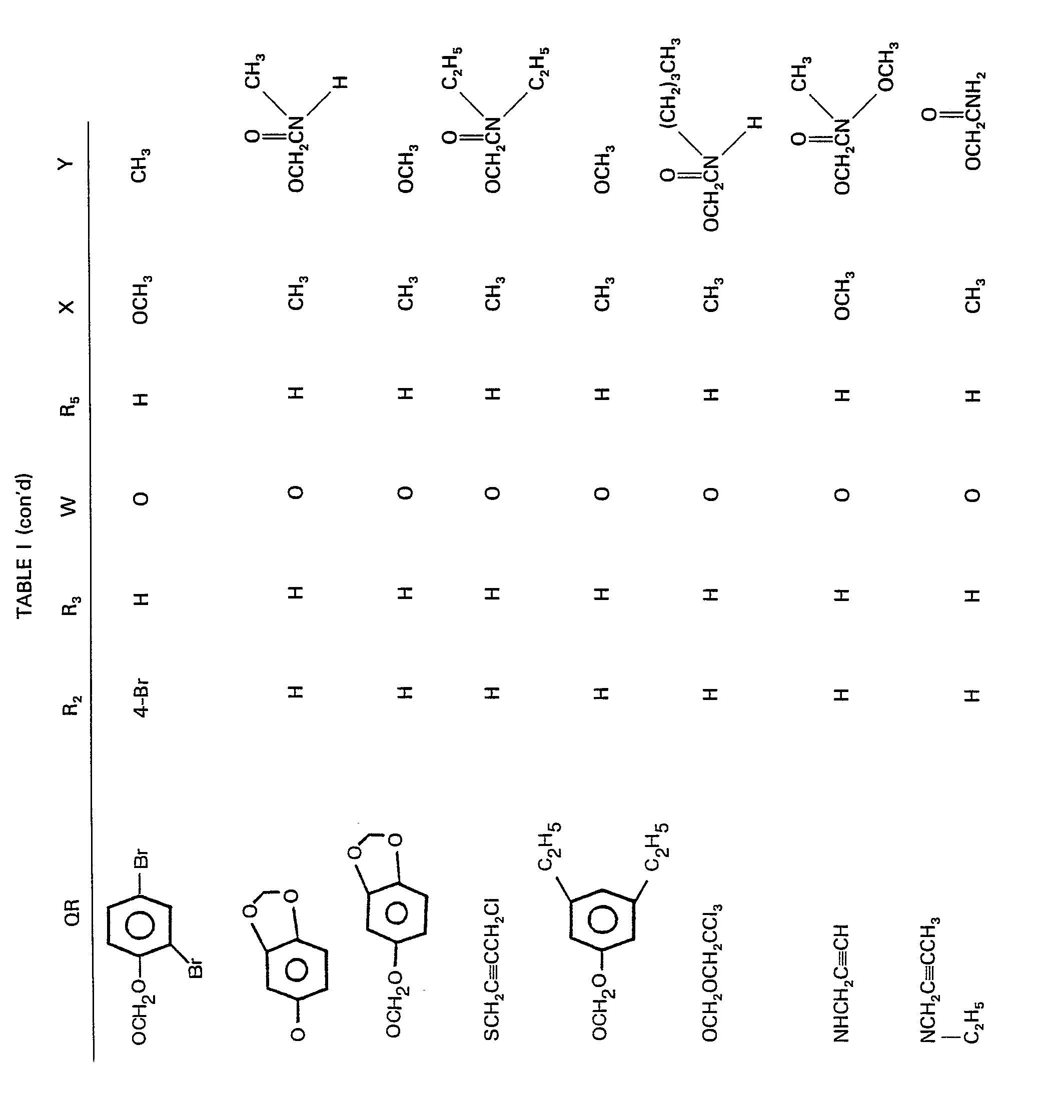

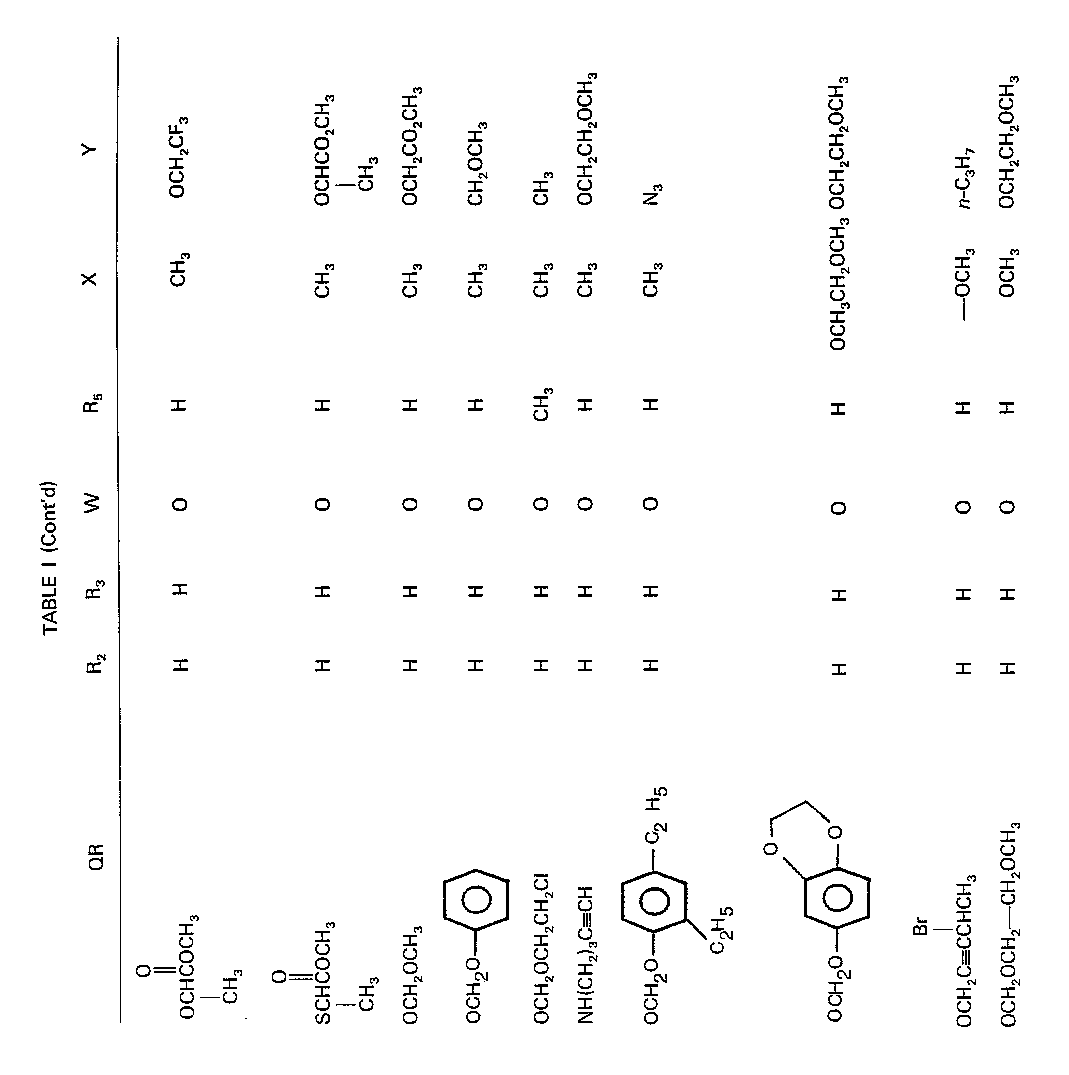

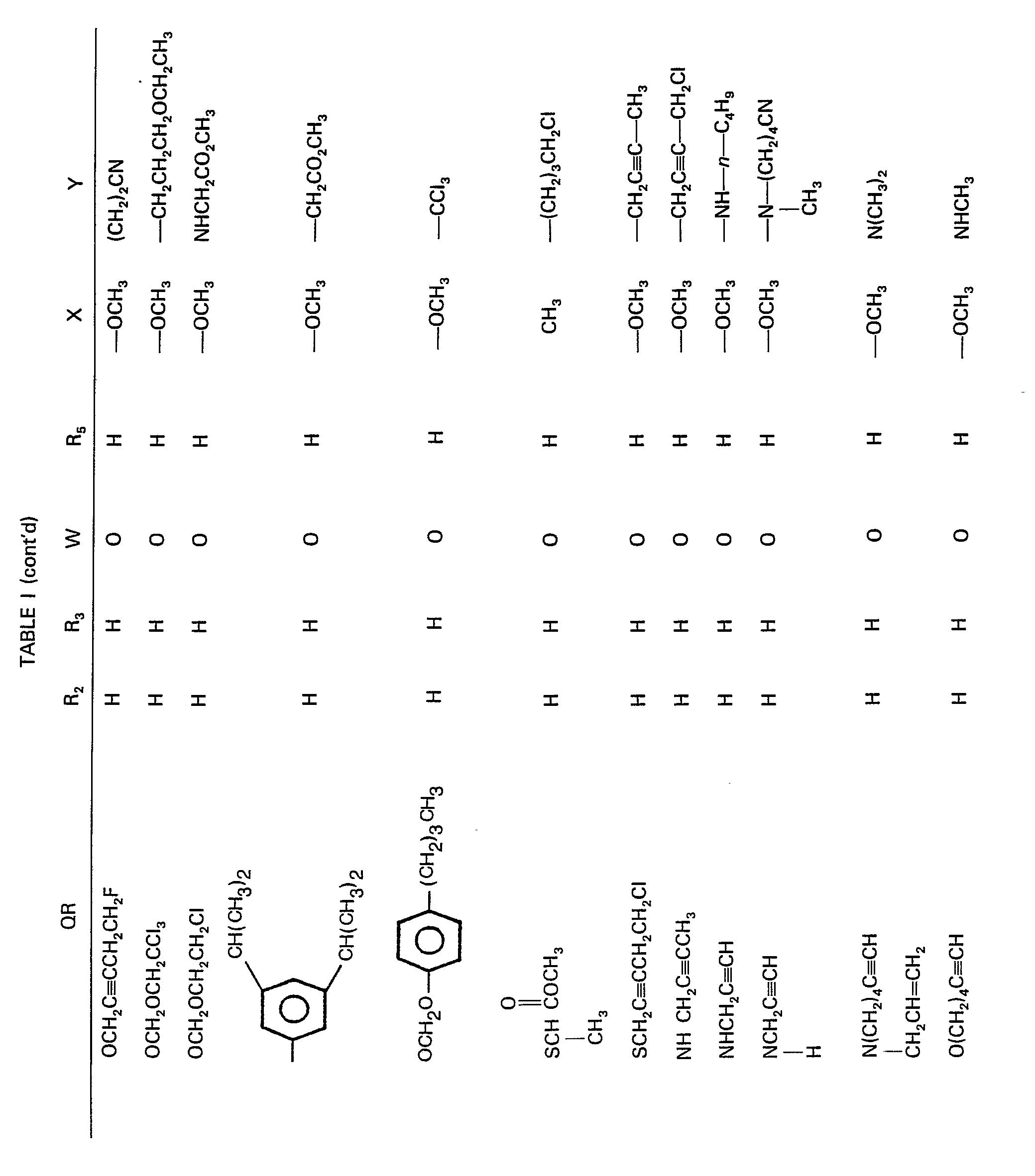

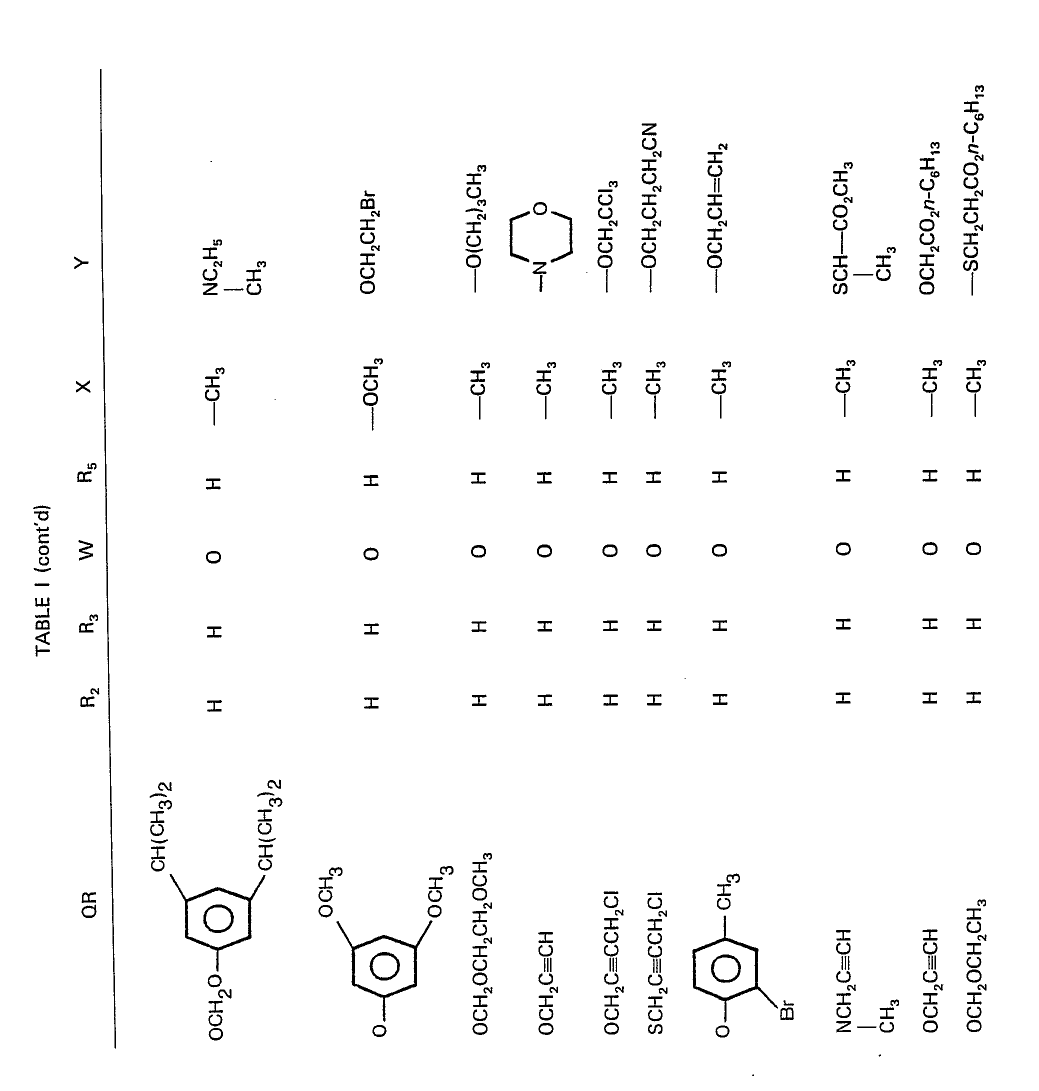

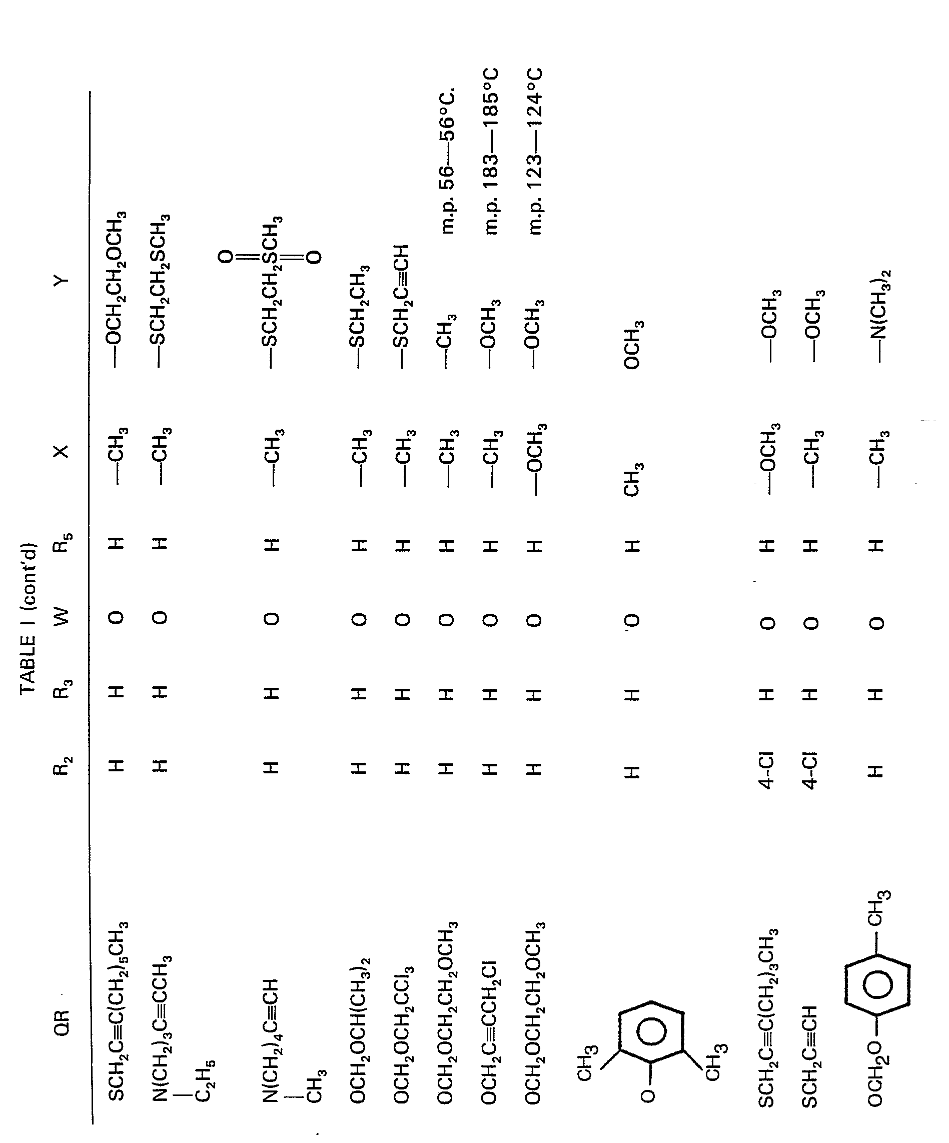

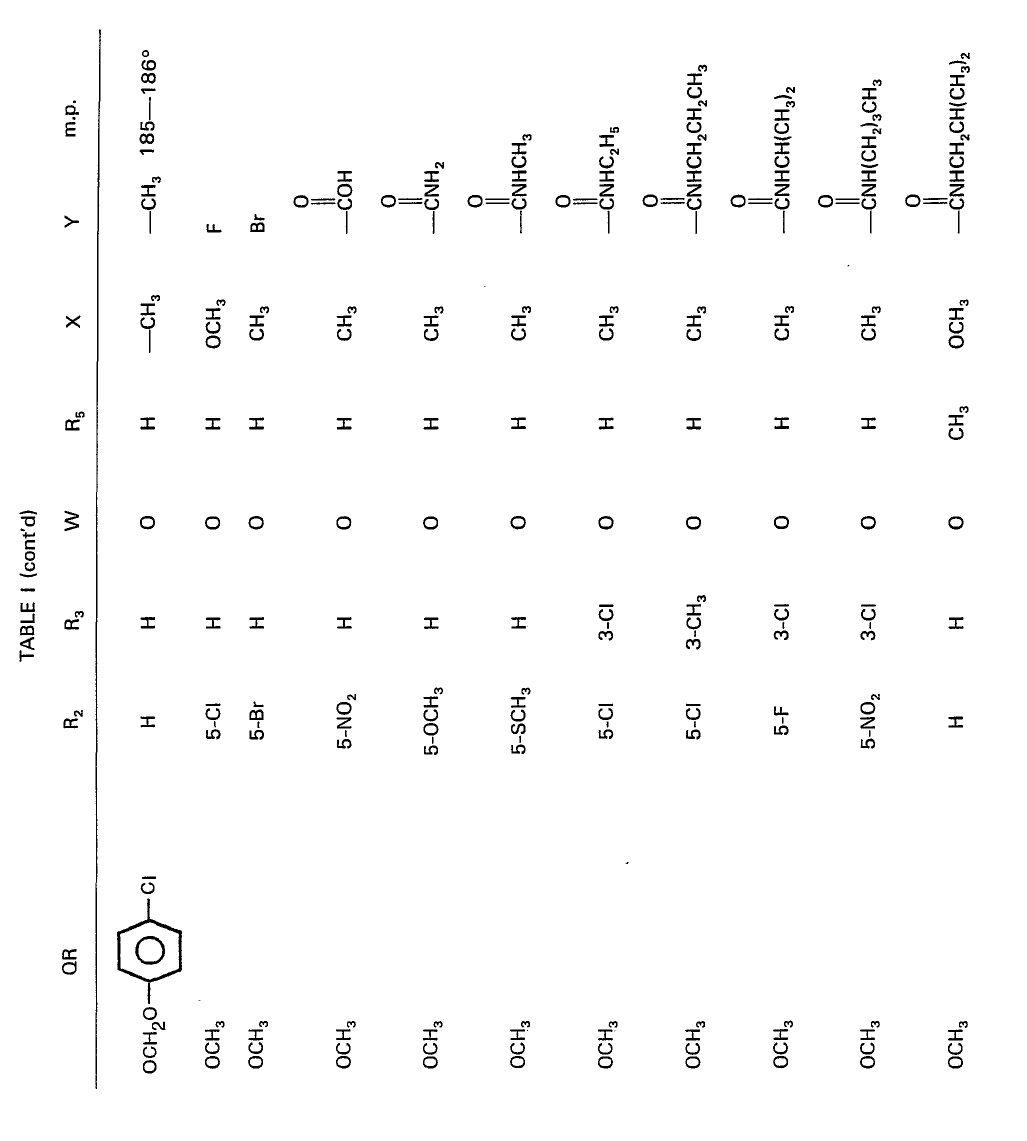

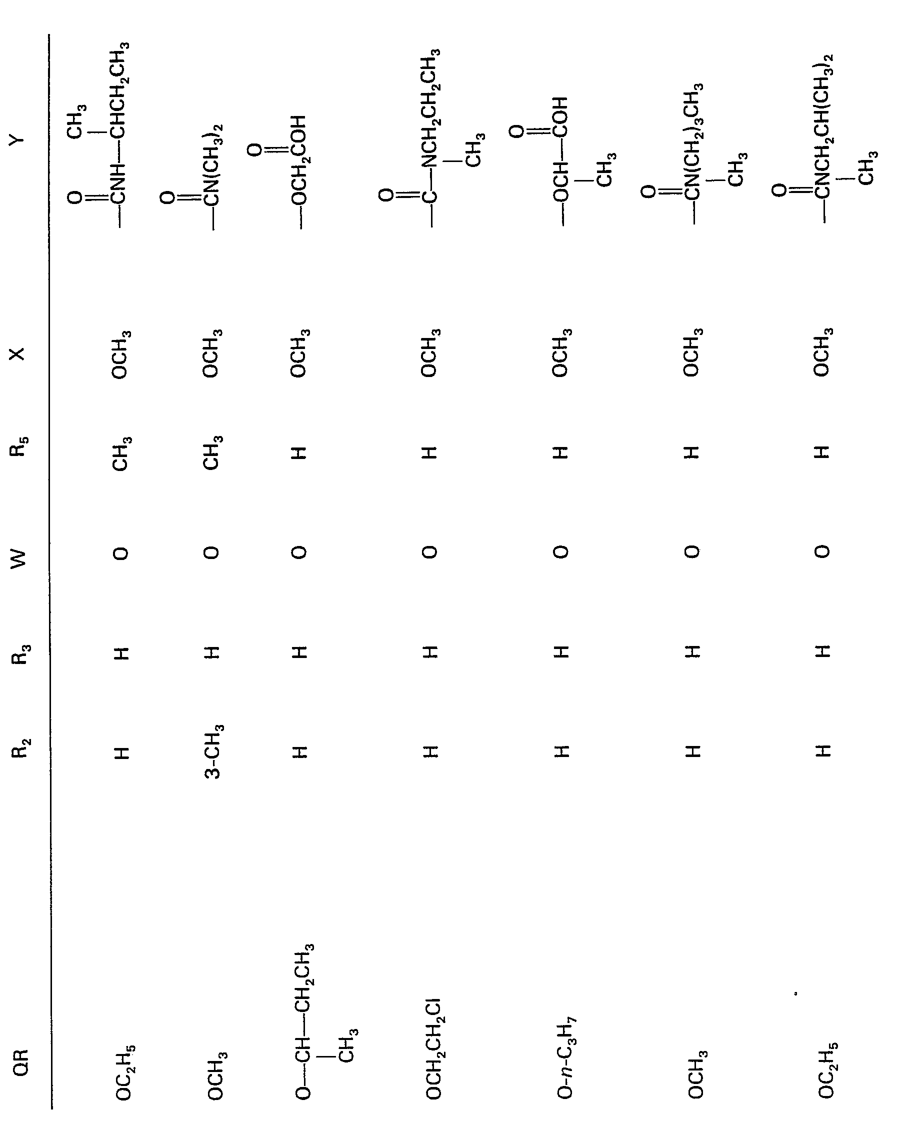

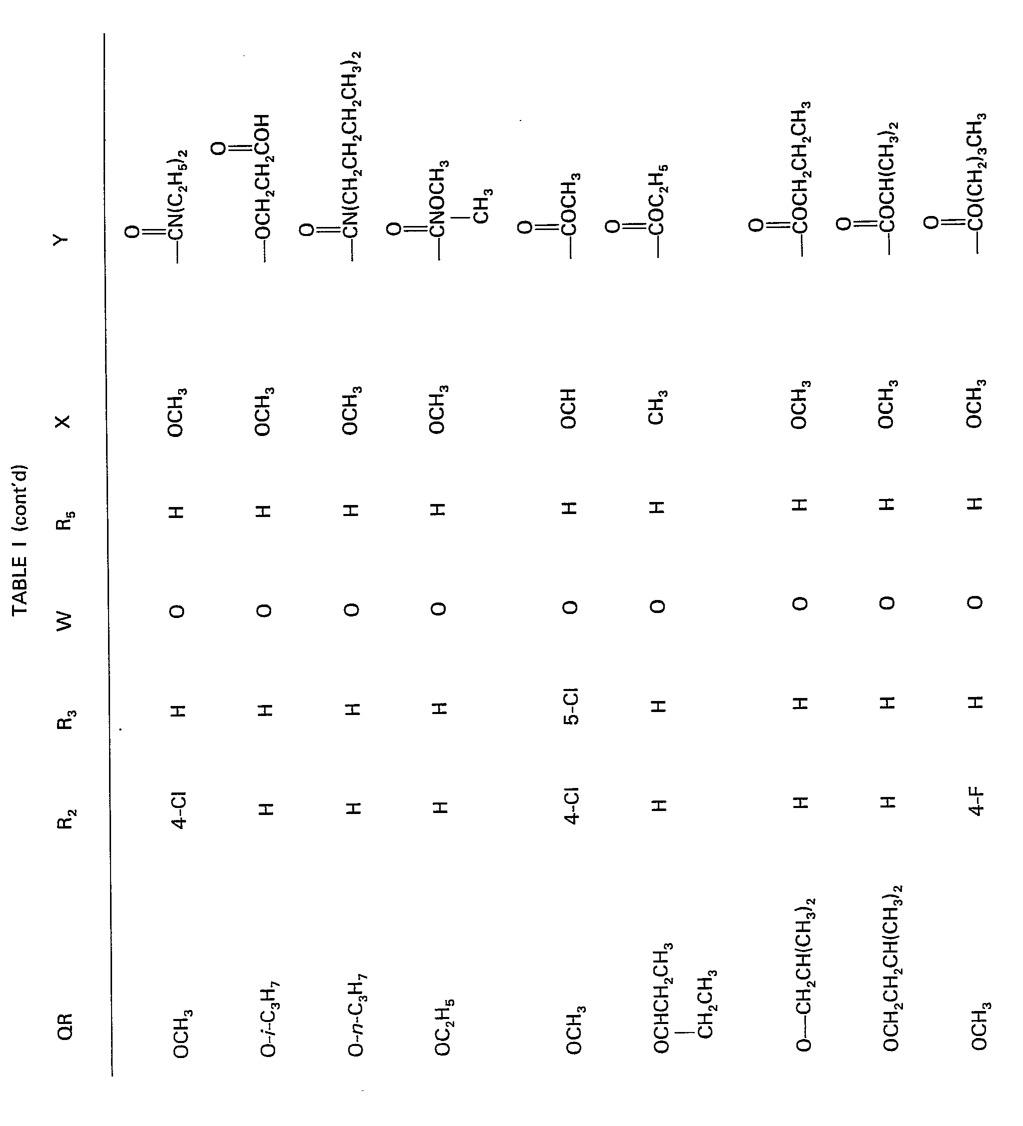

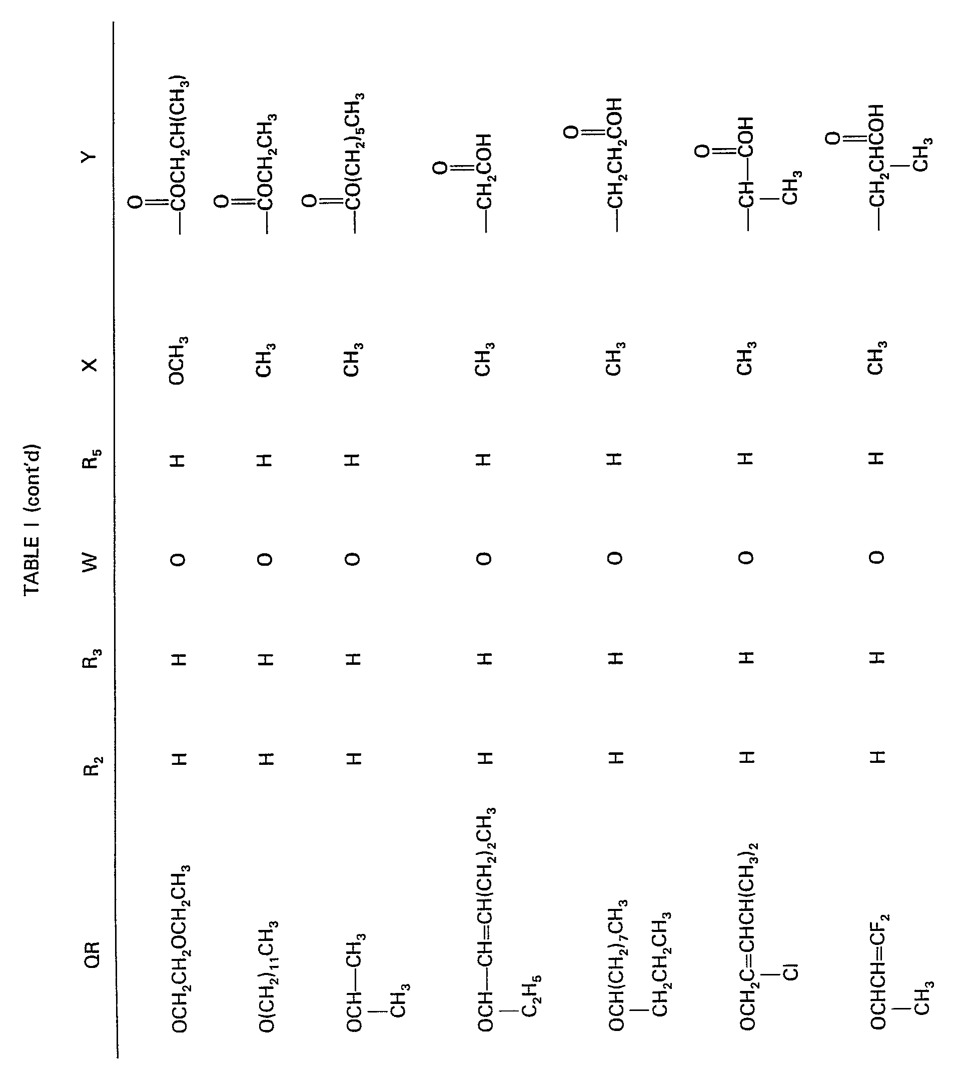

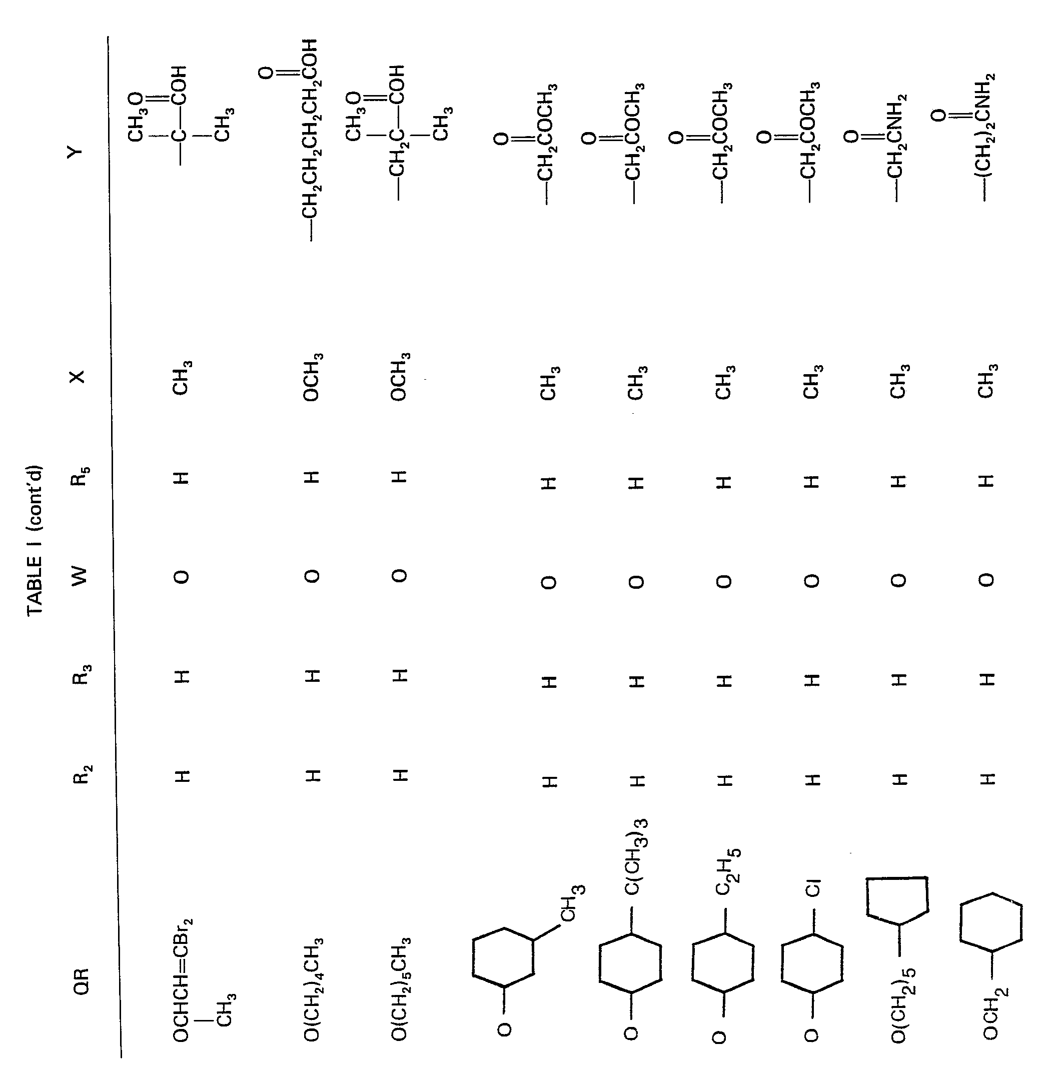

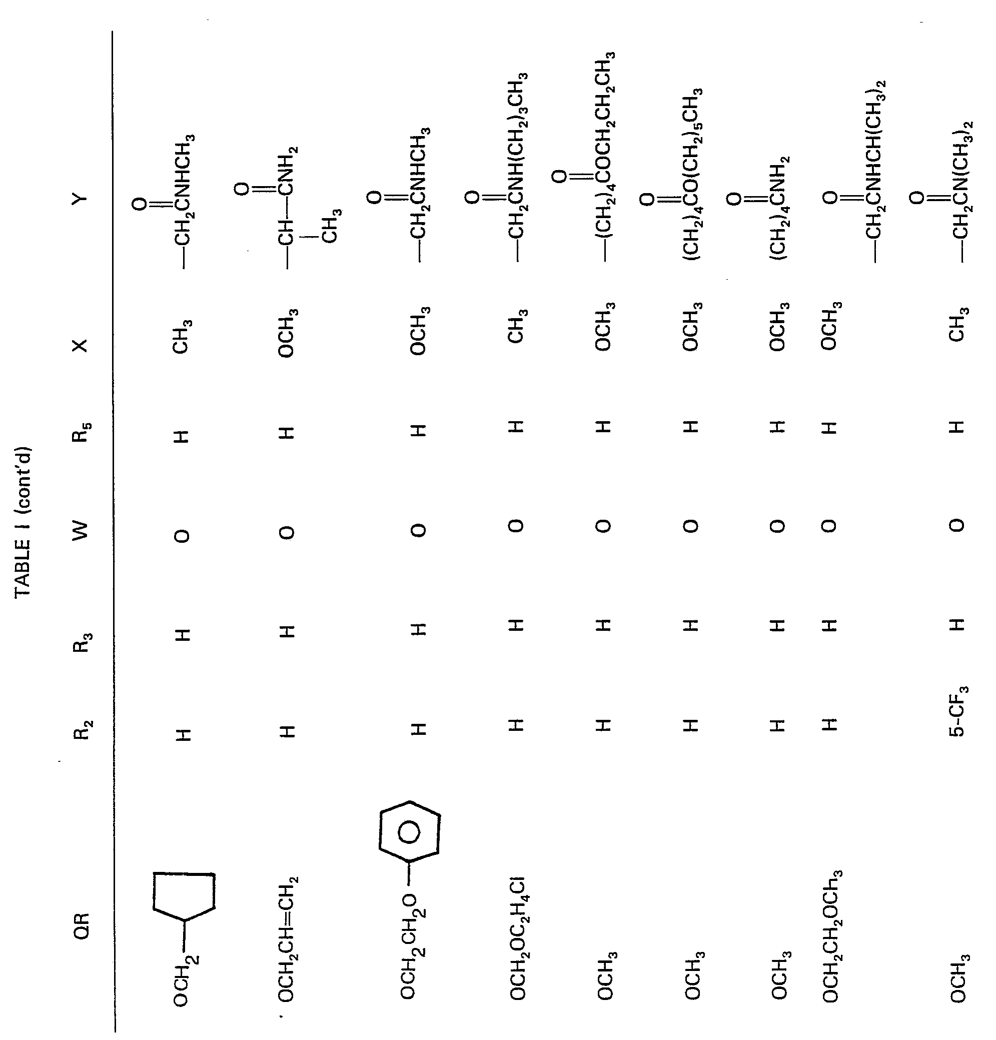

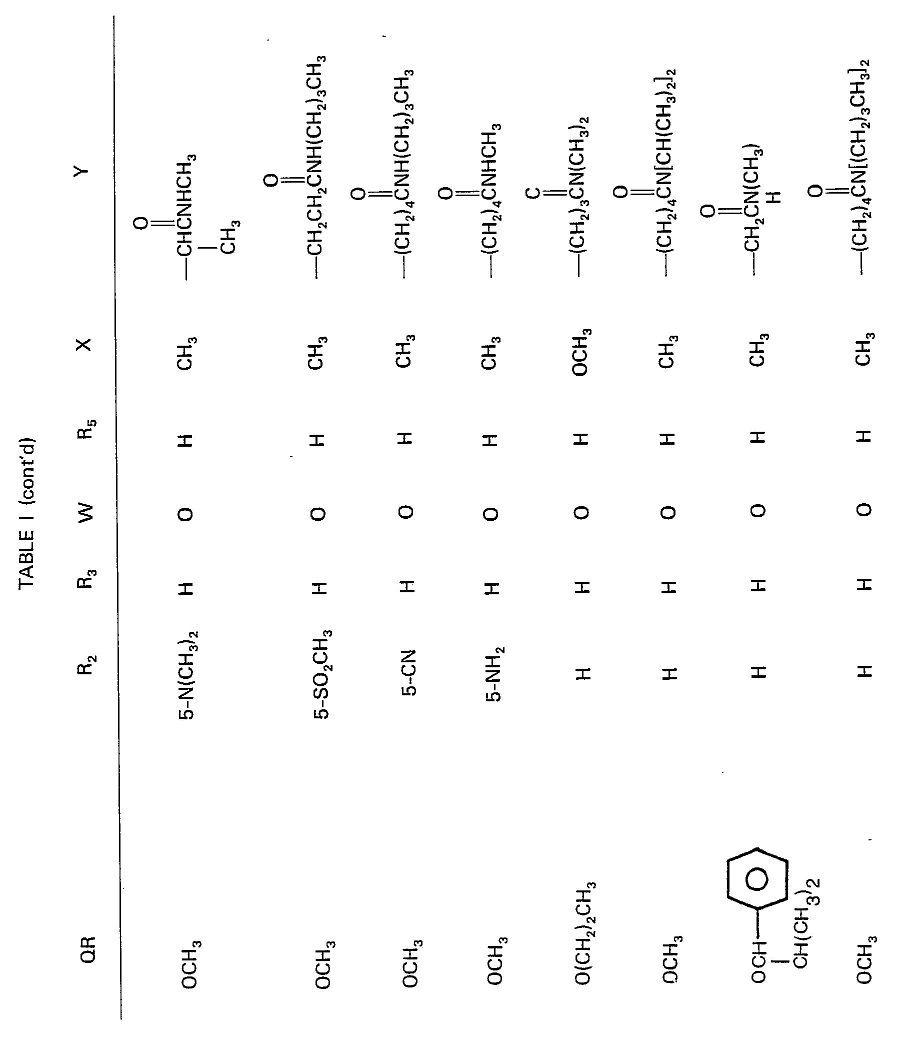

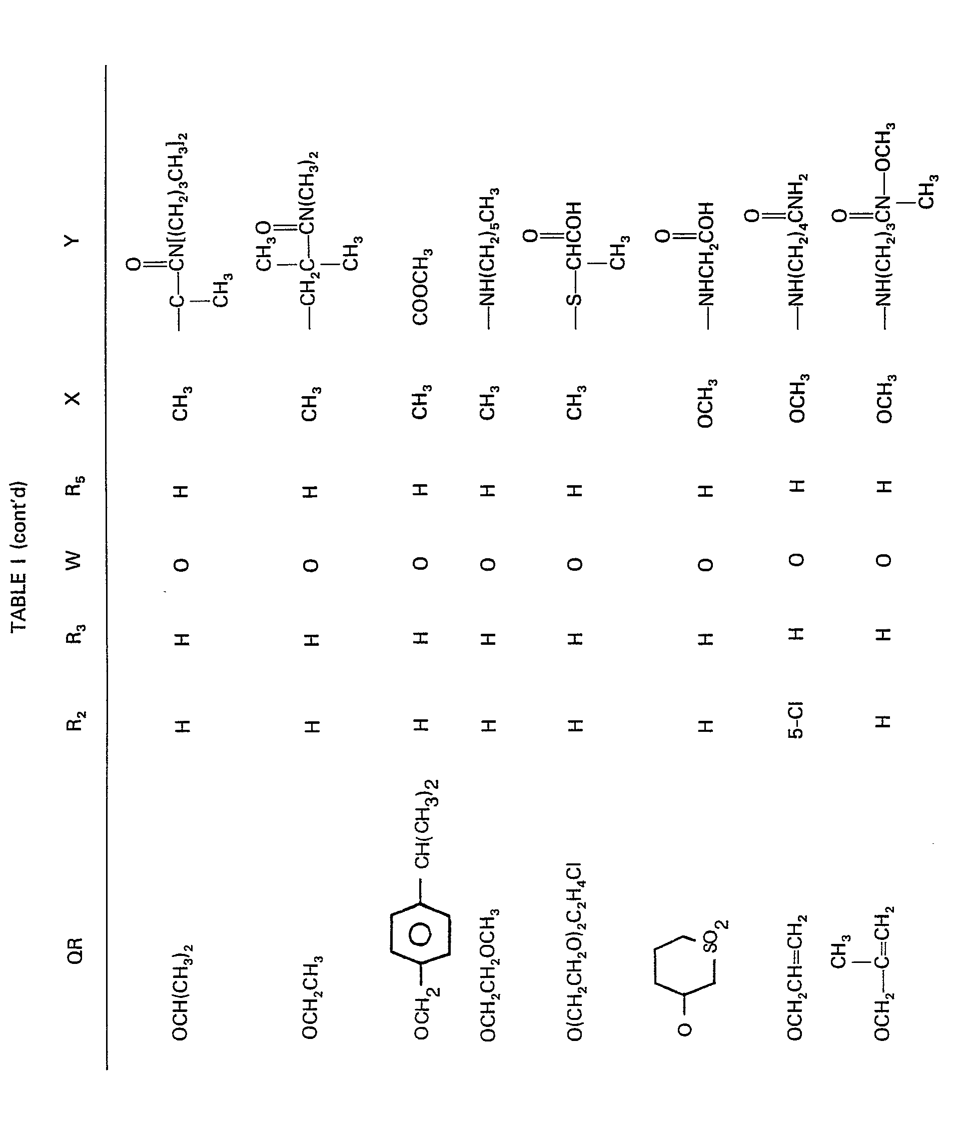

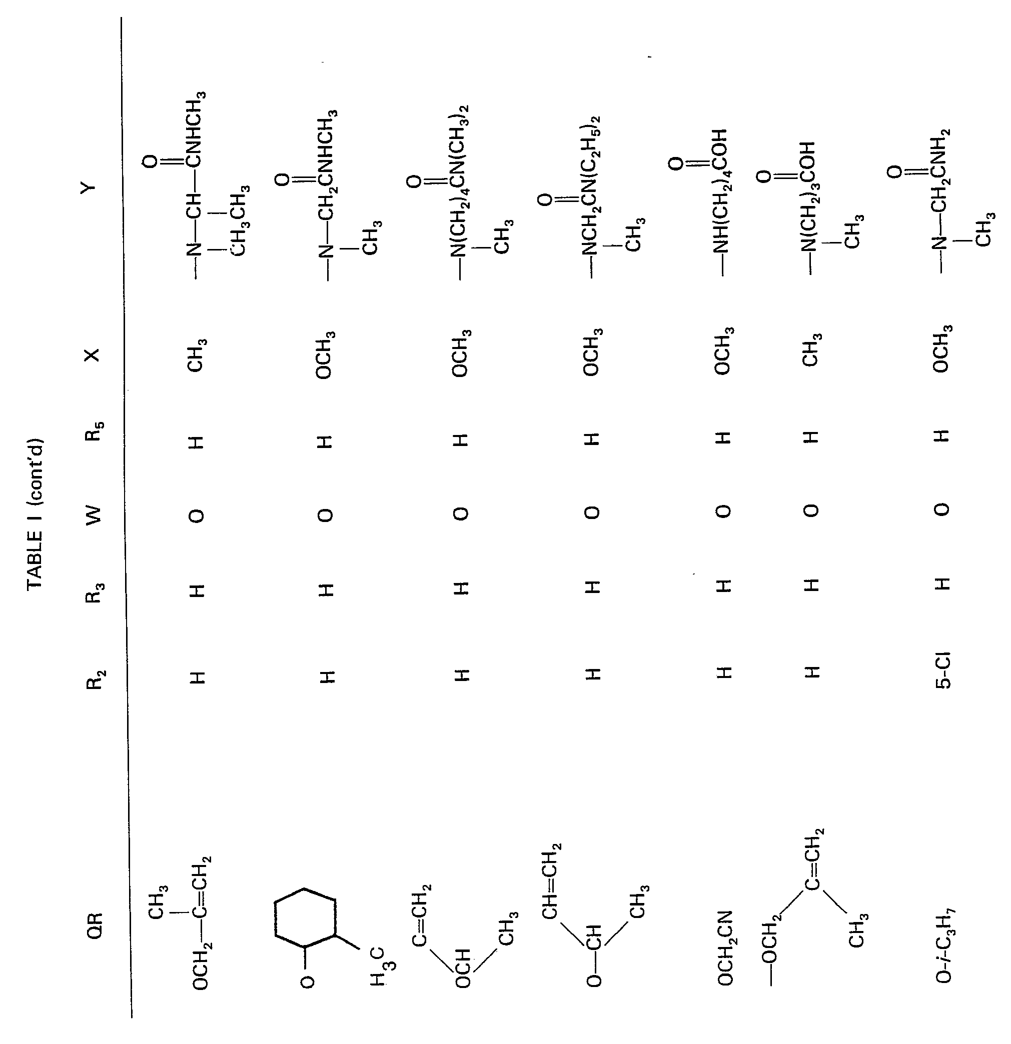

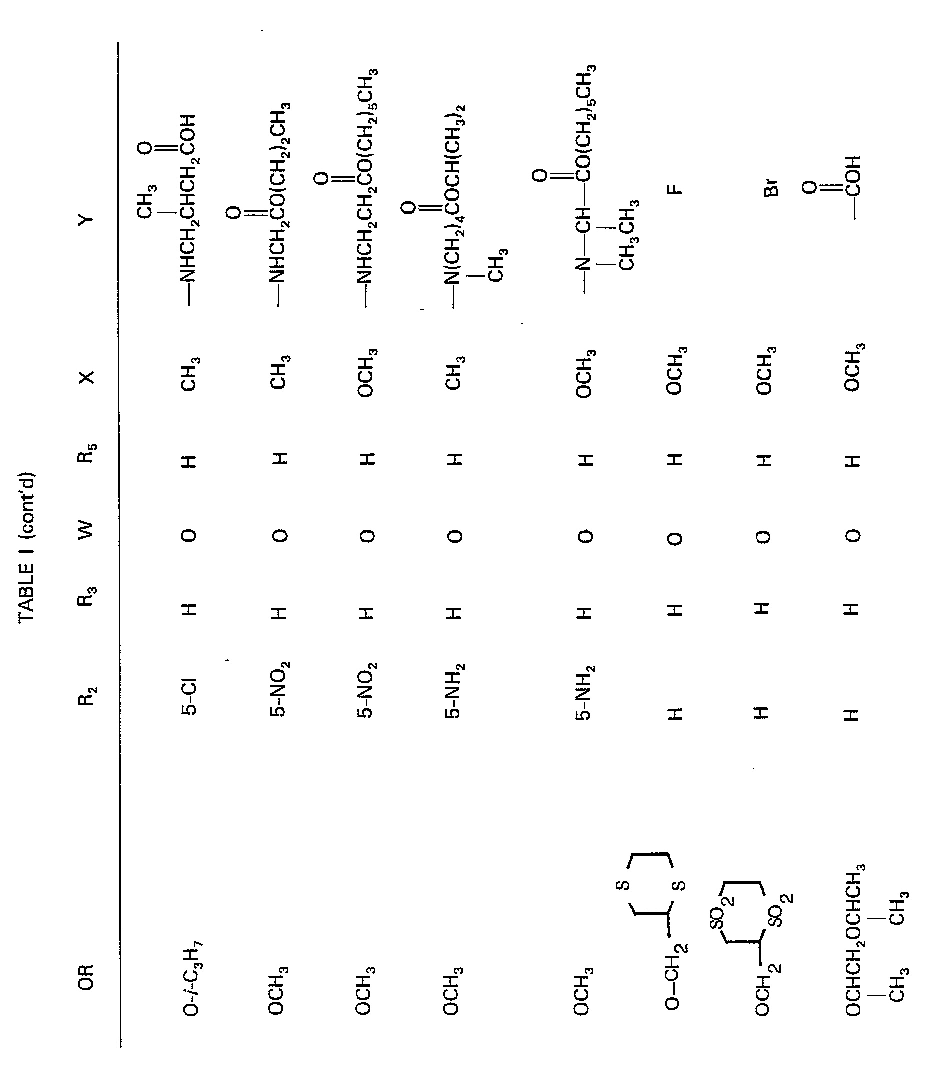

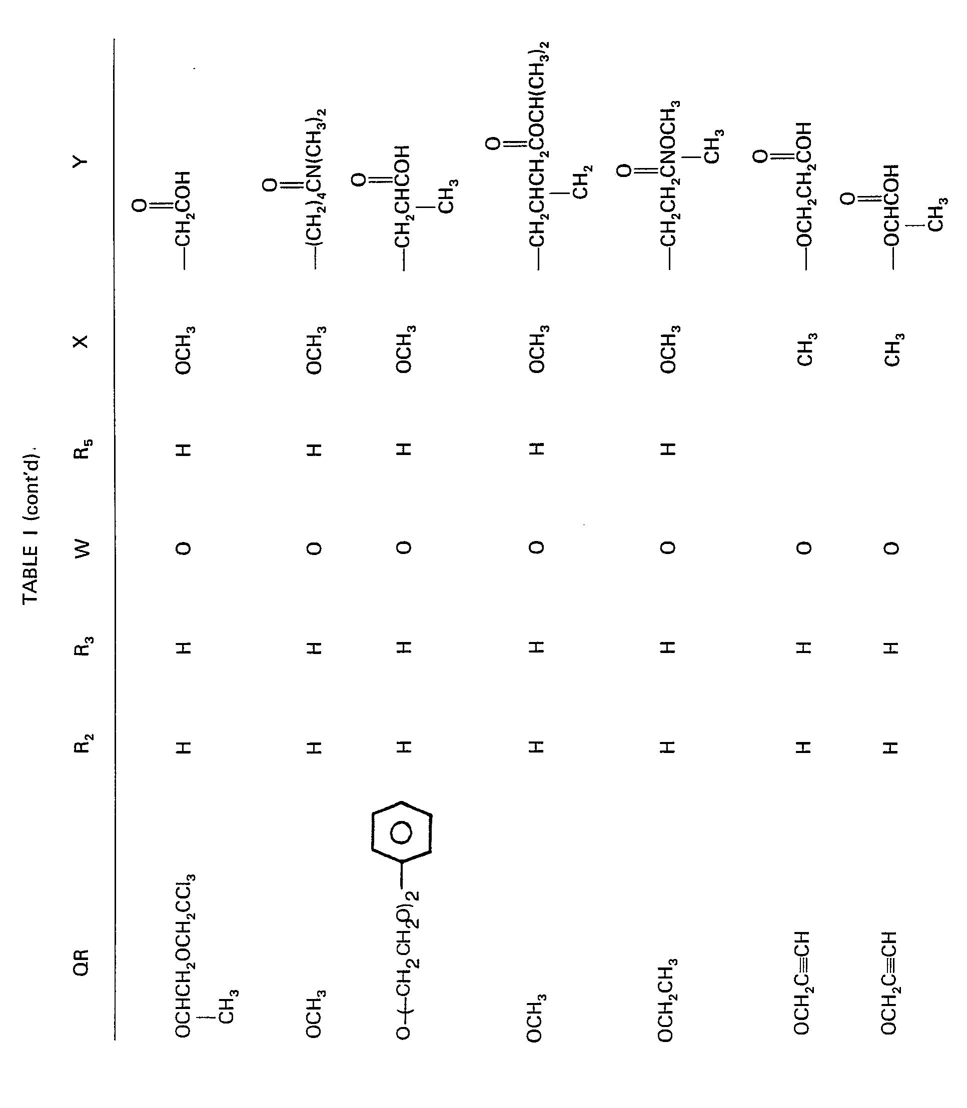

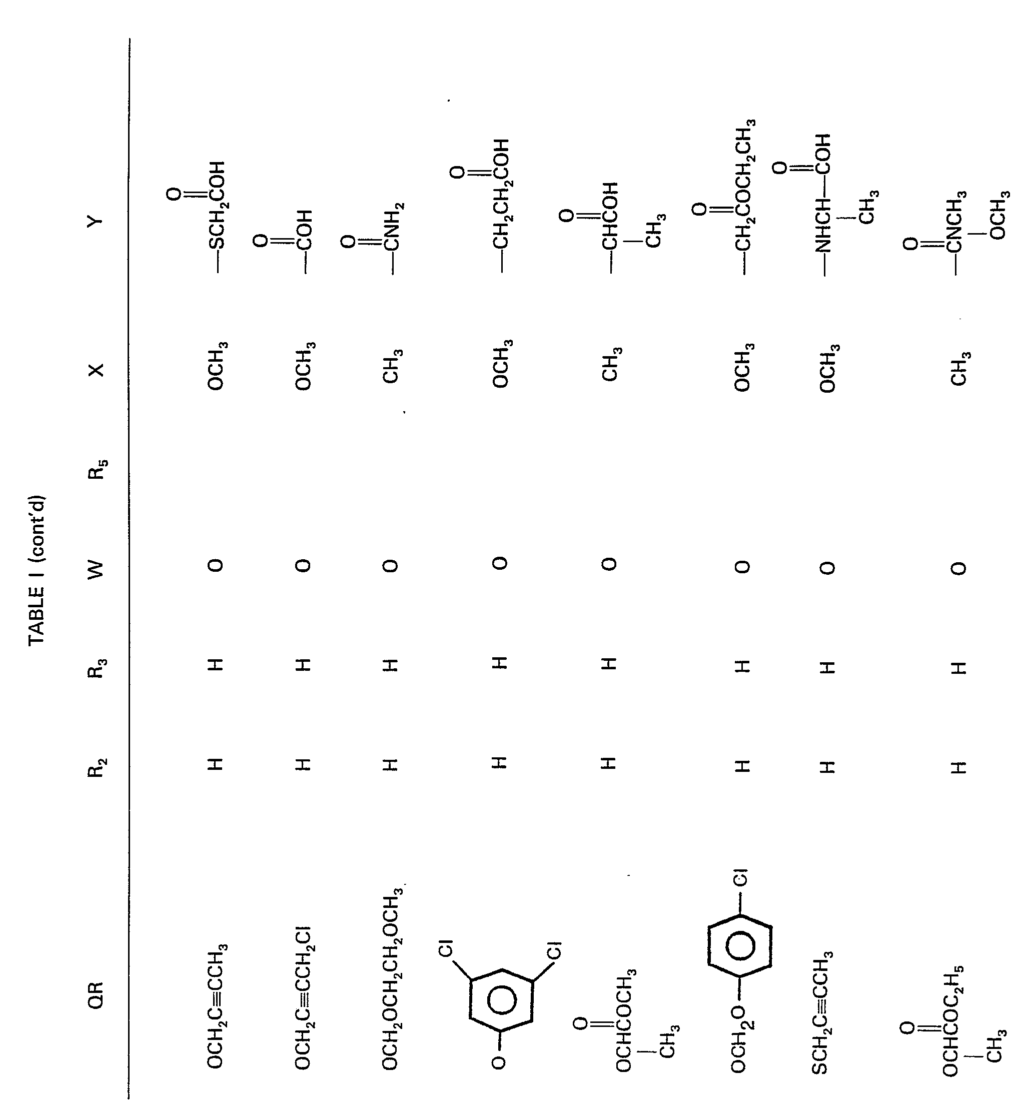

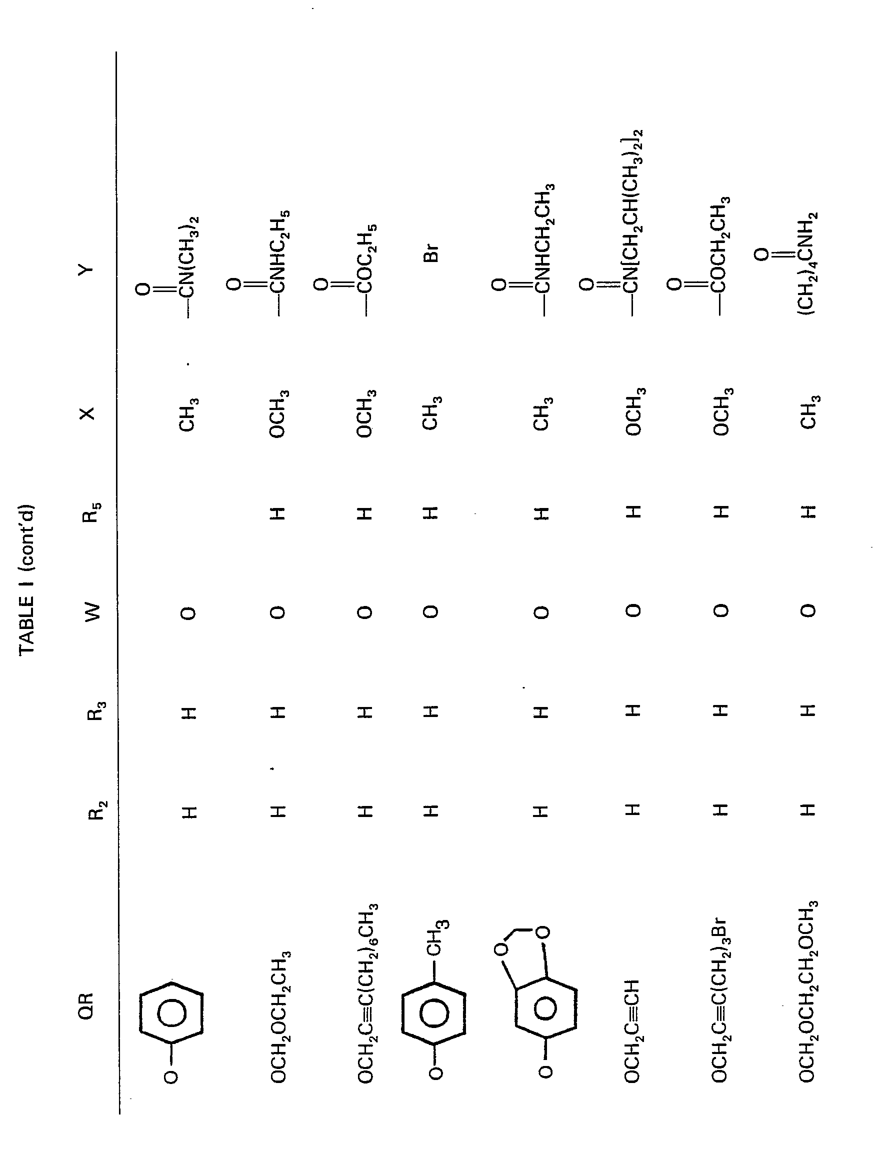

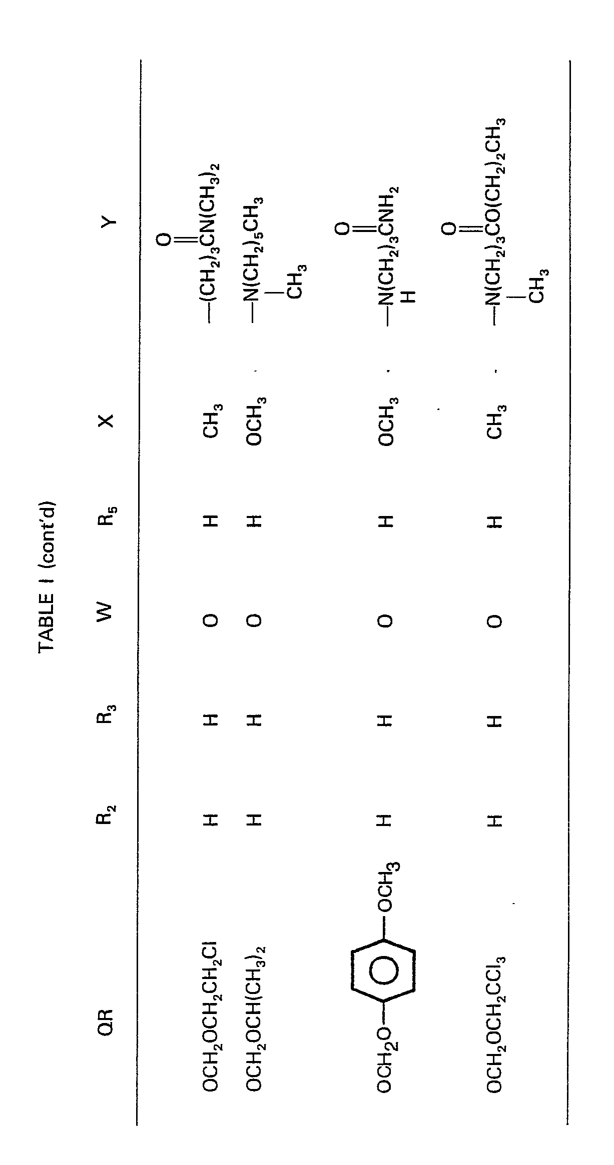

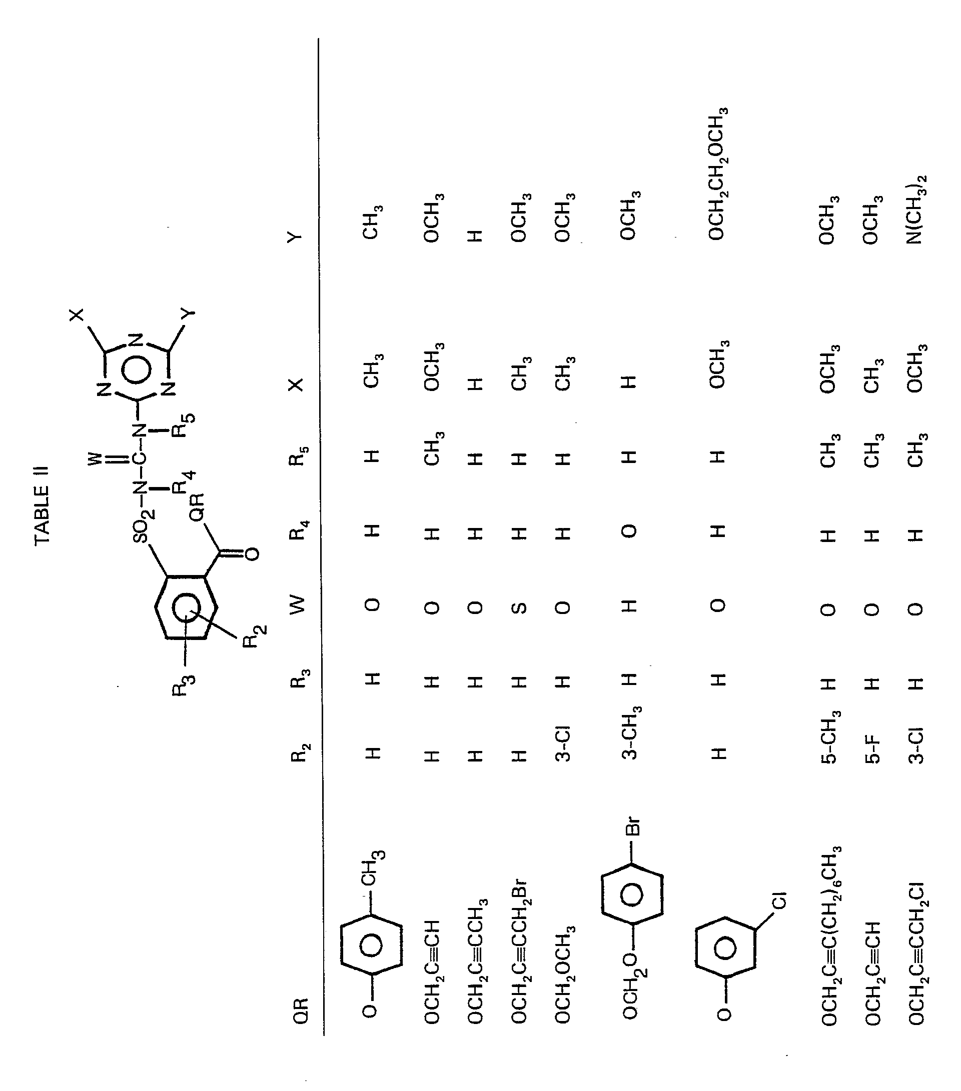

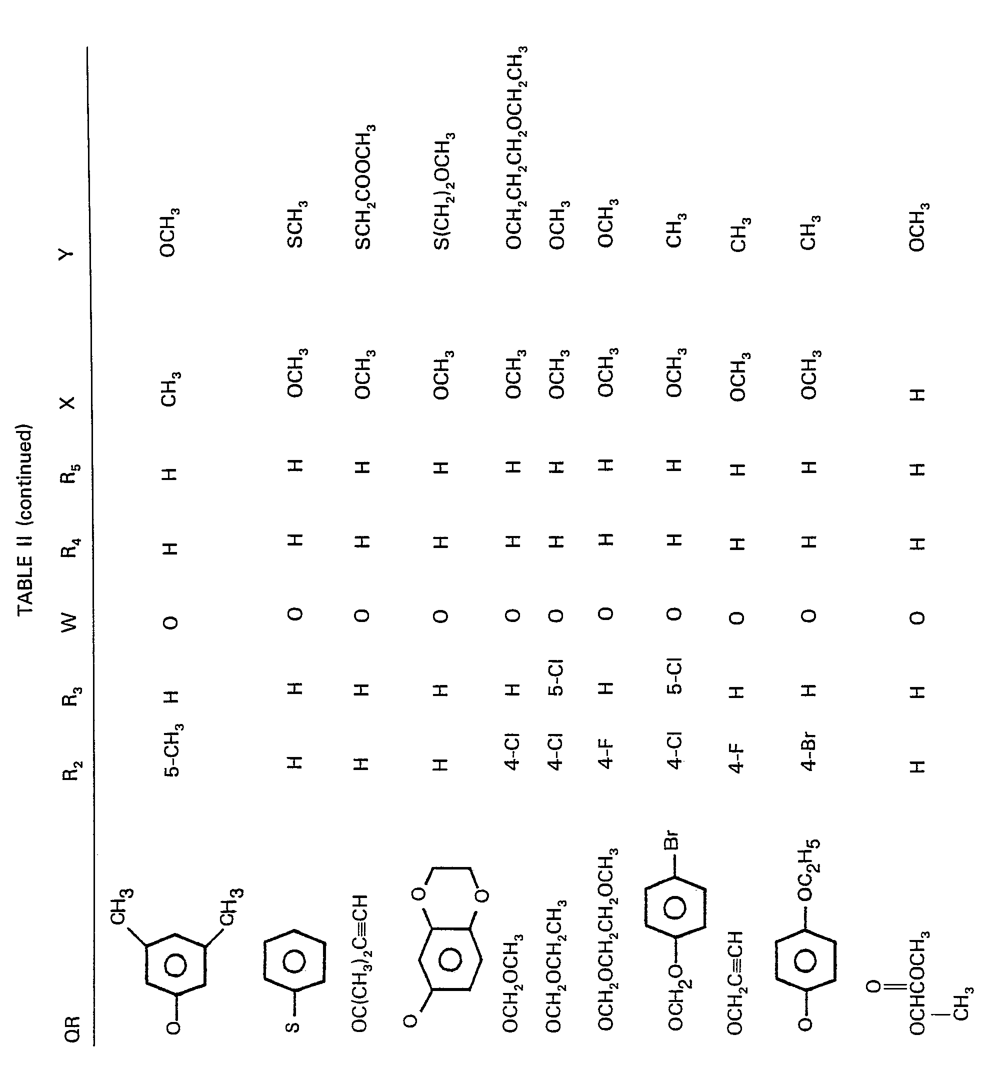

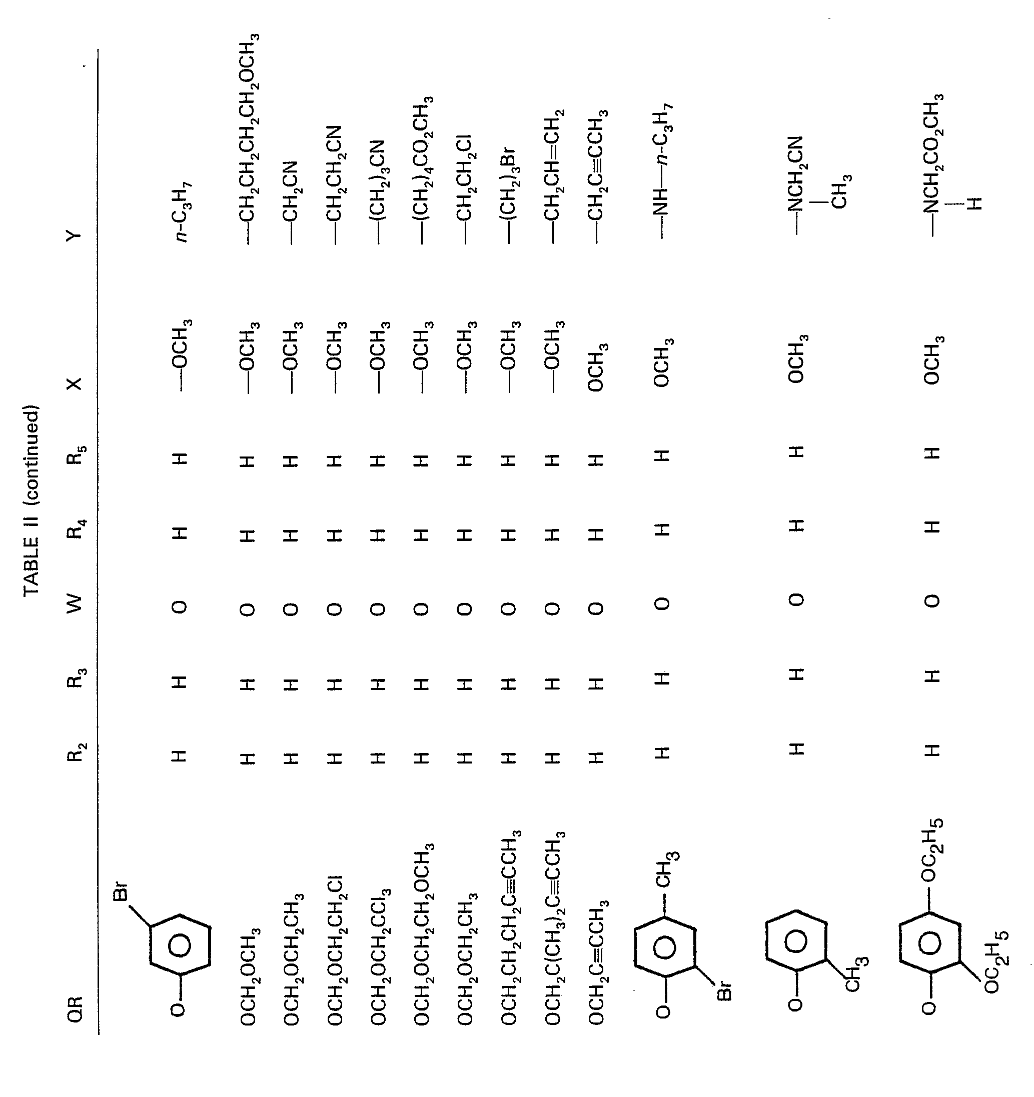

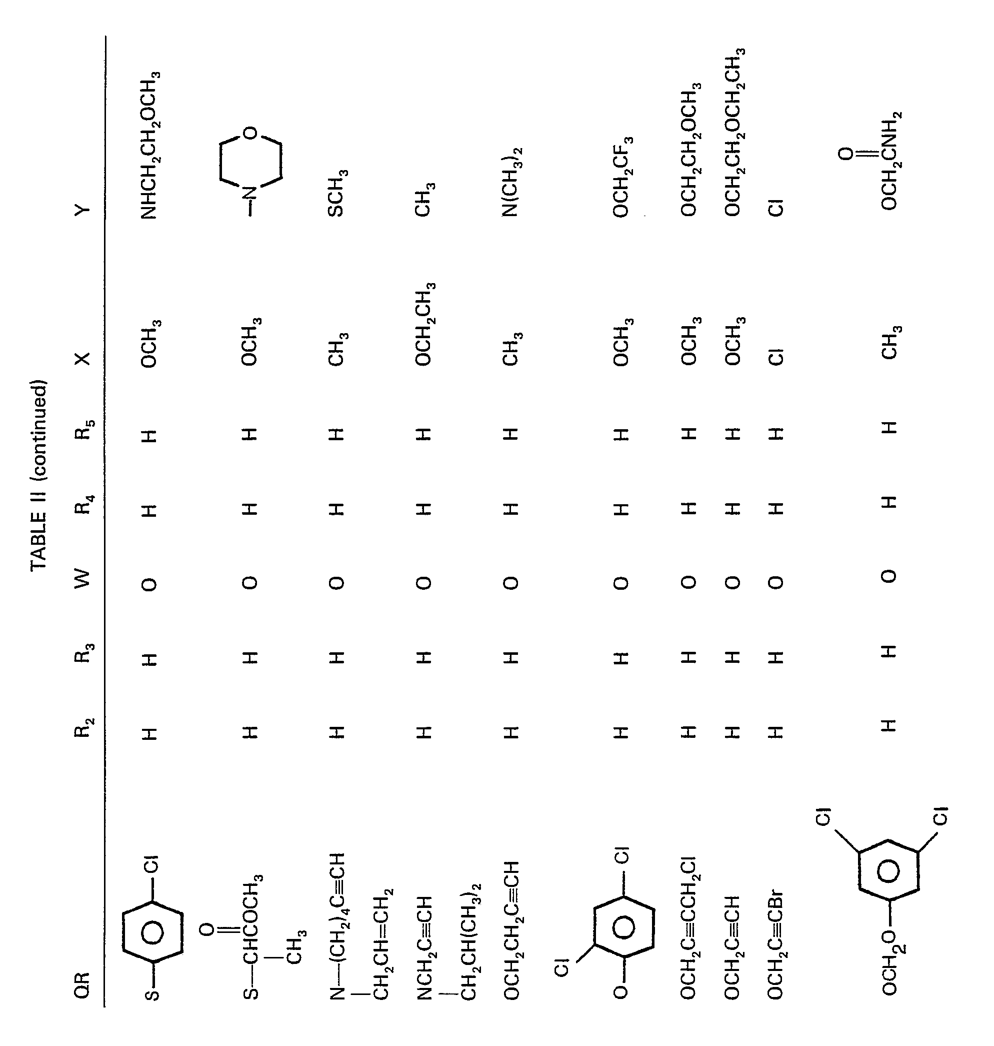

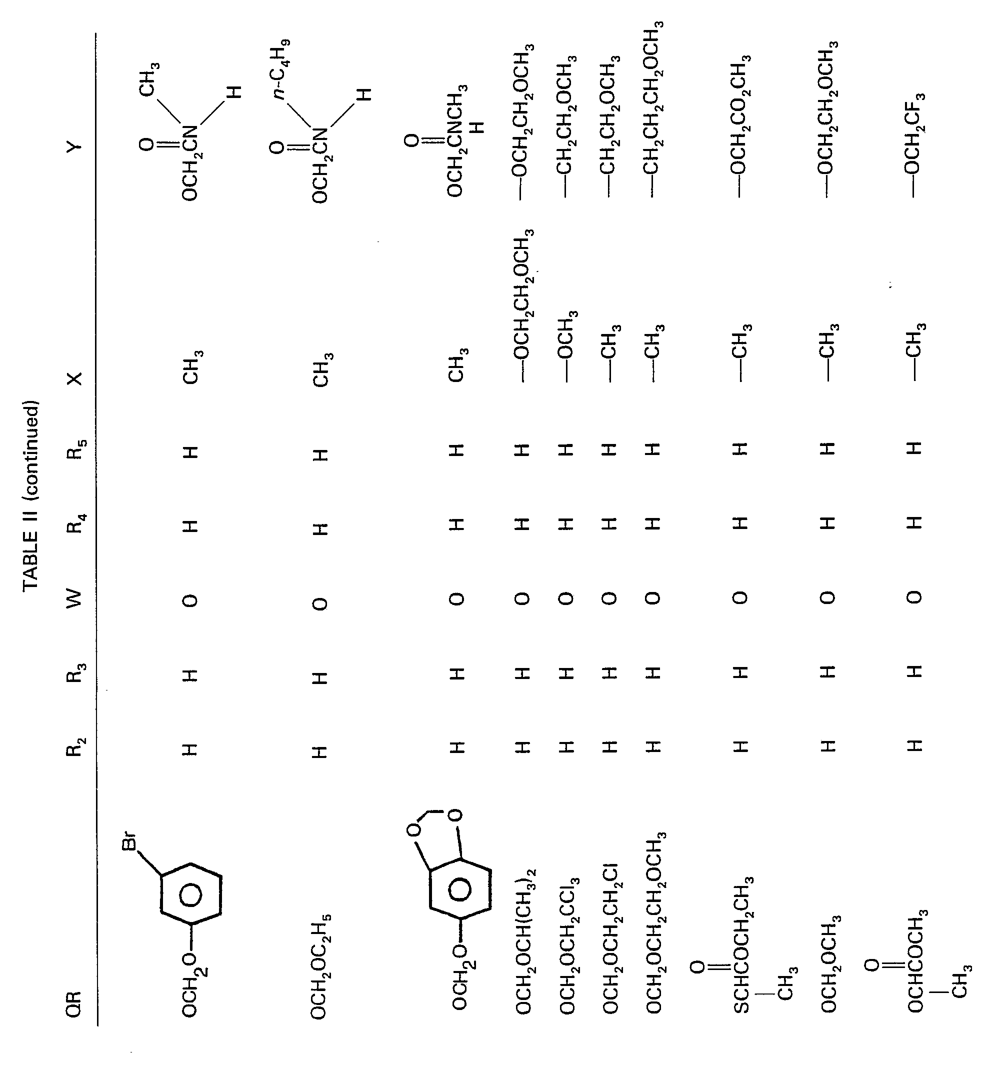

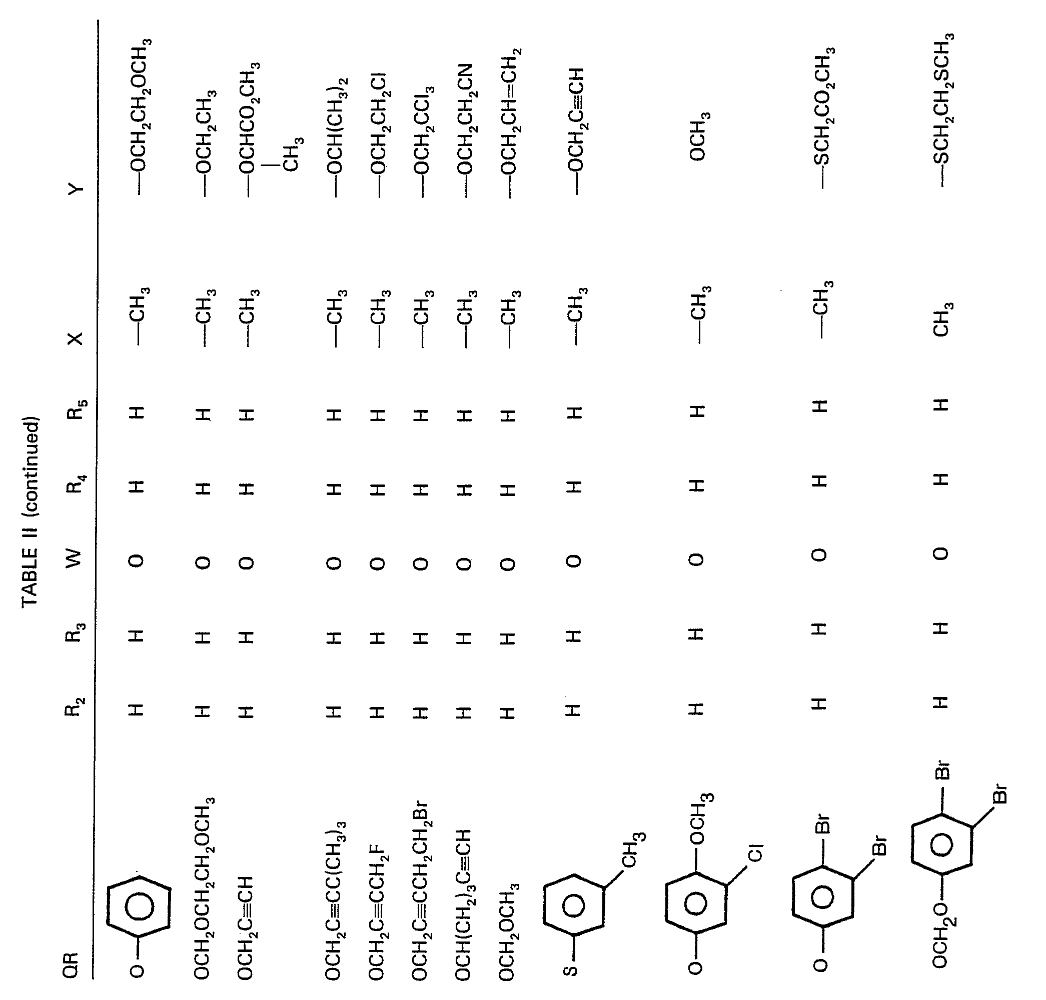

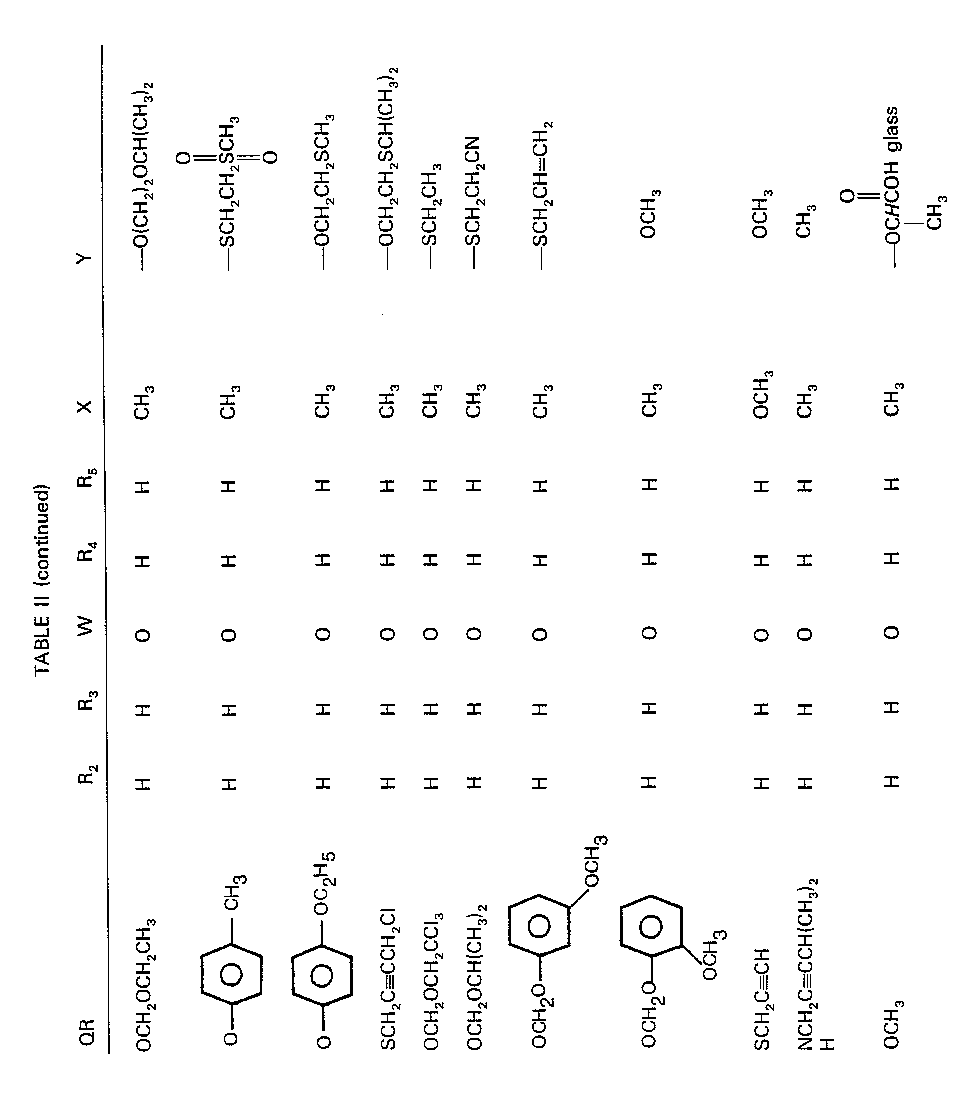

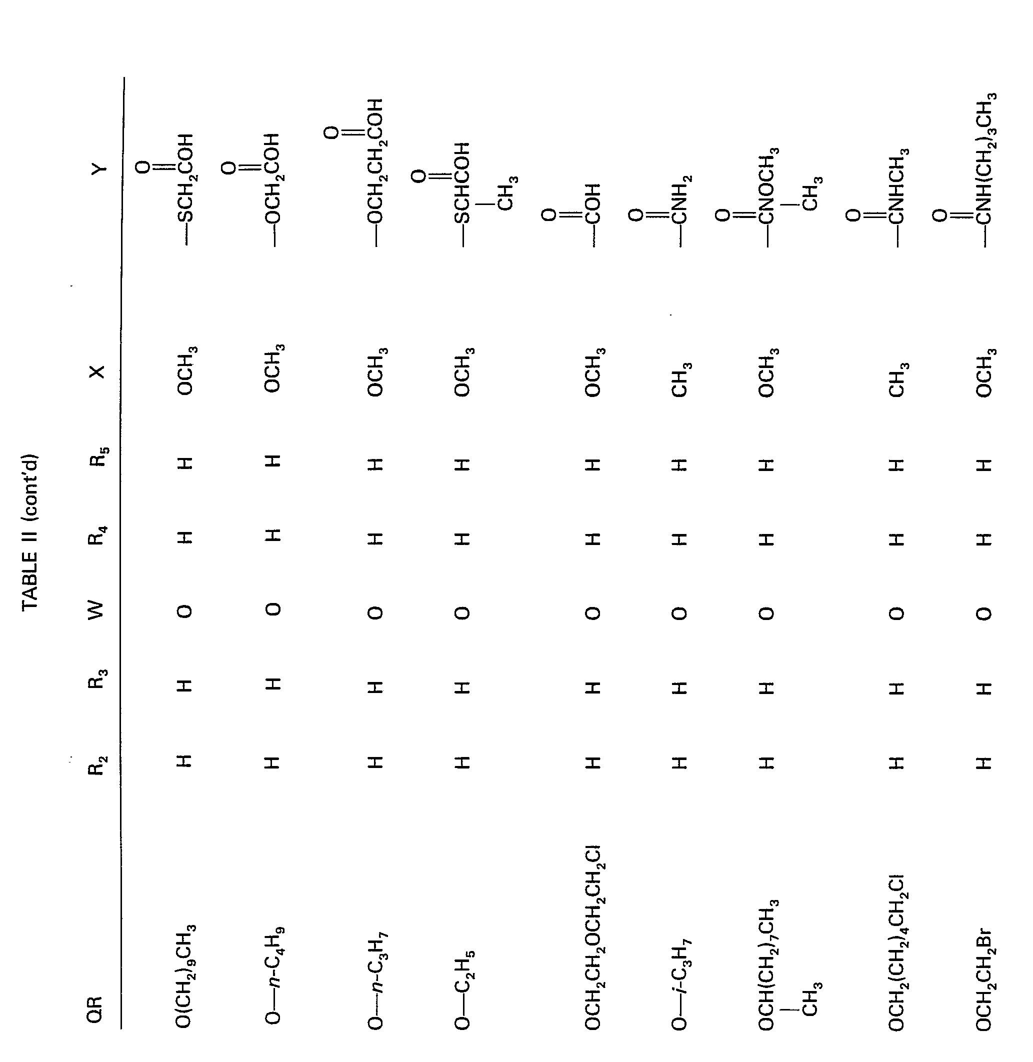

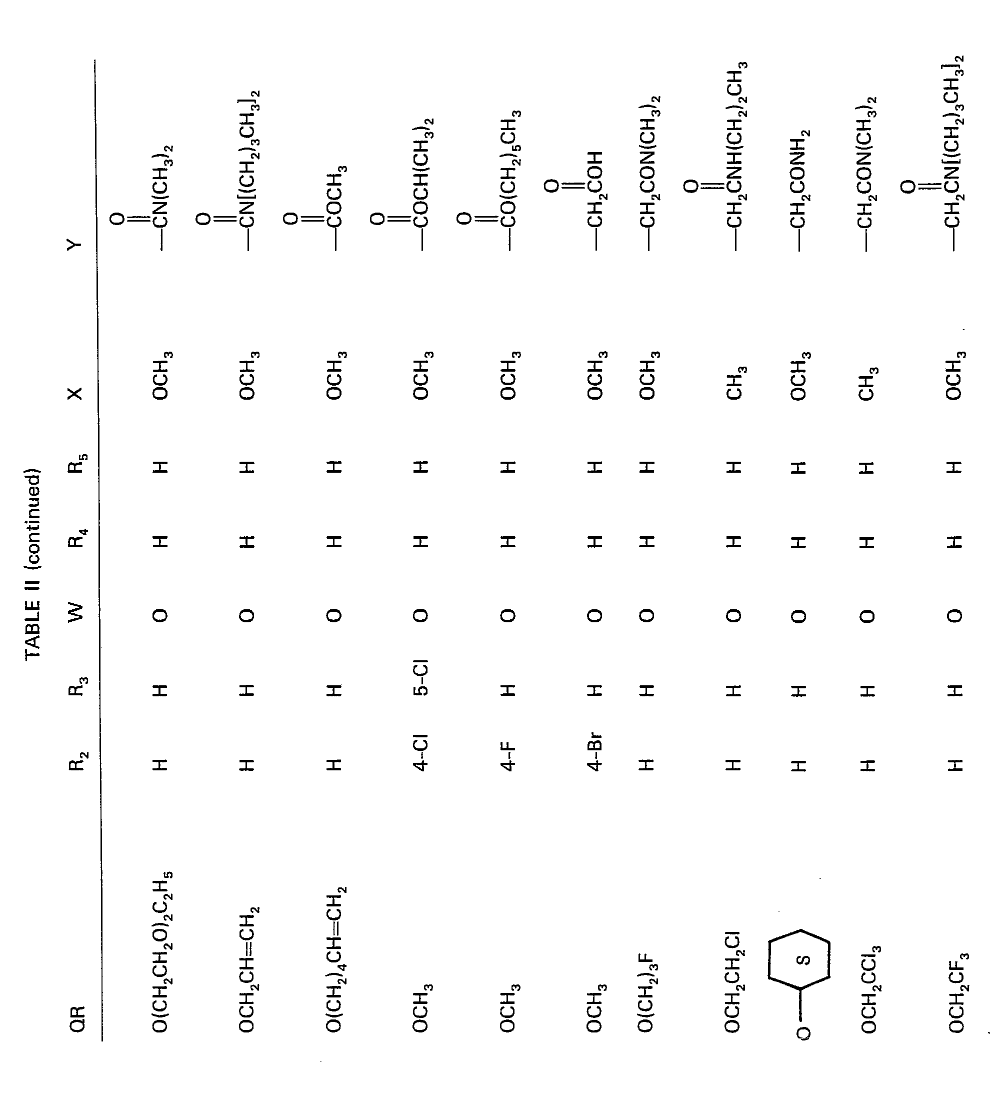

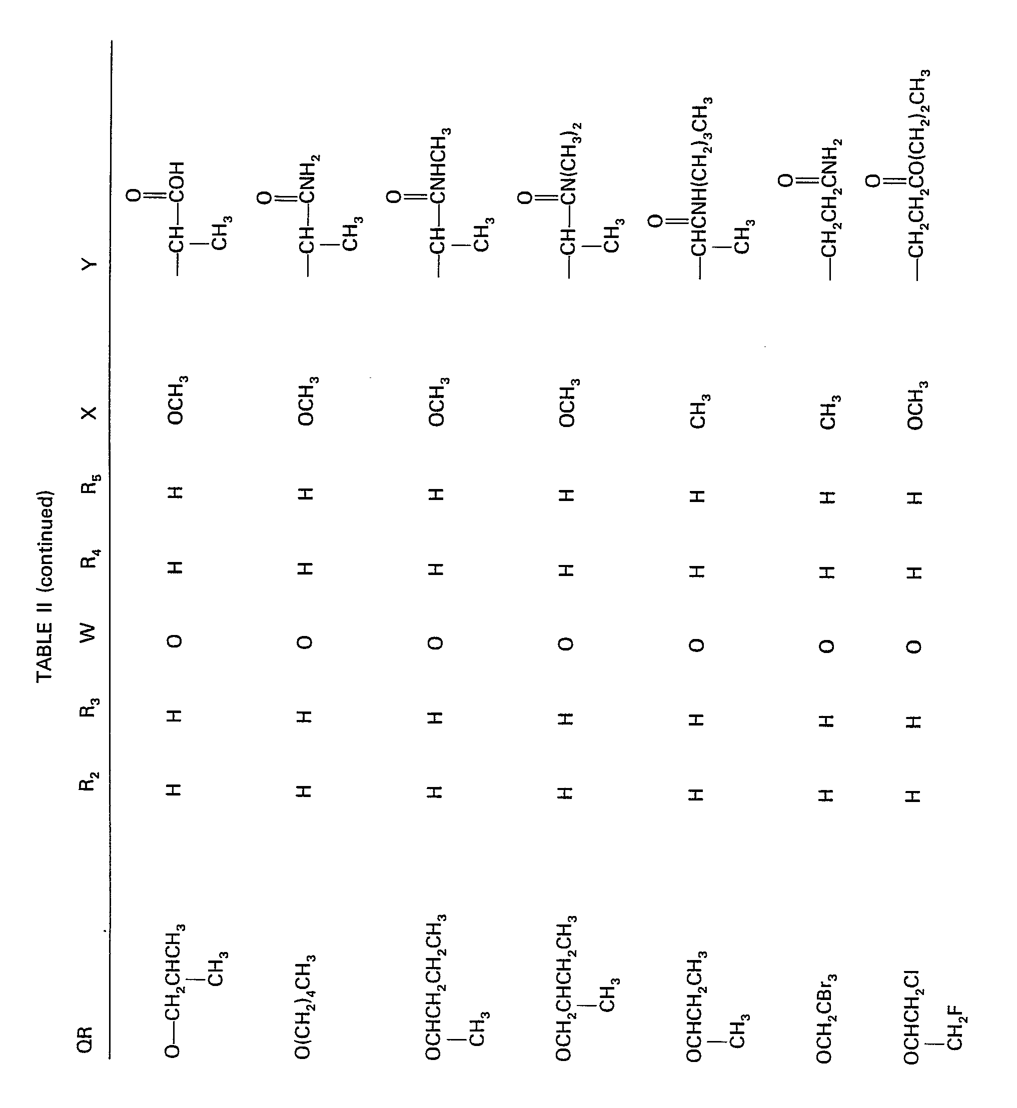

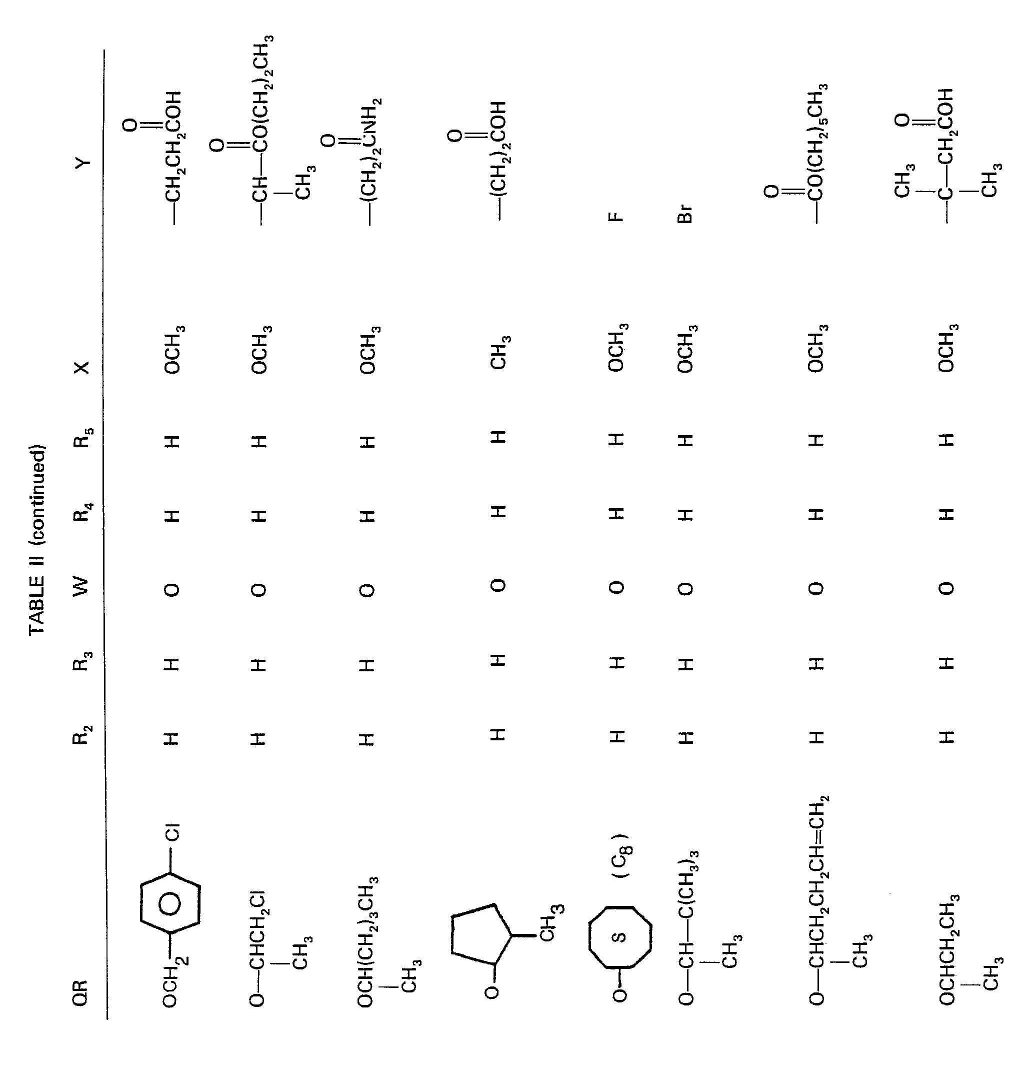

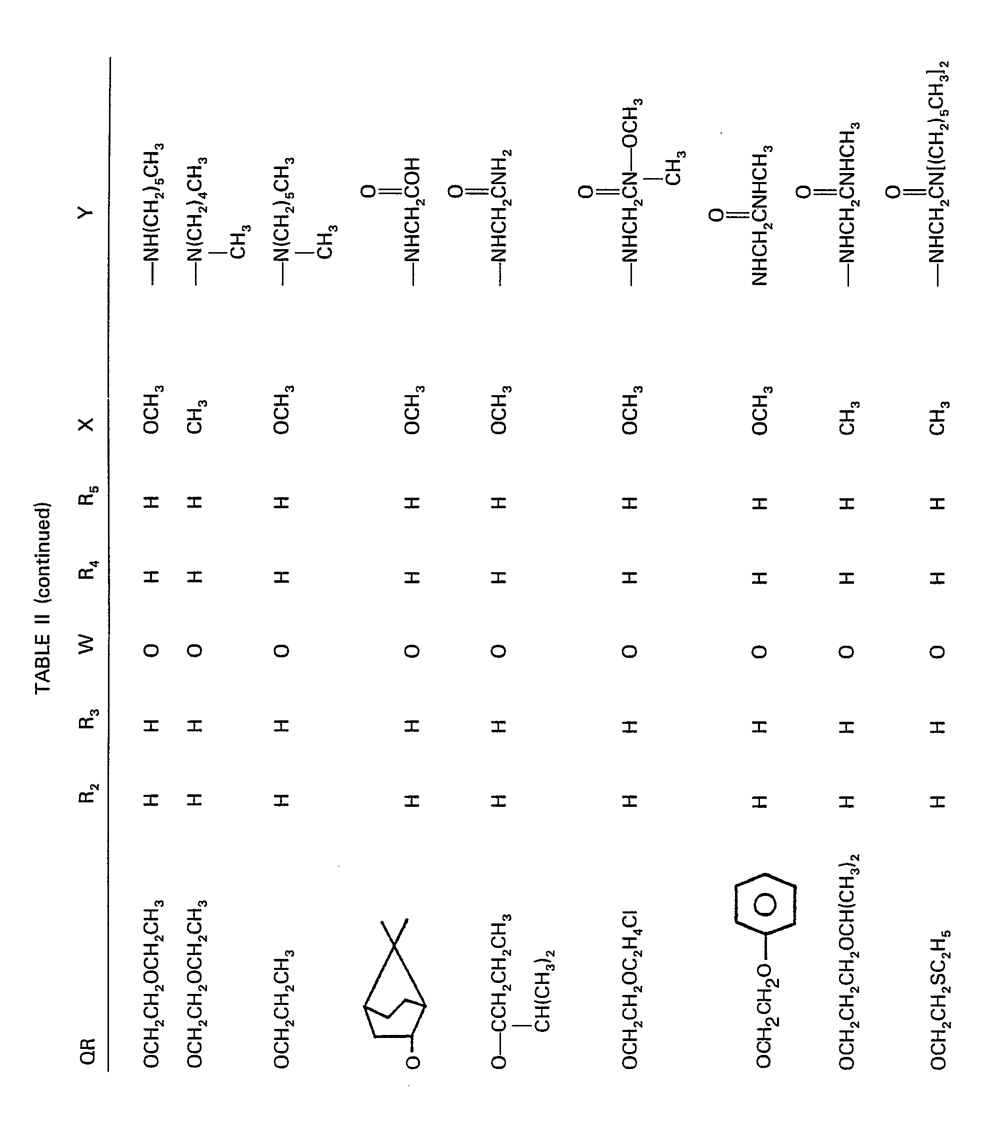

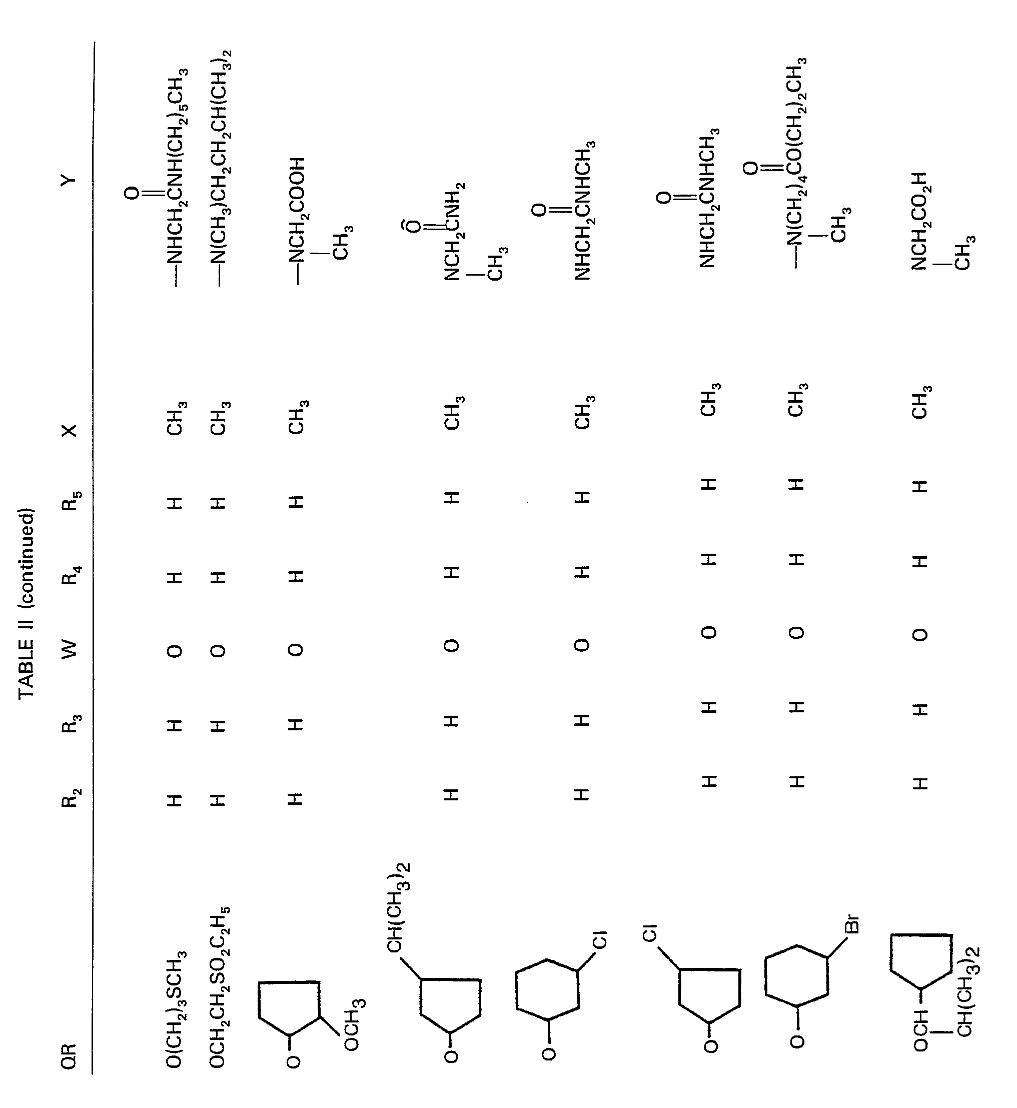

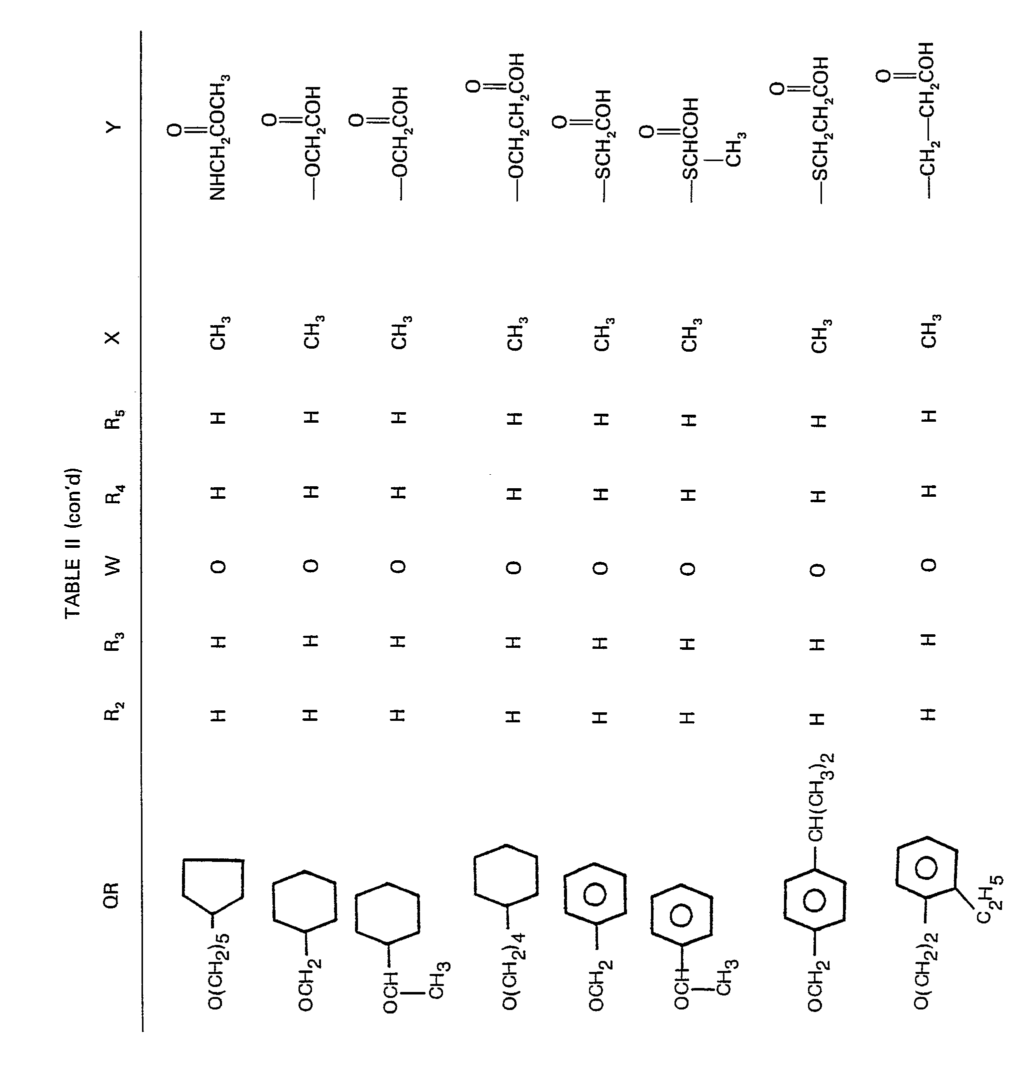

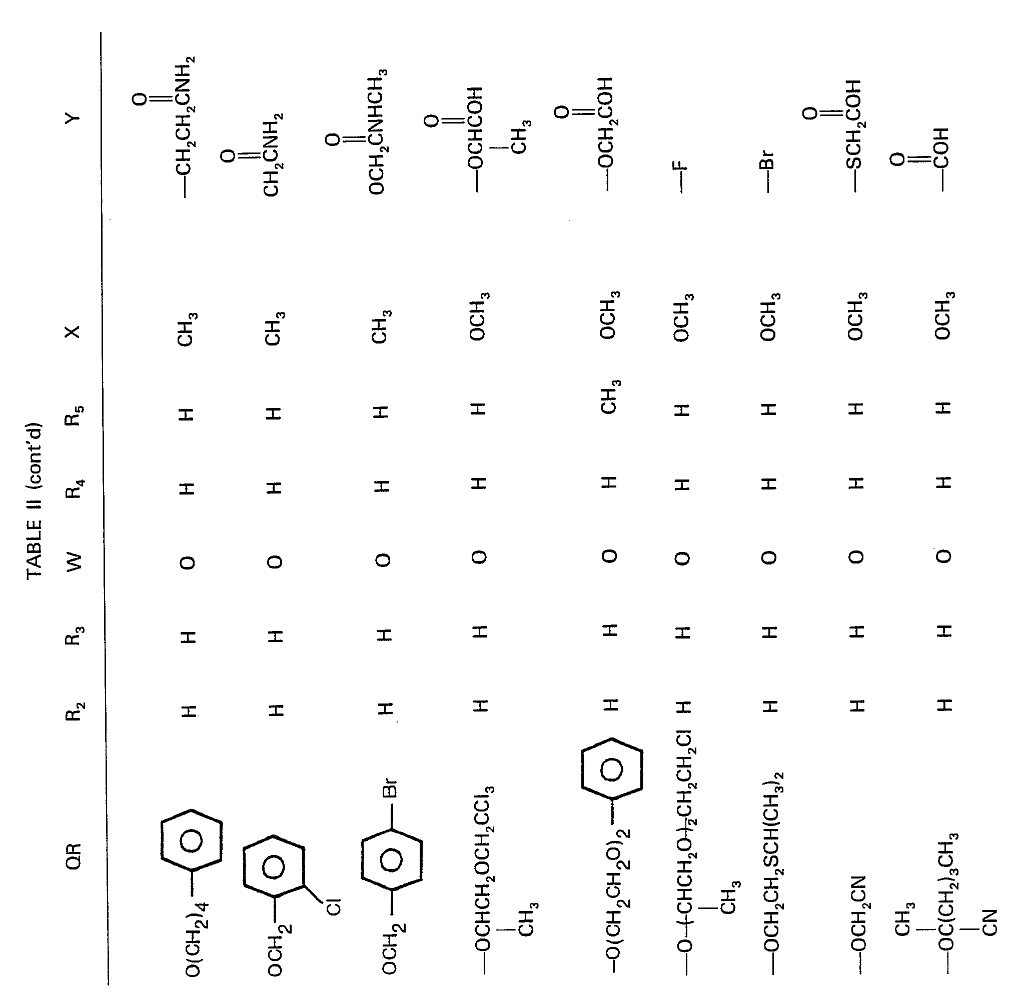

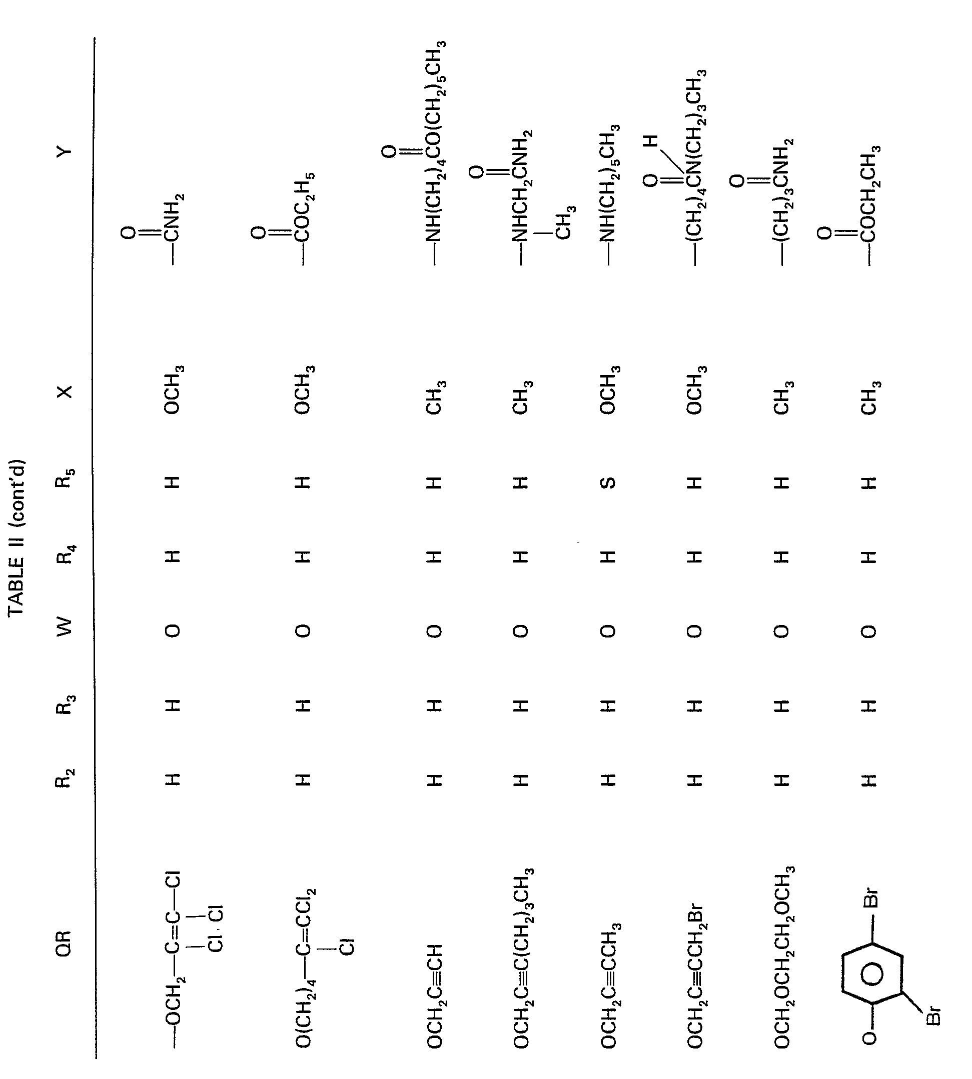

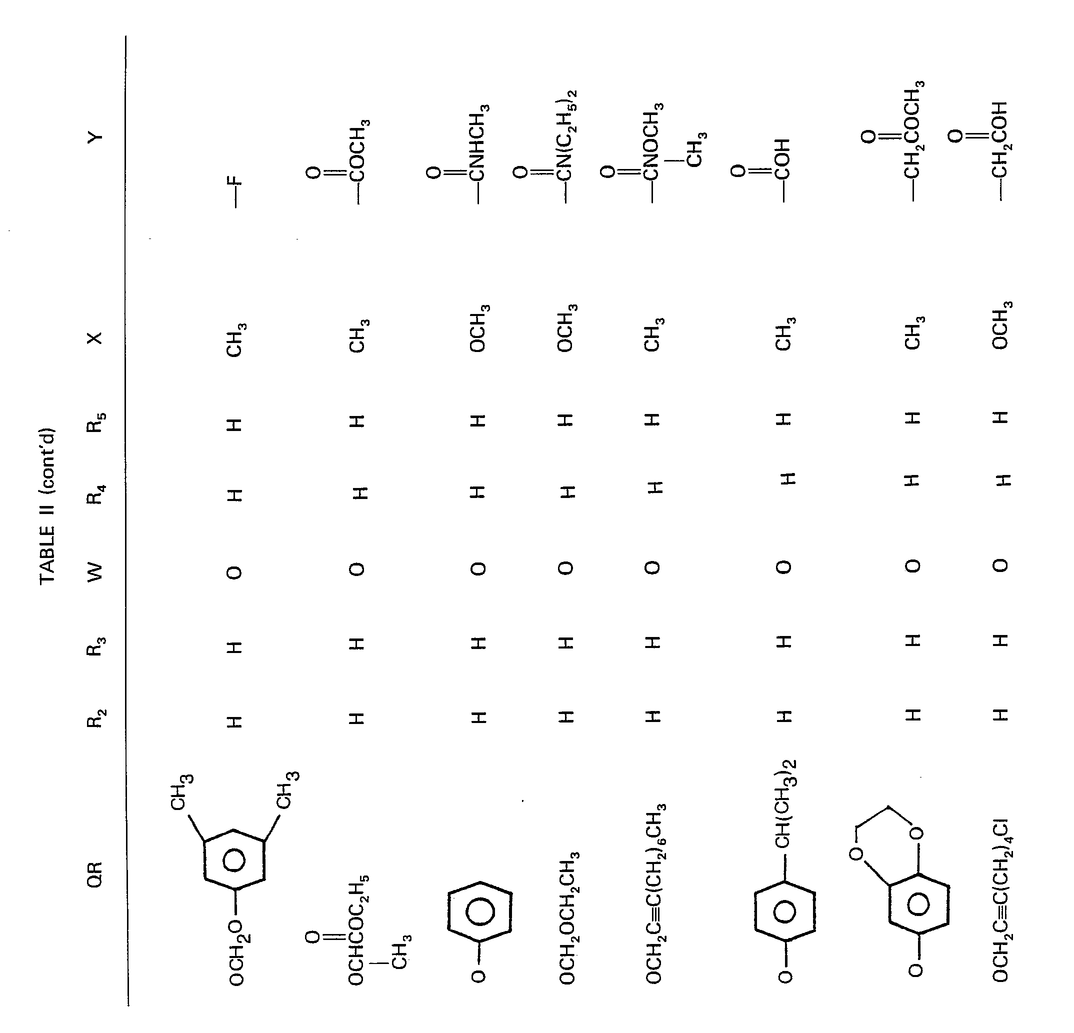

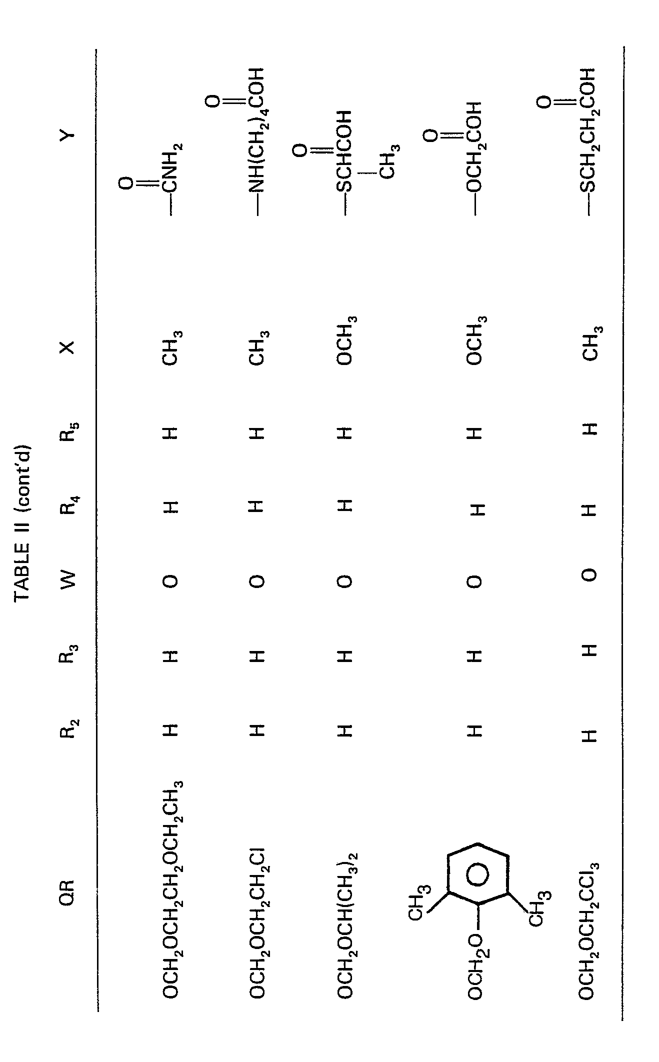

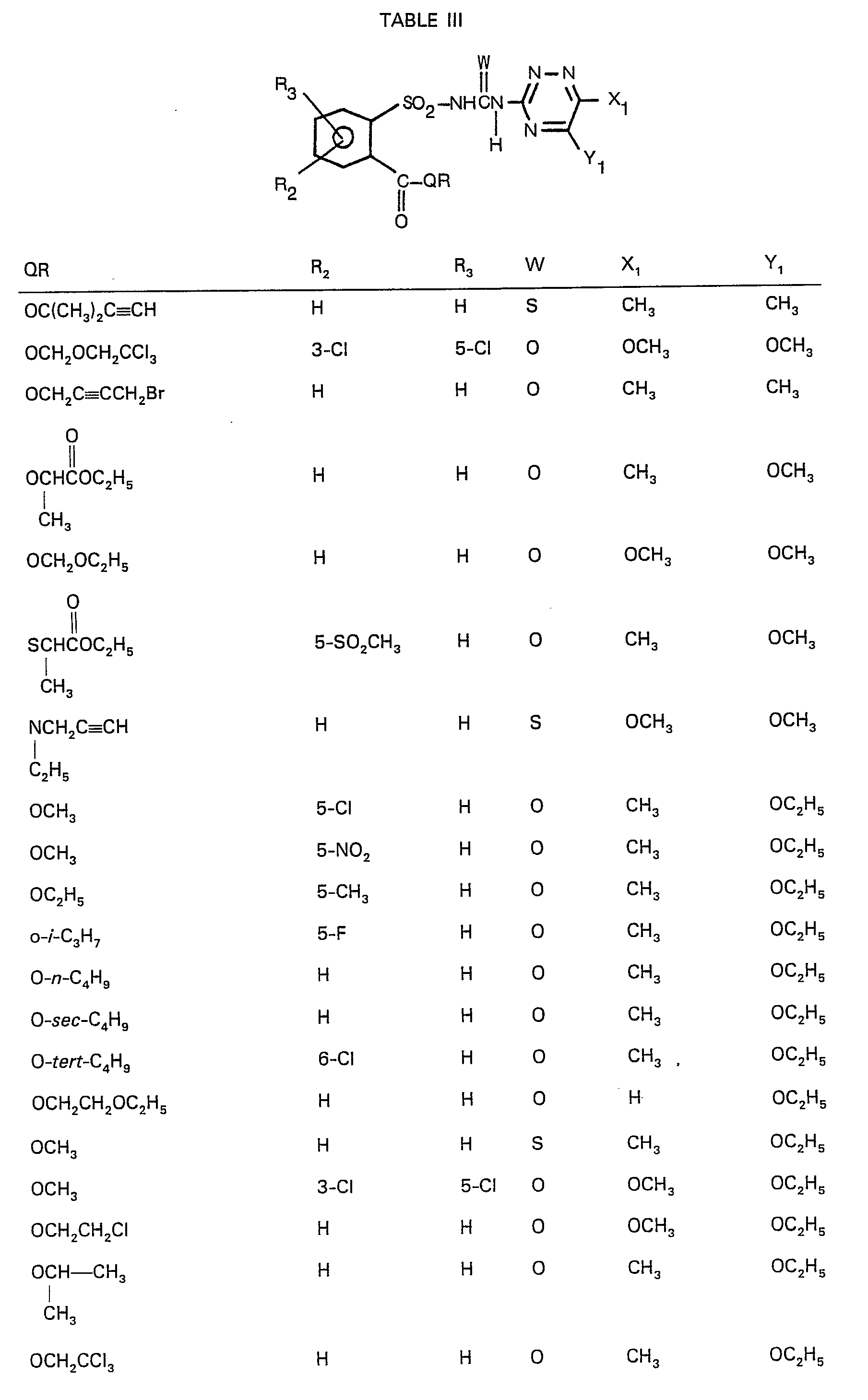

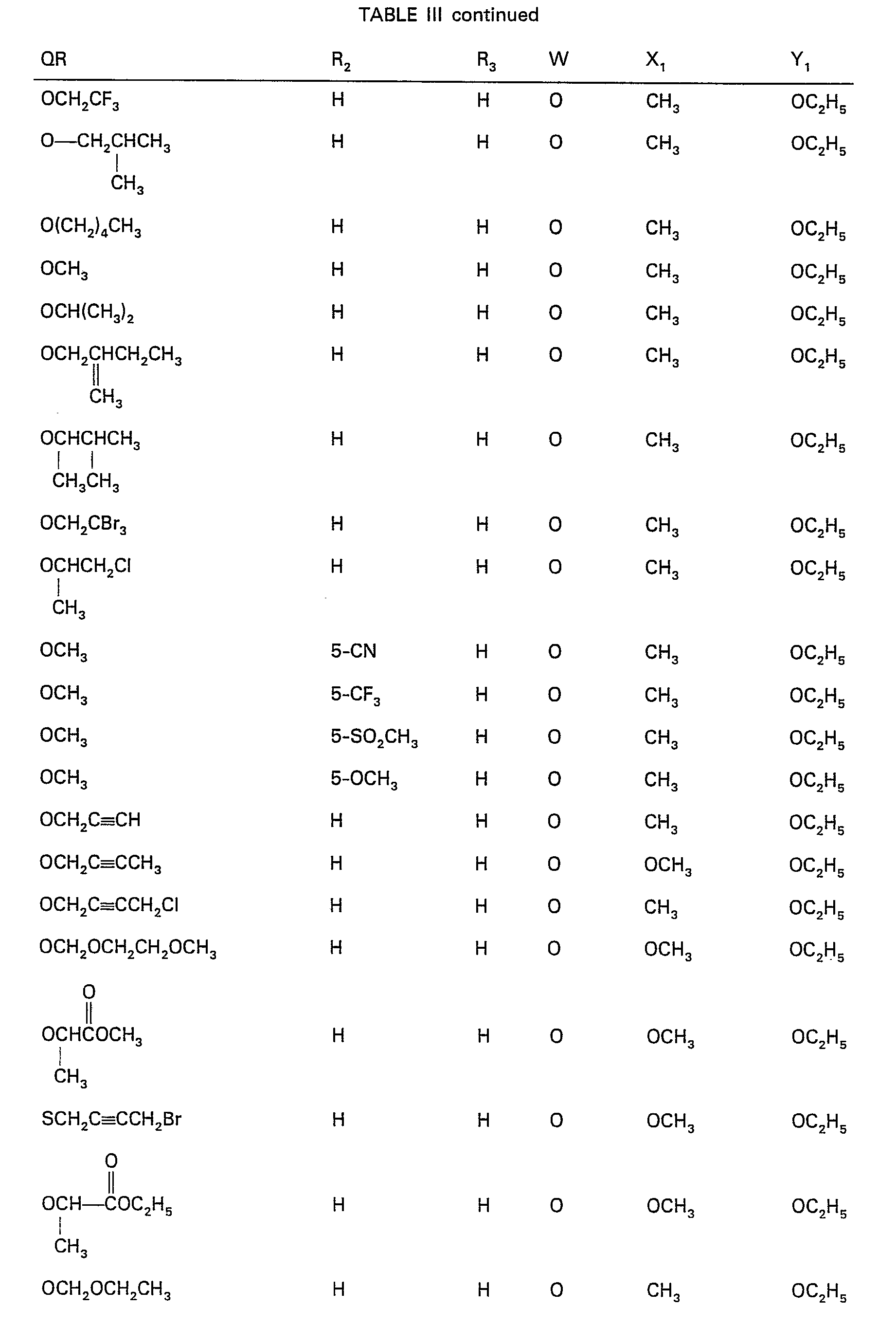

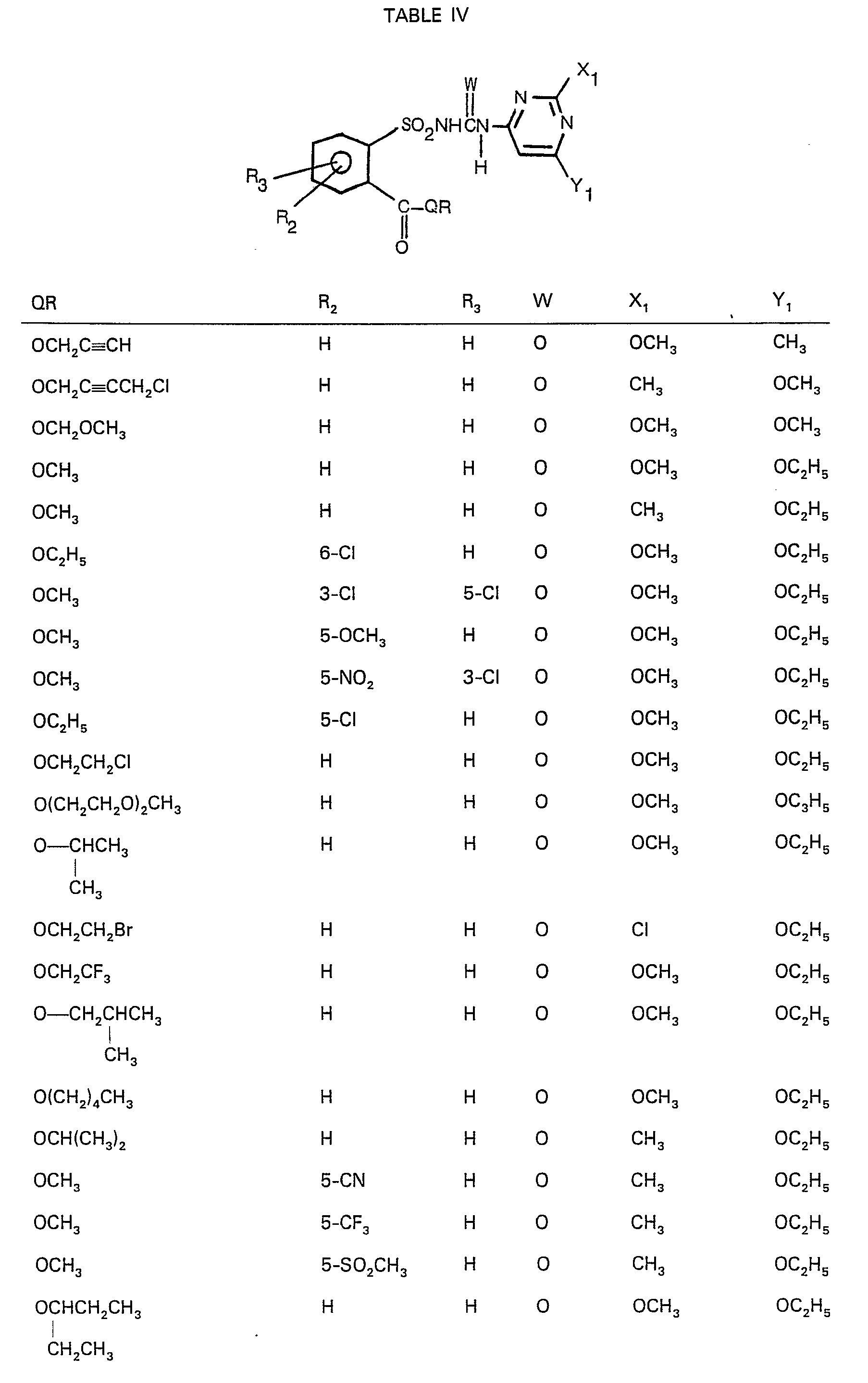

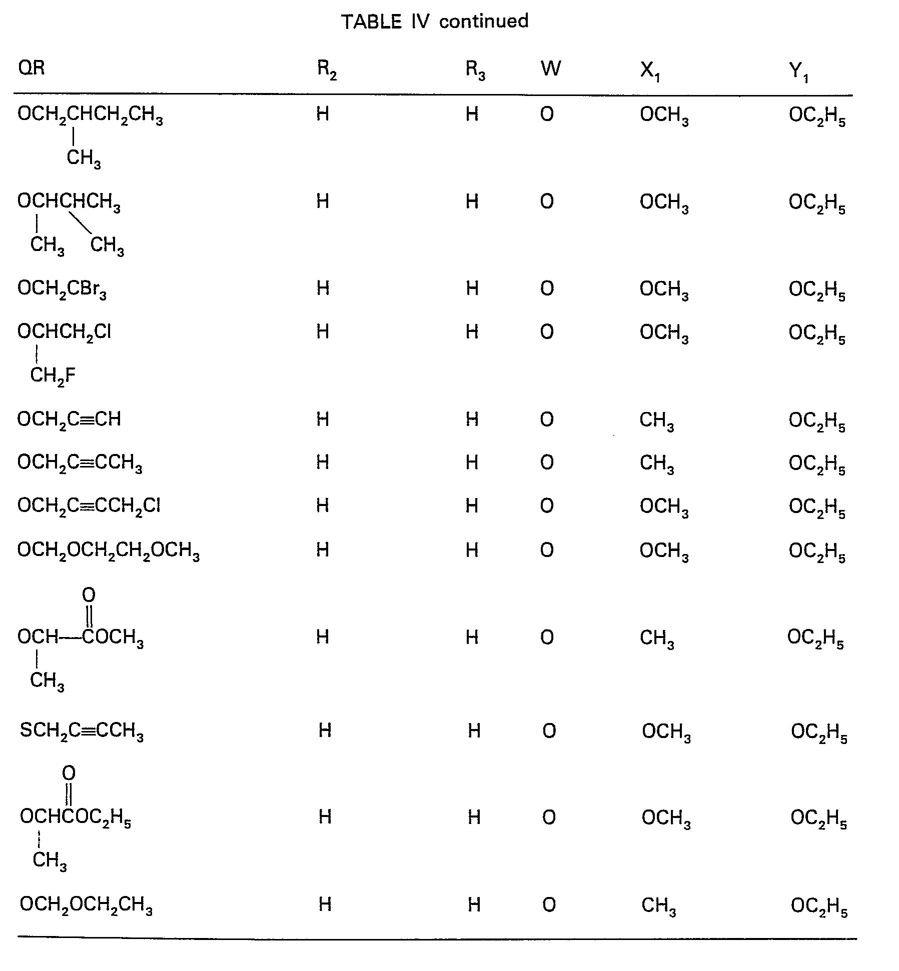

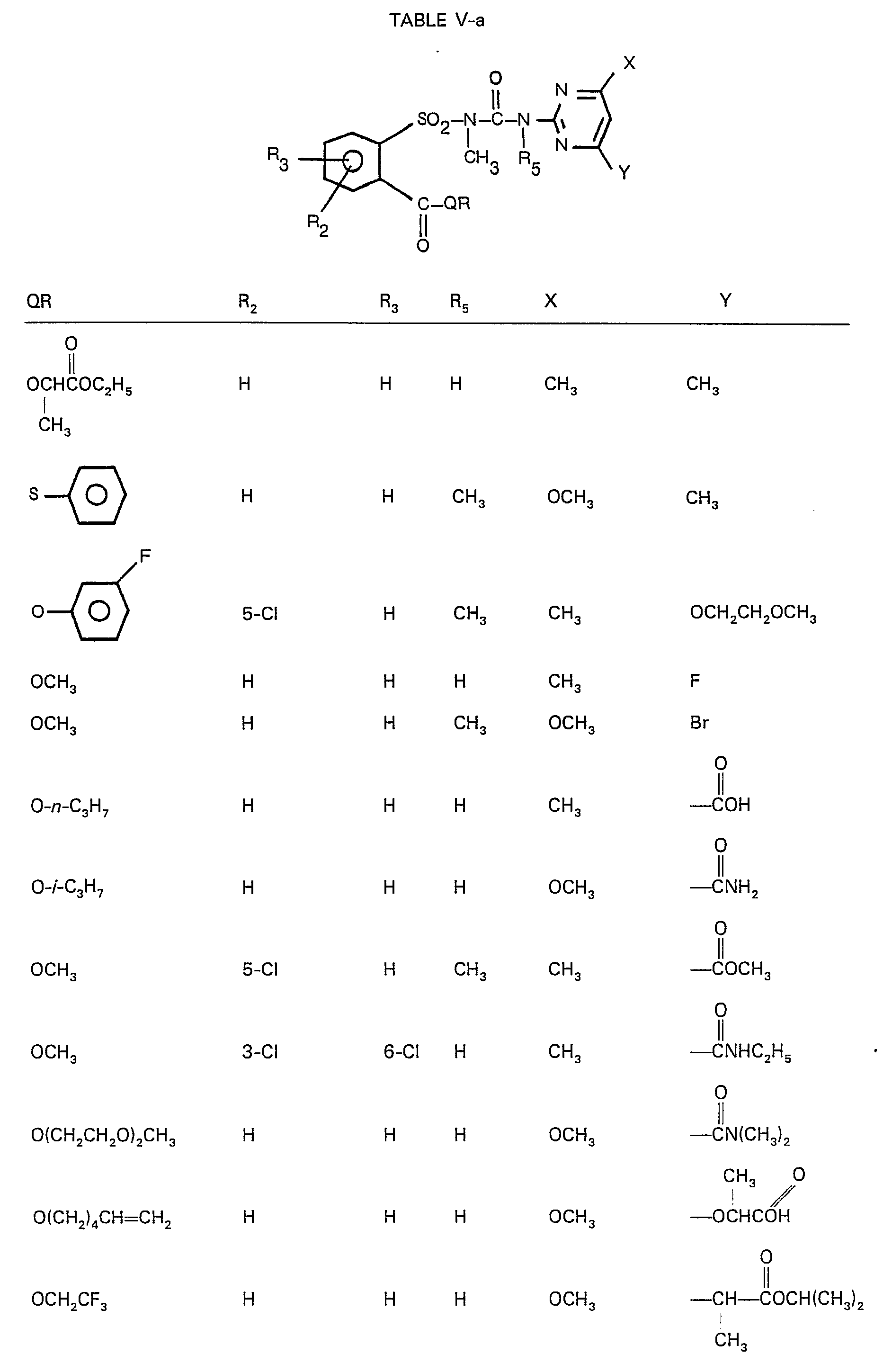

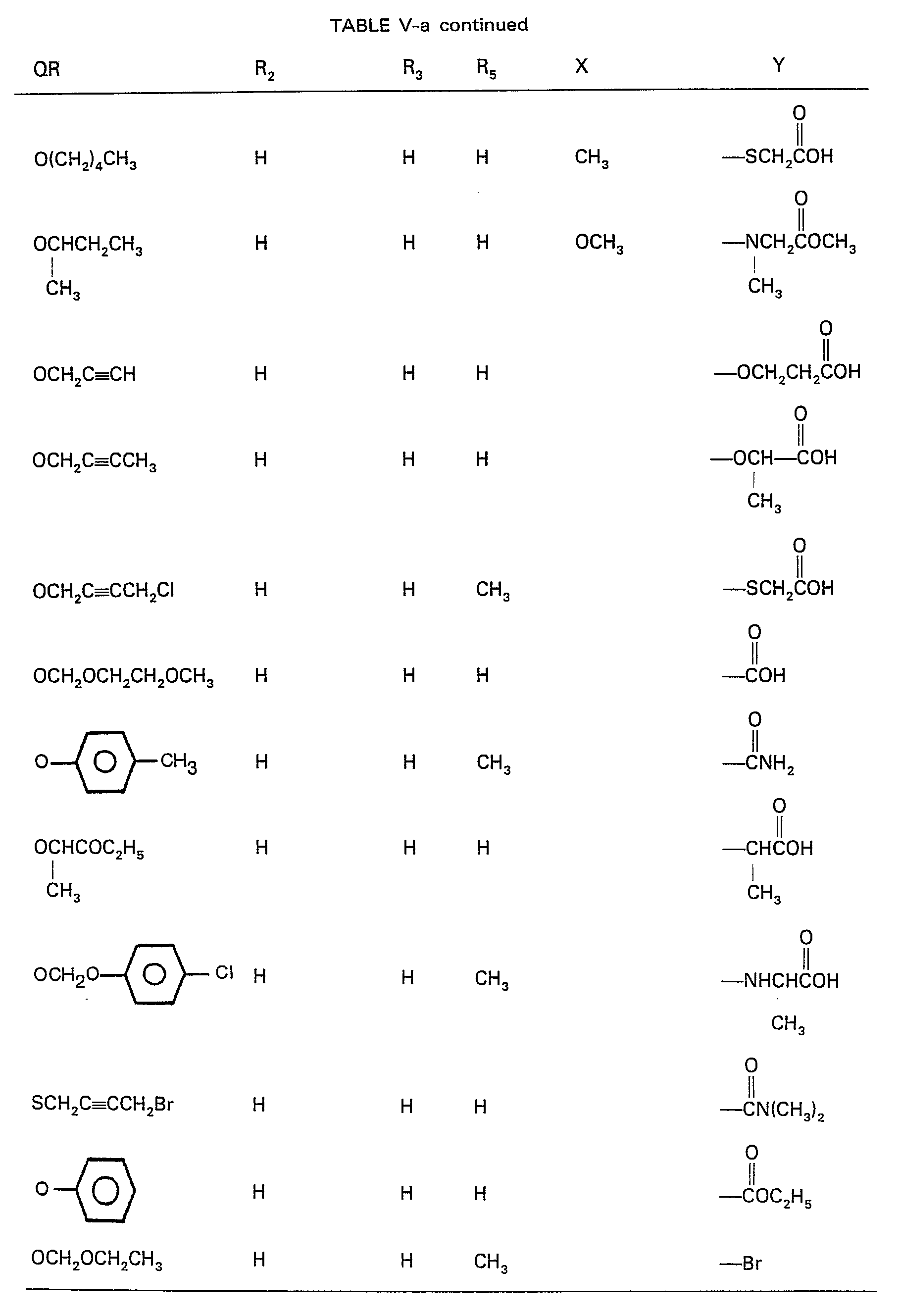

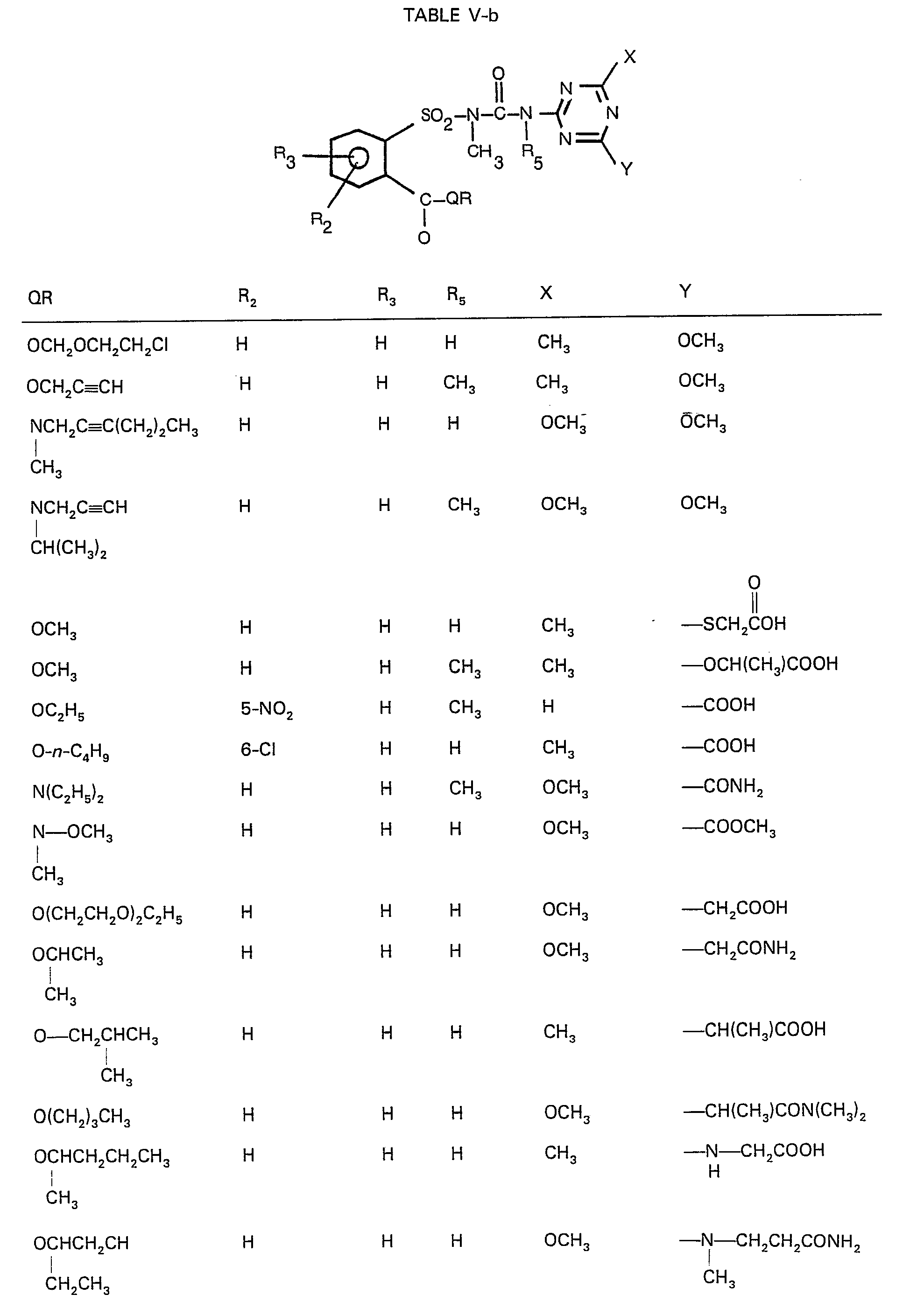

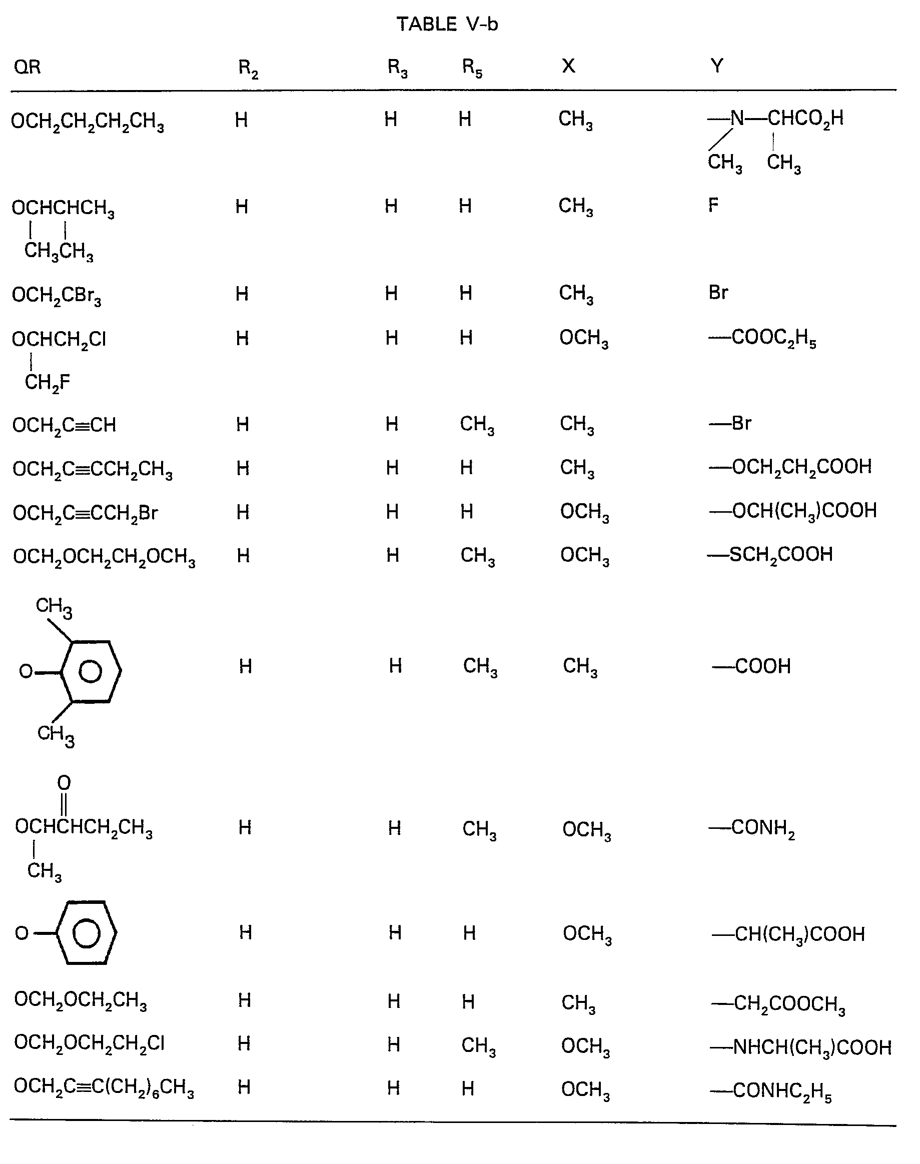

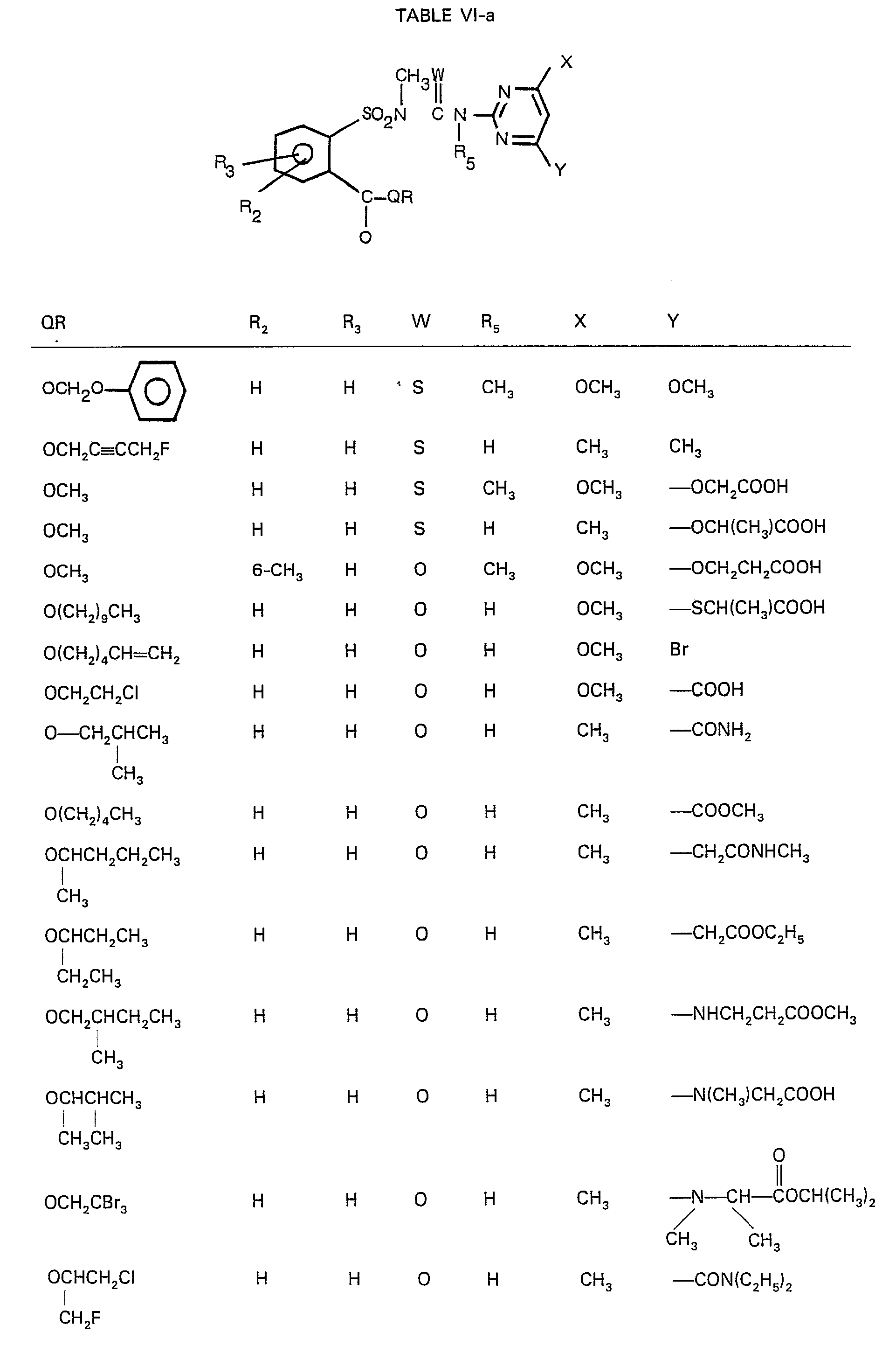

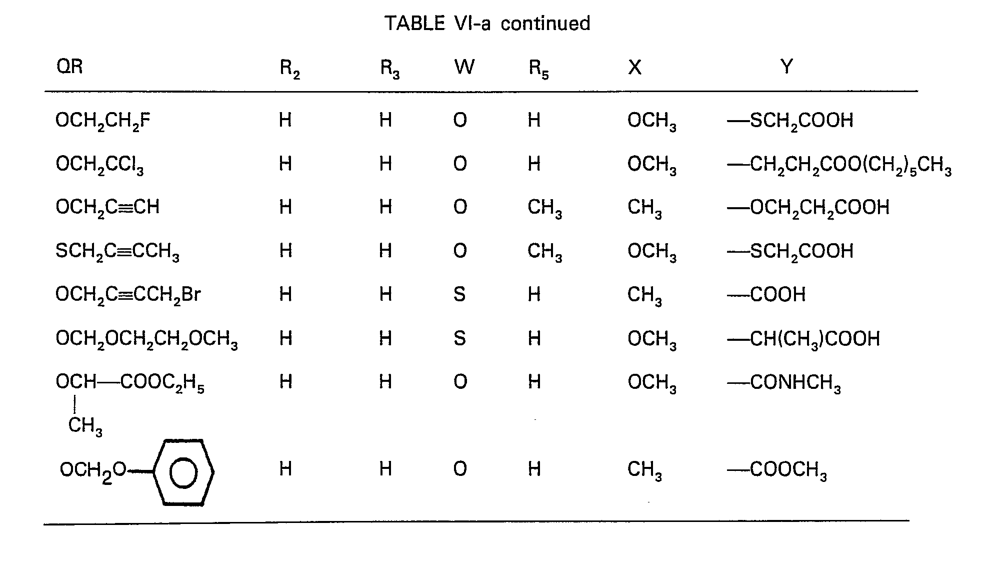

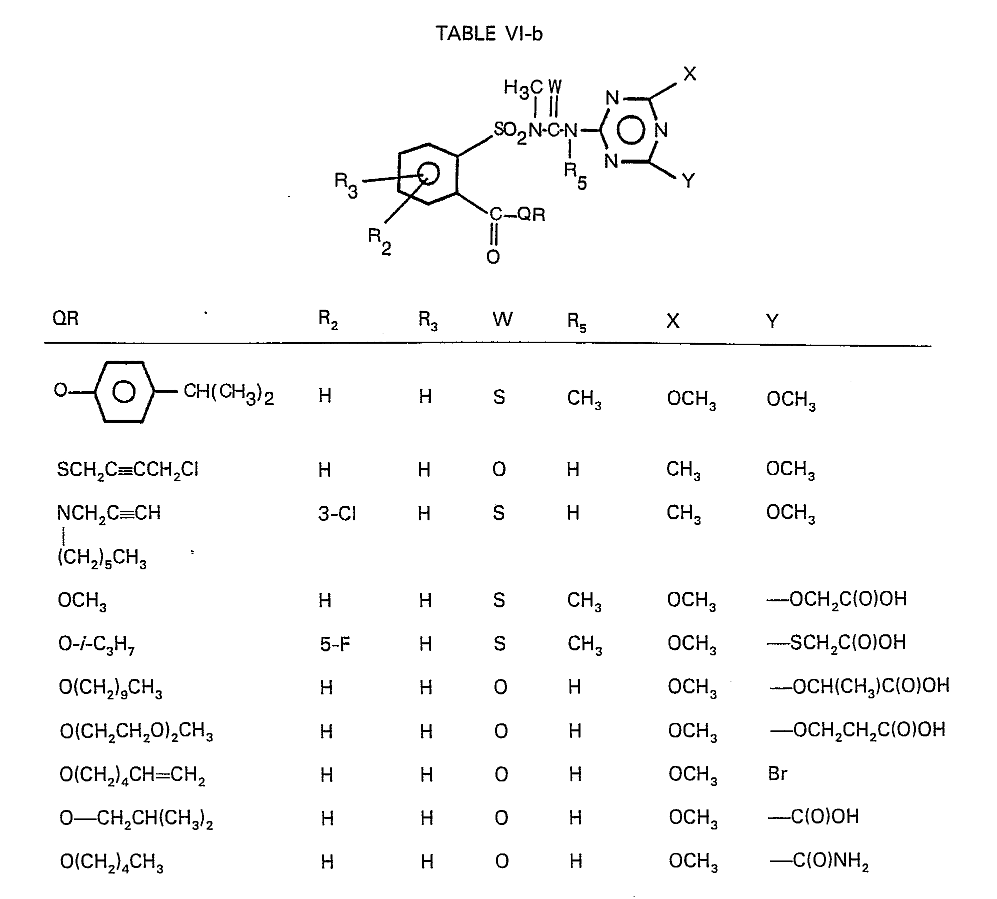

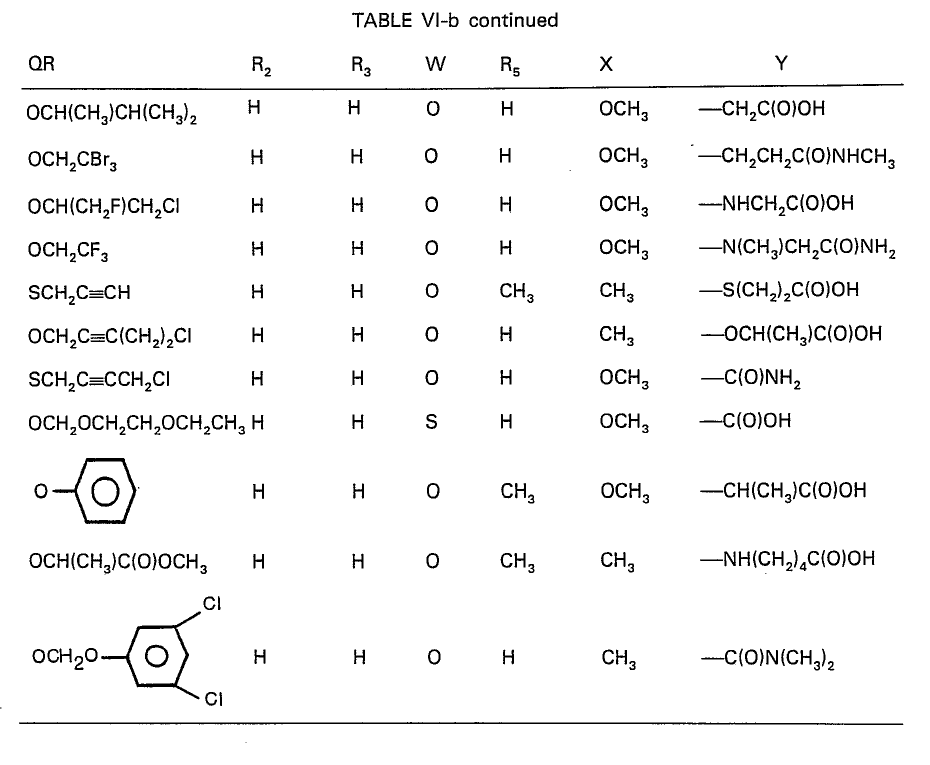

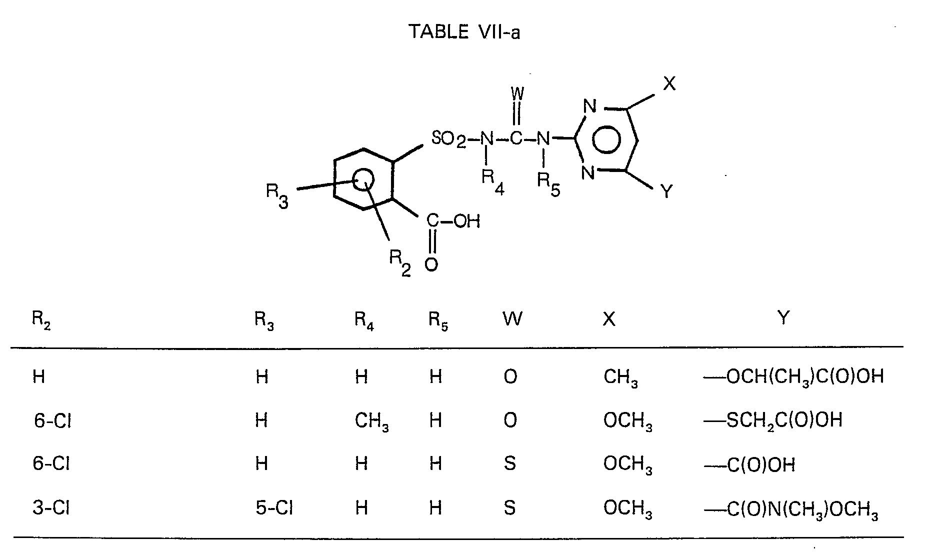

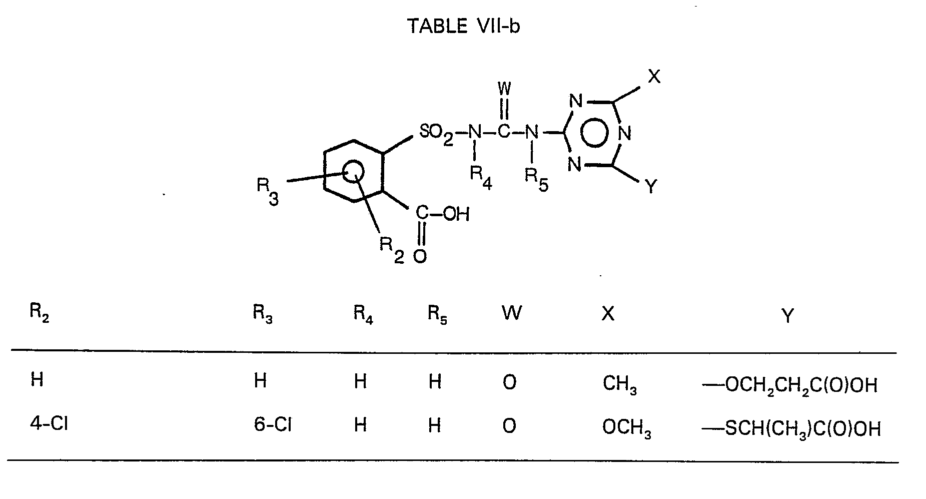

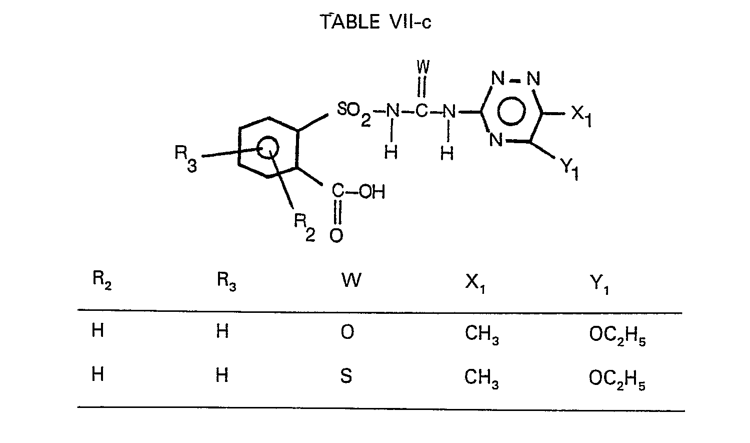

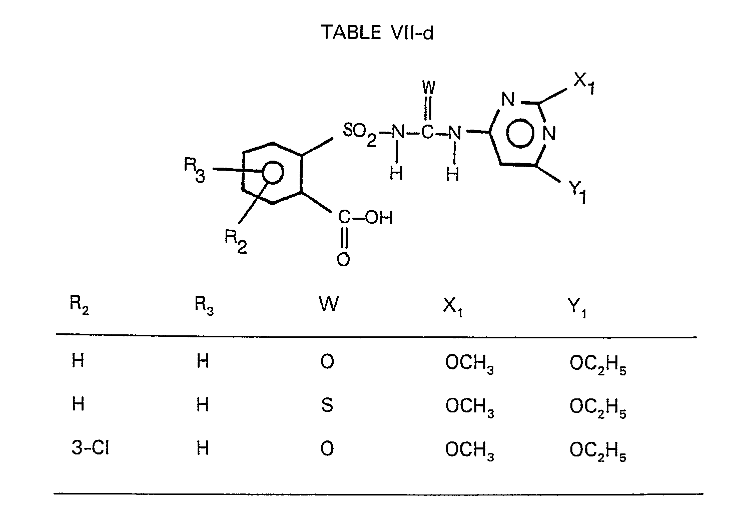

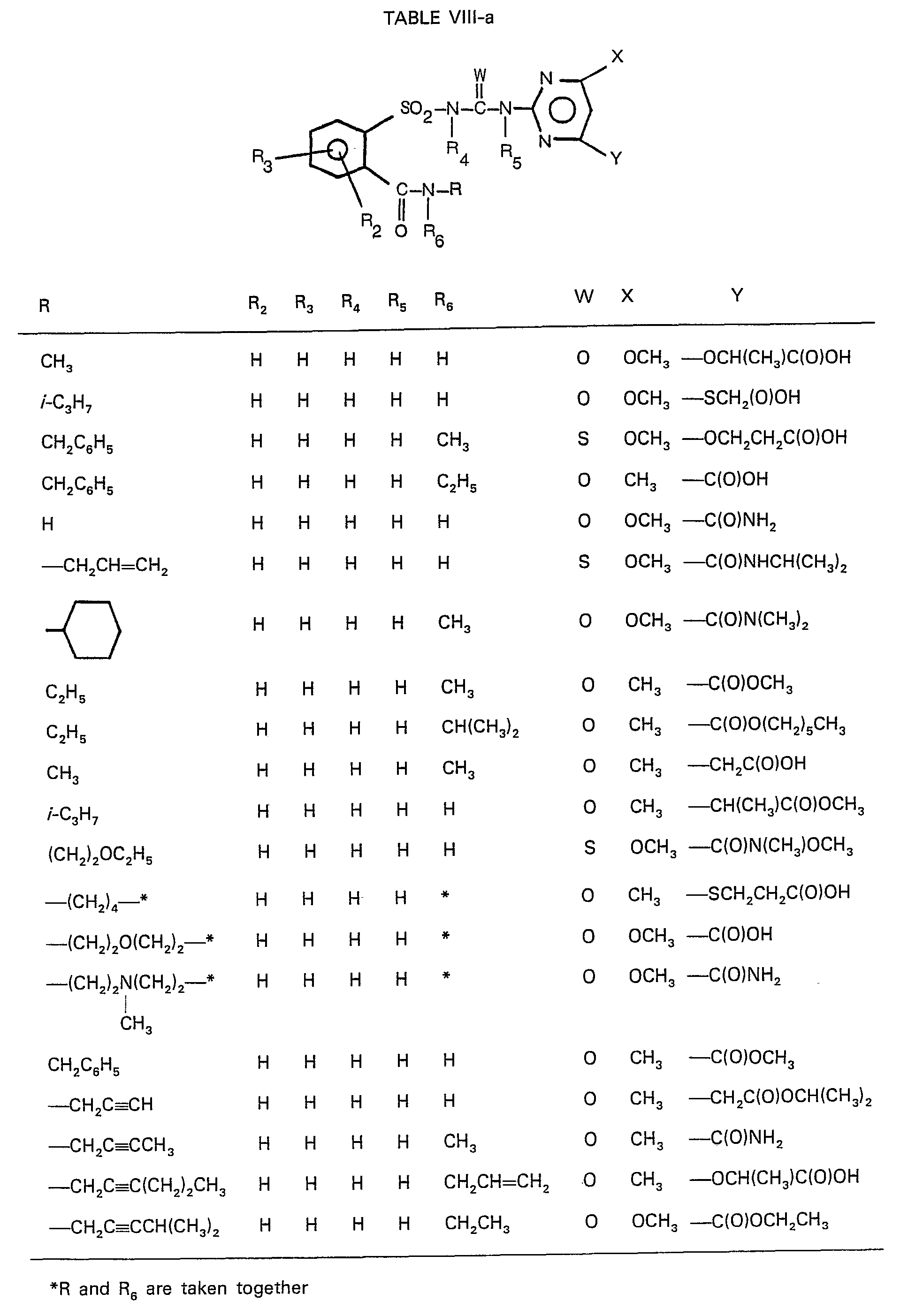

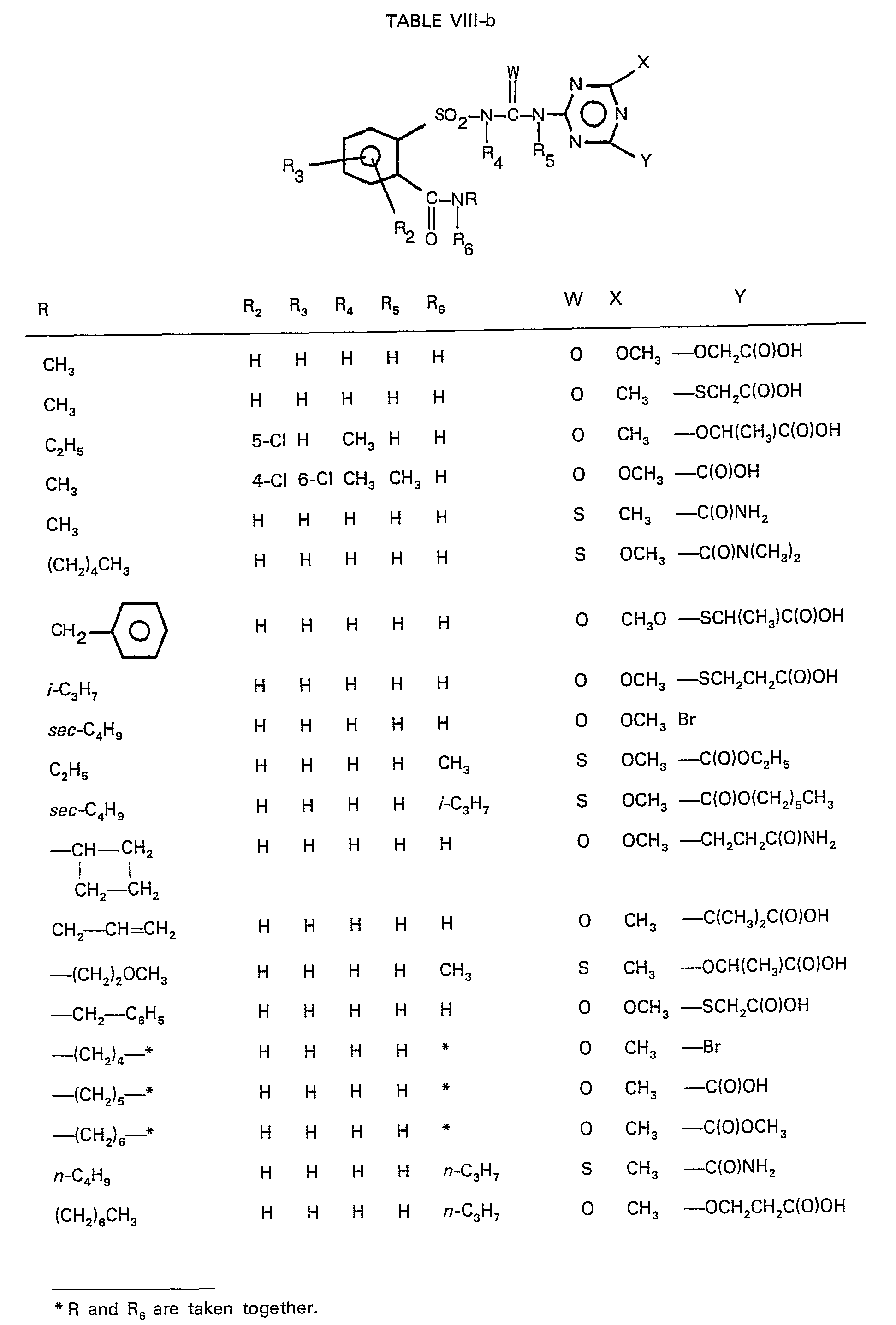

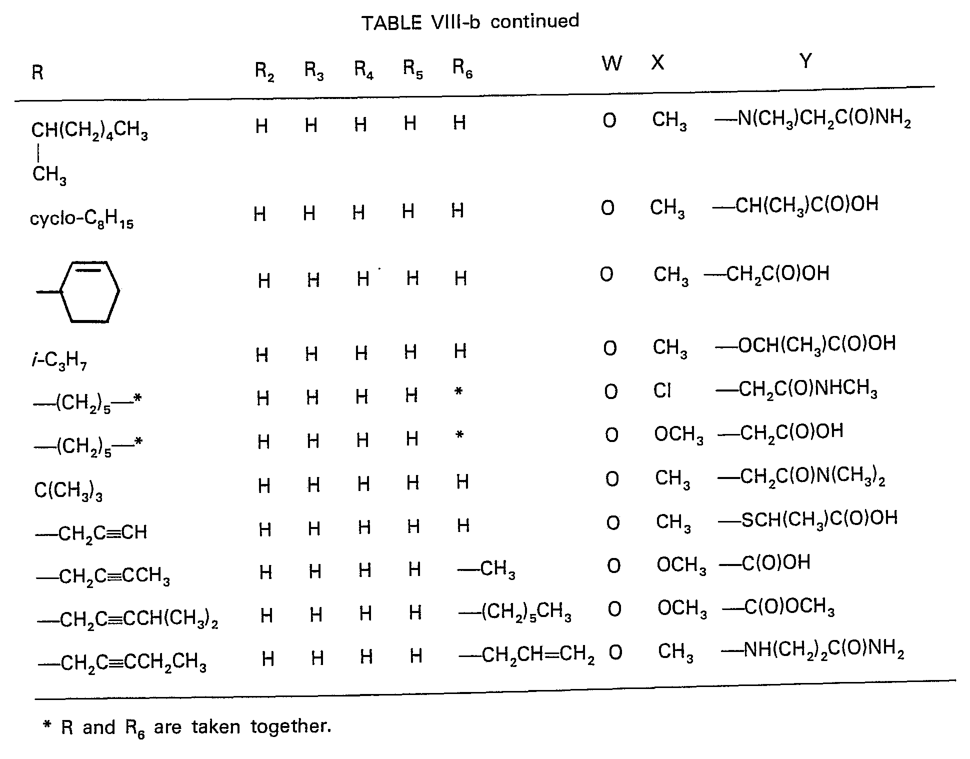

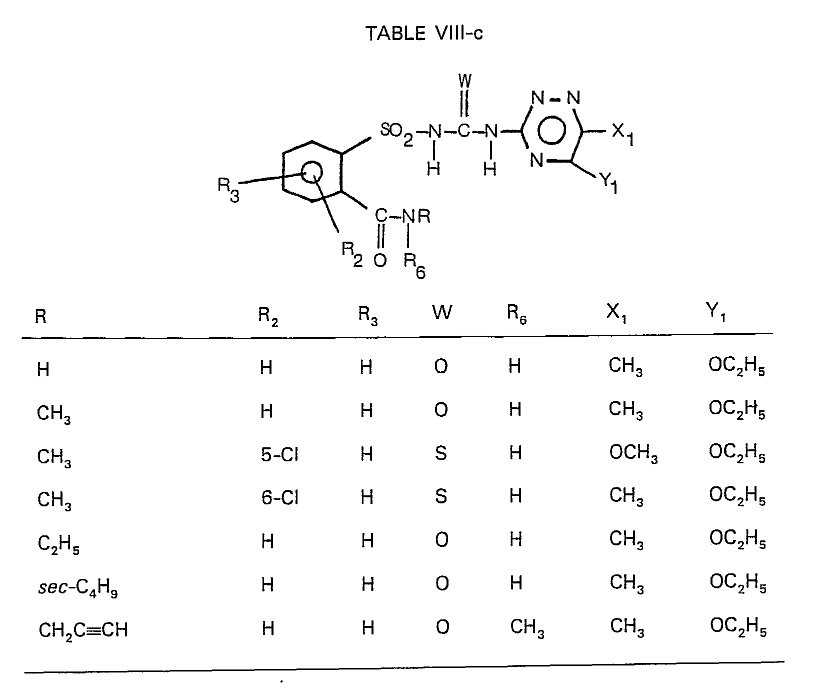

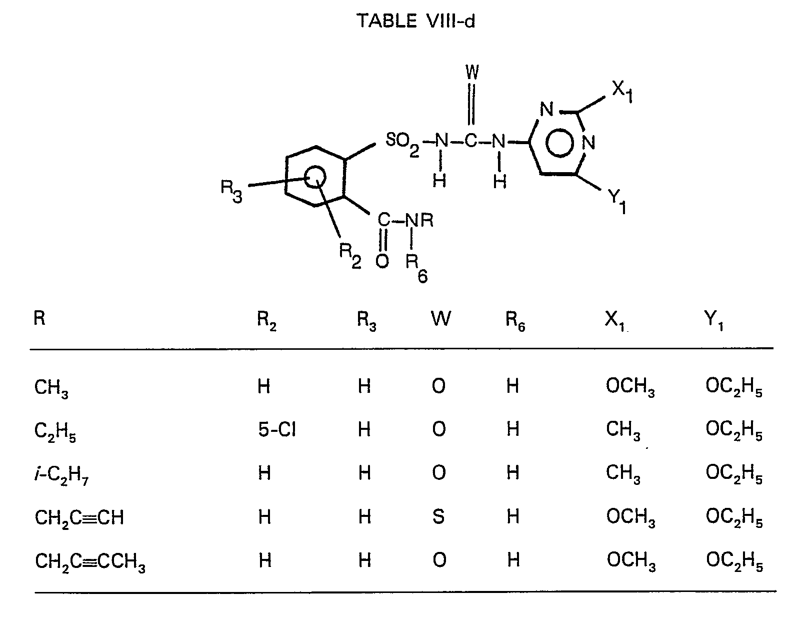

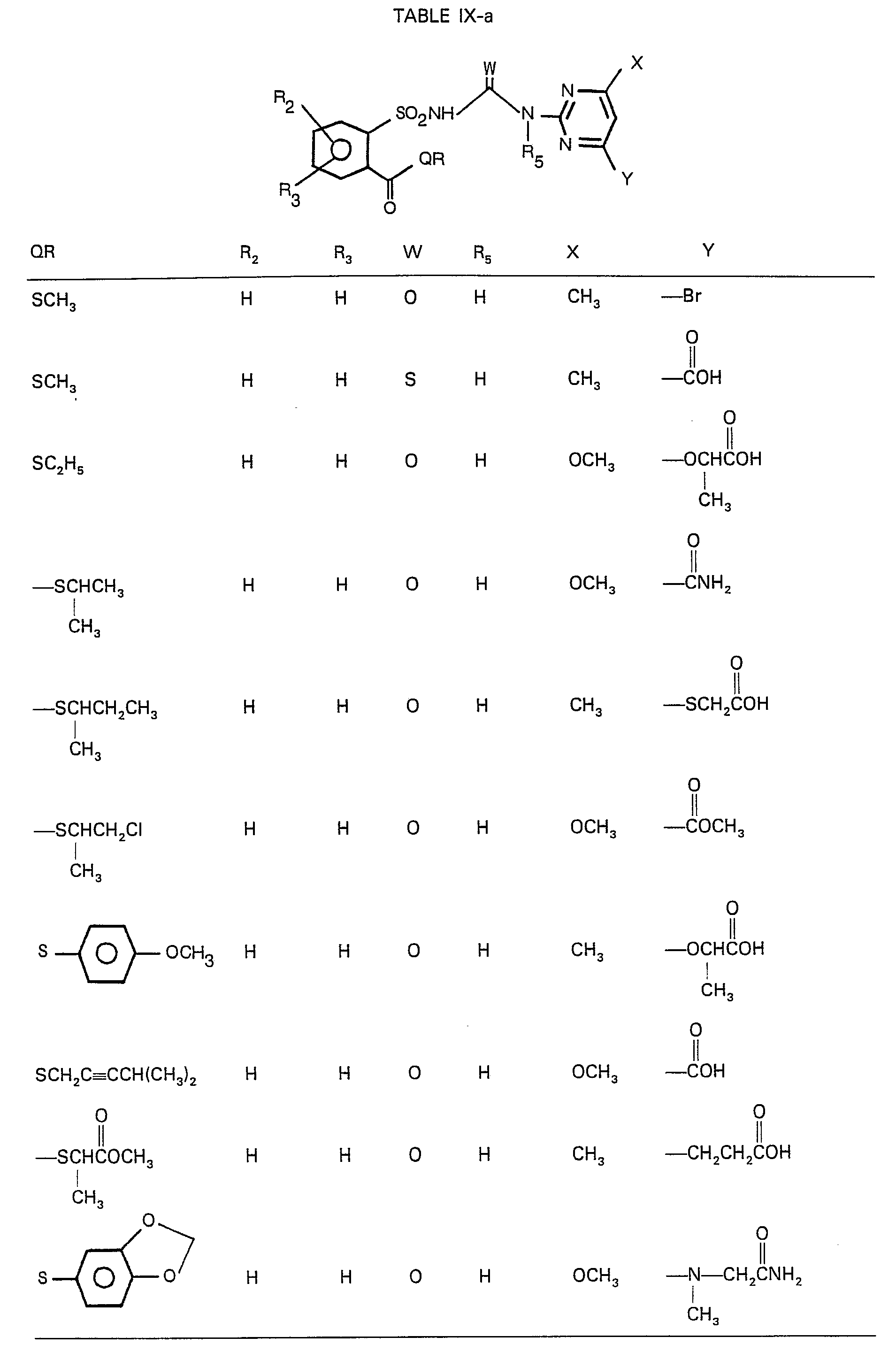

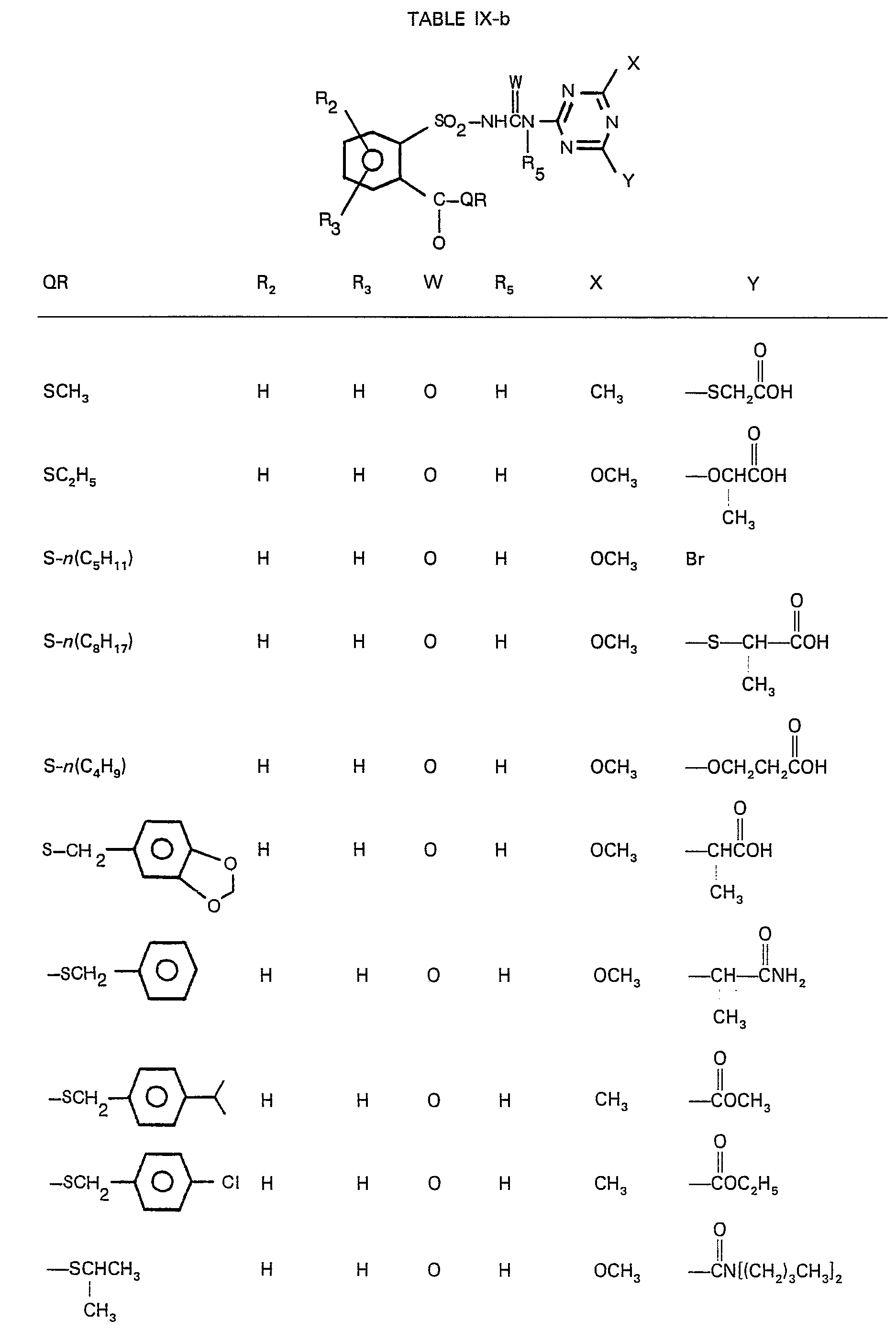

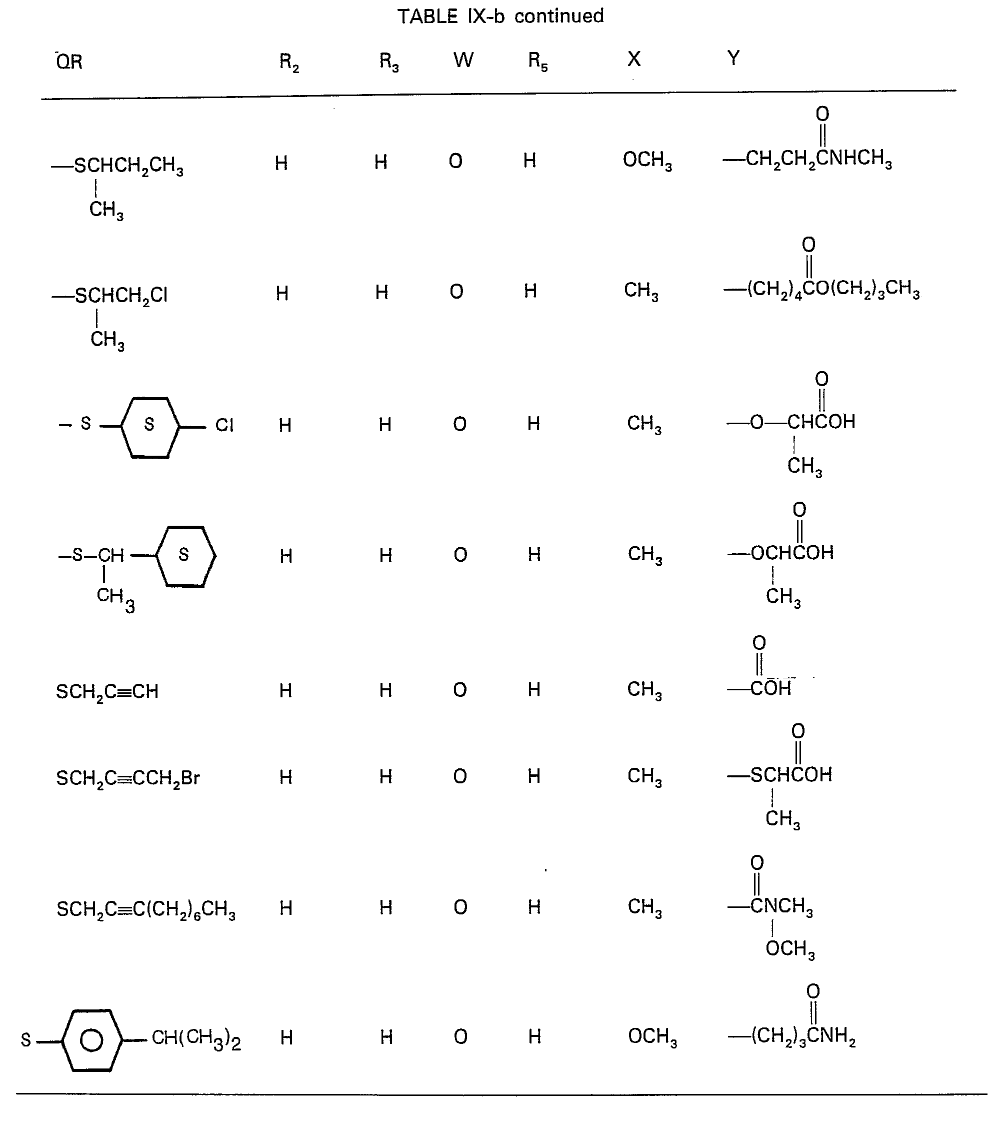

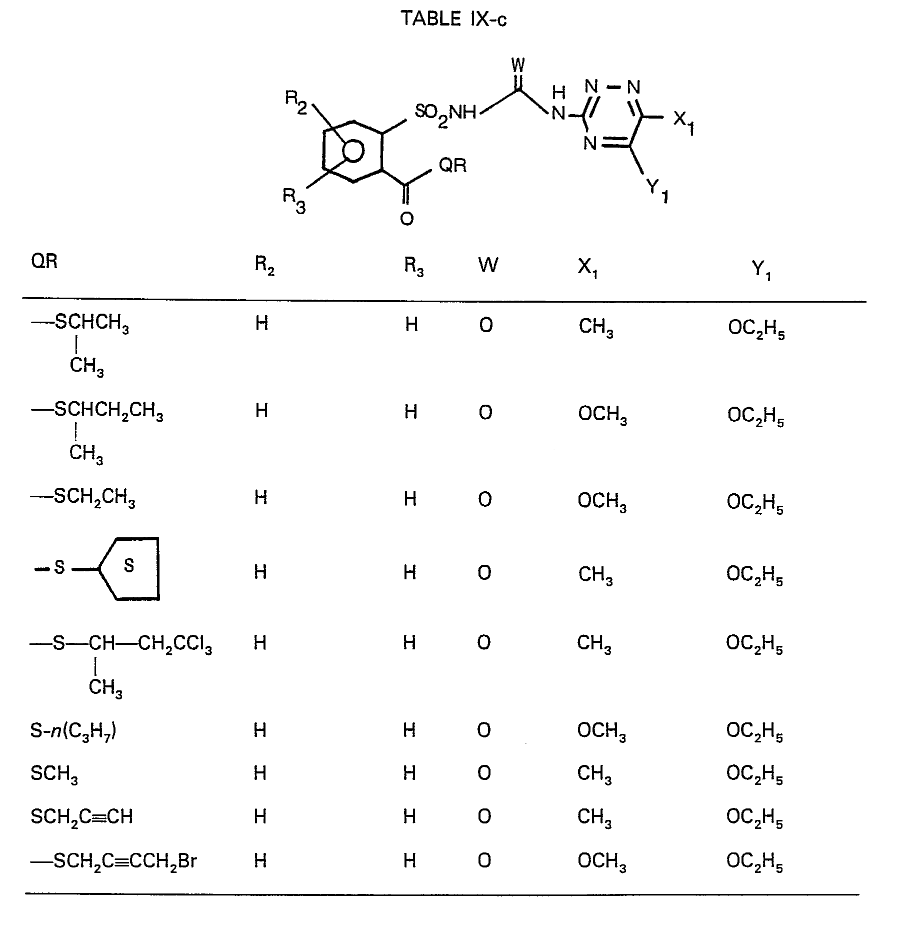

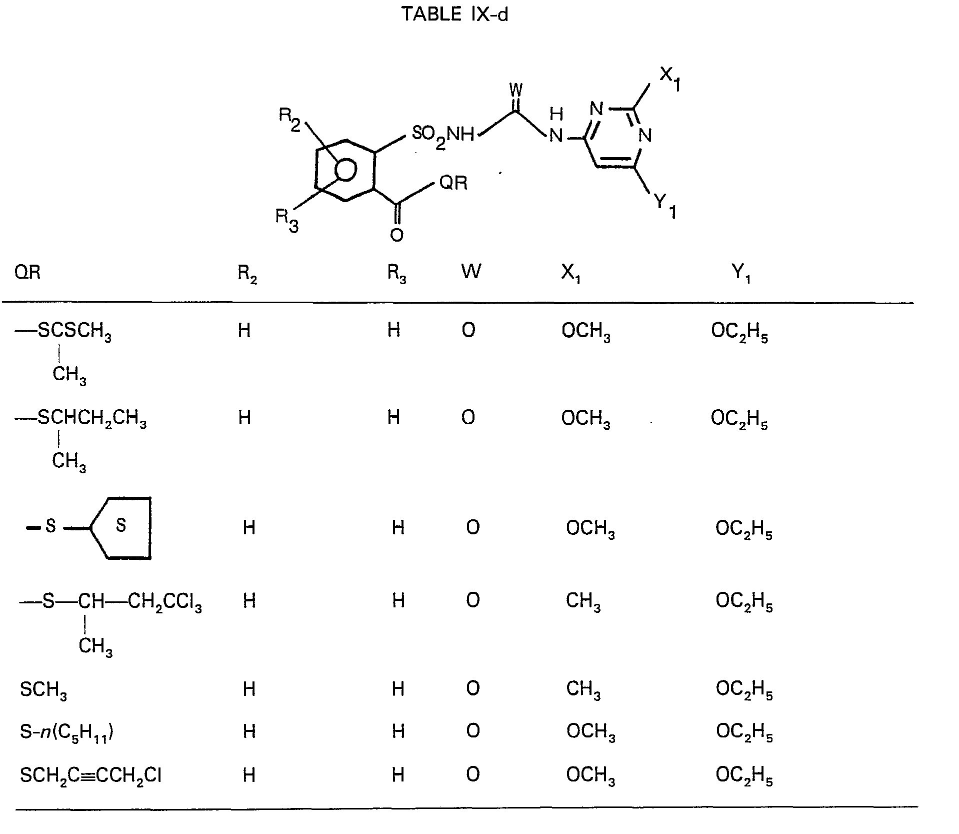

### Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and use at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid diluent(s). More specifically, they will contain these ingredients in the following approximate proportions:

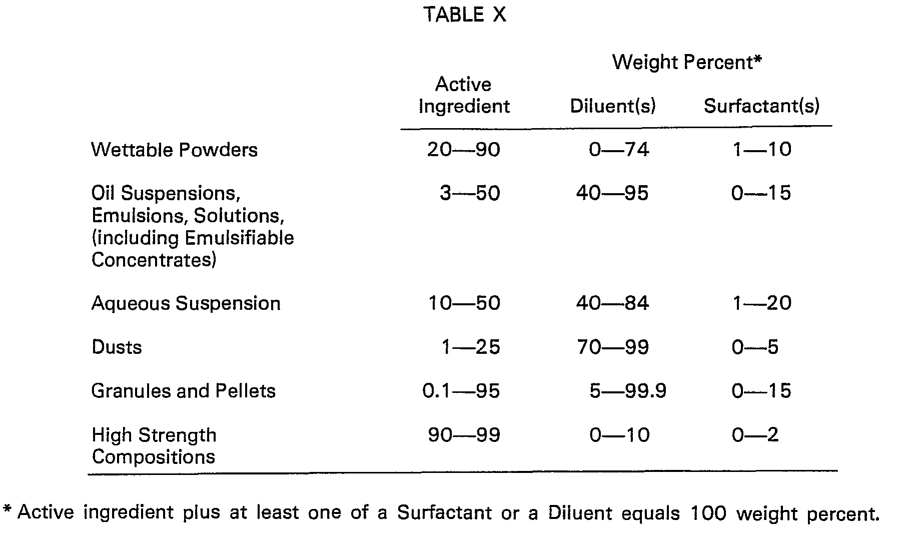

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2 Ed., Interscience, New York, 1950. Solubility under 0.1% is preferred for suspension concentrates; solution concentrates are preferably stable against phase separation at 0 C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", Chemical Engineering, December 4, 1967, pp. 147 ff. and "Perry's Chemical Engineer's Handbook", 5th Ed., McGraw-Hill, New York, 1973, pp. 8-57 ff.

For further information regarding the art of formulation, see for example:
H. M. Loux, U.S. Patent 3,235,361, February 15, 1966, Col. 6, line 16 through Col. 7 line 19 and Examples 10 through 41;
R. W. Luckenbaugh, U.S. Patent 3,309,192, March 14, 1967, Col. 5, line 43 through Col. 7, line 62 and Examples 8, 12, 15,39,41,52,53,58, 132, 138-140, 162-164, 166, 167 and 169-182;
H. Gysin and E. Knusli, U.S. Patent 2,891,855, June 23, 1959, Col. 3, line 66 through Col. 5, line 17 and Examples 1-4;
G. C. Klingman, "Weed Control as a Science", John Wiley & Sons, Inc., New York, 1961, pp. 81-96; and
J. D. Fryer and S. A. Evans, "Weed Control Handbook", 5th Ed., Blackwell Scientific Publications, Oxford, 1968, pp. 101-103.
In the following Examples, all parts are by weight unless otherwise indicated.

### Example 4

### Wettable Powder

### [(2-methoxyethoxy)methyll 2-[[(4-ethoxy-6-methyl-1,3,5-triazin-2-yl)aminocarbonyl]aminosulfonyl]-

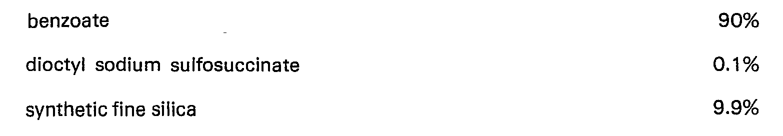

The ingredients are blended and ground in a hammer-mill to produce particles essentially all below 100 microns. The material is sifted through a U.S.S. No. 50 screen and then packaged.

### Example 5

### Wettable Powder

### [(4-chlorophenoxy)methyll 2-[[(4,6-dimethyl-pyrimidin-2-yl)aminocarbonyl]aminosulfonyl]-

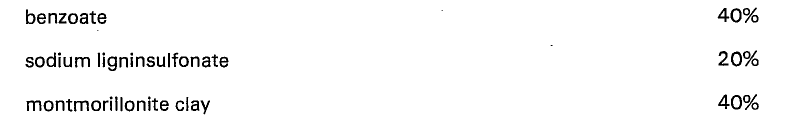

The ingredients are thoroughly blended, coarsely hammer-milled and then air-milled to produce particles essentially all below 10 microns in size. Ther material is reblended and then packaged.

### Example 6

### Oil Suspension

### (4-chloro-2-butynyl) 2-[[(4-methoxy-6-methylprimidin-2-yl)aminocarbonyl]aminosulfonyl]-

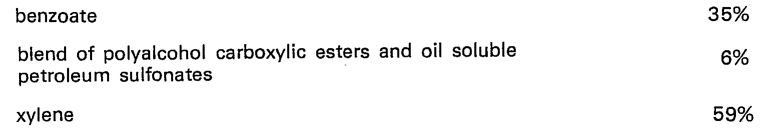

The ingredients are combined and ground together in a sand mill to produce particles essentially all below 5 microns. The product can be used directly extended with oils, or emulsified in water.

### Example 7

### Solution

### [(2-methoxyethoxy)methyl] 2-[[(4,6-dimethylpyrimidin-2-yl)aminocarbonyl]aminosulfonyl]benzoate,

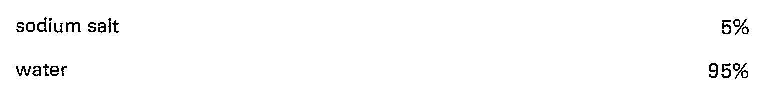

The salt is added directly to the water with stirring to produce the solution, which may then be packaged for use.

### Example 8

### Low Strength Granule

### methyl 2-[[[4-(1-carboxyethyl)-6-methyl-1,3,5-triazin-2-yl]aminocarbonyl]aminosulfonyl]-

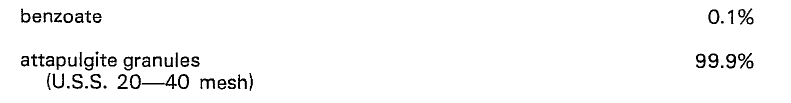

The active ingredient is dissolved in a solvent and the solution is sprayed upon dedusted granules in a double-cone blender. After spraying of the solution has been completed, the material is warmed to evaporate the solvent. The material is allowed to cool and then packaged.

### Example 9

### Dust

### [(2-methoxyethoxy)methyl] 2-[[(4,6-dimethoxypyrimidin-2-yl)aminocarbonyl]aminosulfonyl]-

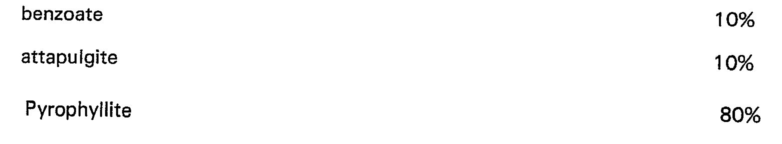

The active ingredient is blended with attapulgite and then passed through a hammer-mill to produce particles substantially all below 200 microns. The ground concentrate is then blended with powdered pyrophyllite until homogeneous.

### Utility

The compounds of the present invention are active herbicides. They have utility for broad- spectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for the selective pre- or post-emergence weed control in crops, such as wheat and barley.

The rates of application for the compounds of the invention are determined by a number of factors, including their use as selective or general herbicides, the crop species involved, the types of weeds to be controlled, weather and climate, formulations selected, mode of application, amount of foliage present, ect. In general terms, the subject compounds should be applied at levels of around 0.06 to 10 kg/ha, the lower rates being suggested for use on lighter soilds and/or those having a low organic matter content, for selective weel control or for situations where only short-term persistence is required.

The compounds of the invention may be used in combination with any other commerical herbicide examples of which are those of the triazine, triazole, uracil, urea, amide, diphenylether, carbamate and bipyridylium types.

For use as a plant growth regulant, the compounds will be used in such manner and amount as to be effective but substantially non-phytotoxic to the desirable plant species whose growth is to be regulated.

The herbicidal properties of the subject compounds were discovered in a number of greenhouse tests. The test procedures and results follow.

### Test A

Seeds of crabgrass (Digitaria spp.), barnyardgrass (Echinochloa crusgal_{/}tï, wild oats (Avena fatua), cassia (Cassia tora), morningglory (Ipomoea spp.), Cocklebur (Xanthium spp.), sorghum, corn, soybean, rice, wheat and nutsedge tubers (Cyperus rotundus) were planted in a growth medium and treated pre-emergence with the chemicals dissolved in a non-phytotoxic solvent solution of the compounds of Table A. At the same time, cotton having five leaves (including cotyledonary ones), bush beans with the third trifoliate leaf expanding, crabgrass, barnyardgrass and wild oats with two leaves, cassia with. three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three-five leaves were sprayed. Treated plants and controls were maintained in a greenhouse for sixteen days, then all species were compared to controls and visually rated for response to treatment.

The ratings are based on a numerical scale extending from 0 = no injury to 10 = complete kill. The accompanying descriptive symbols have the following meanings:
C = chlorosis/necrosis;
G = growth retardation;
H =formative effects;
E = emergence inhibition;
U = unusual pigmentation;
X = axillary stimulation;
6Y = abscised flowers or buds; and
6F = delayed flowering.

The data are summarized in Table A.

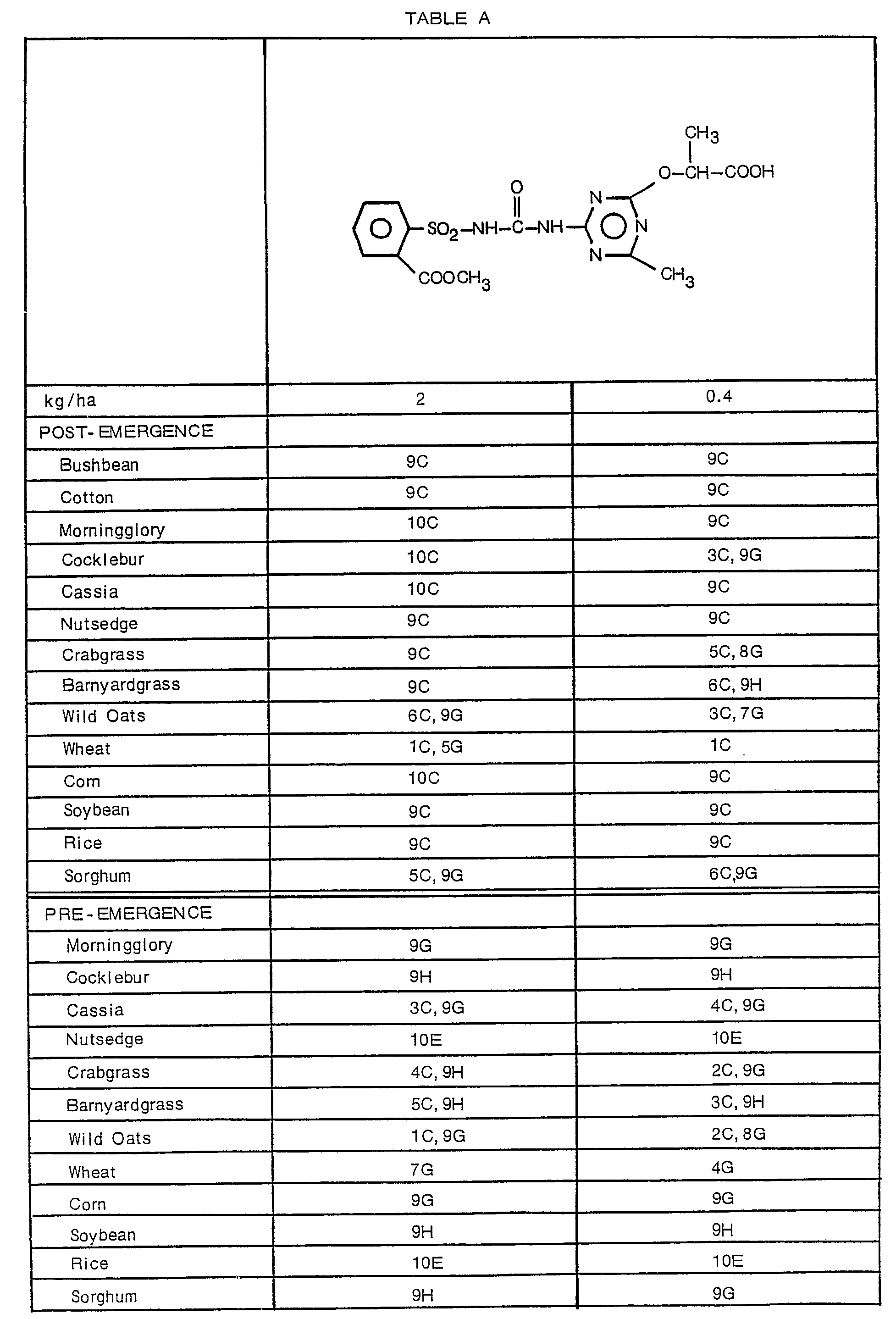

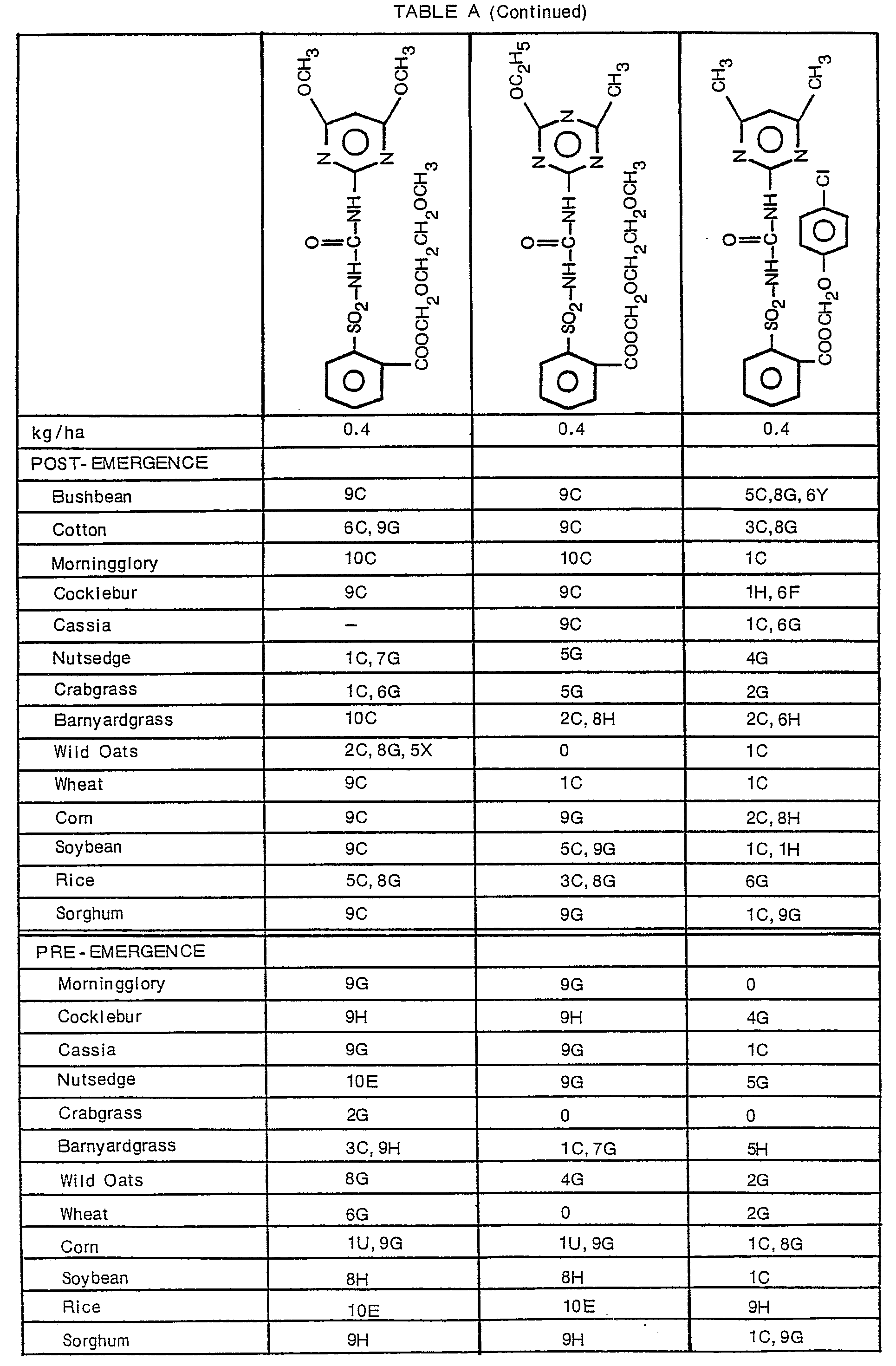

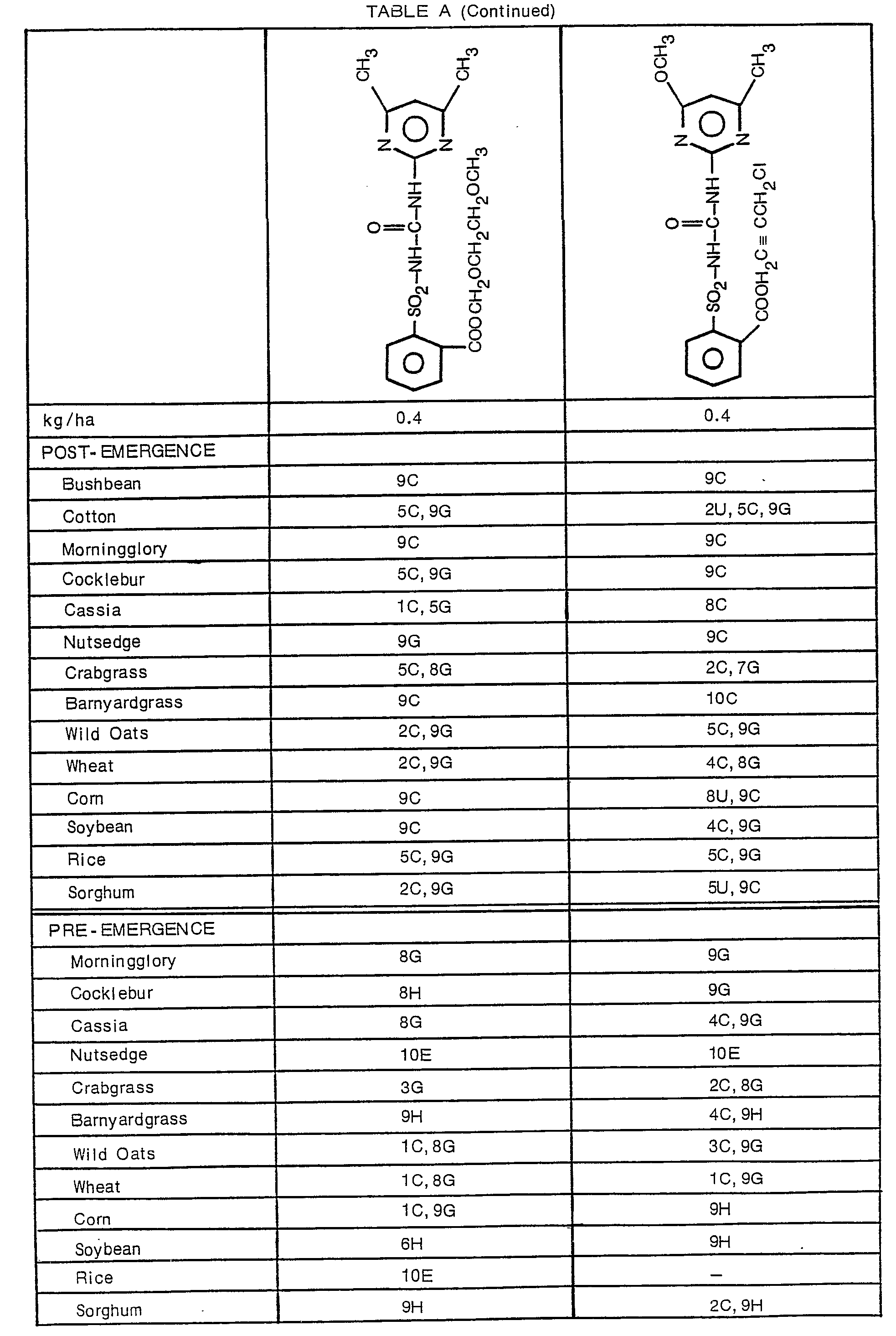

### Test B

Two plastic bulb pans were filled with fertilized and limed Fallsington silt loam soil. One pan was planted with corn, sorghum, Kentucky bluegrass and several grassy weeds. The other pan was planted with cotton, soybeans, purple nutsedge (Cyperus rotundus), and several broadleaf weeds. The following grassy and broadleaf weeds were planted: crabgrass (Digitaria sanguinalis), barnyardgrass (Echinochloa crusgalli), wild oats (Avena fatua), johnsongrass (Sorghum halepense), dallisgrass (Paspalum dilatatum), giant foxtail (Setaria faberii), cheatgrass (Bromus secalinus), mustard (Brassica arvensis), cocklebur (Xanthium pennsylvanicum), pigweed (Amaranthus retroflexus), morningglory (Ipomoea hederacea), cassia (Cassia tora), teaweed (Sida spinosa), velvetleaf (Abutilon theophrasti), and jimsonweed (Datura stramonium). A 12.5 cm diameter plastic pot was also filled with prepared soil and planted with rice and wheat. Another 12.5 cm pot was planted with sugarbeets. The above four containers were treated pre-emergence with several test compounds within the scope of the invention.

Twenty-eight days after treatment, the plants were evaluated and visually rated for response to the chemical treatments utilizing the rating system described previously for Test A. The data are summarized in Table B. Note that certain compounds are useful as pre-emergence treatments for weed control in wheat.

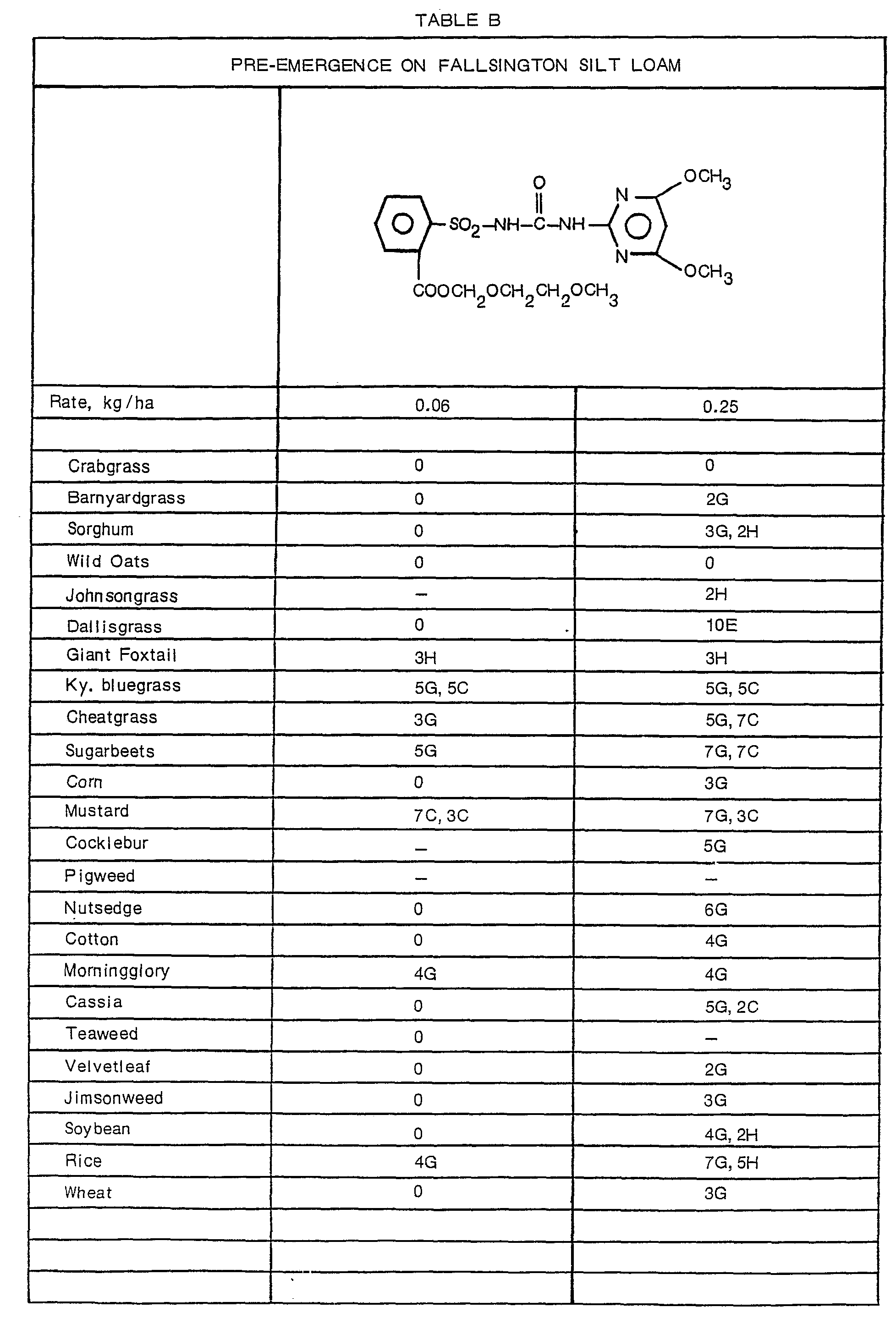

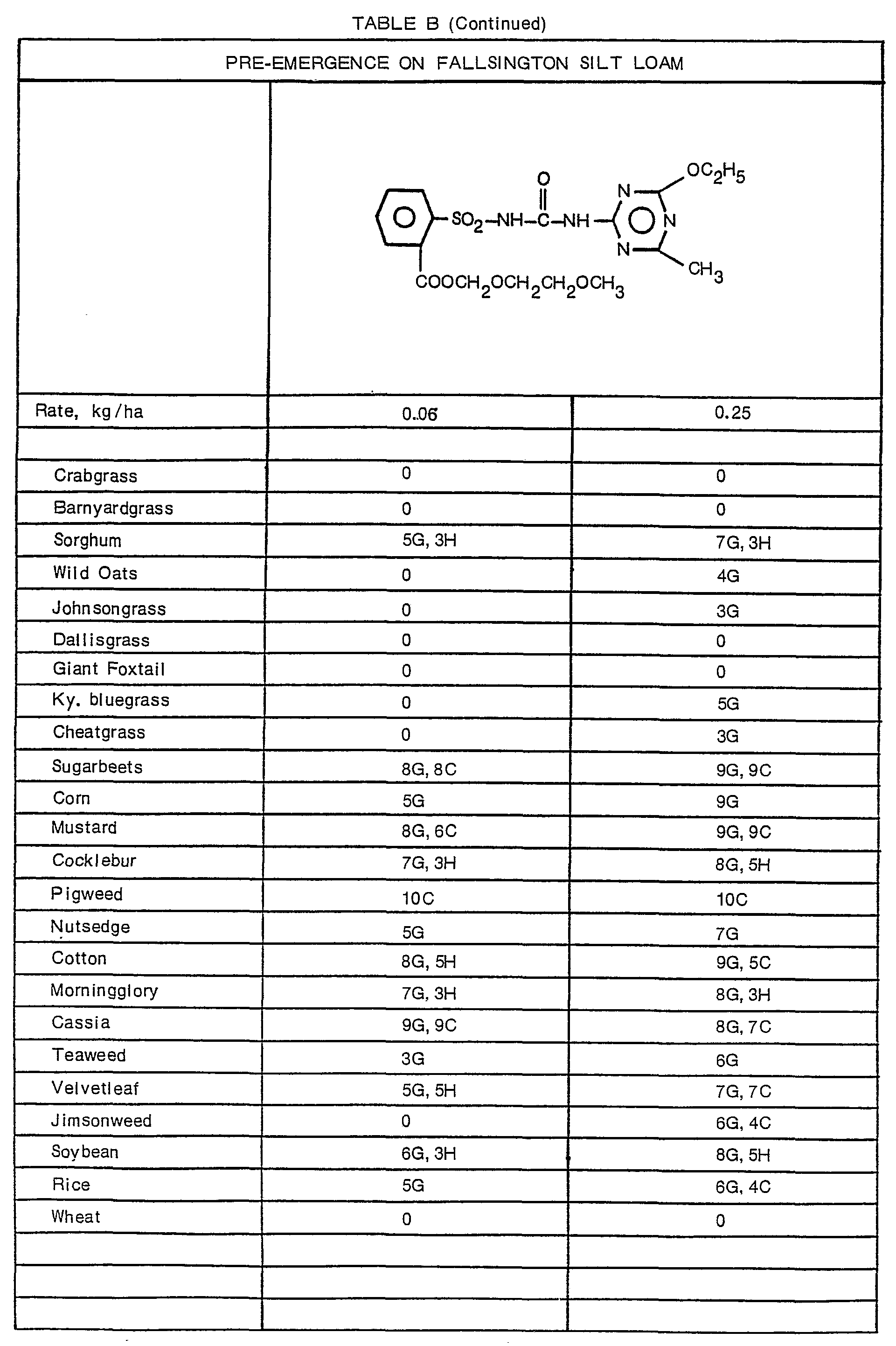

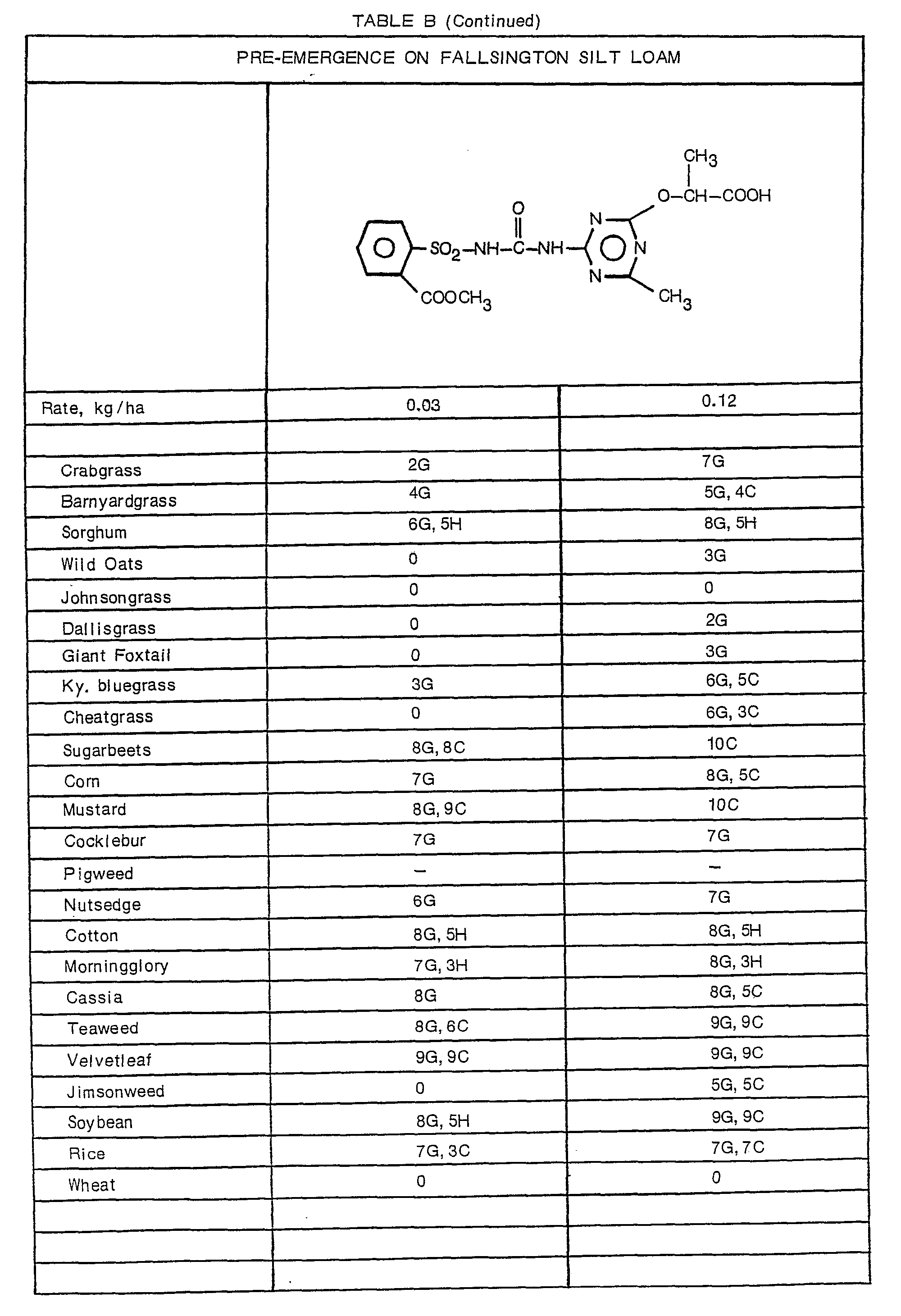

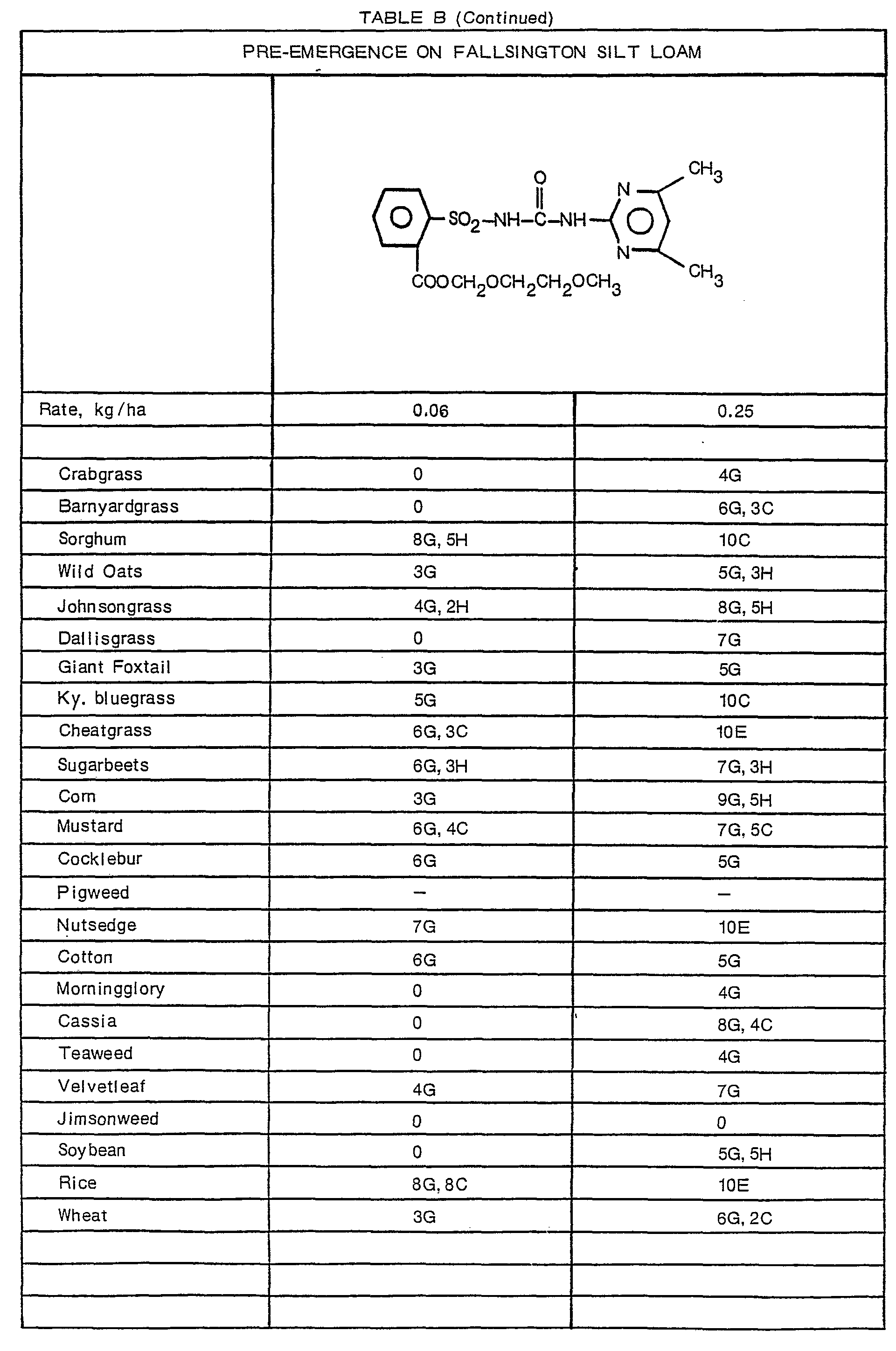

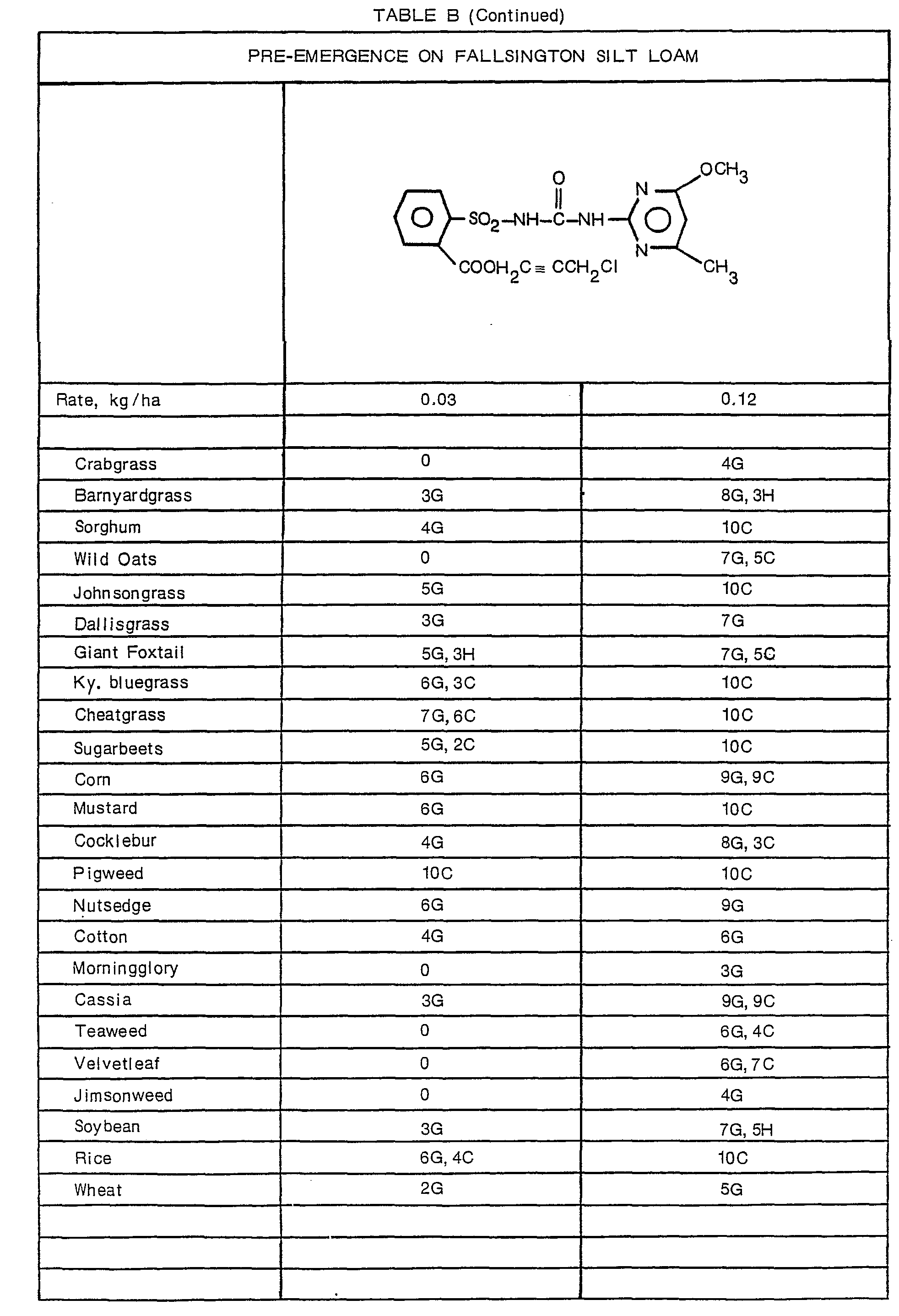

### Test C

Twenty-five cm diameter plastic pots filled with Fallsington silt loam were planted with soybeams, cotton, alfalfa, corn, rice, wheat, sorghum, velvetleaf (Abutilon theophrasti), sesbania (Sesbania exaltata), Cassia (Cassia tora), morningglory (Ipomoea hederacea), jimsonweed (Datura stramonium), cocklebur (Xanthium pennsylvanicum), crabgrass (Digitaria spp.), nutsedge (Cyperus rotundus), barnyardgrass (Echinochloa crusgalli), giant foxtail (Setaria faberii) and wild oats (Avena fatua). Approximately two weeks after planting, the young plants and the soil around them were sprayed overall with the test chemicals dissolved in a non-phytotoxic solvent. Two weeks after treatment, all species were compared to untreated controls and visually rated for response to treatment. The rating system was as described previously for Test A. The data are presented in Table C. The compounds tested by this procedure are useful for the post-emergence control of weeds in wheat.

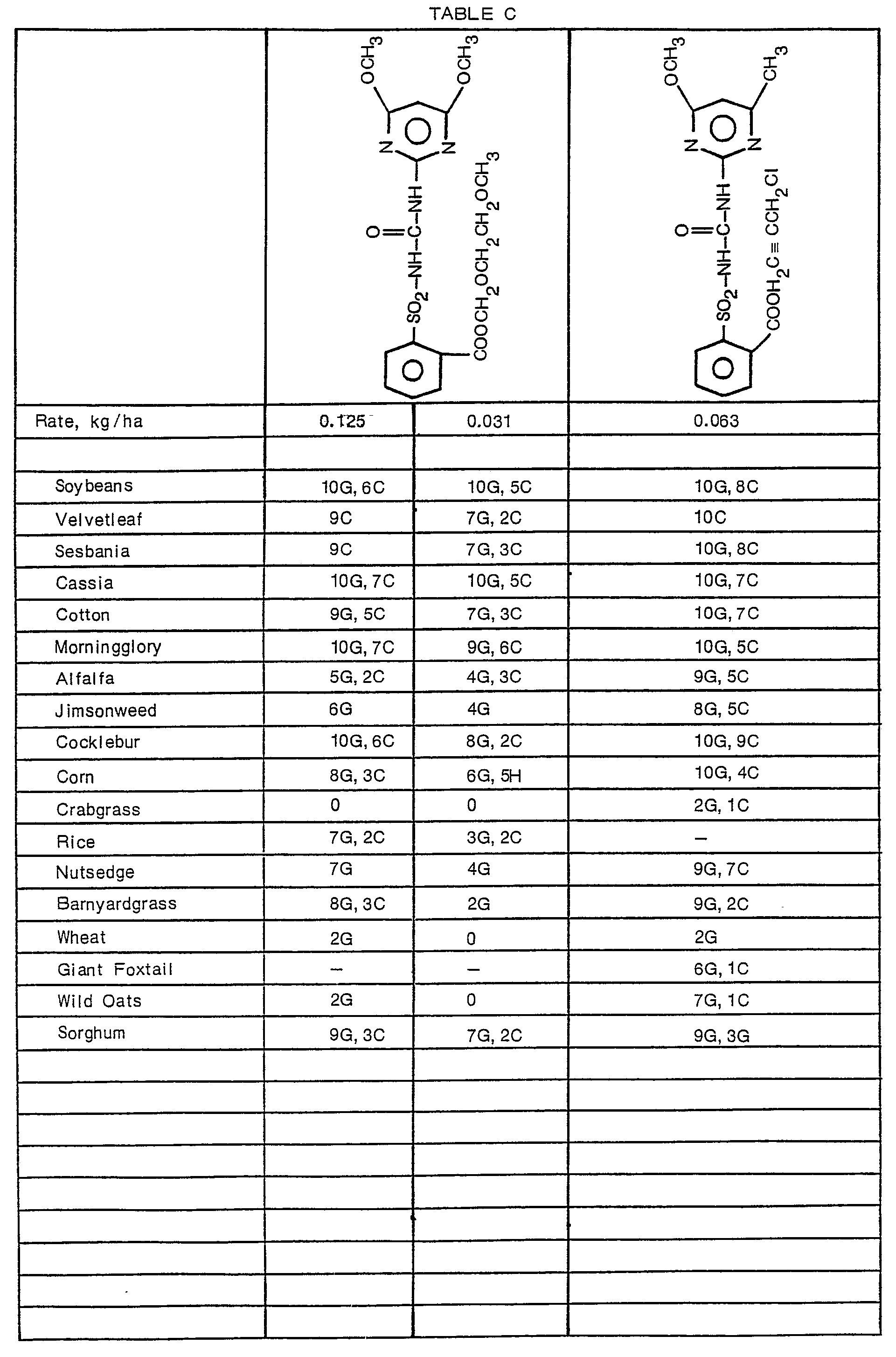

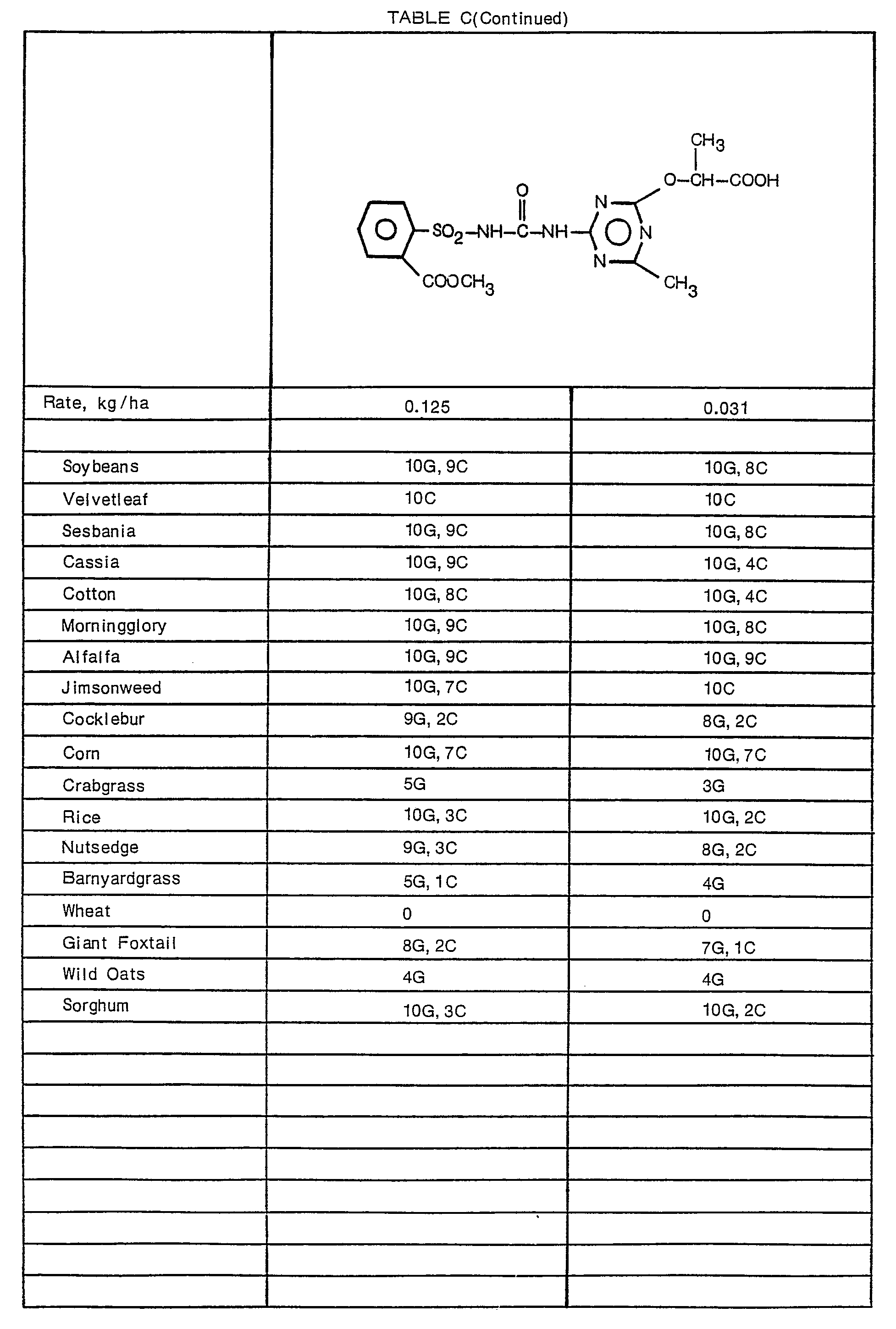

### Test D

Two ten-inch in diameter plastic pans lined with polyethylene liners were filled with prepared Fallsington silt loam soil. One pan was planted with seeds of wheat (Triticum aestivum), barley (Hordeum vulgare), wild oats (Avena fatua), downy brome (Bromus tectorum), cheatgrass (Bromus secalinus), blackgrass (Alopecurus myosuroides), annual bluegrass (Poa annua), green foxtail (Setaria viridis), quackgrass (Agropyron repens), Italian ryegrass (Lolium multiflorum) and ripgut brome (Bromus rigidus). The other pan was planted with seeds of Russian thistle (Salsola kali), tansy mustard (Descuraina pinnata), smartweed (Polygonum pennsylvanicum), tumble mustard (Sisymbrium altissium) kochia (Kochia scoparia), shepherd's purse (Capsella bursa-pastoris), Matricaria inodora, black nightshade (Solanum nigrum), yellow rocket (Barbarea vulgaris) wild mustard (Brassica kaber) and wild buckwheat (Polygonum convolvulus). The above pans were treated pre-emergence. At the same time two pans in which the above plant species were growing were treated post-emergence. Plant height at the time of treatment ranged from 1-15 cm depending on plant species.

The compounds applied were diluted with a non-phytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table D. The rating system used was as described for Test A. The data indicate the utility of the test compounds for pre- and post-emergence weed control in crops such as wheat and barley.

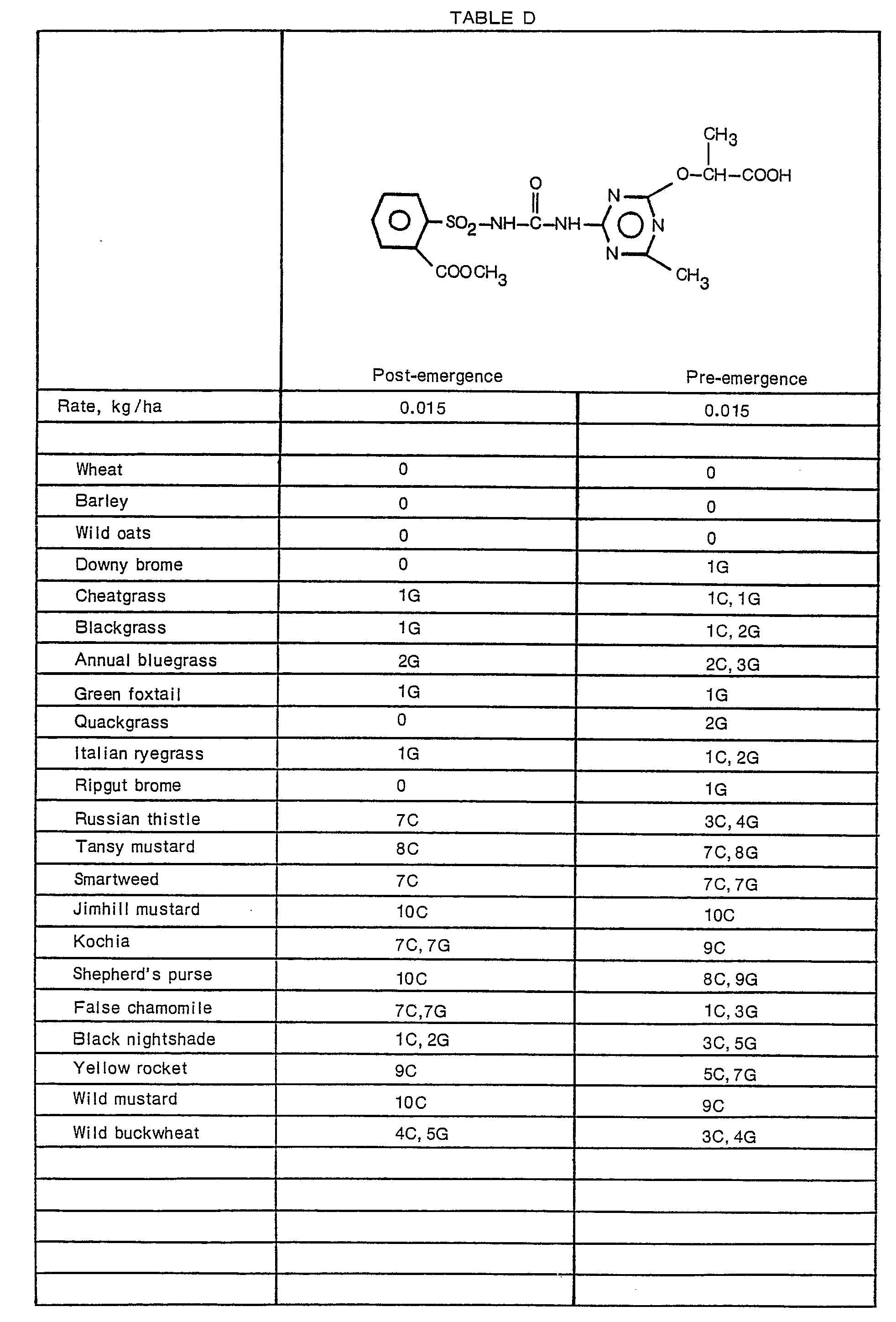

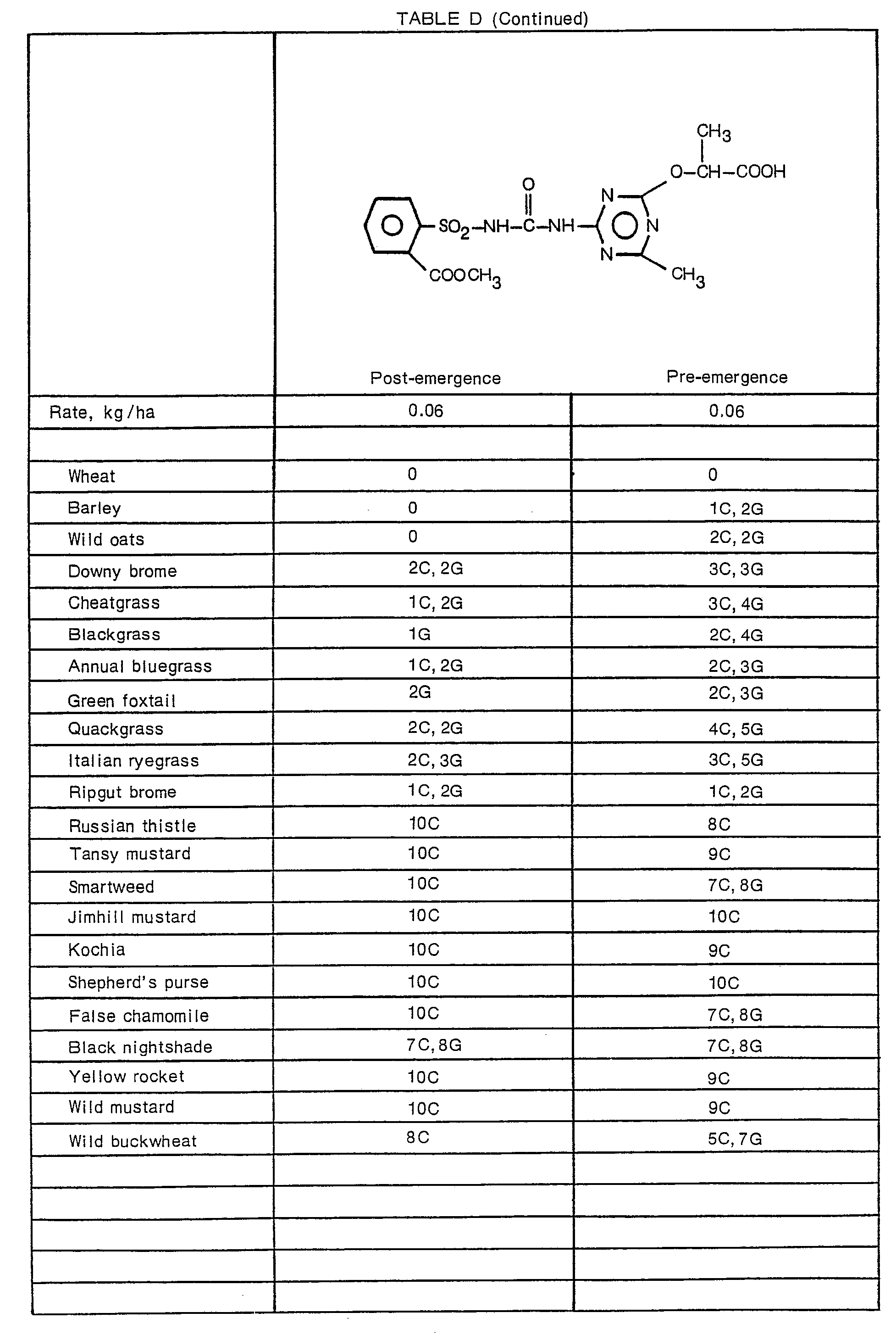

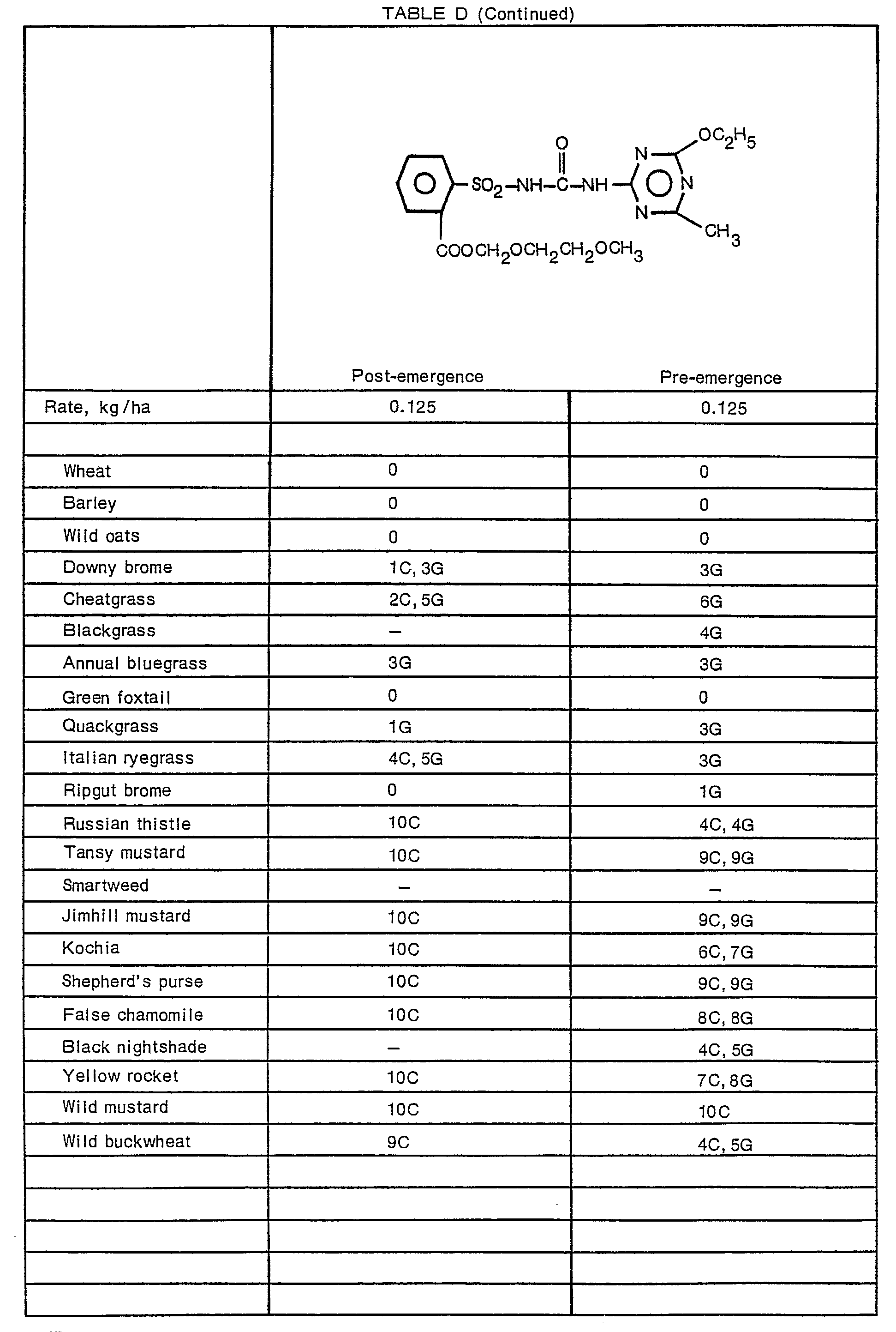

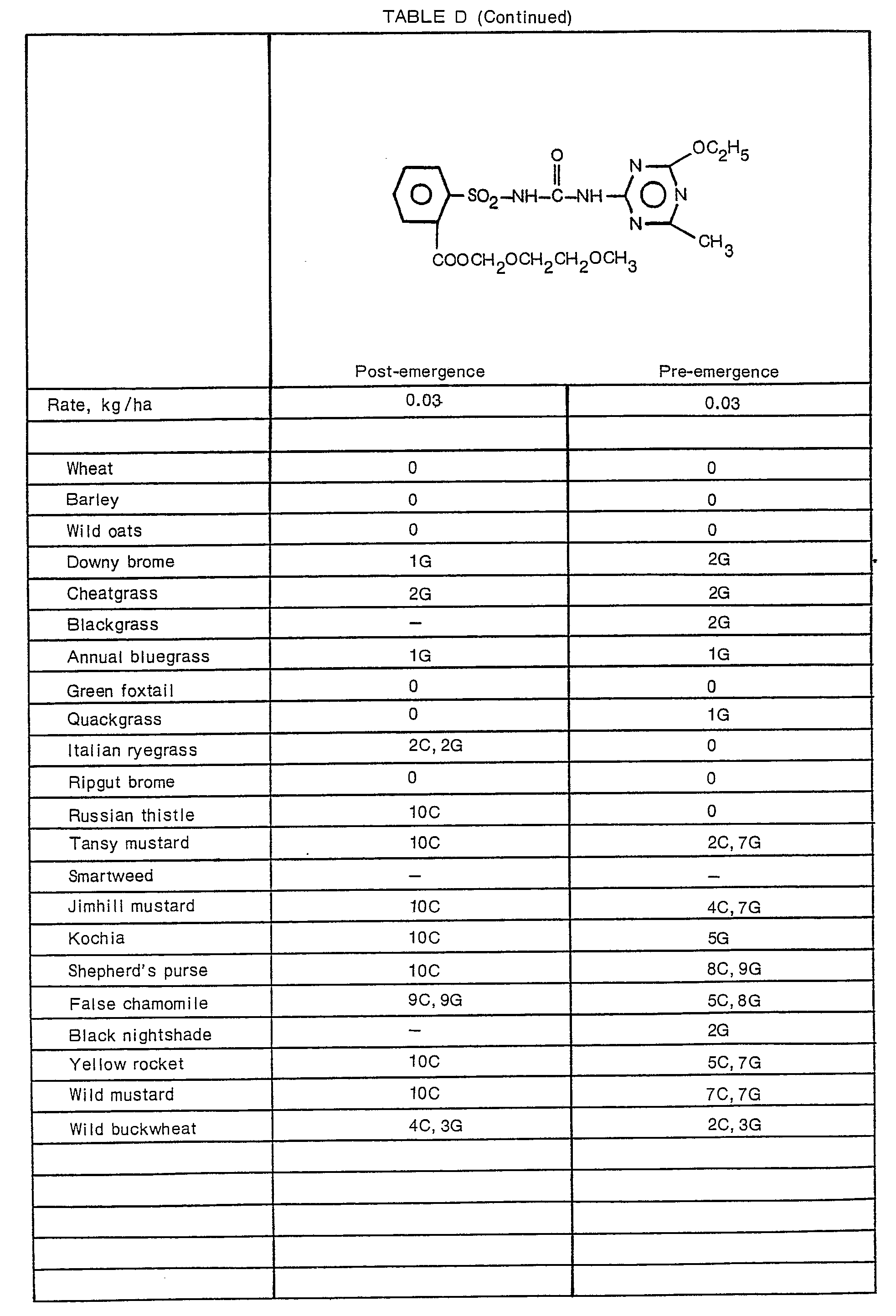

### Test E

Purple nutsedge (Cyperus rotundus) tubers were planted about 2 cm deep in Fallsington silt loam soil contained in 10 cm diameter plastic pots. Five tubers were planted in each pot. Compounds of this invention were dissolved in a non-phytotoxic diluent and sprayed at 560 1/ha in four methods of application: soil surface, tuber/soil, soil incorporated and post-emergence. The soil surface spray consisted of spraying the compound on the surface of the firmed covering soil. The tuber/soil spray consisted of spraying the compound on exposed tubers and subtending soil before adding the untreated covering soil. Soil incorporated treatment consisted in mixing the compound with the covering soil before using it to cover the tubers. The post-emergence treatment was sprayed on nutsedge foliage and the surrounding soil surface when nutsedge had emerged and grown to a height of about 12 cm. Pots receiving the post-emergence treatments were placed directly in the greenhouse. Pots receiving the other treatments were misted with about 0.3 cm water before being transferred to the greenhouse. Response ratings assessed after four weeks are recorded in Table E based on the same rating system as described in procedure A. It may be seen that the test compound has utility for the control of nutsedge.

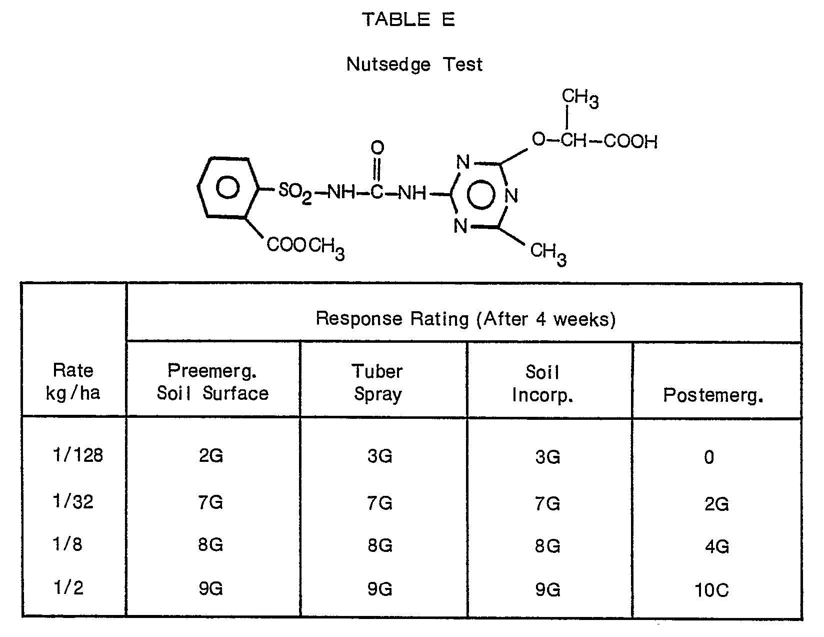

## Claims (Claims for the following Contracting State(s) : s: BE CH DE FR GB IT LI LU NL SE)

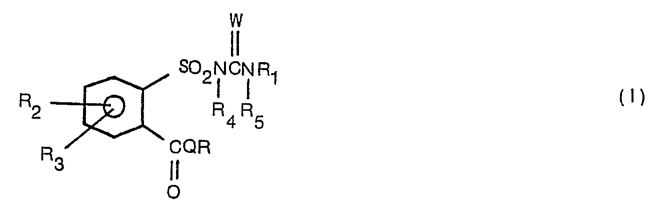

wherein
Q is O, S or 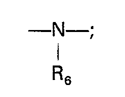 when Q is 0 or S then R is C₁―C₁₂ alkyl; C₃―C₁₀ alkenyl; C₂―C₆ alkyl substituted with one to four substituents selected from 0-3 atoms of F, CI or Br, 0-2 methoxy groups and 0-1 cyano groups; -CH₂CN; ―CH₂CO₂CH₃; ―CH₂CO₂C₂H₅; C₃―C₆ alkenyl substituted with 1-3 atoms of F, CI or Br; C₅―C₈ cycloalkyl; C₅―C₈ cycloalkenyl; C₅―C₆ cycloalkyl substituted with any of one to four methyl groups, OCH₃, alkyl of C₂―C₄, F, CI or Br; C₄―C₁₀ cycloalkylalkyl; C₄―C₈ cycloalkylalkyl with 1-2 CH₃; C₇―C₁₀ bicycloalkyl; C₇―C₁₀ bicycloalkenyl; C₁₀ tricycloalkyl, C₁₀ tricycloalkenyl; 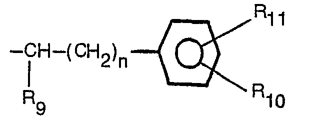 where R₉ is C₁―C₃ alkyl or hydrogen, R₁₀ and R₁₁ are independently hydrogen, C₁―C₃ alkyl, Cl, Br, -OCH₃ or ―OC₂H₅, or R₁₀ and R₁₁ may be taken together to form a 5 or 6 member ring: 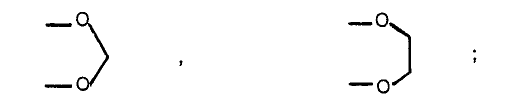 and n is 0, 1, 2 or 3 provided that R has a total number of carbon atoms of less than or equal to 12; 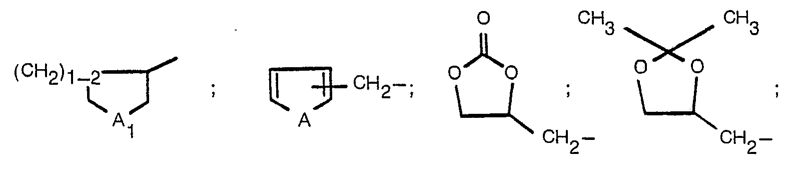 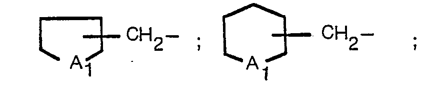 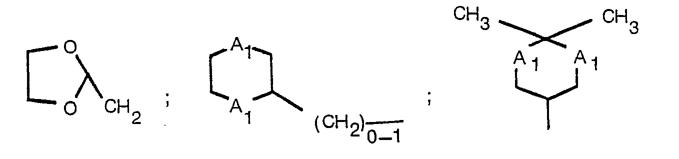
A is 0 or S;
A₁ is 0, S or SO₂; when Q is 0 or S then
1) R may also be C₃―C₁₀ alkynyl; C₃―C₆ alkynyl substituted with F, CI or Br; 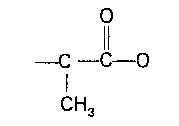 (C₁―C₂ alkyl); or 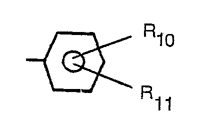 where R₁₀ and R₁₁ are as previously defined; when Q is 0, then R is H, M, ―CH₂CH₂OR₇, ―CH₂CH₂CH₂OR₇, 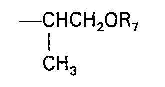 where R₇ is -CH₂CH₃, ―CH(CH₃)₂, phenyl, ―CH₂CH₂Cl or ―CH₂CO₃; (̵CH₂CH₂O)̵ₙ,―R₈ or 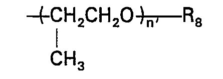 where R₈ is -CH₃, -CH₂CH₃, ―CH(CH₃)₂, phenyl, -CH₂CH₂CI or ―CH₂CCl₃, and n' is 2 or 3; provided that R has a total number of carbon atoms of less than or equal to 13 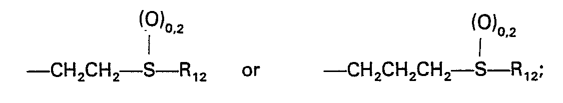 where R₁₂ is -CH₃, -CH₂CH₃, ―CH(CH₃)₂, or phenyl; and when Q is O.
₂) _{R} may also be CH₂OR'₈ where R'₈ is CH₃―, CH_{3C}H₂, (CH₃)₂CH-, ClCH₂CH₂―, Cl₃CCH₂, 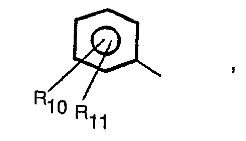 CH₃CH₂OCH₂CH₂ or CH₃OCH₂CH₂; where R₁₀ and R₁₁ are as previously defined; when Q is 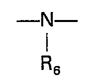 then R is hydrogen; C₁―C₁₂ alkyl; (̵CH₂CH₂O)̵ₙ,―R₁₂, ―CH₂CH₂CH₂OR₁₂ where R₁₂ is as defined above and n"' is 1-3; C₃―C₁₀ alkenyl; C₃―C₈ cycloalkyl; C₅―C₆ cycloalkenyl; C₅―C₈ cycloalkyl substituted with 1 to 3 substituents selected from 0-2 ―OCH₃, 0-3 ―CH₃ and ―C₂H₅; trifluoromethylcyclohexyl; C₄―C₁₀ cycloalkylalkyl; C₄―C₈ cycloalkylalkyl substituted with 1-2 -CH₃; -CH₂CN; -CH₂CH₂CN; 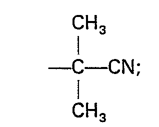 -OCH₃; ―N(CH₃)₂; or 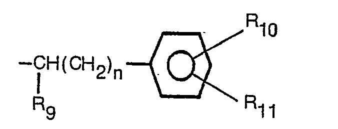 where n, R₉, R₁₀ and R₁₁ are as defined above; 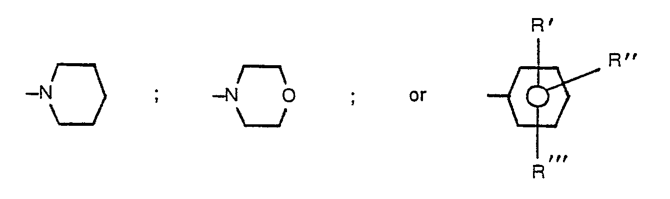 where
R' is hydrogen, C₁―C₄ alkyl, -OCH₃, F, Br, Cl, -CF₃, CN, N0₂, ―SO₂CH₃, -SCH₃ or -N(CH₃)₂;
R" is hydrogen, C₁―C₄ alkyl, -OCH₃, F, Br or Cl;
R"' is hydrogen, -CH₃, Cl, F or Br;
R₆ is hydrogen, C₁―C₆ alkyl, allyl, ―CH₂CN; or ―CH₂CH₂CN; or R₆ and R can be taken together to form ―(CH₂)₄―, ―(CH₂)₅―, ―(CH₂)₆―, ―CH₂CH₂OCH₂CH₂―; or 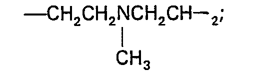 with the proviso that when R is -OCH₃ or OCH₂CH₃ then R₆ is H or -CH₃; when R₆ is ―CH₂CH₂CN or -CH₂CN then R is -CH₂CH₂CN or CH₂CN; and R and R₆ have a total number of carbon atoms of less than or equal to 13; and when Q is 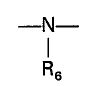
3) R may also be C₃―C₆ alkynyl; 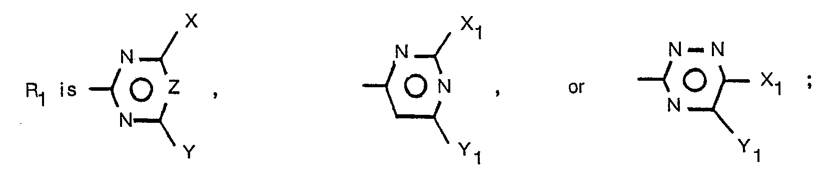
R₂ is H, Cl, Br, F, C₁―C₃ alkyl, -NO₂, ―SO₂CH₃, -OCH₃, -SCH₃, -CF₃, ―N(CH₃)₂, -NH₂, or -CN;
R₃ is H, Cl, Br, F or CH₃;
R₄ is H, or -CH₃;
R₅ is H, -CH₃, or -OCH₃;
M is an alkali metal;
W is oxygen or sulfur;
X is H, Cl, -CH₃, -OCH₃, ―OCH₂CH₃ or ―OCH₂CH₂OCH₃;
Y is H; Cl; C₁―C₄ alkyl; C₁―C₄ alkyl substituted with OCH₃, OC₂H₅, CN, CO₂CH₃, CO₂H₂H₅ or 1―3 atoms of F, CI or Br; C₃―C₄ alkenyl; CH₂C≡CR₁₃; A(CH₂)ₙ,―A₁(C₁―C₃ alkyl); 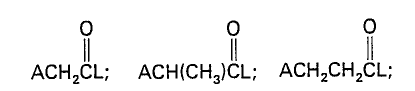 SCN; N₃; NR₁₆R₁₇; OR₁₄ or SR₁₅;
wherein
R₁₃ is H, CH₃ or CH₂Cl;
n' is A and A₁ are as previously defined
L is NH₂, N(OCH₃)CH₃, NH(C₁―C₄ alkyl), N(C₁―C₄ alkyl)₂, or C₁―C₆ alkoxy;
R₁₄ is C₁―C₄ alkyl; C₂―C₄ alkyl substituted with 1-3 atoms of F, Cl or Br; C₁―C₄ alkyl substituted with CN; C₃―C₄ alkenyl; CH₂C=CR₁₃ wherein R₁₃ is as defined above; or 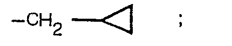
R₁₅ is C₁―C₄ alkyl, cyanomethyl, cyanoethyl allyl or propargyl;
R₁₆ is H or CH₃;
R¹⁷ is H; OCH₃; C₁―C₄ alkyl; C₁―C₄ alkyl substituted with CN, CO₂CH₃ or CO₂C₂H₅; C₃―C₄ alkenyl; or C₂―C₃ alkyl substituted with either OCH₃ or OC₂H₅; or
R₁₆ and R₁₇ may be taken together to form CH₂CH₂CH₂CH₂ or CH₂CH₂OCH₂CH₂
with the proviso that when Y contains 4 or more carbon atoms, then R contains not more than 4 carbon atoms; when X is Cl, then Y is Cl; and when X and Y are both H, then R contains not more than 4 carbon atoms,
4) Y may also be F, Br, 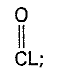 C₁―C₄ alkyl substituted with 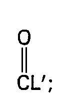 or NR₁₆R₁₇,;
wherein
L is as defined above or OH;
R₁₆ is as defined above;
L' is NH₂, OH, N(OCH₃)CH₃, NH(C₁―C₈ alkyl), N(C₁―C₄ alkyl)₂ or C₃―C₆ alkoxy; and R₁₇, is C₅―C₆ alkyl or C₁―C₄ alkyl substituted with 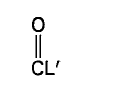 wherein L' is as defined above;
Z is CH or N;
Y₁ is H, -OCH₃ or -CH₃;
5) Y₁ may also be OCH₂CH₃; and
X₁ is H, Cl, -OCH₃, ―OCH₂CH₃ or -CH₃; providing that X₁ and Y₁ are not both simultaneously hydrogen and when R₁ is 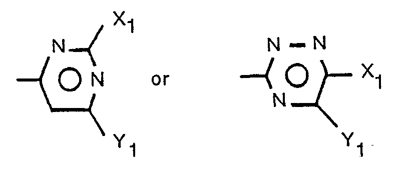 then R₄ and R₅ are both H and R contains 5 or less carbon atoms, and
a) when Y is not as defined in 4), then R must be as defined in 1), 2) or 3;
b) when Y₁ is not as defined in 5), then R must be as defined in 1), 2) or 3; and agriculturally suitable salts thereof.

2. A compound of Claim 1 in which 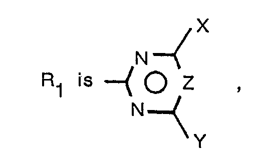
R₄ and R₅ are H; and
W is 0.

3. A compound of Claim 2 in which Q is oxygen.

4. A compound of Claim 3 in which R is C₁―C₆ alkyl, C₃―C₄ alkenyl, CH₂CH₂Cl, CH₂CH₂OCH₃ or CH₂OR'₈, wherein R'₈ is as defined in claim 1.

5. A compound of Claim 4 in which X is CH₃ or OCH₃.

6. A compound of Claim 5 in which Y is CH₃, OCH₃ or

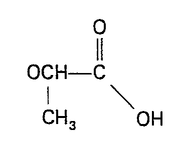

7. A compound of Claim 6 in which R₃ is H

8. A compound of Claim 7 in which R₂ is H.

9. A compound of Claim 8 in which R'₈ is CH₃ or CH₃CH₂ and R is CH₃, CH₂CH₂OCH₃ or CH₂CH₂OC₂H₅.

10. A compound of Claim 1, [(2-methoxyethoxy)methyl] 2-[[(4,6-dimethoxypyrimidin-2-yl)aminocarbonyl]aminosulfonyl]benzoate; and agriculturally suitable salts thereof.

11. A compound of Claim 1, [(2-methoxyethoxy)methyl] 2-[[(4-ethoxy-6-methyl-1,3,5-triazin-2-yl)aminocarbonyl]aminosulfonyl]benzoate; and agriculturally suitable salts thereof.

12. A compound of Claim 1, [(4-chlorophenoxy)methyl] 2-[[(4,6-dimethylpyrimidin-2-yl)aminocarbonyl]aminosulfonyl]benzoate, and agriculturally suitable salts thereof.

13. A compound of claim 1, methyl 2-[[[4-(1-carboxyethoxy)-6-methyl-1,3,5-triazin-2-yl]aminocarbonyl]aminosulfonyl]benzoate; and agriculturally suitable salts thereof.

14. A compound of Claim 1, [(2-methoxyethoxy)methyl] 2-[[(4,6-dimethylpyrimidin-2-yl)aminocarbonyl]aminosulfonyl]benzoate; and agriculturally suitable salts thereof.

15. A compound of Claim 1, (4-chloro-2-butynyl) 2-[[(4-methoxy-6-methylpyrimidin-2-yl)aminocarbonyl]aminosulfonyl]benzoate; and agriculturally suitable salts thereof.

16. A composition suitable for controlling the growth of undesired vegetation comprising an effective amount of a herbicidal compound and at least one of the following: surfactant, solid or liquid diluent, characterised in that said herbicidal compound comprises a compound of any of claims 1 to 15.

17. A method for controlling the growth of undesired vegetation by applying to the locus to be protected an effective amount of a herbicidal compound, characterised in that said herbicidal compound comprises a compound of any of claims 1 to 15.

18. A method for regulating the growth of plants which comprises applying to the locus of said plants an effective but substantially non-phytotoxic amount of a plant growth regulant, characterised in that said plant growth regulant comprises a compound of any of claims 1 to 15.

19. A method for preparing a compound of claim 1 which comprises
(a) reacting a compound of formula 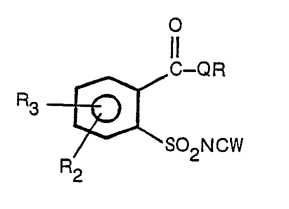 wherein
Q is 0
R is C₁―C₁₂ alkyl; C₃―C₁₀ alkenyl; C₂―C₆ alkyl substituted with one to four substituents selected from 0-3 atoms of F, CI or Br, 0-2 methoxy groups; C₃―C₆ alkenyl substituted with 1-3 atoms of F, CI or Br; C₅―C₈ cycloalkyl; C₅―C₈ cycloalkenyl; C₅―C₆ cycloalkyl substituted with any of one to four methyl groups, methoxy, alkyl of C_{2―4}, F, CI or Br; C₄―C₁₀ cycloalkylalkyl; C₄―C₈ cycloalkylalkyl with 1-2 CH₃; ―CH₂CH₂OR₇; CH₂CH₂CH₂OR₇; 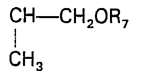 where R₇ is ―CH₂CH₃, CH(CH₃)₂, phenyl, ―CH₂CH₂Cl or ―CH₂CCl₃; (̵-CH₂CH₂O)̵ₙ―R₈ or 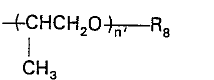 where R₈ is CH₃, ―CH₂CH₃ -CH(CH₃)₂, phenyl, ―CH₂CH₂Cl or -CH₂CCl₃, and n' is 2 or 3;
R₂ is H, Cl, Br, F, C,-C₃ alkyl, -N0₂, -OCH₃, -SCH₃, CF₃, SO₂CH₃, N(CH₃)₂ or CN;
R₃ is H, Cl, Br or CH₃
and W is 0 or S; with an appropriate amino pyrimidine or aminotriazine of formula 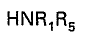 wherein R₁ and R₅ are as defined in claim 1;
(b) reacting a compound of formula 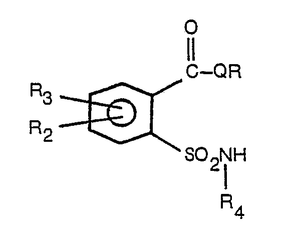 wherein Q, R₂, R₃ and R₄ are as defined in claim 1, and R is as defined in claim 1 other than H or M, with an appropriate triazine or pyrimidine isothiocyanate 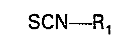 wherein R₁ is as defined in claim 1;
(c) methylating a compound of claim 1 wherein R₄ is H, Q is 0, S or 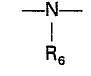 R is as defined in (a) above and W is 0 to obtain a compound of claim 1 wherein R₄ is methyl;
(d) reacting a carbamoyl or thiocarbamoyl chloride of formula 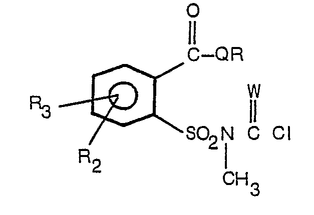 wherein Q and R as as defined in (a) above and W is 0 or S, with the amine HNR₁R₅ defined in (a) above; or
(e) hydrolyzing an ester of claim 1 wherein R is C_{1―12} alkyl to obtain a compound of claim 1 wherein R is H or esterifying a compound of claim 1 wherein R is H to obtain a compound of claim 1 wherein R is other than H.

## Claims (Claims for the following Contracting State(s) : AT)

1. A process for the preparation of a compound, selected from 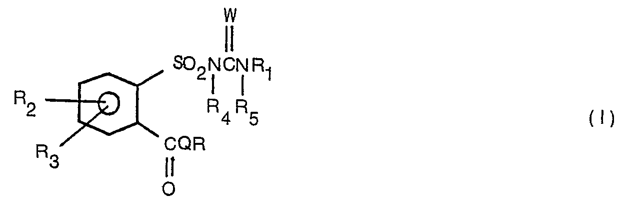 wherein
Q is 0, S or 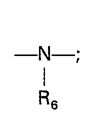 when Q is 0 or S then R is C₁―C₁₂ alkyl; C₃―C₁₀ alkenyl; C₂―C₆ alkyl substituted with one to four substituents selected from 0-3 atoms of F, CI or Br, 0-2 methoxy groups and 0-1 cyano groups; -CH₂CN; ―CH₂CO₂CH₃; ―CH₂CO₂C₂H₅; C₃―C₆ alkenyl substituted with 1-3 atoms of F, CI or Br; C₅―C₈ cycloalkyl; C₅―C₈ cycloalkenyl; C₅―C₆ cycloalkyl substituted with any of one to four methyl groups, OCH₃, alkyl of C₂―C₄, F, CI or Br; C₄―C₁₀ cycloalkylalkyl; C₄―C₈ cycloalkylalkyl with 1-2 CH₃; C₇―C₁₀ bicycloalkyl; C₇―C₁₀ bicycloalkenyl; C₁₀ tricycloalkyl, C₁₀ tricycloalkenyl; 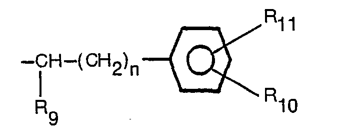 where R₉ is C₁―C₃ alkyl or hydrogen, R₁₀ and R₁₁ are independently hydrogen, C,-C₃ alkyl, Cl, Br, -OCH₃ or ―OC₂H₅, or R₁₀ and R₁₁ may be taken together to form a 5 or 6 member ring: 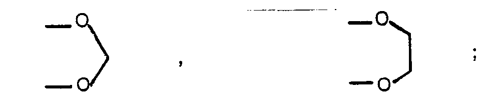 and n is 0, 1, 2 or 3 provided that R has a total number of carbon atoms of less than or equal to 12; 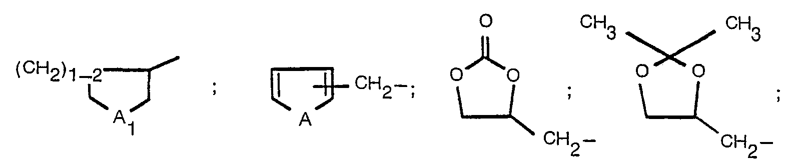 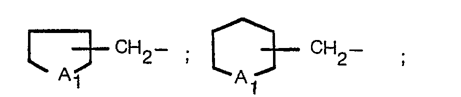 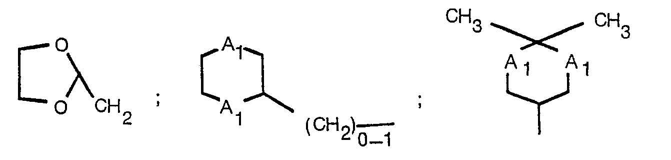
A is 0 or S;
A₁ is 0, S or SO₂; when Q is 0 or S then
1) R may also be C₃―C₁₀ alkynyl; C₃―C₆ alkynyl substituted with F, CI or Br; 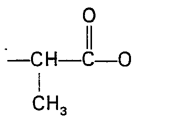 (C₁―C₂ alkyl); or 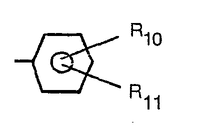 where R₁₀ and R₁₁ are as previously defined; when Q is 0, then R is H, M, ―CH₂CH₂OR₇, ―CH₂CH₂CH₂OR₇, 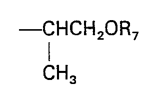 where R₇, is -CH₂CH₃, ―CH(CH₃)₂, phenyl, ―CH₂CH₂Cl or ―CH₂CCl₃; (̵CH₂CH₂O)̵ₙ―R₈ or 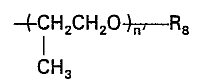 where R₈ is -CH₃, -CH₂CH₃, ―CH(CH₃)₂, phenyl, ―CH₂CH₂Cl or ―CH₂CCl₃, and n' is 2 or 3; provided that R has a total number of carbon atoms of less than or equal to 13 carbon atoms; 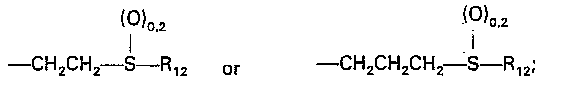 where R₁₂ is -CH₃, -CH₂CH₃, ―CH(CH₃)₂ or phenyl; and when Q is O.
2) R may also be CH₂OR'₈ where R'₈ is CH₃― CH₃CH₂, (CH₃)₂CH― ClCH₂CH₂―, CI₃CCH₂, 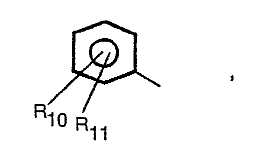 CH₃CH₂OCH₂CH₂ or CH₃OCH₂CH₂; where R₁₀ and R₁₁ are as previously defined; when Q is 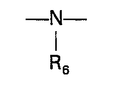 then R is hydrogen; C₁―C₁₂ alkyl; (̵CH₂CH₂O)̵ₙ―R₁₂, ―CH₂CH₂CH₂OR₁₂ where R₁₂ is as defined above and n''' is 1-3; C₃―C₁₀ alkehyl; C₃―C₈ cycloalkyl; C₅―C₆ cycloalkenyl; C₅―C₈ cycloalkyl substituted with 1 to 3 substituents selected from 0-2 -OCH₃, 0-3 ―CH₃ and ―C₂H₅; trifluoromethylcyclohexyl; C₄―C₁₀ cycloalkylalkyl; C₄―C₈ cycloalkylalkyl substituted with 1-2 -CH₃; -CH₂CN; -CH₂^{CH}₂^{CN}; 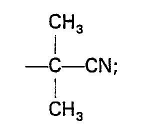 -OCH₃; ―N(CH₃)₂; 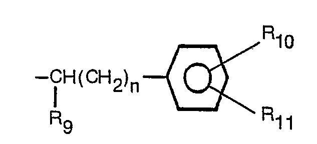 where n, R₉, R₁₀ and R₁₁ are as defined above; 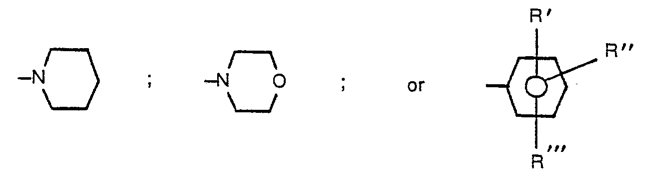 where
R' is hydrogen, C₁―C₄ alkyl, -OCH₃, F, Br, Cl, -CF₃, CN, N0₂, ―SO₂CH₃, -SCH₃ or -N(CH₃)₂;
R" is hydrogen, C₁―C₄ alkyl, -OCH₃, F, Br or Cl;
R''' is hydrogen, -CH₃, Cl, F or Br;
R₆ is hydrogen, C₁―C₆ alkyl, allyl, -CH₂CN; or ―CH₂CH₂CN; or R₆ and R can be taken together to form ―(CH₂)₄―, ―(CH₂)₅―, ―(CH₂)₆―, ―CH₂CH₂OCH₂CH₂―; or 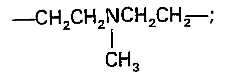 with the proviso that when R is -OCH₃ or OCH₂CH₃ then R₆ is H or -CH₃; when R₆ is -CH₂CH₂CN or -CH₂CN then R is -CH₂CH₂CN or CH₂CN; and R and R₆ have a total number of carbon atoms of less than or equal to 13; and when Q is 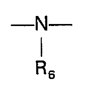
3) R may also be C₃―C₆ alkynyl; 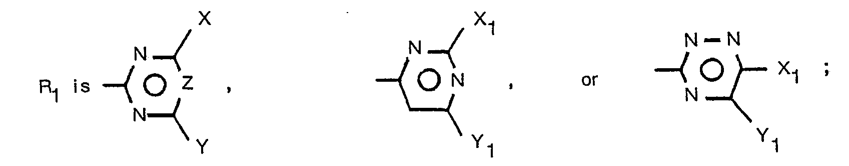
R₂ is H, Cl, Br, F, C₁―C₃ alkyl, ―NO₂, ―SO₂CH₃, -OCH₃, -SCH₃, -CF₃, ―N(CH₃)₂, -NH₂, or -CN;
R₃ is H, Cl, Br, F or CH₃;
R₄ is H, or -CH₃;
R₅ is H, -CH₃, or -OCH₃;
M is an alkali metal;
W is oxygen or sulfur;
X is H, Cl, -CH₃, -OCH₃, ―OCH₂CH₃ or ―OCH₂CH₂OCH₃;
Y is H; CI; C₁―C₄ alkyl; C₁―C₄ alkyl substituted with OCH₃, OC₂H₅, CN, CO₂CH₃, CO₂H₅ or 1-3 atoms of F, CI or Br; C₃―C₄ alkenyl; CH₂C≡CR₁₃; A(CH₂)ₙ,―A₁(C₁―C₃ alkyl); 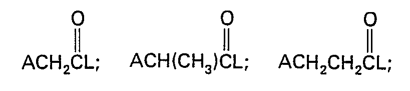 SCN; N₃; NR₁₆R₁₇; OR₁₄ or SR₁₅;
wherein
R₁₃ is H, CH₃ or CH₂Cl;
n' is A and A₁ are as previously defined
L is NH₂, N(OCH₃)CH₃, NH(C₁―C₄ alkyl), N(C₁―C₄ alkyl)₂, or C₁―C₆ alkoxy;
R₁₄ is C₁―C₄ alkyl; C₂―C₄ alkyl substituted with 1-3 atoms of F, CI or Br; C₁―C₄ alkyl substituted with CN; C₃―C₄ alkenyl; CH₂C=CR₁,₃; or 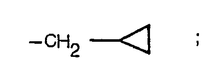
R₅ is C₁―C₄ alkyl, cyanomethyl, cyanoethyl allyl or propargyl;
R₁₆ is H or CH₃;
R¹⁷ is H; OCH₃; C₁―C₄ alkyl; C₁―C₄ alkyl substituted with CN, CO₂CH₃ or CO₂C₂H₅; C₃―C₄ alkenyl; or C₂―C₃ alkyl substituted with either OCH₃ or OC₂H₅; or
R₁₆ and R₁₇ may be taken together to form CH₂CH₂CH₂CH₂ or CH₂CH₂OCH₂CH₂
with the proviso that when Y contains 4 or more carbon atoms, then R contains not more than 4 carbon atoms; when X is Cl, then Y is CI; and when X and Y are both H, then R contains not more than 4 carbon atoms,
4) Y may also be F, Br, 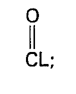 C₁―C₄ alkyl substituted with 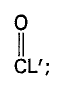 or NR₁₆R₁₇,;
wherein
L is as defined above or OH;
R₁₆ is as defined above;
L' is NH₂, OH, N(OCH₃)CH₃, NH(C₁―C₄ alkyl), N(C₁―C₄ alkyl)₂ or C₃―C₆ alkoxy; and
R₁₇, is C₅―C₆ alkyl or C₁―C₄ alkyl substituted with 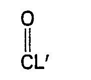 wherein L' is as defined above;
Z is CH or N;
Y₁ is H, -OCH₃ or -CH₃;
5) Y₁ may also be OCH₂CH₃; and
X, is H, Cl, -OCH₃, -OCH₂CH₃ or -CH₃; providing that X₁ and Y₁ are not both simultaneously hydrogen and when R₁ is 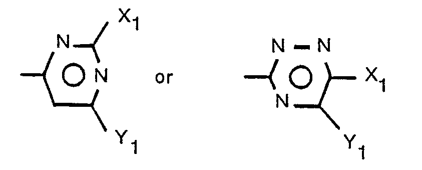 then R₄ and R₅ are both H and R contains 5 or less carbon atoms, and
a) when Y is not as defined in 4), then R must be as defined in 1), 2) or 3;
b) when Y₁ is not as defined in 5), then R must be as defined in 1), 2) or 3; or an agriculturally suitable salt thereof; which comprises
(a) reacting a compound of formula 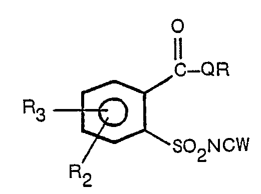 wherein
Q is 0
R is C₁―C₁₂ alkyl; C₃―C₁₀ alkenyl; C₂―C₆ alkyl substituted with one to four substituents selected from 0-3 atoms of F, Cl or Br, 0-2 methoxy groups; C₃―C₆ alkenyl substituted with 1-3 atoms of F, CI or Br; C₅―C₈ cycloalkyl; C₅―C₈ cycloalkenyl; C₈―C₆ cycloalkyl substituted with any of one to four methyl groups, methoxy, alkyl of C_{2-4'} F, CI or Br; C₄―C₁₀ cycloalkylalkyl; C₄―C₈, cycloalkylalkyl with 1-2 CH₃; ―CH₂CH₂OR₇; CH₂CH₂CH₂OR₇; 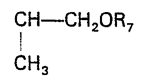 where R₇ is -CH₂CH₃, CH(CH₃)₂, phenyl, ―CH₂CH₂Cl or ―CH₂CCl₃; (̵CH₂CH₂O)̵ₙ―R₈ or 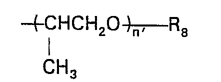 where R₈ is CH₃, ―CH₂CH₃ -CH(CH₃)₂, phenyl, ―CH₂CH₂Cl or ―CH₂CCl₃ and n' is 2 or 3;
R₂ is H, Cl, Br, F, C₁―C₃ alkyl, -N0₂, -OCH₃, -SCH₃, CF₃, SO₂CH₃, N(CH₃)₂ or CN;
R₃ is H, Cl, Br or CH₃
and W is 0 or S; with an appropriate amino pyrimidine or aminotriazine of formula 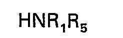 wherein R₁ and R₅ are as defined above;
(b) reacting a compound of formula 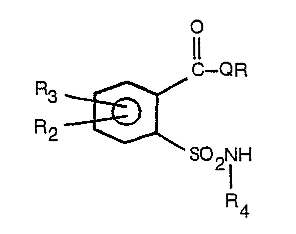 wherein Q, R₂, R₃ and R₄ are as defined for formula (I), and R is as defined for formula (I) other than H or M, with an appropriate triazine or pyrimidine isothiocyanate 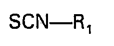 wherein R₁ is as defined for formula (I);
(c) methylating a compound of formula (I) wherein R₄ is H, Q is 0, S or 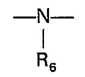 R is as defined in (a) above and W is O to obtain a compound of formula (I) wherein R₄ is methyl;
(d) reacting a carbamoyl or thiocarbamoyl chloride of formula 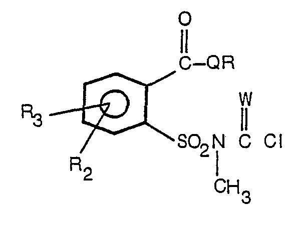 wherein Q and R as as defined in (a) above and W is 0 or S, with the amine HNR₁R₅ defined in (a) above; or
(e) hydrolyzing an ester of formula (I) wherein R is C₁―C₁₂ alkyl to obtain a compound of formula (I) wherein R is H, or esterifying a compound of formula (I) wherein R is H to obtain a compound of formula (I) wherein R is other than H.

2. A process of Claim 1 in which 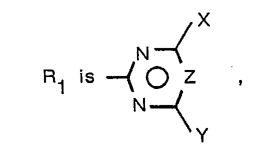
R₄ and R₅ are H; and
W is O.

3. A process of Claim 2 in which Q is oxygen.

4. A process of Claim 3 in which R is C₁―C₆ alkyl, C₃―C₄ alkenyl, CH₂CH₂Cl, CH₂CH₂OCH₃ or CH₂OR'₈, wherein R'₈ is as defined in claim 1.

5. A process of Claim 4 in which X is CH₃ or OCH₃.

6. A process of Claim 5 in which Y is CH₃, OCH₃ or

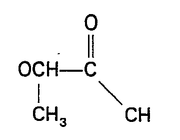

7. A process of Claim 6 in which R₃ is H.

8. A process of Claim 7 in which R₂ is H.

9. A process of Claim 8 in which R'₈ is CH₃ or CH₃CH₂ and R is CH₃, CH₂CH₂OCH₃ or CH₂CH₂CO₂H₅.

10. A process of Claim 1 wherein the product is a compound selected from [(2-methoxyethoxy)methyl] 2-[[(4,6-dimethoxypyrimidin-2-yl)aminocarbonyl]aminosulfonyl]benzoate;
[(2-methoxyethoxy)methyl] 2-[[(4-ethoxy-6-methyl-1,3,5-triazin-2-yl)aminocarbonyl]aminosulfonyl]-benzoate;
[(4-chlorophenoxy)methyl] 2-[[(4,6-dimethylpyrimidin-2-yl)aminocarbonyl]aminosulfonyl]benzoate;
methyl 2-[[[4-( 1-carboxyethoxy)-6-methyl-1,3,5-triazin-2-yl]aminocarbonyl]aminosulfonyl]benzoate;
[(2-methoxyethoxy)methyll 2-[[(4,6-dimethylpyrimidin-2-yl)aminocarbonyl]aminosulfonyl]benzoate;
(4-chloro-2-butynyl) 2-[[(4-methoxy-6-methylpyrimidin-2-yl)aminocarbonyl]aminosulfonyl]benzoate;
or an agriculturally suitable salt of any of these.

11. A method for controlling the growth of undesired vegetation by applying to the locus to be protected an effective amount of a herbicidal compound, characterised in that said herbicidal compound comprises a compound of formula (I) as defined in any of Claims 1 to 9.

12. The method of claim 11 wherein said herbicidal compound comprises a compound as defined in Claim10.

13. A method for regulating the growth of plants which comprises applying to the locus of said plants an effective but substantially non-phytotoxic amount of a plant growth regulant, characterised in that said plant growth regulator comprises a compound of formula (I) as defined in any of Claims 1 to 10.

## Patentansprüche (Patentansprüche für folgende(n) Vertragsstaat(en) : BE CH DE FR GB IT LI LU NL SE)

1. Verbindung ausgewählt aus 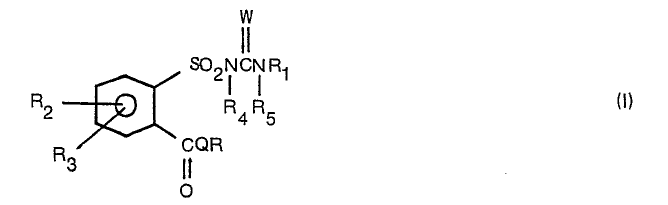 worin
Q 0, S oder 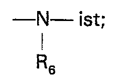 wenn Q 0 oder S ist, dann ist R C₁―C₁₂-Alkyl; C₃―C₁₀-Alkenyl; C₂―C₆-Alkyl substituiert mit einem bis vier Substituenten ausgewählt aus 0-3 Atomen von F, CI oder Br, 0-2 Methoxygruppen und 0-1 Cyanogruppen, ―CH₂CN; -CH₂CO₂CH₃; ―CH₂CO₂C₂H₅; C₃―C₈-Alkenyl substituiert mit 1-3 Atomen von F, CI oder Br; C₅―C₈-Cycloalkyl; C₅―C₈-Cycloalkenyl; C₅―C₆-Cycloalkyl substituiert mit beliebigen von eins bis vier Methylgruppen, OCH₃, Alkyl mit C₂―C₄, F, CI oder Br; C₄―C₁₀-Cycloalkylalkyl; C₄―C₈-Cycloalkylalkyl mit 1-2 CH₃; C₇―C₁₀-Bicycloalkyl; C₇―C₁₀-Bicycloalkenyl; C₁₀-Tricycloalkyl, C₁₀-Tricycloalkenyl; 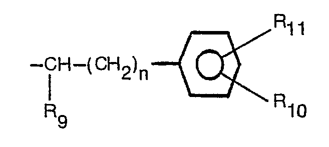 worin R₉ C₁―C₃-Alkyl oder Wasserstoff ist, R₁₀ und R₁₁ unabhängig Wasserstoff, C₁―C₃-Alkyl, Cl, Br, -OCH₃ oder ―OC₂H₅ sind, oder R₁₀ und R₁₁ zusammengenommen einen 5- oder 6-gliedrigen Ring bilden können; 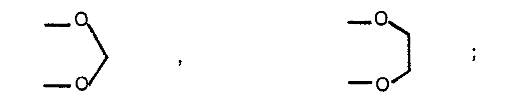 und n 0, 1, 2 oder 3 ist, vorausgesetzt, das R eine Kohlenstoffatomgesamtanzahl von weniger als oder gleich 12 aufweist; 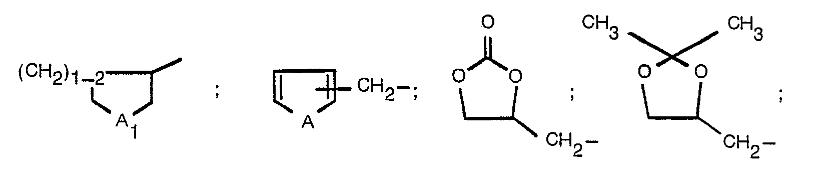 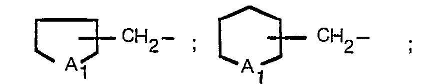 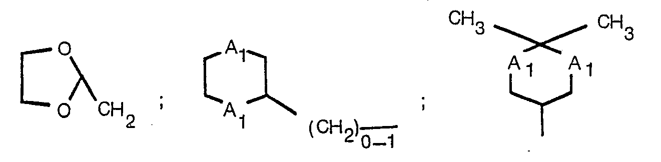
A 0 oder S ist;
A₁ O, S oder SO₂ ist; wenn Q 0 oder S ist, dann
1) kann R auch sein: C₃―C₁₀-Alkinyl; C₃―C₆-Alkinyl substituiert mit F, CI oder Br; 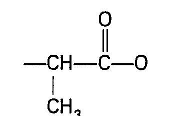 (C₁―C₂-Alkyl); oder 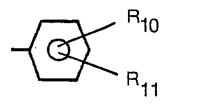 worin R₁₀ und R₁₁ wie vorstehend definiert sind; wenn Q 0 ist, dann ist R H, M, -CH₂CH₂OR₇, ―CH₂CH₂CH₂OR₇, 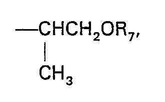 worin R₇ die Bedeutung hat von ―CH₂CH₃, ―CH(CH₃)₂, Phenyl, ―CH₂CH₂Cl oder ―CH₂CCl; (̵-CH₂CH₂O)̵ₙ―R₈ oder 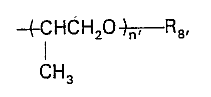 worin R₈ -CH₃, ―CH₃CH₃, ―CH(CH₃)₂, Phenyl, ―CH₂CH₂Cl oder ―CH₂CCl₃ ist, und n' 2 oder 3 ist; vorausgesetzt, daß R eine Kohlenstoffatomgesamtanzahl von weniger als oder gleich 13 aufweist; 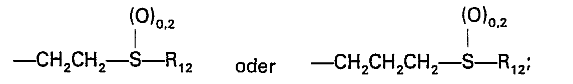 worin R₁₂ -CH₃, ―CH₂CH₃, ―CH(CH₃)₂, oder Phenyl ist; und worin Q 0 ist;
2) kann R auch sein: CH₂OR'₈, worin R'₈ CH₃― CH₃CH₂, (CH₃)₂CH―, ClCH₂CH₂―, Cl₃CCH₂, 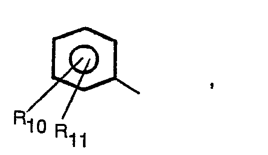 CH₃CH₂OCH₂CH₂ oder CH₃OCH₂CH₂ ist;
worin R₁₀ und R₁₁ wie vorstehend definiert sind; wenn Q 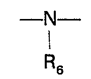 ist, dann ist R Wasserstoff; C₁―C₁₂-Alkyl; (̵CH₂CH₂O)̵ₙ―R₁₂, ―CH₂CH₂CH₂OR₁₂, worin R₁₂ wie vorstehend definiert ist und n''' 1-3 ist; C₃―C₁₀-Alkenyl; C₃―C₈-Cycloalkyl; C₅―C₆-Cycloalkenyl: C₅―C₈-Cycloalkyl substituiert mit 1 bis 3 Substituenten ausgewählt aus 0-2 -OCH₃, 0-3 -CH₃ und ―C₂H₅; Trifluormethylcyclohexyl; C₄―C₁₀-Cycloalkylalkyl; C₄―C₈-Cycloalkylkalkyl substituiert mit 1-2 -CH₃; ―CH₂CN; -CH₂CH₂CN; 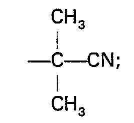 -OCH₃; -N(CH₃)₂; oder 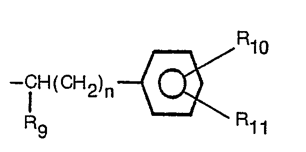 worin n, Rg, R₁₀ und R₁₁ wie vorstehend definiert sind; 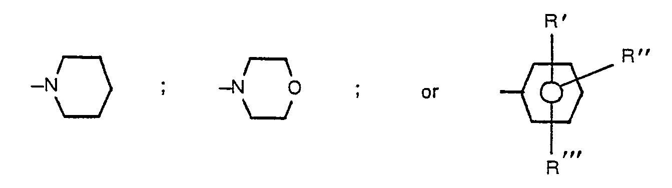 worin
R' Wasserstoff, C₁―C₄-Alkyl, -OCH₃, F, Br, Cl, -CF₃, CN, NO₂, ―SO₂CH₃, -SCH₃ oder ―N(CH₃)₂ ist; R" Wasserstoff, C₁―C₄-Alkyl, -OCH₃, F, Br, oder CI ist;
R''' Wasserstoff, -CH₃, Cl, F oder Br ist;
R₆ Wasserstoff, C₁―C₆-Alkyl, Allyl, ―CH₂₁CN; oder ―CH₂CH₂CN ist; oder R₆ und R zusammengenommen bilden können: ―(CH₂)₄―, ―(CH₂)₅―,―(CH₂)₆―, ―CH₂CH₂OCH₂CH₂―: oder 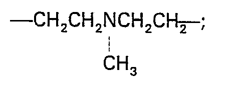 mit der Maßgabe, daß wenn R ―OCH₃ oder OCH₂CH₃ ist, dann R₆ H oder -CH₃ ist; wenn R₆ -CH₂CH₂CN oder -CH₂CN ist, dann R -CH₂CH₂CN oder CH₂CN ist; und R und R₆ eine Gesamtkohlenstoffatomanzahl von weniger als oder gleich 13 aufweisen; und wenn Q 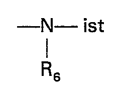
3) R₃ auch sein kann: C₃―C₆-Alkinyl; 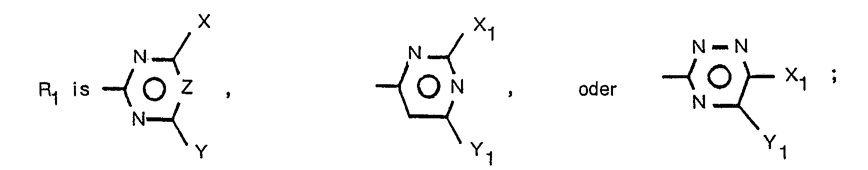
R₂ ist H, Cl, Br, F, C₁―C₃-Alkyl, ―NO₂, ―SO₂CH₃, -OCH₃, ―SCH₃, -CF₃, ―N(CH₃)₂ ―NH₂, oder -CN;
R₃ ist H, CI, Br, F oder CH₃;
R₄ ist H oder -CH₃;
R₅ ist H, -CH₃ oder -OCH₃;
M ist ein Alkalimetall;
W ist Sauerstoff oder Schwefel;
X ist H, CI, -CH₃, ―OCH₃, ―OCH₃CH₃ oder ―OCH₂CH₂OCH₃;
Y ist H; Cl; C₁―C₄-Alkyl; C₁―C₄-Alkyl substituiert mit OCH₃, OC₂H₅, CN, C0₂CH₃, CO₂C₂H₅ oder 1-3 Atomen von F, CI, oder Br; C₃―C₄-Alkenyl; CH₂C≡CR₁₃; A(CH₂)ₙ,―A₁(C₁―C₃-Alkyl); 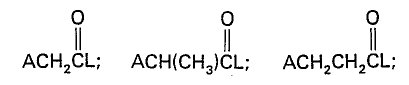 SCN; N₃; NR₁₆R₁₇; OR₁₄ oder SR₁₅;
worin
R₁₃ H, CH₃ oder CH₂Cl ist;
n', A und A₁ wie vorstehend definiert sind;
L NH₂, N(OCH₃)CH₃, NH(C₁―C₄-Alkyl), N(C₁―C₄-Alkyl)₂ oder C₁―C₆-Alkoxy ist;
R₁₄ C₁C₄-Alkyl; C₂―C₄-alkyl substituiert mit 1-3 Atomen von F, CI oder Br; C₁―C₄-Alkyl substituiert mit CN; C₃-C₄₋Alkenyl; CH₂C=CR₁₃, worin R₁₃ wie vorstehend definiert ist, 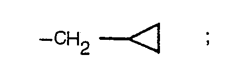 oder ist;
R₁₅ C₁―C₄-Alkyl, Cyanomethyl, Cyanoethyl, Alkyl oder Propargyl ist;
R₁₆ H oder CH₃ ist;
R₁₇ H; OCH₃; C₁―C₄-Alkyl; C₁―C₄-Allyl substituiert mit CN, CO₂CH₃ oder CO₂C₂H₅; C₃―C₄-Alkenyl; oder C₂―C₃-Alkyl substituiert mit entweder OCH₃ oder OC₂H₅; ist oder
R₁₆ und R₁₇ zusammengenommen bilden können CH₂CH₂CH₂CH₂ oder CH₂CH₂OCH₂CH₂, mit der Maßgabe, daß wenn Y 4 oder mehr Kohlenstoffatome enthält, dann R nicht mehr als 4 Kohlenstoffatome enthält; wenn X CI ist, dann Y CI ist; und wenn X und Y beide H sind, dann R nicht mehr als 4 Kohlenstoffatome enthält,
4) Y auch F, Br, 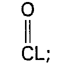 C₁―C₄-Alkyl substituiert mit 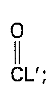 oder NR₁₆R₁₇' sein kann; worin L wie vorstehend definiert oder OH ist;
R₁₆ wie vorstehend definiert ist;
L' NH₂, OH, N(OCH₃)CH₃, NH(C₁―C₄-Alkyl), N(C₁―C₄-Alkyl)₂ oder C₃―C₆-Alkoxy ist; und
R₁₇' C₅―C₆-Alkyl oder C₁―C₄-Alkyl substituiert mit 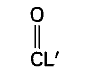 ist, worin L' wie vorstehend definiert ist;
Z CH oder N ist;
Y₁ H, -OCH₃ oder ―CH₃ ist;
5) Y₁ auch OCH₂CH₃ sein kann; und
X₁ H, CI, -OCH₃, ―OCH₂CH₃ oder -CH₃ ist;
vorausgesetzt, daß X₁ und Y₁ nicht beide gleichzeitig Wasserstoff sind, und wenn R₁ 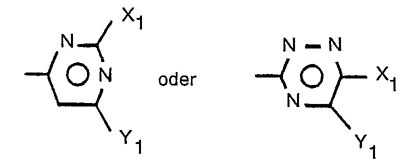 ist, dann R₄ und R₅ beide H sind und R 5 oder weniger Kohlenstoffatome enthält und
a) wenn Y nicht wie in 4) definiert ist, dann R wie in 1), 2) oder 3) definiert sein muß;
b) wenn Y₁ nicht wie in 5) definiert ist, dann R wie in 1), 2) oder 3) definiert sein muß; und landwirtschaftlich brauchbare Salze davon.

2. Verbindung nach Anspruch 1, worin 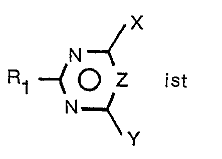
R₄ und R₅ H sind; und
WO ist.

3. Verbindung nach Anspruch 2, worin Q Sauerstoff ist.

4. Verbindung nach Anspruch 3, worin R C₁―C₆-Alkyl, C₃-C₄₋Alkenyl, CH₂CH₂Cl, CH₂CH₂OCH₃ oder CH₂OR'₈ ist, worin R'₈ wie in Anspruch 1 definiert ist.

5. Verbindung nach Anspruch 4, worin X CH₃ oder OCH₃ ist.

6. Verbindung nach Anspruch 5, worin Y CH₃, OCH₃ oder

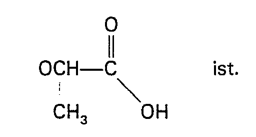

7. Verbindung nach Anspruch 6, worin R₃ H ist.

8. Verbindung nach Anspruch 7, worin R₂ H ist.

9. Verbindung nach Anspruch 8, worin R'₈ CH₃ oder CH₃CH₂ ist, und R CH₃, CH₂CH₂OCH₃ oder CH₂CH₂0C₂H₅ ist.

10. Verbindung nach Anspruch 1, nämlich [(2-Methoxyethoxy)-methyl]-2-[[(4,6-dimethoxypyrimidin-2-yl)-aminocarbonyl]-aminosulfonyl]-benzoat; und landwirtschaftlich brauchbare Salze davon.

11. Verbindung nach Anspruch 1, nämlich [(2-Methoxyethoxy)-methyl]-2-[[(4-ethoxy-6-methyl-1,3,5-triazin-2-yl)-aminocarbonyl]-aminosulfonyl]-benzoat; und landwirtschaftlich brauchbare Salze davon.

12. Verbindung nach Anspruch 1, nämlich [(4-Chlorphenoxy)-methyl]-2-[[(4,6-dimethyl- pyrimidin-2-yl)-aminocarbonyl]-aminosulfonyl]-benzoat; und landwirtschaftlich brauchbare Salze davon.

13. Verbindung nach Anspruch 1, nämlich Methyl-2-[[[4-(1-carboxyethoxy)-6-methyl-1,3,5-triazin-2-yl-]-aminocarbonyl]-aminosulfonyl]-benzoat; und landwirthschaftlich brauchbare Salze davon.

14. Verbindung nach Anspruch 1, nämlich [(2-Methoxyethoxy)-methyl]-2-[[(4,6-dimethyl- pyrimidin-2-yl)-aminocarbonyl]-aminosulfonyl]-benzoat; und landwirtschaftlich brauchbare Salze davon.

15. Verbindung nach Anspruch 1, nämlich (4-Chlor-2-butinyl)-2-[[(4-methoxy-6-methylpyrimidin-2-yl)-aminocarbonyl]-aminosulfonyl]-benzoat; und landwirtschaftlich brauchbare Salze davon.

16. Zusammensetzung geeignet zur Bekämpfung des Wachstums unerwünschter Vegetation, enthaltend eine wirksame Menge einer herbiziden Verbindung und mindestens eines der folgenden: oberflächenaktives Mittel, festes oder flüssiges Verdünnungsmittel, dadurch gekennzeichnet, daß die herbizide Verbindung eine Verbindung gemäß einem der Ansprüche 1 bis 15 umfaßt.

17. Verfahren zur Bekämpfung des Wachstums unerwünschter Vegetation durch Auftrag auf den zu schützenden Ort von einer wirksamen Menge einer herbiziden Verbindung, dadurch gekennzeichnet, daß die herbizide Verbindung eine Verbindung gemäß einem der Ansprüche 1 bis 15 umfaßt.

18. Verfahren zur Regulierung des Wachstums von Pflanzen durch Auftrag auf den Ort dieser Pflanzen von einer wirksamen jedoch im wesentlichen nicht-phytotoxischen Menge eines das Pflanzenwachstum regulierenden Mittels, dadurch gekennzeichnet, daß das das Pflanzenwachstum regulierende Mittel eine Verbindung gemäß einem der Ansprüche 1 bis 15 umfaßt.

19. Verfahren zur Herstellung einer Verbindung nach Anspruch 1, durch (a) Reaktion einer Verbindung der Formel 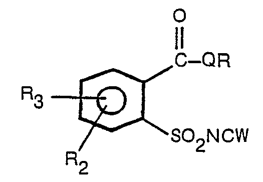 worin
Q 0 ist;
R C₁―C₁₂-Alkyl; C₃―C₁₀-Alkenyl; C₂―C₆Alkyl substituiert mit einem bis vier Substituenten ausgewählt aus 0-3 Atomen von F, CI oder Br, 0-2 Methoxygruppen; C_{3―}C₆-Alkenyl substituiert mit 1-3 Atomen von F, CI oder Br; C₅―C₈-Cycloalkyl; C₅―C₈-Cycloalkenyl; C₅―C₆-Cycloalkyl substituiert mit beliebigen von 1 bis 4 Methylgruppen, Methoxy, Alkyl mit C₂₋₄, F, CI oder Br; C₄―C₁₀-Cycloalkylalkyl; C₄―C₈-Cycloalkylalkyl mit 1-2 CH₃; ―CH₂CH₂OR₇; CH₂CH₂CH₂OR₇; 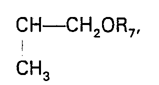 worin R₇ -CH₂CH₃, CH(CH₃)₂, Phenyl, ―CH₂CH₂Cl oder ―CH₂CCl₃ ist; (̵CH₂CH₂O)̵ₙ―R₈ oder 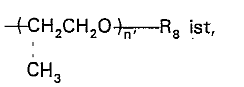 worin Rₐ CH₃, ―CH₂CH₃ ―CH(CH₃)₂, Phenyl, ―CH₂CH₂Cl oder ―CH₂CCl₃ ist,
und n' 2 oder 3 ist;
R₂ H, CI, Br, F, C₁―C₃-Alkyl, ―NO₂ -OCH₃, -SCH₃, CF₃, SO₂CH₃, N(CH₃)₂ oder CN ist;
R₃ H, CI, Br oder CH₃ ist
und W 0 oder S ist;
mit einem geeigneten Aminopyrimidin oder Aminotriazin der Formel
HNR₁R₅
worin R₁ und R₅ wie in Anspruch 1 definiert sind;
(b) Reaktion einer Verbindung der Formel 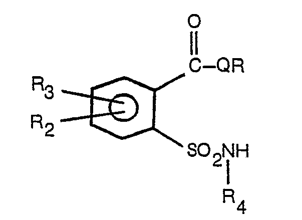 worin Q, R_{2'} R₃ und R₄ wie in Anspruch 1 definiert sind, und R wie in Anspruch 1 definiert ist, jedoch unterschiedlich von H oder M, mit einem geeigneten Triazin- oder Pyrimidin-isothiocyanat 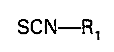 worin R, wie in Anspruch 1 definiert ist;
(c) Methylieren einer Verbindung nach Anspruch 1, worin R₄ H ist, Q 0, S oder 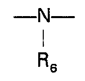 ist, R wie in
(a) vorstehend definiert ist und W 0 ist, unter Erzielung einer Verbindung nach Anspruch 1, worin R₄ Methyl ist;
(d) Umsetzung eines Carbamoyl- oder Thiocarbamoylchlorids der Formel 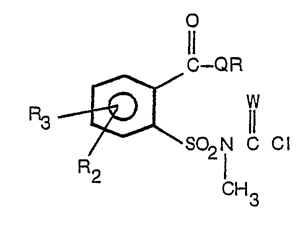 worin Q und R wie in (a) vorstehend definiert sind, und W 0 oder S ist, mit dem Amin HNR₁R₅, wie vorstehend in (a) definiert; oder
(e) Hydrolysieren eines Esters nach Anspruch 1, worin R C₁₋₁₂-Alkyl ist, unter Erzielung einer Verbindung nach Anspruch 1, worin R H ist oder Veresterung einer Verbindung nach Anspruch 1, worin R H ist, unter Erzielung einer Verbindung nach Anspruch 1, worin R von H unterschiedlich ist.

## Patentansprüche (Patentansprüche für folgende(n) Vertragsstaat(en) : AT)

1. Verfahren zur Herstellung einer Verbindung ausgewählt aus 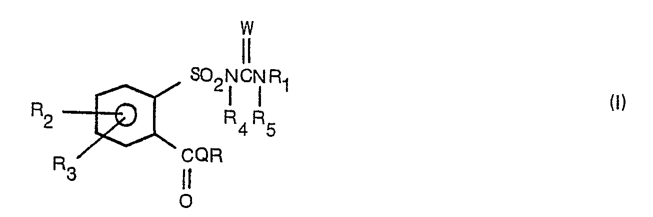 worin
Q 0, S oder 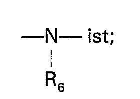 wenn Q 0 oder S ist, dann ist R C₁―C₁₂-Alkyl; C₃―C₁₀-Alkenyl; C₂―C₆-Alkyl substituiert mit einem bis vier Substituenten ausgewählt aus 0-3 Atomen von F, CI oder Br, 0-2 Methoxygruppen und 0-1 Cyanogruppen, -CH₂CN; ―CH₂CO₂CH₃; ―CH₂CO₂C₂H₅; C₃―C₆-Alkenyl substituiert mit 1-3 Atomen von F, CI oder Br; C₅―C₈-Cycloalkyl; C₅―C₈-Cycloalkenyl; C₅―C₆-Cycloalkyl substituiert mit beliebigen von eins bis vier Methylgruppen, OCH₃, Alkyl mit C₂―C₄, F, CI oder Br; C₄―C₁₀ Cycloalkylalkyl; C₄―C₈-Cycloalkylalkyl mit 1-2 CH₃; C₇―C₁₀-Bicycloalkyl; C₇―C₁₀-Bicycloalkenyl; C₁₀-Tricycloalkyl, C₁₀-Tricycloalkenyl; 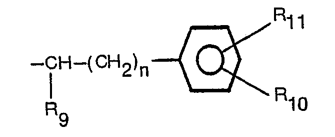 worin R₉ C₁―C₃-Alkyl oder Wasserstoff ist, R₁₀ und R₁₁ unabhängig Wasserstoff, C₁―C₃-Alkyl, Cl, Br, -OCH₃ oder ―OC₂H₅ sind, oder R₁₀ und R₁₁ zusammengenommen einen 5- oder 6-gliedrigen Ring bilden können; 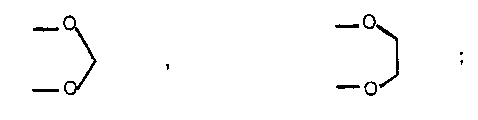 und n 0, 1, 2 oder 3 ist, vorausgesetzt, das R eine Kohlenstoffatomgesamtanzahl von weniger als oder gleich 12 aufweist; 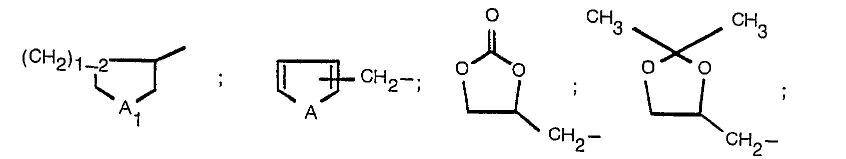 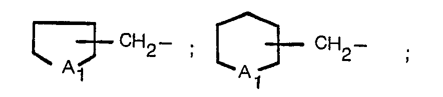 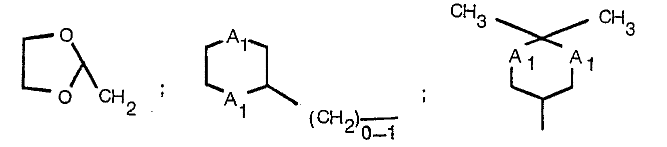
A 0 oder S ist;
A₁ O, S oder S0₂ ist; wenn Q 0 oder S ist, dann
1) kann R auch sein C₃―C₁₀-Alkinyl; C₃―C₆-Alkinyl substituiert mit F, CI oder Br; 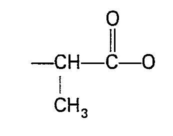 (C₁―C₂-Alkyl); oder 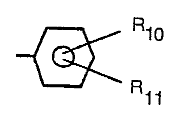 worin R₁₀ und R₁₁ wie vorstehend definiert sind; wenn Q 0 ist, dann ist R H, M, ―CH₂CH₂OR₇, ―CH₂CH₂CH₂OR₇, 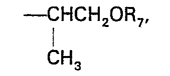 worin R₇ die Bedeutung hat von ―CH₂CH₃, ―CH(CH₃)₂, Phenyl, ―CH₂CH₂Cl oder ―CH₂CCl; (̵CH₂CH₂O)̵ₙ-R₈ oder 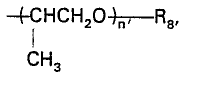 worin R₈ -CH₃, ―CH₂CH₃, ―CH(CH₃)₂, Phenyl, ―CH₂CH₂Cl oder ―CH₂CCl₃ ist, und n' 2 oder 3 ist; vorausgesetzt, das R eine Kohlenstoffatomgesamtanzahl von weniger als oder gleich 13 aufweist; 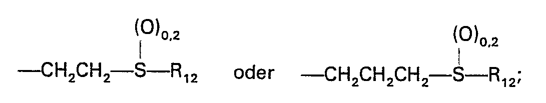 worin R₁₂ -CH₃, ―CH₂CH₃, -CH(CH₃)₂ oder Phenyl ist; und worin Q 0 ist;
2) kann R auch sein CH₂OR₈, worin R'₈ CH₃―, CH₃CH₂, (CH₃)₂CH―, CICH₃CH₂―, Cl₃CCH₂, 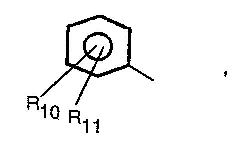 CH₃CH₂OCH₂CH₂ oder CH₃OCH₂CH₂ ist;
worin R₁₀ und R₁₁ wie vorstehend definiert sind; wenn Q 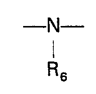 ist, dann ist R Wasserstoff; C₁―C₁₂-Alkyl; (̵CH₂CH₂O)̵ₙR₁₂, ―CH₂CH₂CH₂OR₂, worin R₁₂ wie vorstehend definiert ist und n"' 1-3 ist; C₃―C₁₀-Alkenyl; C₃―C₈-Cycloalkyl; C₅―C₆-Cydoalkenyl; C₅―C₈-Cycloalkyl substituiert mit 1 bis 3 Substituenten ausgewählt aus 0-2 -OCH₃, 0-3 -CH₃ und ―C₂H₅; Trifluormethylcyclohexyl; C₄―C₁₀-Cycloalkylalkyl; C₄―C₈-Cycloalkylkalkyl substituiert mit 1-2 -CH₃; ―CH₂CN; ―CH₂CH₂CN; 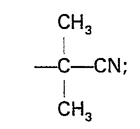 -OCH₃; ―N(CH₃)₂; oder 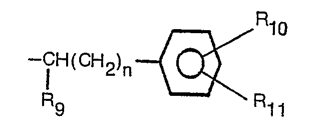 worin n, R₉, R₁₀ und R₁₁ wie vorstehend definiert sind; 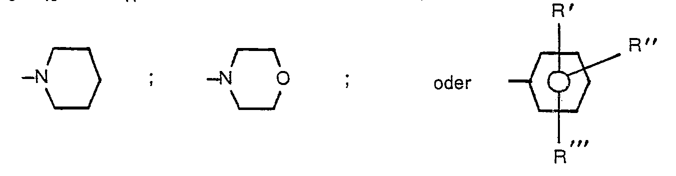 worin R' Wasserstoff, C₁―C₄-Alkyl, -OCH₃, F, Br, Cl, -CF₃, CN, NO₂, ―SO₂CH₃, -SCH₃ oder -N(CH₃)₂ ist; R" Wasserstoff, C₁―C₄-Alkyl, -OCH₃, F, Br, oder Cl ist;
R"' Wasserstoff, -CH₃, CI, F oder Br ist;
R₆ Wasserstoff, C₁―C₆-Alkyl, Allyl, -CH₂CN; oder -CH CH₂CN ist; oder R₆ und R zusammengenommen bilden können: ―(CH₂)₄―, ―(CH₂)₅―, ―(CH₂)₆―, ―CH₂CH₂OCH₂CH₂―; oder 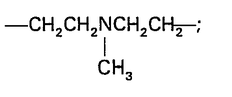 mit der Maßgabe, daß wenn R -OCH₃ oder OCH₂CH₃ ist, dann R₆ H oder -CH₃ ist; wenn R₆ ―CH₂CH₂CN oder -CH₂CN ist, dann R -CH₂CH₂CN oder CH₂CN ist; und R und R₆ eine Gesamtkohlenstoffanzahl von weniger als oder gleich 13 aufweisen; und wenn Q 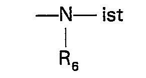
3) R₃ auch sein kann: C₃―C₆-Alkinyl; 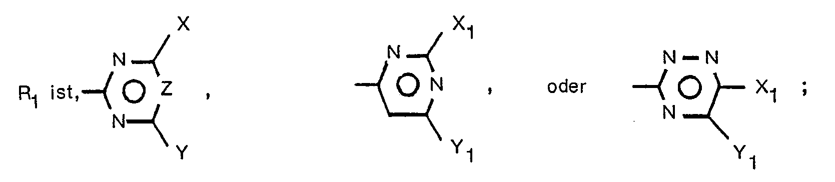
R₂ ist H, CI, Br, F, C₁-C₃-Alkyl, -N0₂, ―SO₂CH₃, -OCH₃, -SCH₃, -CF₃, ―N(CH₃)₂, -NH₂, oder -CN;
R₃ ist H, Cl, Br, F oder CH₃;
R₄ ist H oder -CH₃;
R₅ ist H, -CH₃ oder -OCH₃;
M ist ein Alkalimetall;
W ist Sauerstoff oder Schwefel;
X ist H, Cl, -CH₃, ―OCH₃, ―OCH₂CH₃ oder ―OCH₂CH₂OCH₃;
Y ist H; Cl; C₁―C₄-Alikyl; C₁-C₄-Alkyl substituiert mit OCH₃, OC₂H₅, CN, CO₂CH₃, CO₂C₂H₅ oder 1-3 Atomen von F, Cl, oder Br; C₃―C₄-Alkenyl: CH₂C≡CR₁₃; A(CH₂)ₙ―A₁(C₁―C₃-Alkyl); 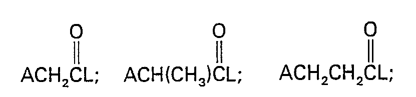 SCN; N₃; NR₁₆R₁₇; OR₁₄ oder SR₁₅;
worin
R₁₃ H, CH₃ oder CH₂Cl ist;
n', A und A₁ wie vorstehend definiert sind;
L NH₂, N(OCH₃)CH₃, NH(C₁―C₄-Alkyl), N(C₁―C₄-Alkyl)₂ oder C₁―C₆-Alkoxy ist;
R₁₄ C₁―C₄-Alkyl; C₂―C₄-Alkyl substituiert mit 1-3 Atomen von F, Cl oder Br; C₁―C₄-Alkyl substituiert mit CN; C₃―C₄-Alkenyl; CH₂C=CR₁₃, oder -C2_{H} 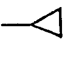 ist;
R₁₅ C₁―C₄-Alkyl, Cyanomethyl, Cyanoethyl, Allyl oder Propargyl ist;
R₁₆ H oder CH₃ ist;
R₁₇ H; OCH₃; C₁―C₄-Alkyl; C₁―C₄-Allyl substituiert mit CN, CO₂CH₃ oder CO₂C₂H₅; C₃―C₄-Alkenyl; oder C₂―C₃-Alkyl substituiert mit entweder OCH₃ oder OC₂H₅; ist oder
R₁₆ und R₁₇ zusammengenommen bilden können CH₂CH₂CH₂CH₂ oder CH₂CH₂OCH₂CH₂,
mit der Maßgabe, daß wenn Y 4 oder mehr Kohlenstoffatome enthält, dann R nicht mehr als 4 Kohlenstoffatome enthält; wenn X CI ist, dann Y CI ist; und wenn X und Y beide H sind, dann R nicht mehr als 4 Kohlenstoffatome enthält,
4) Y auch F, Br, 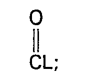 C₁―C₄-Alkyl substituiert mit 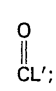 oder NR₁₆R₁₇' sein kann; worin L wie vorstehend definiert oder OH ist;
R₁₆ wie vorstehend definiert ist;
L' NH₂, OH, N(OCH₃)CH₃, NH(C₁―C₄-Alkyl), N(C₁―C₄-Alkyl)₂ oder C₃―C₆-Alkoxy ist; und
R₁₇' C₆―C₆-Alkyl oder C₁―C₄-Alkyl substituiert mit 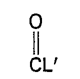 ist, worin L' wie vorstehend definiert ist;
Z CH oder N ist;
Y₁ H, ―OCH₃ oder -CH₃ ist;
5) Y₁ auch OCH₂CH₃ sein kann; und
X₁ H, CI, -OCH₃, ―OCH₂CH₃ oder -CH₃ ist;
vorausgesetzt, daß X₁ und Y₁ nicht beide gleichzeitig Wasserstoff sind, und wenn R₁ 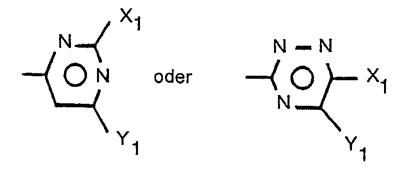 ist, dann R₄ und R₅ beide H sind und R 5 oder weniger Kohlenstoffatome enthält und
a) wenn Y nicht wie in 4) definiert ist, dann R wie in 1), 2) oder 3) definiert sein muß;
b) wenn Y₁ nicht wie in 5) definiert ist, dann R wie in 1), 2) oder 3) definiert sein muß; oder der landwirtschaftlich brauchbaren Salze davon; durch
(a) Reaktion einer Verbindung der Formel 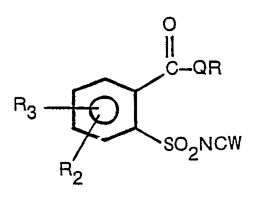 worin
Q 0 ist;
R C₁―C₁₂-Alkyl; C₃―C₁₀-Alkenyl; C₂―C₆Alkyl substituiert mit einem bis vier Substituenten ausgewählt aus 0-3 Atomen von F, CI oder Br, 0-2 Methoxygruppen; C₃―C₆-Alkenyl substituiert mit 1-3 Atomen von F, CI oder Br; C₅―C₈-Cycloalkyl; C₅―C₈-Cycloalkenyl; C₅―C₆-Cycloalkyl substituiert mit beliebigen von 1 bis 4 Methylgruppen, Methoxy, Alkyl mit C₂₋₄, F, CI oder Br; C₄―C₁₀-Cycloalkylalkyl; C₄―C₈-Cycloalkylalkyl mit 1-2 CH₃; ―CH₂CH₂OR₇; CH₂CH₂CH₂OR₇; 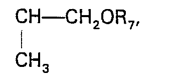 worin R₇= ―C2_{H}CH₃, CH(CH₃)₂, Phenyl, ―CH₂CH₂Cl oder ―CH₂CCl₃; (̵CH₂CH₂O)̵ₙR₈ oder 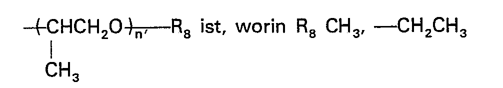 ―CH(CH₃)₂, Phenyl, ―CH₂CH₂Cl oder ―CH₂CCl₃ ist,
und n' 2 oder 3 ist;
R₂ H, Cl, Br, F, C₁―C₃-Alkyl, ―NO₂, -OCH₃, -SCH₃, CF₃, SO₂CH₃, N(CH₃)₂ oder CN ist;
R₃ H, CI, Br oder CH₃ ist;
und W 0 oder S ist;
mit einem geeigneten Aminopyrimidin oder Aminotriazin der Formel 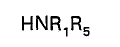 worin R₁ und R₅ wie in Anspruch 1 definiert sind;
(b) Reaktion einer Verbindung der Formel 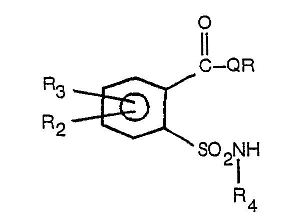 worin Q, R₂, R₃ und R₄ wie für Formel I definiert sind, und R wie für Formel I definiert ist, jedoch unterschiedlich von H oder M, mit einem geeigneten Triazin oder Pyrimidin-isothiocyanat 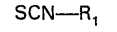 worin R₁ wie für Formel I definiert ist;
(c) Methylieren einer Verbindung der Formel I, worin R₄ H ist, Q 0, S oder 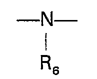 ist, R wie in
(a) vorstehend definiert ist und W 0 ist, unter Erzielung einer Verbindung der Formel I, worin R₄ Methyl ist;
(d) Umsetzung eines Carbamoyl- oder Thiocarbamoylchlorids der Formel 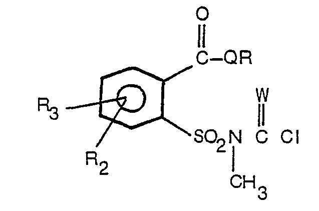 worin Q und R wie in (a) vorstehend definiert sind, und W 0 oder S ist, mit dem Amin HNR₁R₅, wie vorstehend in (a) definiert; oder
(e) Hydrolysieren eines Esters der Formel I, worin R C₁₋₁₂-Alkyl ist, unter Erzielung einer Verbindung der Formel I, worin R H ist oder Veresterung einer Verbindung der Formel I, worin R H ist, unter Erzielung einer Verbindung der Formel I, worin R von H unterschiedlich ist.

2. Verfahren nach Anspruch 1, bei dem 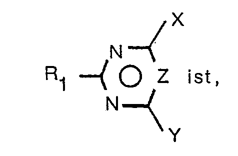
R₄ und R₅ H sind; und
W 0 ist.

3. Verfahren nach Anspruch 2, worin Q Sauerstoff ist.

4. Verfahren nach Anspruch 3, worin R C₁―C₆-Alkyl, C₃―C₄-Alkenyl, CH₂CH₂Cl. CH₂CH₂OCH₃ oder CH₂OR'₈ ist, worin R'₈ wie in Anspruch 1 definiert ist.

5. Verfahren nach Anspruch 4, bei dem X CH₃ oder OCH₃ ist.

6. Verfahren nach Anspruch 5, bei dem Y CH₃, OCH₃ oder 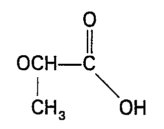 ist.

7. Verfahren nach Anspruch 6, bei dem R₃ H ist.

8. Verfahren nach Anspruch 7, bei dem R₂ H ist.

9. Verfahren nach Anspruch 8, bei dem R'₈ CH₃ oder CH₃CH₂ ist und R CH₃, CH₂CH₂OCH₃ oder CH₂CH₂OC₂H₅ ist.

10. Verfahren nach Anspruch 1, bei dem des Produkt eine Verbindung ist, ausgewählt aus [(2-Methoxyethoxy)methyl]-2-[[4,6-dimethoxypyrimidin-2-yl)-aminocarbonyl]-aminosulfonyl]-benzoat;
[(2-Methoxyethoxy)-methyl]-2-[[(4-ethoxy-6-methyl-1,3,5-triazian-2-yl)-aminocarbonyl]-aminosulfonyl]-benzoat;
[(4-Chlorphenoxy)-methyl]-2-[[(4,6-dimethylpyrimidin-2-yl)-aminocarbonyl]-aminosulfonyl]-benzoat;
Methyl-2-[[[4-(1-carboxyethoxyl-6-methyl-1,3,5-triazin-2-yl]-aminocarbonyl]-aminosulfonyl]-benzoat;
[(2-Methoxyethoxy)-methyl]-2-[[(4,6-dimethylpyrimidin-2-yl)-aminocarbonyl]-aminosulfonyl]-benzoat;
(4-Chlor-2-butinyl)-2-[[(4-methoxy-6-methylpyrimidin-2-yl)-aminocarbonyl]-aminosulfonyl]-benzoat;
oder einem landwirtschaftlich brauchbaren Salz von einem beliebigen davon.

11. Verfahren zur Bekämpfung des Wachstums unerwünschter Vegetation durch Auftrag auf den zu schützenden Ort von einer wirksamen Menge einer herbiziden Verbindung, dadurch gekennzeichnet, daß die herbizide Verbindung eine Verbindung der Formel (I), wie in einem der Ansprüche 1 bis 9 definiert, umfaßt.

12. Verfahren nach Anspruch 11, bei dem die herbizide Verbindung eine Verbindung, wie in Anspruch 10 definiert, umfaßt.

13. Verfahren zur Regulierung des Pflanzenwachstums durch Auftrag auf den Ort dieser Pflanzen von einer wirksamen, jedoch im wesentlichen nicht-phytotoxischen, Menge eines des Pflanzenwachstum regulierenden Mittels, dadurch gekennzeichnet, daß das das Pflanzenwachstum regulierende Mittel, eine Verbindung der Formel (1), wie in einem der Ansprüche 1 bis 10 definiert, umfaßt.

## Revendications (Revendications pour l'(les) Etat(s) contractant(s) suivant(s) : BE CH DE FR GB IT LI LU NL SE)

1. Un composé choisi parmi ceux de formule: 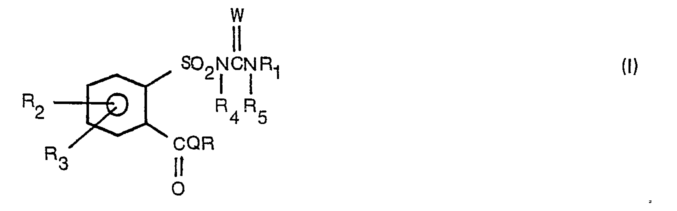 dans laquelle:
Q est 0, S ou 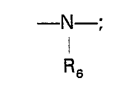 lorsque Q est 0 ou S, alors R est un groupe alkyle en C₁ à C₁₂; alcényle en C₃ à C₁₀; alkyle en C₂ à C₆ substitué par un à quatre substituants choisis entre 0 à 3 atomes de F, Cl ou Br, 0 à 2 groupes méthoxy et 0 à 1 groupe cyano; -CH₂CN; ―CH₂CO₂CH₃; ―CH₂CO₂C₂H₅; alcén^{y}le en C₃ à C₆ substitué par 1 à 3 atomes de F, Clou Br; cycloalkyle en C₅ à C₈; cycloalcényle en C₅ à Cₐ; cyclo- ayle en C₅ à C₆ substitué par un à quatre groupes méthyle, OCH₃, alkyle en C₂ à C₄, F, Ci ou Br; cycloalkylalkyle en C₄ à C₁₀; cycloalkylalkyle en C₄ à C₈ contenant 1 à 2 groupes CH₃; bicycloalkyle en C₇ à C₁₀; bicycloalcényle en C₇ à C₁₀; tricycloalkyle en C₁₀; tricycloalcényle en C₁₀; 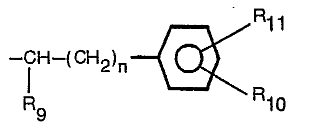 dans lequel R₉ est un groupe alkyle en C₁ à C₃ ou l'hydrogène, R₁₀ et R₁₁ sont indépendamment l'hydrogène, un groupe alkyle en C₁ à C₃, CI, Br, -OCH₃ ou ―OC₂H₅, ou R₁₀ et R₁₁ peuvent être pris ensemble pour former un noyau à 5 ou 6 chaînons: 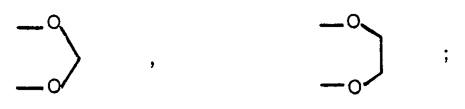 et n vaut 0, 1, 2 ou 3, avec la condition que R a un nombre total d'atomes de carbone inférieur ou égal à 12; 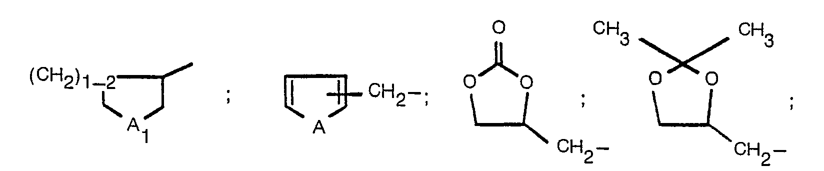 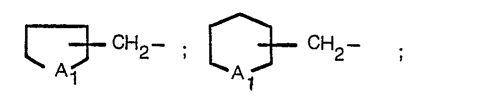 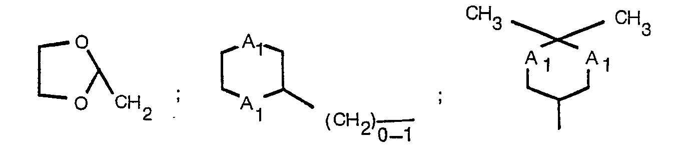
A est O ou S;
A₁ est 0, S ou SO₂; quand Q est O ou S, alors
1 ) R peut être aussi un groupe alcynyle en C₃ à C₁₀; alcynyle en C₃ à C₆ substitué par F, CI ou Br; 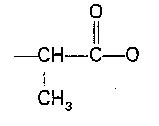 (alkyle en C₁―C₂); ou 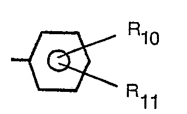 où R₁₀ et R₁₁ sont tels que définis plus haut; quand Q est 0, alors R est H, M, ―CH₂CH₂OR₇, ―CH₂CH₂CH₂OR₇, 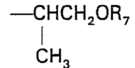 où R₇ est ―CH₂CH₃, ―CH(CH₃)₂, phényle, ―CH₂CH₂Cl ou ―CH₂CCl₃; (̵CH₂CH₂O)ₙ,R₈ ou 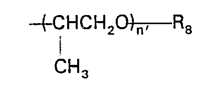 où R₈ est -CH₃, ―CH₂CH₃, ―CH(CH₃)₂, phényle, ―C2_{H}CH₂Cl ou ―CH₂CCl₃ et n' vaut 2 ou 3; avec la condition que R ait un nombre total d'atomes de carbone inférieur ou égal à 13, 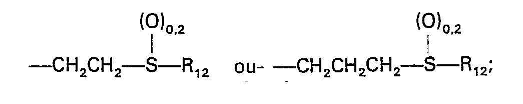 où R₁₂ est -CH₃, ―CH₂CH₃, -CH(CH₃)₂ ou phényle; et quand Q est 0,
2) R peut être aussi CH₂OR'₈ où R' est CH₃― CH₃CH₂, (CH₃)₂CH―, OCH₂CH₂―, CI₃CCH₂, 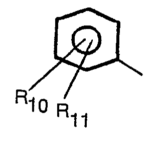 CH₃CH₂OCH₂CH₂ ou CH₃OCH₂CH₂; où R₁₀ et R₁₁ sont tels que définis plus haut; quand Q est 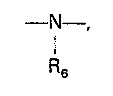 alors R est l'hydrogène; un groupe alkyle en C₁ à C₁₂; (̵CH₂CH₂O)̵ₙR₁₂, ―CH₂CH₂CH₂OR₁₂ où R₁₂ est tel que défini plus haut et n"' vaut de 1 à 3; alcényle en C₃ à C₁₀; cycloalkyle en C₃ à C₈; cycloalcényle en C₅ à C₆; cycloalkyle en C₅ à C₈ substitué par 1 à 3 substituants choisis entre 0 à 2 groupes -OCH₃, 0 à 3 groupes -CH₃ et ―C₂H₅; trifluorométhylcyclohexyle; cycloalkylalkyle en C₄ à C₁₀; cycloalkylalkyle en C₄ à C₈ substitué par 1 à 2 groupes -CH₃; -CH₂CN; ―CH₂CH₂CN; 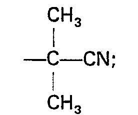 -OCH₃; ―N(CH₃)₂; ou 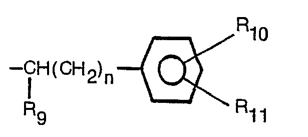 où n, R₉, R₁₀ et R₁₁ sont tels que définis plus haut; 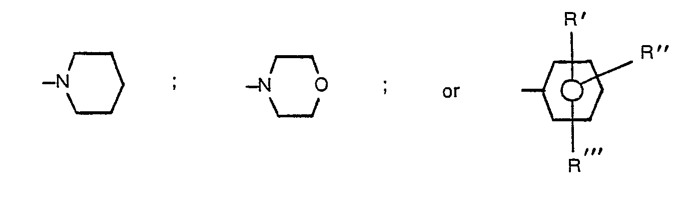 où
R' est l'hydrogène, un groupe alkyle en C₁ à C₄, -OCH₃, F, Br, CI, ―CF₃, CN, NO₂, ―SO₂CH₃, -SCH₃ ou -N(CH₃)₂;
R" est l'hydrogène, un groupe alkyle en C₁ à C₄, -OCH₃, F, Br ou CI;
R"' est l'hydrogène, -CH₃, CI, F ou Br;
R₆ est l'hydrogène, un groupe alkyle en C₁ à C₆; allyle, ―CH₂CN; ou ―CH₂CH₂CN; ou R₆ et R peuvent être pris ensemble pour former ―(CH₂)₄―, ―(CH₂)₅―, ―(CH₂)₆―, ―CH₂CH₂OCH₂CH₂― ou 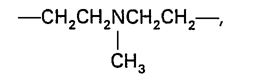 avec les conditions que, lorsque R est -OCH₃ ou ―OCH₂CH₃, alors R₆ est H ou -CH₃; que, lorsque R₆ est ―CH₂CH₂CN ou -CH₂CN, alors R est -CH₂CH₂CN ou -CH₂CN; et R et R₆ ont un nombre total d'atomes de carbone inférieur ou égal à 13; et quand Q est 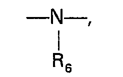
3) R peut aussi être un groupe alcynyle en C₃ à C₆; R₁ est 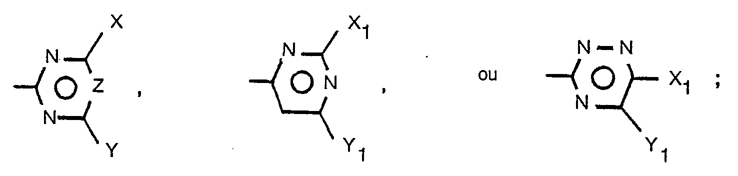
R₂ est H, CI, Br, F, un groupe alkyle en C₁ à C₃, ―NO₂ ―SO₂CH₃, -OCH₃, ―SCH₃ -CF₃, ―Nl(CH₃)₂, -NH₂ ou -CN;
R₃ est H, CI, Br, F ou CH₃;
R₄ est H ou -CH₃;
R₅ est H, -CH₃ ou -OCH₃;
M est un métal alcalin;
W est l'oxygène ou le soufre;
X est H, CI, -CH₃, -OCH₃, -OCH₂CH₃ ou ―OCH₂CH₂OCH₃;
Y est H, CI, un groupe alkyle en C₁ à C₄; un groupe alkyle en C₁ à C₄ substitué par OCH₃, OC₂H₅, 'CN, CO₂CH₃, CO₂C₂H₅ ou par 1 à 3 atomes de F, Clou Br; un groupe alcényle en C₃ à C₄; CH₂C≡CR₁₃; A(CH₂)̵ₙA₁ (alkyle en C, à C₃); 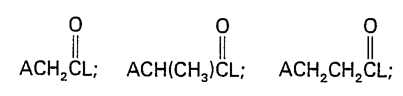 SCN; N₃; MR₁₆R₁₇; OR₁₄ ou SR₁₅;
où
R₁₃ est H, CH₃ ou CH₂Cl;
n', A et A₁ sont tels que définis plus haut;
L est NH₂, N(OCH₃)CH₃, NH(alkyle en C₁ à C₄), N(alkyle en C₁ à C₄)₂ ou un groupe alcoxy en C₁ à
^{C6;} R₁₄ est un groupe alkyle en C₁ à C₄; un groupe alkyle substitué par 1 à 3 atomes de F, CI ou Br; un groupe alkyle en C₁ à C₄ substitué par CN; un groupe alcényle en C₃ à C₄; un groupe CH₂C=CR₁₃ où R₁₃ est tel que défini plus haut, ou un groupe 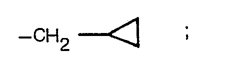
R₁₅ est un groupe alkyle en C₁ à C₄, cyanométhyle, cyanoéthyle, allyle ou propargyle;
R₁₆ est H ou CH₃;
R₁₇ est H; OCH₃; un groupe alkyle en C₁ à C₄; un groupe alkyle en C₁ à C₄ substitué par CN, CO₂C₂H₅; un groupe alcényle en C₃ à C₄; ou un groupe alkyle en C₂ à C₃ substitué par OCH₃ ou par OC₂H₅; ou
R₁₆ et R₁₇ peuvent être pris ensemble pour former CH₂CH₂CH₂CH₂ ou CH₂CH₂0CH₂CH₂, avec les conditions que, lorsque Y contient 4 atomes de carbone ou avantage, R ne contient pas plus de 4 atomes de carbone; lorsque X est CI, alors Y est CI; et, lorsque X et Y sont tous deux H, alors R ne contient pas plus de 4 atomes de carbone,
4) Y peut aussi être F, Br, 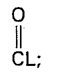 un groupe alkyle substitué par 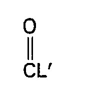 ou NR₁₆R₁₇, où L est tel que défini plus haut ou OH; R₁₆ est tel que défini plus haut; L' est NH₂, OH, N(OCH₃)CH₃, NH(alkyle en C₁ à C₄), N(alkyle en C₁ à C₄)₂ ou un groupe alcoxy en C₃ à C₆; et R₁₇ est un groupe alkyle en C₅ à C₆ ou un groupe alkyle en C₁ à C₄ substitué par 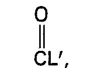 où L' est tel que défini plus haut;
Z est CH ou N;
Y₁ est H, -OCH3 ou ―CH₃;
5) Y₁ peut aussi être OCH₂CH₃; et
X₁ est H, CI, -OCH₃, ―OCH₂CH₃ ou ―CH₃; avec les conditions que X₁ et Y, ne sont pas tous deux simultanément des atomes d'hydrogène, et que, lorsque R₁ est 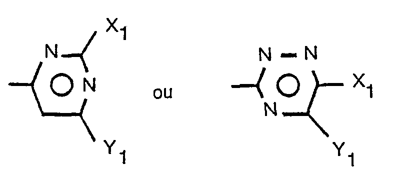 alors R₄ et R₅ sont tous deux H et R contient 5 atomes de carbone ou moins, et que
a) quand Y n'est pas tel que défini en 4), alors R doit être tel que défini en 1 ), 2) ou 3;
b) quand Y₁ n'est pas tel que défini en 5), alors R doit être tel que défini en 1), 2) ou 3; et ses sels appropriés en agriculture.

2. Un composé selon la revendication 1, dans lequel 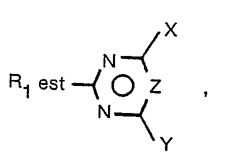
R₄ et R₅ sont H;
W est O.

3. Un composé selon la revendication 2, dans lequel Q est l'oxygene.

4. Un composé selon la revendication 3, dans lequel R est un groupe alkyle en C₁ à C₆, un groupe alcén^{y}le en C₃ à C₄, CH₂CH₂CI, CH₂CH₂OCH₃ ou CH₂OR'₈ dans lequel R' est tel que défini à la revendication 1.

5. Un composé selon la revendication 4, dans lequel X est CH₃ ou OCH₃.

6. Un composé selon la revendication 5, dans lequel Y est CH₃, OCH₃ ou

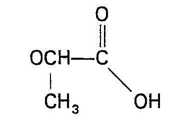

7. Un composé selon la revendication 6, dans lequel R₃ est H.

8. Un composé selon la revendication 7, dans lequel R₂ est H.

9. Un composé selon la revendication 8, dans lequel R'8 est CH₃ ou CH₃CH₂ et R est CH₃, CH₂CH₂OCH₃ ou CH₂CH₂OC₂H₅.

10. Un composé selon la revendication 1, qui est le 2-[[(4,6-diméthoxypyrimidin-2-yi)-amino- carbonyl]-aminosulfonyl]-benzoate de [(2-méthoxyéthoxy)-méthyle]; et ses sels appropriés en agriculture.

11. Un composé selon la revendication 1, qui est le 2-[[(4-éthoxy-6-méthyl-1,3,5-triazin-2-yl)-aminocarbonyl]-aminosulfonyl]-benzoate de [(2-méthoxyéthoxy)-méthyle]; et ses sels appropriés en agriculture.

12. Un composé selon la revendication 1, qui est le 2-[[(4,6-diméthylpyrimidin-2-yl)-amino- carbonyl]-aminosulfonyl]-benzoate de [(4-chlorophénoxy)-méthyle]; et ses sels appropriés en agriculture.

13. Un composé selon la revendication 1, qui est le 2-[[[4-(1-carboxyéthoxy)-6-méthyl-1,3,5-triazin-2-yl]-aminocarbonyl]-aminosulfonyl]-benzoate de méthyle; et ses sels appropriés en agriculture.

14. Un composé selon la revendication 1, qui est le 2-[[(4,6-diméthylpyrimidin-2-yl)-amino- carbonyl]-aminosulfonyl]-benzoate de [(2-méthoxyéthoxy)-méthyle]; et ses sels appropriés en agriculture.

15. Un composé selon la revendication 1, qui est le 2-[[(4-méthoxy-6-méthylpyrimidin-2-yl)-aminocarbonyl]-aminosulfonyl]-benzoate de (4-chloro-2-butynyle); et ses sels appropriés en agriculture.

16. Une composition propre à maîtriser la croissance de végétation indésirable, comprenant une quantité efficace d'un composé herbicide et au moins un des ingrédients suivants: agent tensio-actif, diluant solide ou liquide, caractérisée en ce que le composé herbicide comprend un composé selon l'une quelconque des revendications 1 à 15.

17. Procédé pour maîtriser la croissance de végétation indésirable en appliquant à l'endroit à protéger une quantité efficace d'un composé herbicide, caractérisé en ce que le composé herbicide comprend un composé selon l'une quelconque des revendications 1 à 15.

18. Procédé de régulation de la croissance des plantes qui consiste à appliquer à l'endroit de ces plantes une quantité efficace mais pratiquement non phytoxique d'un régulateur de croissance des plantes, caractérisé en ce que le régulateur de croissance des plantes comprend un composé selon l'une quelconque des revendications 1 à 15.

19. Procédé de préparation d'un composé selon la revendication 1, qui consiste:
(a) à faire réagir un composé de formule: 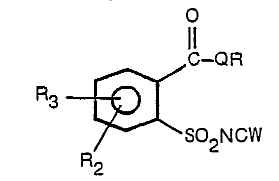 dans laquelle
Q est 0,
R est un groupe alkyle en Ci à C₁₂; un groupe alcényle en C₃ à C₁₀; un groupe alkyle en C₂ à C₆ substitué par un à quatre substituants choisis parmi 0 à 3 atomes de F, CI ou Br et 0 à 2 groupes méthoxy; un groupe alcényle en C₃ à C₆ substitué par 1 à 3 atomes de F, CI ou Br; un groupe cycloalkyle en C₅ à C₈; un groupe cycloalcényle en C₅ à C₈; un groupe cycloalkyle en C₅ à C₆ substitué par un à quatre groupes méthyle, méthoxy, alkyle en C₂ à C₄, F, CI ou Br; un groupe cycloalkylalkyle en C₄ à C₁₀; un groupe cycloalkylalkyle en C₄ à C₈ comportant 1 à 2 groupes CH₃; -CH₂CH₂OR₇; CH₂CH₂CH₂OR₇; 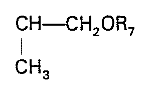 dans lesquels R₇ est ―CH₂CH₃, CH(CH₃)₂, phényle ―CH₂CH₂Cl ou ―CH₂CCl₃; (̵CH₂CH₂O)̵ₙ R₈ ou 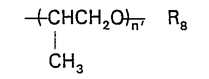 où R₈ est CH₃, ―CH₂CH₃, ―CH(CH₃)₂, phényle, ―CH₂CH₂Cl ou ―CH₂CCl₃ et n' vaut 2 ou 3;
_{CN;} R₂ est H, CI, Br, F, un groupe alkyle en C₁ à C₃, ―NO₂, -OCH₃, -SCH₃, CF₃, SO₂CH₃, N(CH₃)₂ ou
R₃ est H, CI, Br ou CH₃
et W est 0 ou S; avec une aminopyrimidine ou une aminotriazine appropriée de formule 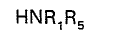 dans laquelle R₁ et R₅ sont tels que définis à la revendication 1 ;
(b) à faire réagir un composé de formule: 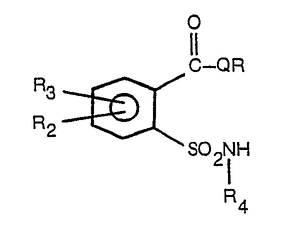 dans laquelle Q, R_{2'} R₃ et R₄ sont tels que définis à la revendication 1, et R est tel que défini à la revendication 1, autre que H ou M, avec un isothiocyanate de triazine ou de pyrimidine approprié 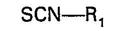 dans lequel R, est tel que défini à la revendication 1;
(c) à méthyler un composé selon la revendication 1 dans lequel R₄ est H, Q est 0, S ou 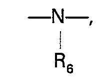 R est tel que défini en (a) ci-dessus et W est 0, pour obtenir un composé selon la revendication 1 dans lequel R₄ est un groupe méthyle;
(d) à faire réagir un chlorure de carbamoyle ou de thiocarbamoyle de formule: 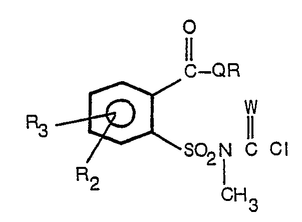 dans laquelle Q et R sont tels que définis en (a) ci-dessus et W est 0 ou S, avec l'amine HNR₁R₅ définie en (a) ci-dessus;
ou
(e) à hydrolyser un ester selon la revendication 1 dans lequel R est un groupe alkyle en C₁ à C₁₂ pour obtenir un composé selon la revendication 1 dans lequel R est H, ou à estérifier un composé selon la revendication 1 dans lequel R est H pour obtenir un composé selon la revendication 1 dans lequel R est autre que H.

## Revendications (Revendications pour l'(les) Etat(s) contractant(s) suivant(s) : AT)

1. Un procede de preparation d'un composé choisi parmi ceux de formule: 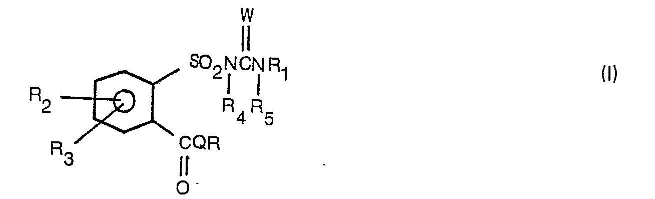 dans laquelle:
Q est 0, S ou 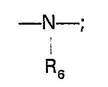 lorsque Q est O ou S, alors R est un groupe alkyle en C, à C₁₂; alcényle en C₃ à C₁₀; alkyle en C₂ à C₆ substitué par un à quatre substituants choisis entre 0 à 3 atomes de F, CI ou Br, 0 à 2 groupes méthoxy et 0 à 1 groupe cyano; ―CH₂CN; ―CH₂CO₂CH₃; ―CH₂CO₂CH₃; ―CH₂CO₂C₂Hₛ; alcényle en C₃ à C₆ substitué par 1 à 3 atomes de F, CI ou Br, cycloalkyle en C₅ à C₈; cycloalcényle en C₅ à C₈; cycloalkyle en C₅ à C₆ substitué par un à quatre groupes méthyle, OCH₃ alkyle en C₂ à C₄, F, Clou Br; cycloalkylalkyle en C₄ à C₁₀; cycloalkylalkyle en C₄ à C₈ contenant 1 à 2 groupes CH₃; bicycloalkyle en C₇ à C₁₀; bicycloalcényle en C₇ à C₁₀; tricycloalkyle en C₁₀; tricycloalcényle en C₁₀; 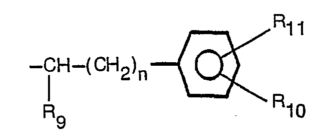 dans lequel R₉ est un groupe alkyle en C₁ à C₃ ou l'hydrogène, R₁₀ et R₁₁ sont indépendamment l'hydrogène, un groupe alkyle en C₁ à C₃, CI, Br, -OCH₃ ou ―OC₂H₅, ou R₁₀ et R₁₁ peuvent être pris ensemble pour former un noyau à 5 ou 6 chaînons: 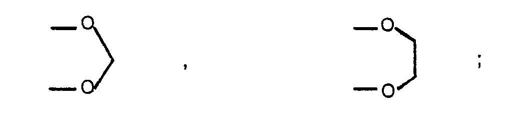 et n vaut 0, 1, 2 ou 3, avec la condition que R a un nombre total d'atomes de carbone inférieur ou égal à 12; 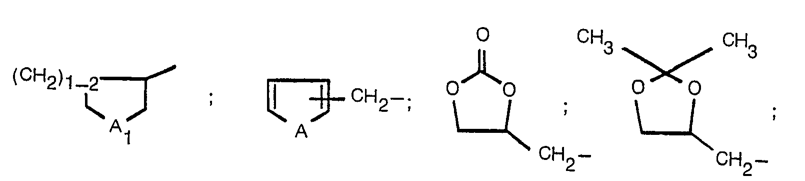 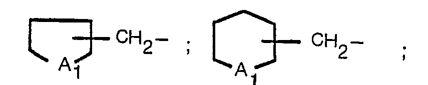 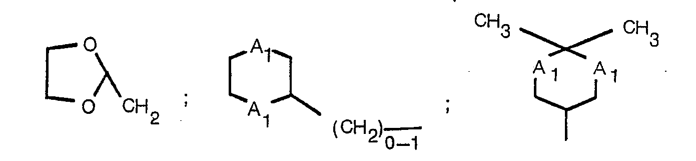
A est 0 ou S;
A₁ est 0, S ou SO₂; quand Q est 0 ou S, alors
1 ) R peut être aussi un groupe alcynyle en C₃ à C₁₀; alcynyle en C₃ à C₆ substitué par F, Ci ou Br; 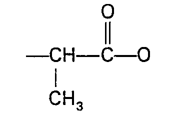 (alkyle en C₁―C₂); ou 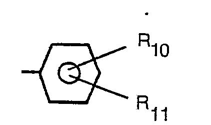 où R₁₀ et R₁₁ sont tels que définis plus haut; quand Q est 0, alors R est H, M, ―CH₂CH₂OR₇, ―CH₂CH₂CH₂OR₇, 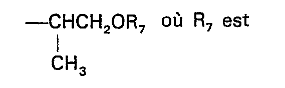 ―CH₂CH₃, ―CH(CH₃)₂, phényle, ―CH₂CH₂Cl ou ―CH₂CCl₃; (̵CH₂CH₂O)̵_{n,} R₈ ou 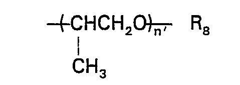 où R₈ est ―CH₃, ―CH₂CH₃, ―CH(CH₃)₂, phényle, ―CH₂CH₂Cl ou ―CH₂CCl₃ et n' vaut 2 ou 3; avec la condition que R ait un nombre total d'atomes de carbone inférieur ou égal à 13, 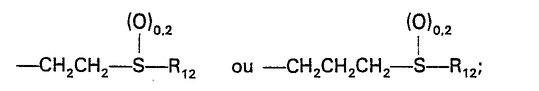 où R₁₂ est -CH₃, ―CH₂CH₃, -CH(CH₃)₂ ou phényle; et quand Q est 0,
2) R peut être aussi CH₂OR'₈ où R'₈ est CH₃―, CH₃CH₂, (CH₃)₂CH-, ClCH₂CH₂―, Cl₃CCH₂, 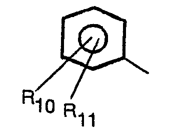 CH₃CH₂OCH₂CH₂ ou CH₃OCH₂CH₂; où R₁₀ et R₁₁ sont tels que définis plus haut; quand Q est 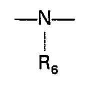 alors R est l'hydrogène; un groupe alkyle en C₁ à C₁₂; (̵CH₂CH₂O)̵ₙ R₁₂, ―CH₂CH₂CH₂OR₁₂ où R₁₂ est tel que défini plus haut et n"' vaut de 1 à 3; alcényle en C₃ à C₁₀; cycloalkyle en C₃ à C₈; cycloalcényle en C₅ à C₆; cycloalkyle en C₅ à C₈ substitué par 1 à 3 substituants choisis entre 0 à 2 groupes -OCH₃, 0 à 3 groupes -CH₃ et ―2_{C}H₅; trifluorométhylcyclohexyle; cycloalkylalkyle en C₄ à C₁₀; cycloalkylalkyle en C₄ à C₈ substitué par 1 à 2 groupes -CH₃; -CH₂CN; -CH₂CH₂CN; 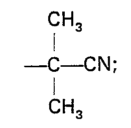 -OCH₃; ―N(CH₃)₂; ou 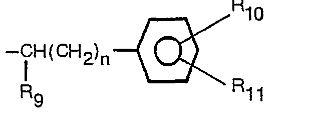 où n, R₉, R₁₀ et R₁₁ sont tels que définis plus haut; 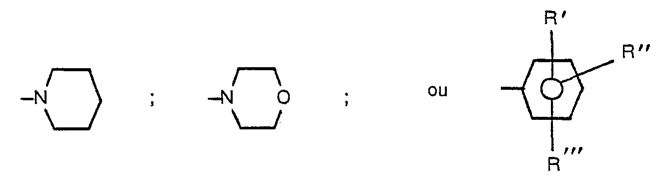 où
R' est l'hydrogène, un groupe alkyle en C, à C₄, -OCH₃, F, Br, CI, -CF₃, CN, NO₂, ―SO₂CH₃, -SCH₃ ou ―N(CH₃)₂;
R" est l'hydrogène, un groupe alkyle en C₁ à C₄, -OCH₃, F, Br ou CI;
R"' est l'hydrogène, -CH₃, CI, ^{F} ou ^{Br;}
R₆ est l'hydrogène, un groupe alkyle en C, à C₆, allyle, -CH₂CN; ou ―CH₂CH₂CN; ou R₆ et R peuvent être pris ensemble pour former ―(CH₂)₄―, ―(CH₂)₅―, ―(CH₂)₆―, ―CH₂CH₂OCH₂CH₂―; ou 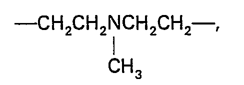 avec les conditions que, lorsque R est ―OCH₃ ou ―OCH₂CH₃, alors R₆ est H ou -CH₃; que, lorsque R₆ ,est ―CH₂CH₂CN ou -CH₂CN, alors R est -CH₂CH₂CN ou ―CH₂CN et R et R₆ ont un nombre total d'atomes de carbone inférieur ou égal à 13; et quand Q est 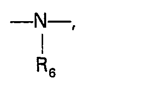
3) R peut aussi être un groupe alcynyle en C₃ à C₆; R₁ est 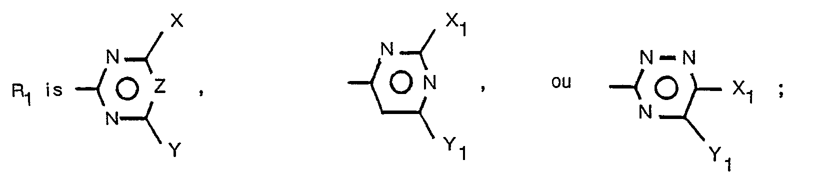
R₂ est H, CI, Br, F, un groupe alkyle en C, à C₃, ―NO₂, ―SO₂CH₃, -OCH₃, -SCH₃, -CF₃, ―N(CH₃)₂, -NH₂ ou -CN;
R₃ est H, CI, Br, F ou CH₃;
R₄ est H ou -CH₃;
R₅ est H, -CH₃ ou -OCH₃;
M est un métal alcalin;
W est l'oxygène ou le soufre;
X est H, CI, -CH₃, ―OCH₃, ―OCH₂CH₃ ou ―OCH₂CH₂OCH₃;
Y est H, CI, un groupe alkyle en C₁ à C₄; un groupe alkyle en C₁ à C₄ substitué par OCH₃, OC₂H₅, CN, CO₂CH₃, CO₂C₂H₅ ou par 1 à 3 atomes de F, CI ou Br; un groupe alcényle en C₃ à C₄; CH₂C≡CR₁₃; A(CH₂)ₙ, A₁ (alkyle en C, à C₃); 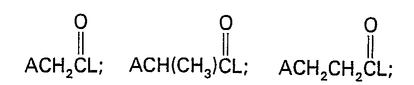 SCN; N₃; MR₁₆R₁₇; OR₁₄ ou SR₁₅;
où
R₁₃ est H, CH₃ ou CH₂Cl;
n', A et A₁ sont tels que définis plus haut;
_{C6;} L est NH₂, N(OCH₃)CH₃, NH(alkyle en C, à C₄), N(alkyle en C₁ à C₄)₂ ou un groupe alcoxy en C₁ à
R₁₄ est un groupe alkyle en C₁ à C₄; un groupe alkyle substitué par 1 à 3 atomes de F, CI oui Br; un groupe alkyle en C₁ à C₄ substitué par CN; un groupe alcényle en C₃ à C₄; un groupe CH₂C=CR₁₃ où R₁₃ est tel que défini plus haut, ou un groupe 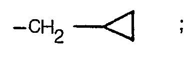
R₁₅ est un groupe alkyle en C₁ à C₄, cyanométhyle, cyanoéthyle, allyle ou propargyle;
R₁₆ est H ou CH₃;
R₁₇ est H; OCH₃; un groupe alkyle en C₁ à C₄; un groupe alkyle en C₁ à C₄ substitué par CN, CO₂C₂H₅; un groupe alcényle en C₃ à C₄; ou un groupe alkyle en C₂ à C₃ substitué par OCH₃ ou par OC₂H₅; ou
R₁₆ et R₁₇ peuvent être pris ensemble pour former CH₂CH₂CH₂CH₂ ou CH₂CH₂OCH₂CH₂, avec les conditions que, lorsque Y contient 4 atomes de carbone ou avantage, R ne contient pas plus de 4 atomes de carbone; lorsque X est CI, alors Y est CI; et, lorsque X et Y sont tous deux H, alors R ne contient pas plus de 4 atomes de carbone,
4) Y peut aussi être F, Br, 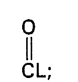 un groupe alkyle substitué par 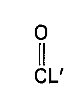 ou NR₁₆R₁₇, où L est tel que défini plus haut ou OH; R₁₆ est tel que défini plus haut; L' est NH₂, OH, N(OCH₃)CH₃, NH(alkyle en C₁ à C₄), N(alkyle en C₁ à C₄)₂ ou un groupe alcoxy en C₃ à C₆; et R₁₇. est un groupe alkyle en C₅ à C₆ ou un groupe alkyle en C₁ à C₄ substitué par 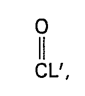 où L' est tel que défini plus haut;
Z est CH ou N;
Y₁ est H, -OCH₃ ou -CH₃;
5) Y₁ peut aussi être OCH₂CH₃; et
X₁ est H, CI, ―OCH₃, ―OCH₂CH₃ ou ―CH₃; avec les conditions que X₁ et Y₁ ne sont pas tous deux simultanément des atomes d'hydrogène, et que, lorsque R₁ est 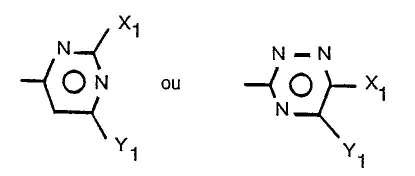 / .alors R₄ et R₅ sont tous deux H et R contient 5 atomes de carbone ou moins, et que
a) quand Y n'est pas tel que défini en 4), alors R doit être tel que défini en 1), 2) ou 3;
b) quand Y₁ n'est pas tel que défini en 5), alors R doit être tel que défini en 1), 2) ou 3; ou d'un sel de celui-ci utilisable en agriculture, qui consiste:
(a) à faire réagir un composé de formule: 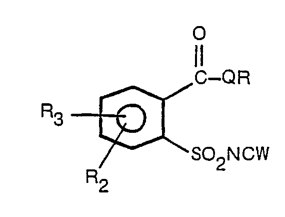 dans laquelle
Q est 0,
R est un groupe alkyle en C₁ à C₁₂; un groupe alcényle en C₃ à C₁₀; un groupe alkyle en C₂ à C₆ substitué par un à quatre substituants choisis parmi 0 à 3 atomes de F, CI ou Br et 0 à 2 groupes méthoxy; un groupe alcényle en C₃ à C₆ substitué par 1 à 3 atomes de F, Clou Br; un groupe cycloalkyle en C₅ à C₈; un groupe cycloalcényle en C₅ à C₈; un groupe cycloalkyle en C₅ à C₆ substitué par un à quatre groupes méthyle, méthoxy, alkyle en C₂ à C₄, F, CI ou Br; un groupe cycloalkylalkyle en C₄ à C₁₀; un groupe cycloalkylalkyle en C₄ à C₈ comportant 1 à 2 groupes CH₃; ―CH₂CH₂OR₇; CH₂CH₂CH₂OR₇; 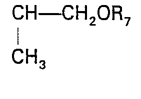 dans lesquels R₇ est ―CH₂CH₃, CH(CH₃)₂, phényle ―CH₂CH₂Cl ou ―CH₂CCl₃; (̵CH₂CH₂O)̵ₙ, R₈ ou 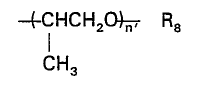 où R₈ est CH₃, ―CH₂CH₃, ―CH(CH₃)₂, phényle, ―CH₂CH₂Cl ou ―CH₂CCl₃ et n' vaut 2 ou 3;
R₂ est H, CI, Br, F, un groupe alkyle en C, à C₃, ―NO₂, -OCH₃, -SCH₃, CF₃, S0₂CH₃, N(CH₃)₂ ou CN;
R₃ est H, CI, Br ou CH₃
et W est 0 ou S; avec une aminopyrimidine ou une aminotriazine appropriée de formule 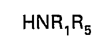 dans laquelle R₁ et R₅ sont tels que définis plus haut,
(b) à faire réagir un composé de formule: 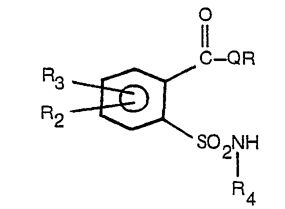 dans laquelle Q, R₂, R₃ et R₄ sont tels que définis pour la formule (I), et R est tel que défini pour la formule (II), autre que H ou M, avec un isothiocyanate de triazine ou de pyrimidine approprié 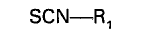 dans lequel R₁ est tel que défini pour la formule (I);
(c) à méthyler un composé de formule (I) dans lequel R₄ est H, Q est 0, S ou 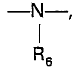 R est tel que défini en (a) ci-dessus et W est 0, pour obtenir un composé de formule (I) dans lequel R₄ est un groupe méthyle;
(d) à faire réagir un chlorure de carbamoyle ou de thiocarbamoyle de formule: 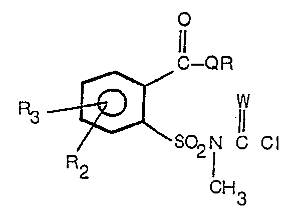 dans laquelle Q et R sont tels que définis en (a) ci-dessus et W est 0 ou S, avec l'amine HNR₁R₅ définie en (a) ci-dessus; ou
(e) à hydrolyser un ester de formule (I) dans lequel R est un groupe alkyle en C₁ à C₁₂ pour obtenir un composé de formule (I) dans lequel R est H, ou à estérifier un composé de formule (I) dans lequel R est H pour obtenir un composé de formule (I) dans lequel R est autre que H.

2. Un procédé selon la revendication 1, dans lequel R est 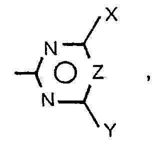
R₄ et R₅ sont H, et W est 0.

3. Un procédé selon la revendication 2, dans lequel Q est l'oxygene.

4. Un prócedé selon la revendication 3, dans lequel R est un groupe alkyle en C, à C₆, un groupe alcényle en C₃ à C₄, CH₂CH₂Cl, CH₂CH₂OCH₃ ou CH₂OR'₈ R'₈ où est tel que défini à la revendication 1.

5. Un procédé selon la revendication 4, dans lequel X est CH₃ ou OCH₃.

6. Un procédé selon la revendication 5, dans lequel Y est CH₃, OCH₃ ou

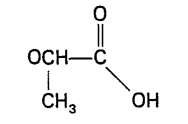

7. Un procédé selon la revendication 6, dans lequel R₃ est H.

8. Un procédé selon la revendication 7, dans lequel R₂ est H.

9. Un procédé selon la revendication 8, dans lequel R'₈ est CH₃ ou CH₃CH₂ et R est CH₃, CH₂CH₂OCH₃ ou CH₂CH₂OC₂H₅.

10. Un procédé selon la revendication 1, dans lequel le produit est un composé choisi parmi:
le 2-[[(4,6-diméthoxypyrimidin-2-yl)-aminocarbonyl]-aminosulfonyl]-benzoate de [(2-méthoxyéthoxy)-méthyle];
le 2-[[(4-éthoxy-6-méthyl-1,3,5-triazin-2-yl)-aminocarbonyl]-aminosulfonyl]-benzoate de [(2-méthoxyéthoxy)-méthyle];
le 2-[[(4,6-diméthylpyrimidin-2-yl)-aminocarbonyl]-aminosulfonyl]-benzoate de [(4-chlorophénoxy)-méthyle];
le 2-[[[4-(1-carboxyéthoxy)-6-méthyl-1,3,5-triazin-2-yl]-aminocarbonyl]-aminocarbonyl]-aminosulfonyl]-benzoate de méthyle;
le 2-[[(4,6-diméthylpyrimidin-2-yl)-aminocarbonyl]-aminosulfonyl]-benzoate de (2-méthoxyéthoxy)-méthyle;
le 2-[[(4-méthoxy-6-méthylpyrimidin-2-yl)-aminocarbonyl]-aminosulfonyl]-benzoate de (4-chloro-2-butynyle);
ou un sel de l'un quelconque de ceux-ci, utilisable en agriculture.

11. Procédé pour maîtriser la croissance de végétation indésirable en appliquant à l'endroit à protéger une quantité efficace d'un composé herbicide, caractérisé en ce que le composé herbicide comprend un composé de formule (I) tel que défini à l'une quelconque des revendications 1 à 9.

12. Procédé selon la revendication 11, dans lequel le composé herbicide comprend un composé tel que défini à la revendication 10.

13. Procédé de régulation de la croissance des plantes, qui consiste à appliquer à l'endroit de ces plantes une quantité efficace mais pratiquement non phytotoxique d'un régulateur de croissance des plantes, caractérisé en ce que le régulateur de croissance des plantes comprend un composé de formule (I) tel que défini à l'une quelconque des revendications 1 à 10.
